# EUROPEAN PATENT APPLICATION

(11) **EP 1 120 414 A1**
(43) Date of publication of application: **01.08.2001**
(21) Application number: 99933165.5
(22) Date of filing: 28.07.1999
(51) Int. Cl.: C07D 401/14, C07D 403/04, C07D 403/14, C07D 471/04, C07D 487/04, C07D 498/06, A61K 31/40, A61K 31/41, A61K 31/415, A61K 31/44, A61K 31/445, A61K 31/47, A61K 31/495, A61K 31/535, A61K 31/54, A61K 31/55

(54) **DISUBSTITUTED MALEIMIDE COMPOUNDS AND MEDICINAL UTILIZATION THEREOF**

(30) Priority: 30.07.1998 JP 21507098
(71) Applicant: Japan Tobacco Inc., Tokyo 105-8422 (JP)
(72) Inventor: INABA, Takashi, Central Phar. Res. Ins. of Japan, Takatsuki-shi, Osaka 569-1125 (JP); TANAKA, Masahiro, Central Phar. Res. Ins. of Japan, Takatsuki-shi, Osaka 569-1125 (JP); SAKODA, Kenji, Central Phar. Res. Ins. of Japan, Takatsuki-shi, Osaka 569-1125 (JP)
(74) Representative: von Kreisler, Alek, Dipl.-Chem.
(86) International application number: JP9904085
(87) International publication number: WO0006564

(57) **Abstract**

The present invention relates to a disubstituted maleimide compound of the formula [I] 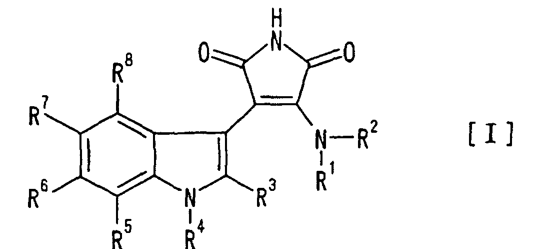 wherein R¹ is hydrogen or alkyl, R² is aryl, cycloalkyl or heterocyclic group, R³, R⁵, R⁶, R⁷ and R⁸ are hydrogen, halogen, hydroxyl group, amino, alkyl or alkoxy, and R⁴ is W or R⁴ and R³, or R⁴ and R⁵ jointly form a ring having a substituent W on the ring wherein W is - (CH₂)ₗ- (Y)ₘ- (CH₂)ₙ-Z, or a pharmaceutically acceptable salt thereof, a pharmaceutical composition containing this compound, and a protein kinase C (PKC) β inhibitor. The compounds of the present invention selectively inhibit PKCβ, and can be safe and effective pharmaceutical or prophylactic agents against the diseases caused by PKC including diabetic complications.

## Description

### TECHNICAL FIELD OF THE INVENTION

The present invention relates to a novel disubstituted maleimide compound, a pharmaceutically acceptable salt thereof, and a pharmaceutical composition containing same as an active ingredient. More particularly, the present invention relates to a novel disubstituted maleimide compound, a pharmaceutically acceptable salt thereof, and a pharmaceutical composition containing same as an active ingredient, which can treat or prevent diabetic complications such as diabetic retinopathy, diabetic nephropathy, diabetic cardiomyopathy, diabetic neuropathy and the like by selectively inhibiting a protein kinase C (PKC) isozyme β activity.

### BACKGROUND OF THE INVENTION

PKC is a kind of serine/threonine protein kinase that plays a central role in various intracellular signal transductions.

The proteins that PKC phosphorylates include receptors (e.g., epidermal growth factor receptor, insulin receptor, interleukin 2 receptor, acetylcholine receptor, adrenergic receptor and the like), a number of membrane proteins (e.g., phospholamban, sodium channel, glucose transporter and the like), actin, myosin and the like that constitute muscles, metabolic enzymes such as glycogen phosphorylase kinase, cytochrome P450 and the like, and many others.

At present, PKC is known to have at least 10 kinds of isozymes. Any of these isozymes have a structure including a kinase domain on the C-terminal side and a regulatory domain on the N-terminal side. The kinase domains of PKCs show high homology between them and also show homology with other protein kinases such as A kinase (cyclic AMP-dependent protein kinase, also called PKA), G kinase (cyclic GMP-dependent protein kinase), tyrosine kinase and the like. The regulatory domain contains a calcium-binding site and a phorbol ester-binding site. PKCs are classified into a group having both of the above sites {α,β (type I, type II), γ}, a group having only the phorbol ester-binding site (δ, ε, θ, η) and a group lacking both sites (ζ, λ).

PKC α,β and γ are activated by metabolite of cell membrane inositol phospholipid such as diacylglycerol (DAG) and the like and calcium. In other words, it is a phospholipid/calcium-dependent serine/threonine protein kinase.

The diseases mediated by the activation of PKC include abnormal blood flow (e.g., lowered retinal blood flow), abnormal vasoconstriction such as hyperpermeability of retinal vessels, promotion of glomerular filtration rate and the like, low constrictive response in renal mesangial cell and increased production of extracellular matrix. In addition, there are various reports on the disease states of abnormal cell proliferation and abnormal gene expression due to activation of transcription factor, hypercardia and fibrillation in cardiac muscle tissue, and the like.

The biological distribution, intracellular distribution and activation mechanism of each isozyme of the PKC family are known to vary, and activation of PKC β is particularly noticeable in retina, heart, aorta and glomerulus in the state of hyperglycemia. This is considered to be attributable to the fact that PKC β has high DAG sensitivity at low calcium concentration when compared to other isozymes, which leads to marked activation of PKC β in the disease state, that is free of increase in calcium concentration and that is mediated by DAG synthetic system, such as diabetes.

Given the central role of PKC in intracellular signal transduction, selective inhibition of PKC β activity is particularly desirable in cell and organ showing high PKC β distribution, in the disease state free of increase in intracellular calcium concentration and in the disease state caused by a factor that selectively activates PKC β. A PKC β selective inhibitor is drawing attention as a target in the development of a safe pharmaceutical agent causing fewer side effects.

Considering the fact with regard to biological distribution of PKC that isozyme α exists in almost every organ, the selective inhibition of PKC β rather than PKC α is particularly one of the desirable modes.

Therefore, a PKC inhibitor is considered to be applicable to various diseases such as diabetic complications, which specifically include diabetic retinopathy, diabetic nephropathy, diabetic cardiomyopathy and diabetic neuropathy, angiopathy such as arteriosclerosis, thrombosis and the like, inflammation, dermatosis, immune diseases such as acquired immunodeficiency syndrome and the like, central nervous system diseases such as Alzheimer's diseases and the like, cancer and the like. In particular, the application to diabetic complications such as diabetic retinopathy, diabetic nephropathy, diabetic cardiomyopathy, diabetic neuropathy and the like, particularly diabetic retinopathy, is expected.

The compounds having a PKC inhibitory action have been reported in large numbers. Some inhibitors thereof are PKC selective as compared to other kinases and the like, but insufficient in selectivity to isozymes α and β. Combined with other reasons, they have not been developed as practical pharmaceutical agents. Such compounds having a PKC inhibitory action include Staurosporine as reported in 1986 by Tamaoki et al. (Biochem. and Biophys. Research Commun. 135 (2), 397-402, 1986).

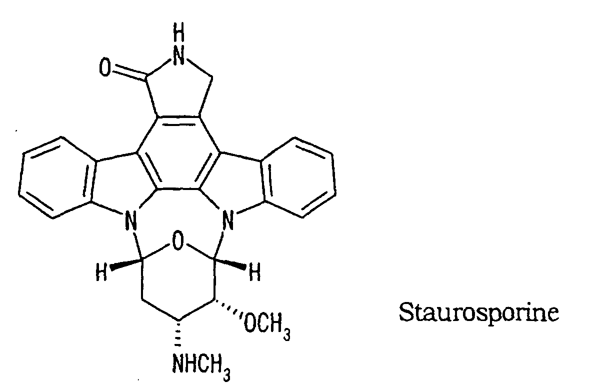

The PKC inhibitory action of the compounds having the following structures has been subsequently reported in large numbers.

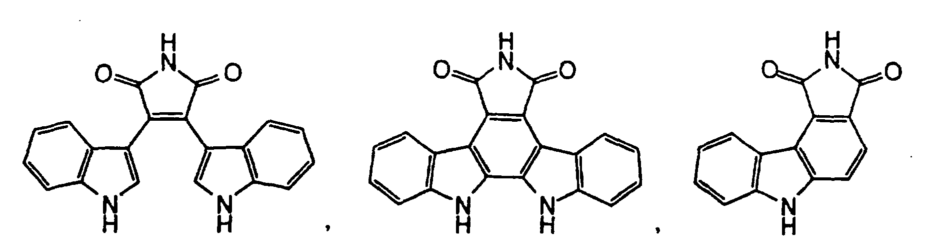

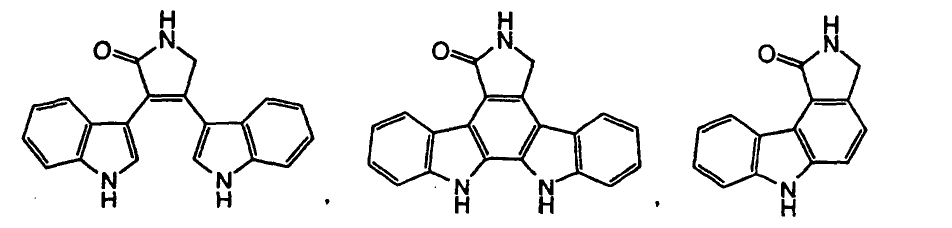

To be specific, Japanese Patent Application under PCT laid-open under *kohyo* No. 9-507066 (Eli Lilly & Co.), Japanese Patent Unexamined Publication No. 8-059666 (F Hoffmann-La Roche AG), EP115350 (Bristol-Myers Co.), WO97/05140 (Ciba-Geigy AG) and the like disclose such compounds.

These compounds are common in that they have an indole structure and a 1H-pyrrole-2,5-dione(maleimide) or 1,2-dihydro-pyrrol-5-one structure, but differ from each other in characteristics: in the level of PKC inhibitory activity, PKC selectivity and PKC isozyme selectivity.

A PKC inhibitor having a relatively similar structure to the present invention compound is disclosed in US 5405864 (Syntex Inc.) wherein the following Compound A and the like are encompassed. Bioorg. Med. Chem. Lett., 4 (24), 2845-2850, 1994 discloses the following Compound B and the like. However, these publications do not teach that they are PKC β selective.

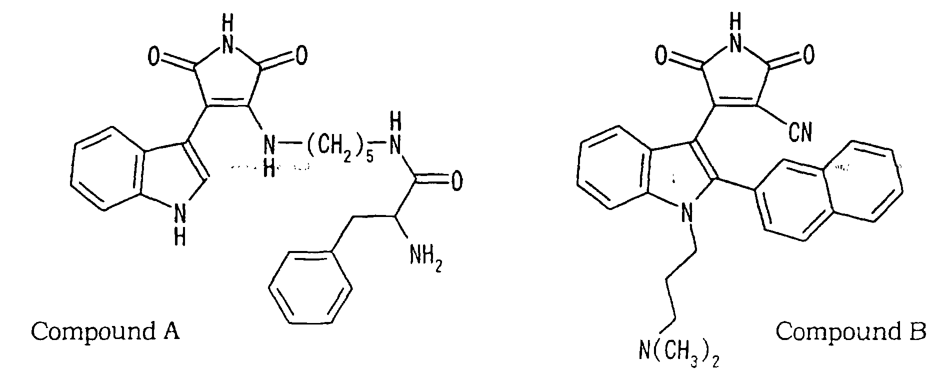

Likewise, as the same PKC inhibitor, Japanese Patent Unexamined Publication No. 2-264776 (F. Hoffmann-La Roche AG) discloses the following Compounds C and D and the like, but again, this publication does not teach that they are PKC β selective. On the other hand, Biochem. J., 294 (2), 335-337, 1993 teaches PKC isozyme activity of the following Compound C and its optically active compound. What it teaches is that the selectivity of this compound is that the isozyme β is several times higher in activity than isozyme ε and it shows higher α inhibitory activity in the comparison of α and β.

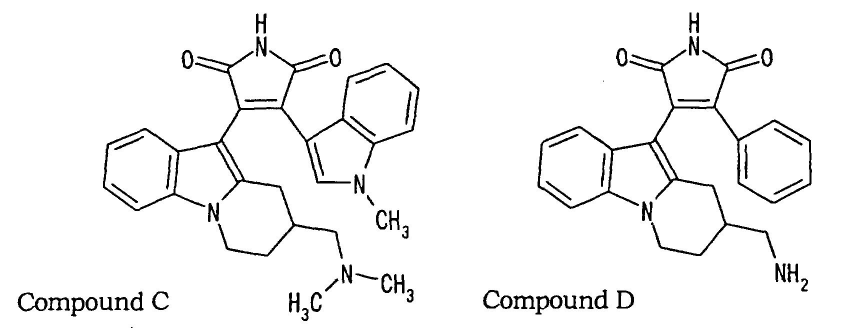

The following compound LY333531 (Japanese Patent Unexamined Publication No. 7-215977, Eli Lilly & Co.) developed as a PKC β selective inhibitor is a known compound.

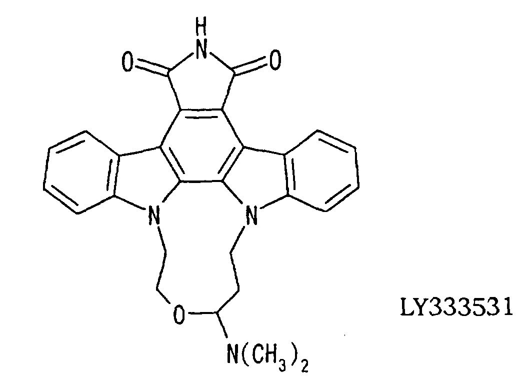

The compound of the present invention differs from all these known compounds in the structure and from most of the known compounds in the characteristics in that it has a PKC β selective action.

Meanwhile, a compound other than a PKC inhibitor and having a similar structure to the compound of the present invention includes the following Compound E disclosed in WO91/13070 (Boehringer Mannheim).

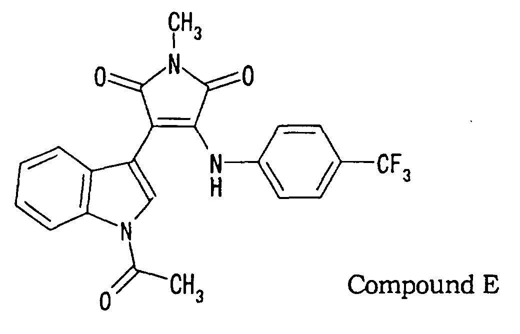

Its use, nevertheless, is as an agent for immune diseases and an antiallergic agent. It does not teach the action to be via PKC inhibition as in the present invention, nor does it disclose the data suggestive thereof.

### DISCLOSURE OF THE INVENTION

From the foregoing findings, it is known that a pharmaceutical agent having a PKC inhibitory action, particularly, a PKC β selective inhibitory action, can be a safe pharmaceutical agent for the normal intracellular signal transduction free of noticeable side effects, particularly an agent for the treatment and prophylaxis of diabetic complications. Accordingly, an object of the present invention is to provide a pharmaceutical agent having a PKC inhibitory action, particularly a pharmaceutical agent having a PKC β selective inhibitory action.

The present inventors have conducted intensive studies in an attempt to find a compound having a high PKC inhibitory action and PKC β isozyme selective inhibitory action, though many similar compounds are known as PKC inhibitors, and completed the present invention.

Accordingly, the present invention provides the following (1) to (14).
(1) A disubstituted maleimide compound of the formula [I] 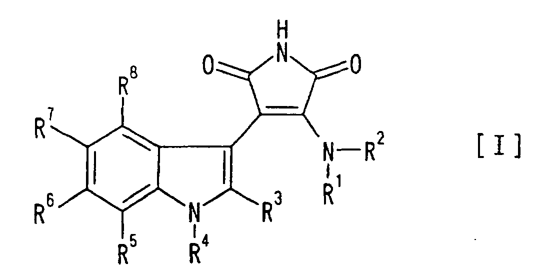 wherein
   R¹ is hydrogen atom or lower alkyl;
   R² is optionally substituted aryl, optionally substituted cycloalkyl or optionally substituted heterocyclic group;
   R³, R⁵, R⁶, R⁷ and R⁸ are the same or different and each is hydrogen atom, halogen atom, hydroxyl group, amino, optionally substituted lower alkyl or optionally substituted lower alkoxy;
   R⁴ is independently W, or R⁴ and R³ jointly form a group of the formula 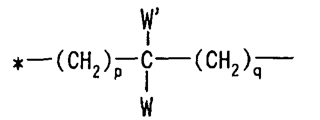 or 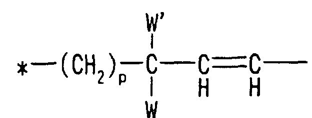 or R⁴ and R⁵ jointly form a group of the formula 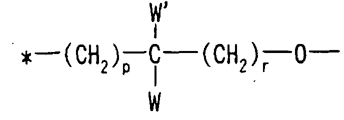
   W is - (CH₂)ₗ-(Y)ₘ- (CH₂)ₙ-Z
   {wherein Y is -CR⁹R⁹' - (wherein R⁹ and R⁹' are the same or different and each is hydrogen atom, hydroxyl group, lower alkyl, lower alkoxy, lower alkylthio, lower alkylamino, di(lower)alkylamino, or heterocyclic group), -NR¹⁰- (wherein R¹⁰ is hydrogen atom or lower alkyl), -O-, -S-, -SO₂-, -CONH-, -NHCO-, -SONH-, -NHSO-, -SO₂NH-, -NHSO₂- or -SO₃-,
   Z is hydrogen atom, halogen atom, hydroxyl group, optionally substituted lower alkoxy, lower alkanoyl, lower alkoxycarbonyl, -NR¹¹R¹² (wherein R¹¹ and R¹² are the same or different and each is hydrogen atom or lower alkyl), optionally substituted amidino, optionally substituted guanidino, carbamoyl, lower alkylaminocarbonyl, optionally substituted aryl, optionally substituted cycloalkyl or optionally substituted heterocyclic group,
   1 is 0 or an integer of 1 to 4, m is 0 or 1, and n is 0 or an integer of 1 to 4},
   W' is hydrogen atom or the same as or different from W and is - (CH₂)ₗ-(Y)ₘ-(CH₂)ₙ-Z (wherein each symbol is as defined above); and
   p, q and r are the same or different and each is 0 or an integer of 1 to 4,
   the above-mentioned symbol 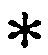 means that the side marked with a 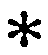 binds to the nitrogen atom of the indole ring,
      or a pharmaceutically acceptable salt thereof.
(2) The disubstituted maleimide compound of the formula [I] of (1) above 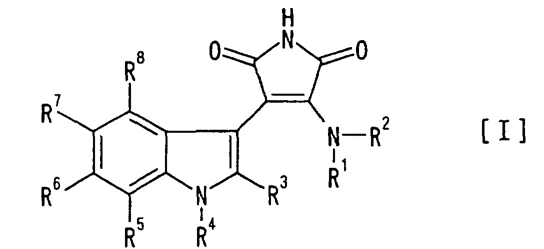 wherein R' is hydrogen atom or C₁-C₆ lower alkyl (wherein C₁-C₆ means having 1 to 6 carbon atoms, hereinafter the same);
   R² is optionally substituted C₆-C₁₈ aryl, optionally substituted C₃-C₈ cycloalkyl or optionally substituted heterocyclic group (wherein said heterocyclic group has 1 to 4 hetero atoms selected from oxygen atom, nitrogen atom and sulfur atom, wherein the number of atoms constituting the ring is 5 to 12);
   R³, R⁵, R⁶, R⁷ and R⁸ are the same or different and each is hydrogen atom, halogen atom, hydroxyl group, amino, optionally substituted C₁-C₆ lower alkyl or optionally substituted C₁-C₆ lower alkoxy;
   R⁴ is independently W, or R⁴ and R³ jointly form a group of the formula 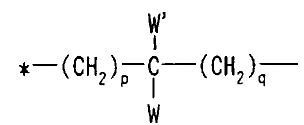 or 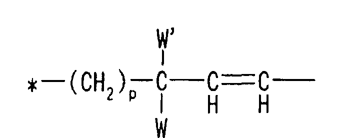 or R⁴ and R⁵ jointly form a group of the formula 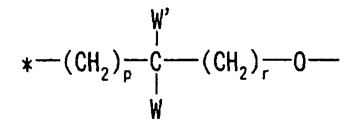
   W is - (CH₂)ₗ- (Y)ₘ- (CH₂)ₙ-Z
   wherein Y is -CR⁹R⁹'- [wherein R⁹ and R⁹' are the same or different and each is hydrogen atom, hydroxyl group, C₁-C₆ lower alkyl, C₁-C₆ lower alkoxy, C₁-C₆ lower alkylthio, C₁-C₆ lower alkylamino, di(C₁-C₆ lower) alkylamino or heterocyclic group (wherein said heterocyclic group has 1 to 4 hetero atoms selected from oxygen atom, nitrogen atom and sulfur atom, wherein the number of atoms constituting the ring is 5 to 12)], -NR¹⁰- (wherein R¹⁰ is hydrogen atom or C₁ -C₆ lower alkyl), -O-, -S-, -SO₂-, -CONH-, -NHCO-, -SONH-, -NHSO-, -SO₂NH-, -NHSO₂- or -SO₃-,
   Z is hydrogen atom, halogen atom, hydroxyl group, optionally substituted C₁-C₆ lower alkoxy, C₁-C₆ lower alkanoyl, C₁-C₆ lower alkoxycarbonyl, -NR¹¹R¹² (wherein R¹¹ and R¹² are the same or different and each is hydrogen atom or C₁-C₆ lower alkyl), optionally substituted amidino, optionally substituted guanidino, carbamoyl, C₁-C₆ lower alkylaminocarbonyl, optionally substituted C₆-C₁₈ aryl, optionally substituted C₃-C₈ cycloalkyl or optionally substituted heterocyclic group (said heterocyclic group is as defined above),
   1 is 0 or an integer of 1 to 4, m is 0 or 1, and n is 0 or an integer of 1 to 4;
   W' is hydrogen atom or the same as or different from W and is - (CH₂)ₗ- (Y)ₘ- (CH₂)ₙ-Z (wherein each symbol is as defined above); and
   p, q and r are the same or different and each is 0 or an integer of 1 to 4,
   the above-mentioned symbol 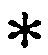 means that the side marked with a 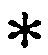 binds to the nitrogen atom of the indole ring, or a pharmaceutically acceptable salt thereof.
(3) The disubstituted maleimide compound of (2) above, wherein R² is optionally substituted C₆-C₁₈ aryl or optionally substituted C₃-C₈ cycloalkyl;
   R³, R⁵, R⁶, R⁷ and R⁸ are the same or different and each is hydrogen atom, optionally substituted C₁-C₆ lower alkyl or optionally substituted C₁-C₆ lower alkoxy;
   Y at W is -CR⁹R^{9'} -, -NR¹⁰- (wherein R⁹, R⁹' and R¹⁰ are as defined in (2)), -O-, -S- or -SO₂-;
   Z at W is hydrogen atom, hydroxyl group, optionally substituted C₁-C₆ lower alkoxy, C₁-C₆ lower alkanoyl, -NR¹¹R¹² (wherein R¹¹ and R¹² are as defined in (2)), optionally substituted amidino or optionally substituted heterocyclic group; and
   W' is hydrogen atom,
   or a pharmaceutically acceptable salt thereof.
(4) The disubstituted maleimide compound of (2) above, wherein R¹ is hydrogen atom, and R² is optionally substituted C₆-C₁₈ aryl, or a pharmaceutically acceptable salt thereof.
(5) The disubstituted maleimide compound of (4) above, wherein R⁴ is independently W or R⁴ and R³ jointly form a group of the formula 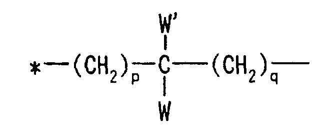 wherein W, p and q are as defined in (2), and W' is hydrogen atom, or a pharmaceutically acceptable salt thereof.
(6) The disubstituted maleimide compound of (5) above, wherein R⁴ and R³ jointly form a group of the formula 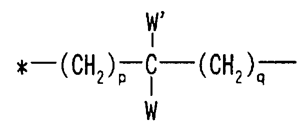 wherein W, p and q are as defined in (2), and W' is hydrogen atom, or a pharmaceutically acceptable salt thereof.
(7) The disubstituted maleimide compound of (6) above, wherein R⁵, R⁶, R⁷ and R⁸ are each hydrogen atom, and R² is phenyl, or a pharmaceutically acceptable salt thereof.
(8) The disubstituted maleimide compound of (7) above, wherein Z at W is hydroxyl group, -NR¹¹R¹² (wherein R¹¹ and R¹² are as defined in (2)) or optionally substituted heterocyclic group, or a pharmaceutically acceptable salt thereof.
(9) The disubstituted maleimide compound of (1) above or a pharmaceutically acceptable salt thereof, which is selected from the group consisting of
   3-(1H-indol-3-yl)-4-[(3-methoxyphenyl)amino]-1H-pyrrole-2,5-dione (Example 1-1),
   3-(1H-indol-3-yl)-4-(phenylamino)-1H-pyrrole-2,5-dione (Example 1-2),
   3-(cyclohexylamino)-4-(1H-indol-3-yl)-1H-pyrrole-2,5-dione (Example 1-3),
   3-(1H-indol-3-yl)-4-[(4-methylphenyl)amino]-1H-pyrrole-2,5-dione (Example 1-4),
   3-(1H-indol-3-yl)-4-[(3-methylphenyl)amino]-1H-pyrrole-2,5-dione (Example 1-5),
   3-[(3-chlorophenyl)amino]-4-(1H-indol-3-yl)-1H-pyrrole-2,5-dione (Example 1-6),
   3-(1H-indol-3-yl)-4-[(4-methoxy-2-methylphenyl)amino]-1H-pyrrole-2,5-dione (Example 1-7),
   3-[(2,4-dimethoxyphenyl)amino)-4-(1H-indol-3-yl)-1H-pyrrole-2,5-dione (Example 1-8),
   3-[(2,4-difluorophenyl)amino]-4-(1H-indol-3-yl)-1H-pyrrole-2,5-dione (Example 1-9),
   3-[(3-bromophenyl)amino]-4-(1H-indol-3-yl)-1H-pyrrole-2,5-dione (Example 1-10),
   3-(1H-indol-3-yl)-4-[(2-methylphenyl)amino]-1H-pyrrole-2,5-dione (Example 1-11),
   3-[(3-fluorophenyl)amino]-4-(1H-indol-3-yl)-1H-pyrrole-2,5-dione (Example 1-12),
   3-(1H-indol-3-yl)-4-[(3-trifluoromethylphenyl)amino]-1H-pyrrole-2,5-dione (Example 1-13),
   3-(1H-indol-3-yl)-4-(biphenyl-3-ylamino)-1H-pyrrole-2,5-dione (Example 1-14),
   3-(1H-indol-3-yl)-4-[(3-phenoxyphenyl)amino]-1H-pyrrole-2,5-dione (Example 1-15),
   3-(1H-indol-3-yl)-4-[(3-isopropylphenyl)amino]-1H-pyrrole-2,5-dione (Example 1-16),
   3-(1H-indol-3-yl)-4-(N-methyl-N-phenylamino)-1H-pyrrole-2,5-dione (Example 1-17),
   3-[1-(3-hydroxypropyl)-1H-indol-3-yl]-4-(phenylamino)-1H-pyrrole-2,5-dione (Example 2-1),
   3-[1-(3-hydroxypropyl)-1H-indol-3-yl]-4-[(3-methylphenyl)amino]-1H-pyrrole-2,5-dione (Example 2-2),
   3-[(3-chlorophenyl)amino]-4-[1-(3-hydroxypropyl)-1H-indol-3-yl]-1H-pyrrole-2,5-dione (Example 2-3),
   3-[1-(2-hydroxyethyl)-1H-indol-3-yl]-4-(phenylamino)-1H-pyrrole-2,5-dione (Example 2-4),
   3-[1-(4-hydroxybutyl)-1H-indol-3-yl]-4-(phenylamino)-1H-pyrrole-2,5-dione (Example 2-5),
   3-[(3,4-dichlorophenyl)amino]-4-[1-(3-hydroxypropyl)-1H-indol-3-yl]-1H-pyrrole-2,5-dione (Example 2-6),
   3-[1-(2-acetoxyethyl)-1H-indol-3-yl]-4-(phenylamino)-1H-pyrrole-2,5-dione (Example 2-7),
   3-[1-(3-dimethylaminopropyl)-1H-indol-3-yl]-4-(phenylamino)-1H-pyrrole-2,5-dione (Example 2-8),
   3-[1-(2-dimethylaminoethyl)-1H-indol-3-yl]-4-(phenylamino)-1H-pyrrole-2,5-dione (Example 2-9),
   3-[1-(4-dimethylaminobutyl)-1H-indol-3-yl]-4-(phenylamino)-1H-pyrrole-2,5-dione (Example 2-10),
   3-[1-(3-dimethylaminopropyl)-1H-indol-3-yl]-4-[(3-methylphenyl)amino]-1H-pyrrole-2,5-dione (Example 2-11),
   3-[1-(3-dimethylaminopropyl)-1H-indol-3-yl]-4-[(3-chlorophenyl)amino]-1H-pyrrole-2,5-dione (Example 2-12),
   3-[1-(3-diethylaminopropyl)-1H-indol-3-yl]-4-(phenylamino)-1H-pyrrole-2,5-dione (Example 2-13),
   3-[1-{3-[N-(2-dimethylaminoethyl)-N-methylamino)propyl}-1H-indol-3-yl]-4-(phenylamino)-1H-pyrrole-2,5-dione (Example 2-14),
   3-[1-{3-[N-ethyl-N-(2-methoxyethyl)amino]propyl}-1H-indol-3-yl]-4-(phenylamino)-1H-pyrrole-2,5-dione (Example 2-15),
   3-[1-{2-[N-(2-dimethylaminoethyl)-N-methylamino]ethyl}-1H-indol-3-yl]-4-(phenylamino)-1H-pyrrole-2,5-dione (Example 2-16),
   3-[1-{3-(N-benzyl-N-ethylamino)propyl}-1H-indol-3-yl)-4-(phenylamino)-1H-pyrrole-2,5-dione (Example 2-17),
   3-[1-{3-[N-ethyl-N-(4-pyridylmethyl)amino]propyl}-1H-indol-3-yl]-4-(phenylamino)-1H-pyrrole-2,5-dione (Example 2-18),
   3-[1-(3-morpholinopropyl)-1H-indol-3-yl]-4-(phenylamino)-1H-pyrrole-2,5-dione (Example 2-19),
   3-[1-(3-piperidinopropyl)-1H-indol-3-yl]-4-(phenylamino)-1H-pyrrole-2,5-dione (Example 2-20),
   3-(phenylamino)-4-[1-(3-thiomorpholinopropyl)-1H-indol-3-yl]-1H-pyrrole-2,5-dione (Example 2-21),
   3-(phenylamino)-4-(1-(3-pyrrolidin-1-ylpropyl)-1H-indol-3-yl]-1H-pyrrole-2,5-dione (Example 2-22),
   3-[1-(3-azacycloheptan-1-ylpropyl)-1H-indol-3-yl]-4-(phenylamino)-1H-pyrrole-2,5-dione (Example 2-23),
   3-[1-{3-(2-carbamoylpyrrolidin-1-yl)propyl}-1H-indol-3-yl]-4-(phenylamino)-1H-pyrrole-2,5-dione (Example 2-24),
   3-[1-{3-(4-hydroxypiperidino)propyl}-1H-indol-3-yl]-4-(phenylamino)-1H-pyrrole-2,5-dione (Example 2-25),
   3-[1-{3-(4-methylpiperazin-1-yl)propyl}-1H-indol-3-yl]-4-(phenylamino)-1H-pyrrole-2,5-dione (Example 2-26),
   3-[(3-chlorophenyl)amino]-4-[1-{4-(4-hydroxypiperidino)butyl}-1H-indol-3-yl]-1H-pyrrole-2,5-dione (Example 2-27),
   3-[1-{5-(4-hydroxypiperidino)pentyl}-1H-indol-3-yl]-4-(phenylamino)-1H-pyrrole-2,5-dione (Example 2-28),
   3-[1-{4-(4-methylpiperazin-1-yl)butyl}-1H-indol-3-yl]-4-(phenylamino)-1H-pyrrole-2,5-dione (Example 2-29),
   3-[1-{3-[3-(tert-butylaminocarbonyl)-decahydro-(4aS,8aS)-isoquinolin-2-yl]propyl}-1H-indol-3-yl]-4-(phenylamino)-1H-pyrrole-2,5-dione (Example 2-30),
   3-(phenylamino)-4-[1-{3-(4-piperidinopiperidino)propyl}-1H-indol-3-yl]-1H-pyrrole-2,5-dione (Example 2-31),
   3-[1-{3-[4-(2-hydroxyethyl)piperazin-1-yl]propyl}-1H-indol-3-yl]-4-(phenylamino)-1H-pyrrole-2,5-dione (Example 2-32),
   3-[1-{3-(4-carbamoylpiperidino)propyl}-1H-indol-3-yl]-4-(phenylamino)-1H-pyrrole-2,5-dione (Example 2-33),
   3-[1-{3-(4-dimethylaminopiperidino)propyl}-1H-indol-3-yl]-4-(phenylamino)-1H-pyrrole-2,5-dione (Example 2-34),
   3-[1-{3-(phenylsulfonyl)propyl}-1H-indol-3-yl]-4-(phenylamino)-1H-pyrrole-2,5-dione (Example 2-35),
   3-[1-(3-imidazol-1-ylpropyl)-1H-indol-3-yl]-4-(phenylamino)-1H-pyrrole-2,5-dione (Example 2-36),
   3-(phenylamino)-4-[1-(3-pyrazol-1-ylpropyl)-1H-indol-3-yl]-1H-pyrrole-2,5-dione (Example 2-37),
   3-(phenylamino)-4-[1-{3-(1,2,4-triazol-1-yl)propyl}-1H-indol-3-yl]-1H-pyrrole-2,5-dione (Example 2-38),
   3-[(3-chlorophenyl)amino]-4-[1-(3-imidazol-1-ylpropyl)-1H-indol-3-yl]-1H-pyrrole-2,5-dione (Example 2-39),
   3-[(3-chlorophenyl)amino]-4-[1-(4-imidazol-1-ylbutyl)-1H-indol-3-yl]-1H-pyrrole-2,5-dione (Example 2-40),
   3-[1-(5-imidazol-1-ylpentyl)-1H-indol-3-yl]-4-(phenylamino)-1H-pyrrole-2,5-dione (Example 2-41),
   3-[(3-chlorophenyl)amino]-4-[1-{3-(2-methylimidazol-1-yl)propyl}-1H-indol-3-yl]-1H-pyrrole-2,5-dione (Example 2-42),
   3-[1-(3-amidinothiopropyl)-1H-indol-3-yl]-4-(phenylamino)-1H-pyrrole-2,5-dione hydrobromide (Example 2-43),
   3-[1-(2,3-dihydroxypropyl)-1H-indol-3-yl]-4-(phenylamino)-1H-pyrrole-2,5-dione (Example 2-44),
   3-[1-{3-(hydroxymethyl)benzyl}-1H-indol-3-yl]-4-(phenylamino)-1H-pyrrole-2,5-dione (Example 2-45),
   3-[1-(3-hydroxypropyl)-5-methoxy-1H-indol-3-yl]-4-(phenylamino)-1H-pyrrole-2,5-dione (Example 2-46),
   3-[1-{2-(4-hydroxypiperidino)ethyl}-1H-indol-3-yl]-4-(phenylamino)-1H-pyrrole-2,5-dione (Example 2-47),
   3-[1-{3-(4-benzylpiperidino)propyl}-1H-indol-3-yl]-4-(phenylamino)-1H-pyrrole-2,5-dione (Example 2-48),
   3-[1-(3-(4-pyrrolidinylpiperidino)propyl}-1H-indol-3-yl]-4-(phenylamino)-1H-pyrrole-2,5-dione (Example 2-49),
   3-[1-{3-[4-(hydroxymethyl)piperidino]propyl}-1H-indol-3-yl]-4-(phenylamino)-1H-pyrrole-2,5-dione (Example 2-50),
   3-[1-{3-[4-(tert-butoxycarbonyl)piperidino]propyl}-1H-indol-3-yl]-4-(phenylamino)-1H-pyrrole-2,5-dione (Example 2-51),
   3-[2-methyl-1-(3-morpholinopropyl)-1H-indol-3-yl]-4-(phenylamino)-1H-pyrrole-2,5-dione (Example 2-52),
   3-[2-methyl-1-(3-piperidinopropyl)-1H-indol-3-yl]-4-(phenylamino)-1H-pyrrole-2,5-dione (Example 2-53),
   3-[1-(3-dimethylaminopropyl)-2-methyl-1H-indol-3-yl]-4-(phenylamino)-1H-pyrrole-2,5-dione (Example 2-54),
   3-[2-methyl-1-(3-pyrrolidin-1-ylpropyl)-1H-indol-3-yl]-4-(phenylamino)-1H-pyrrole-2,5-dione (Example 2-55),
   3-[1-{3-(ethylmethylamino)propyl}-2-methyl-1H-indol-3-yl]-4-(phenylamino)-1H-pyrrole-2,5-dione (Example 2-56),
   3-[1-(3-dimethylaminopropyl)-5-methoxy-1H-indol-3-yl]-4-(phenylamino)-1H-pyrrole-2,5-dione (Example 2-57),
   3-[(3-chlorophenyl)amino]-4-[1-{3-(4-methyl-imidazol-1-yl)propyl}-1H-indol-3-yl]-1H-pyrrole-2,5-dione (Example 2-58),
   3-[(3-chlorophenyl)amino]-4-[1-{3-(5-methyl-imidazol-1-yl)propyl}-1H-indol-3-yl]-1H-pyrrole-2,5-dione (Example 2-59),
   3-[(3-chlorophenyl)amino]-4-[1-{3-(4-hydroxymethyl-imidazol-1-yl)propyl}-1H-indol-3-yl]-1H-pyrrole-2,5-dione and 3-[(3-chlorophenyl)amino]-4-[1-{3-(5-hydroxymethyl-imidazol-1-yl)propyl}-1H-indol-3-yl]-1H-pyrrole-2,5-dione (Example 2-60),
   3-[1-{3-(2-methylimidazol-1-yl)propyl}-1H-indol-3-yl]-4-(phenylamino)-1H-pyrrole-2,5-dione (Example 2-61),
   3-[1-(2-imidazol-1-ylethyl)-1H-indol-3-yl]-4-(phenylamino)-1H-pyrrole-2,5-dione (Example 2-62),
   3-[1-{2-(2-methyl-imidazol-1-yl)ethyl}-1H-indol-3-yl]-4-(phenylamino)-1H-pyrrole-2,5-dione (Example 2-63),
   3-[(4-chlorophenyl)amino]-4-[1-(3-imidazol-1-ylpropyl)-1H-indol-3-yl]-1H-pyrrole-2,5-dione (Example 2-64),
   3-[1-(3-imidazol-1-ylpropyl)-1H-indol-3-yl]-4-[(4-metholyphenyl)amino]-1H-pyrrole-2,5-dione (Example 2-65),
   3-[(4-bromophenyl)amino]-4-[1-(3-imidazol-1-ylpropyl)-1H-indol-3-yl]-1H-pyrrole-2,5-dione (Example 2-66),
   3-[1-(3-imidazol-1-ylpropyl)-1H-indol-3-yl]-4-[(4-trifluoromethylphenyl)amino]-1H-pyrrole-2,5-dione (Example 2-67),
   3-[(4-fluorophenyl)amino]-4-[1-(3-imidazol-1-ylpropyl)-1H-indol-3-yl]-1H-pyrrole-2,5-dione (Example 2-68),
   3-[1-(3-imidazol-1-ylpropyl)-2-methyl-1H-indol-3-yl]-4-(phenylamino)-1H-pyrrole-2,5-dione (Example 2-69),
   3-(cyclohexylamino)-4-[1-(3-imidazol-1-ylpropyl)-1H-indol-3-yl]-1H-pyrrole-2,5-dione (Example 2-70),
   3-(cyclopentylamino)-4-[1-(3-imidazol-1-ylpropyl)-1H-indol-3-yl]-1H-pyrrole-2,5-dione (Example 2-71),
   3-(cycloheptylamino)-4-[1-(3-imidazol-1-ylpropyl)-1H-indol-3-yl]-1H-pyrrole-2,5-dione (Example 2-72),
   3-[1-(3-imidazol-1-ylpropyl)-5-methoxy-1H-indol-3-yl]-4-(phenylamino)-1H-pyrrole-2,5-dione (Example 2-73),
   3-(phenylamino)-4-[1-(3-piperidylmethyl)-1H-indol-3-yl]-1H-pyrrole-2,5-dione (Example 3-1),
   3-(phenylamino)-4-[1-(4-piperidylmethyl)-1H-indol-3-yl]-1H-pyrrole-2,5-dione (Example 3-2),
   3-[1-{(1-methylpiperidin-3-yl)methyl}-1H-indol-3-yl]-4-(phenylamino)-1H-pyrrole-2,5-dione (Example 3-3),
   3-[1-{(1-methylpiperidin-4-yl)methyl}-1H-indol-3-yl]-4-(phenylamino)-1H-pyrrole-2,5-dione (Example 3-4),
   3-[1-{[1-(2,3-dihydroxypropyl)piperidin-4-yl]methyl}-1H-indol-3-yl]-4-(phenylamino)-1H-pyrrole-2,5-dione (Example 3-5),
   3-[1-{(1-carbamoylpiperidin-4-yl)methyl}-1H-indol-3-yl]-4-(phenylamino)-1H-pyrrole-2,5-dione (Example 3-6), and
   3-[1-{(1-amidinopiperidin-4-yl)methyl}-1H-indol-3-yl]-4-(phenylamino)-1H-pyrrole-2,5-dione hydrochloride (Example 3-7).
(10) The disubstituted maleimide compound of (1) above or a pharmaceutically acceptable salt thereof, which is selected from the group consisting of 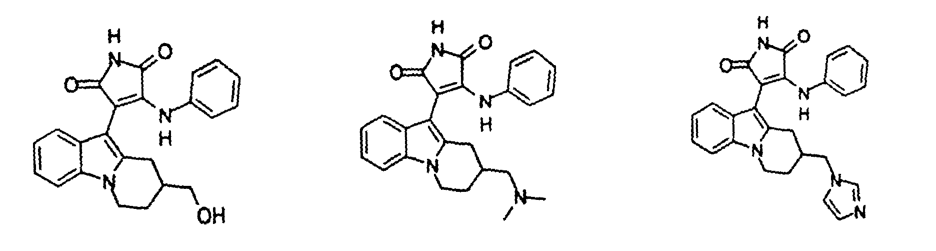 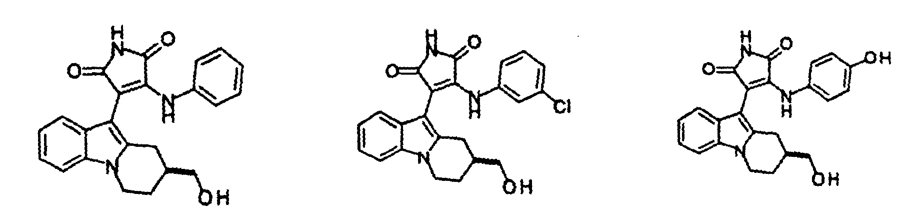 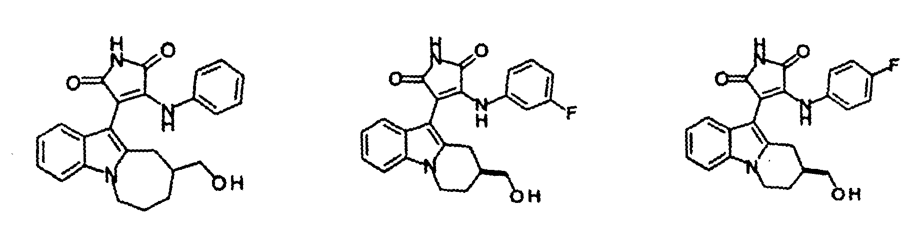 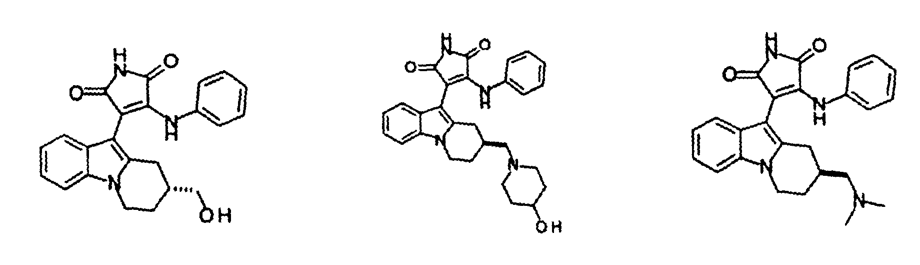 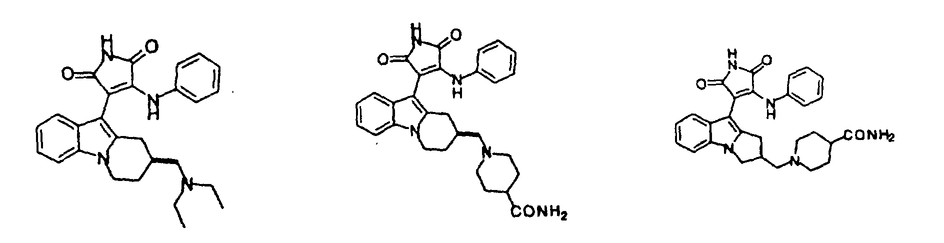 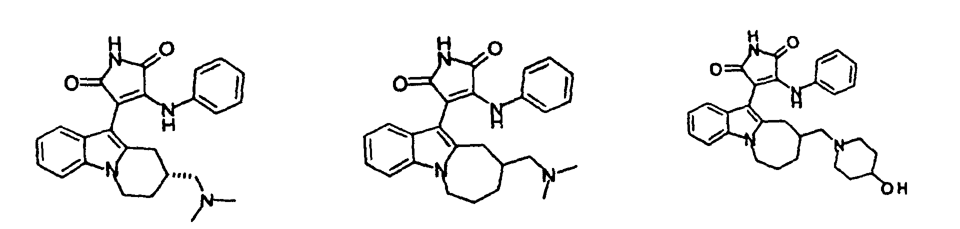 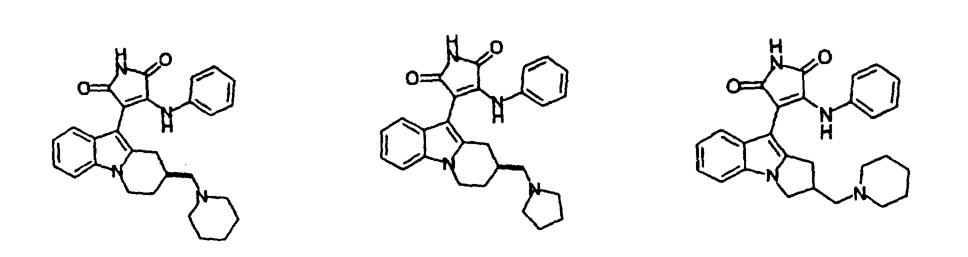 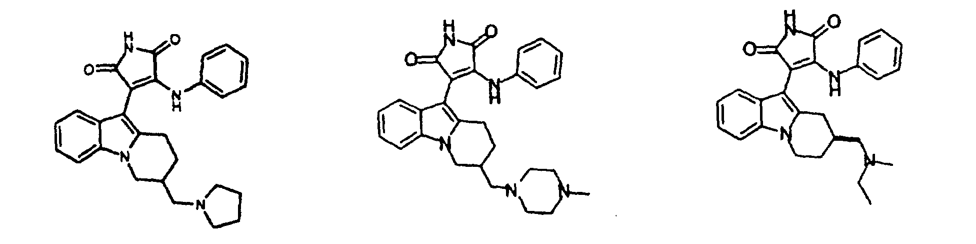 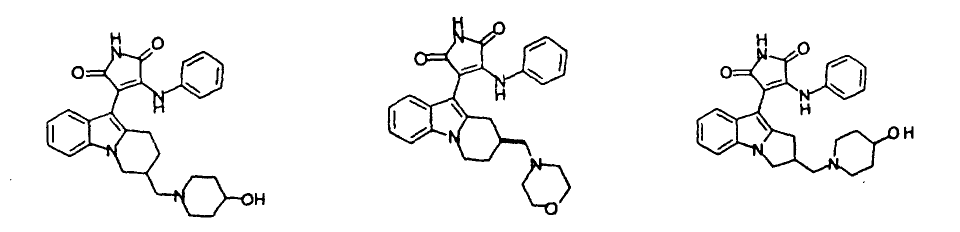 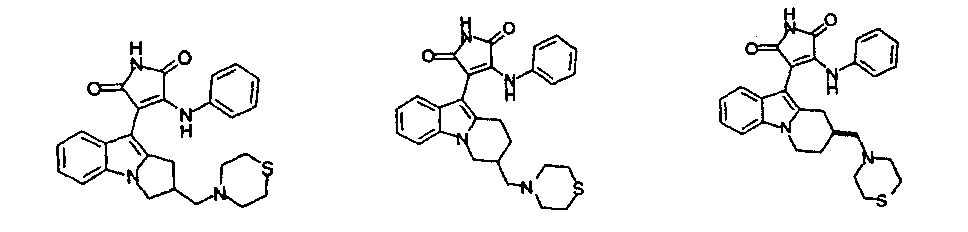 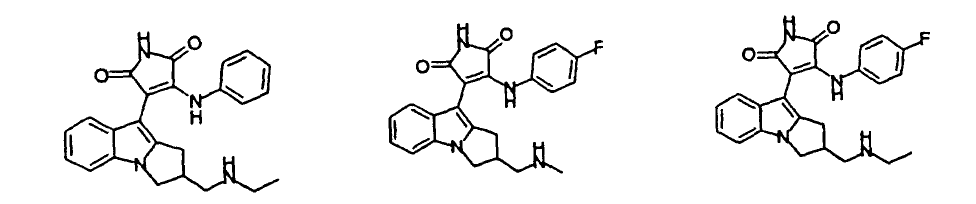 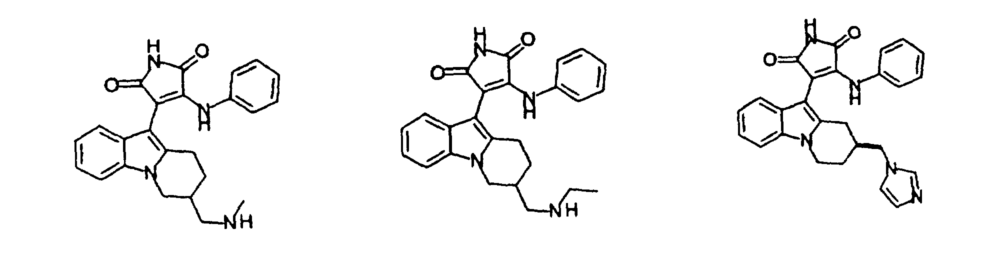 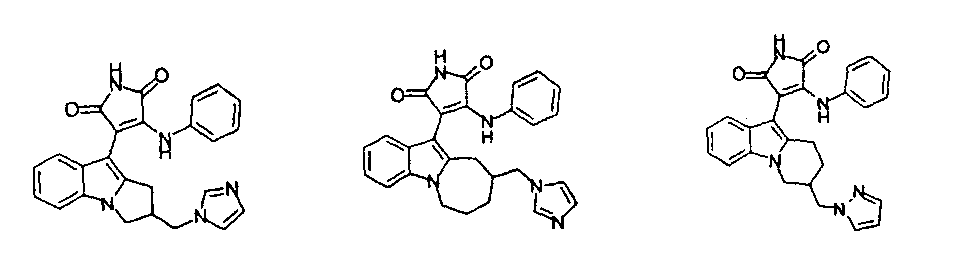 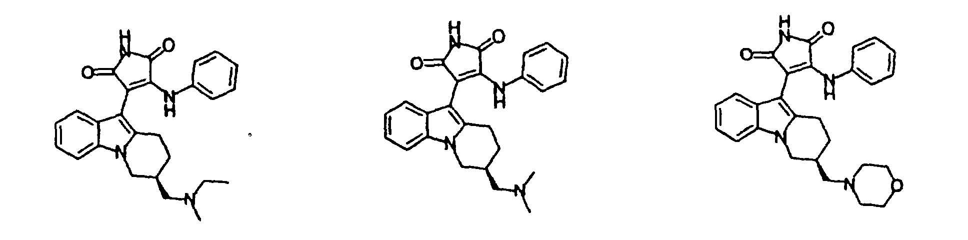 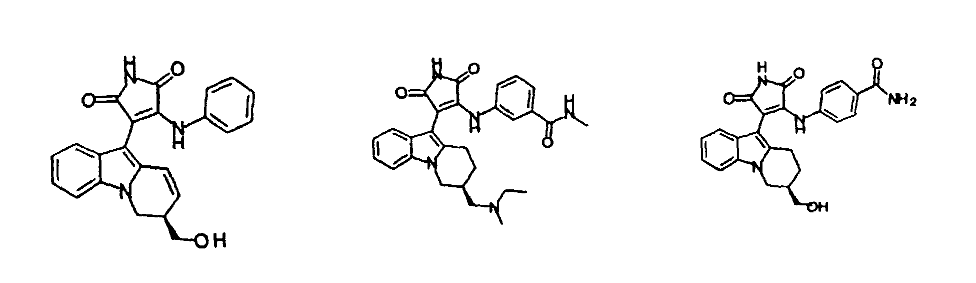 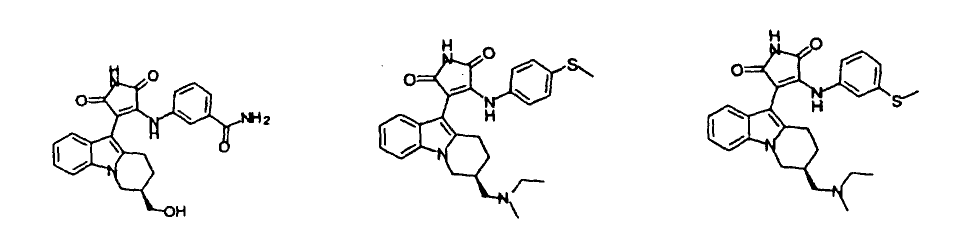 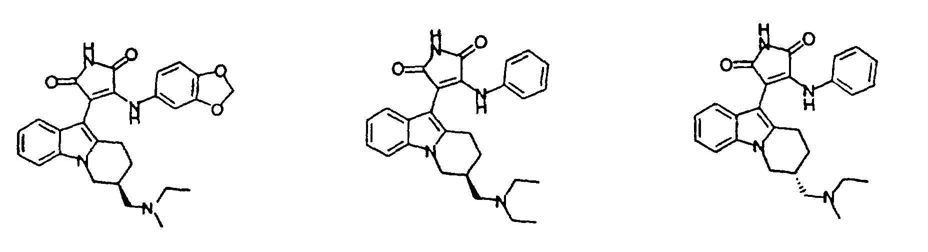 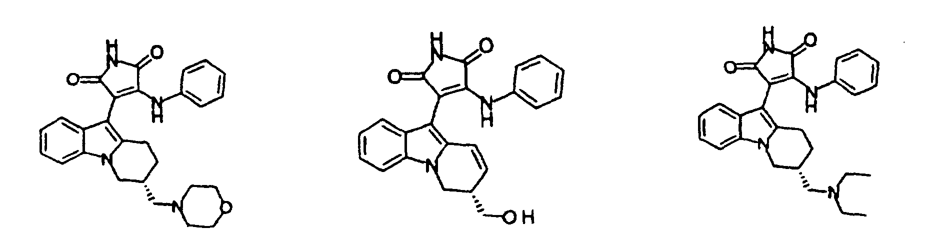 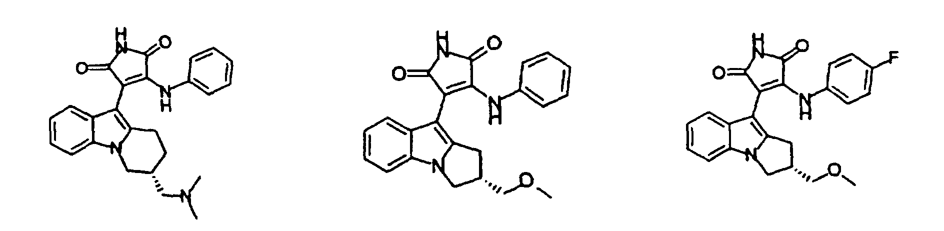 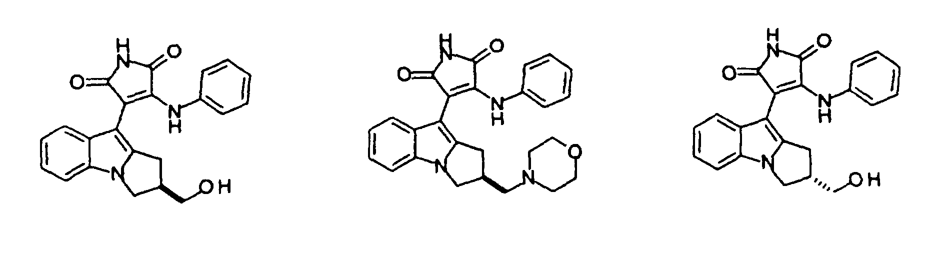 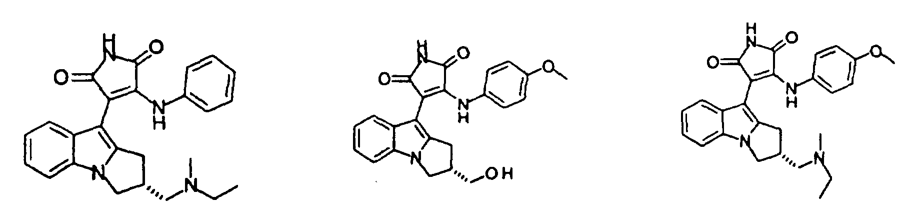 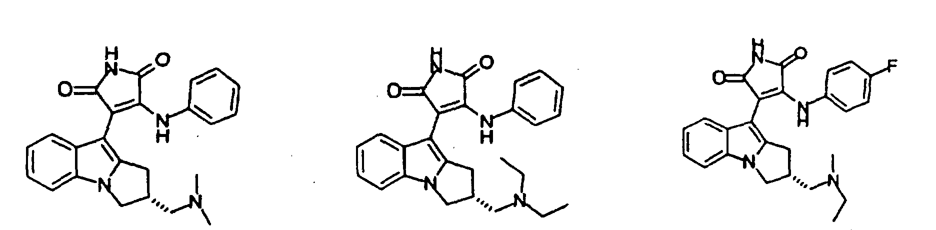 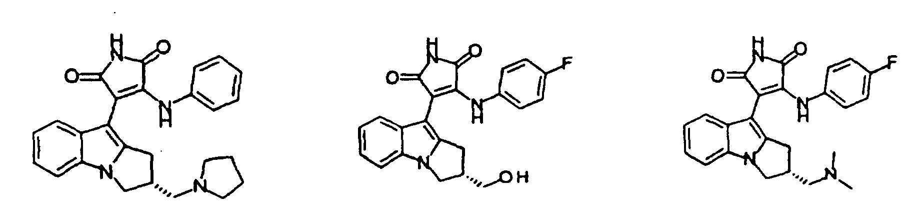 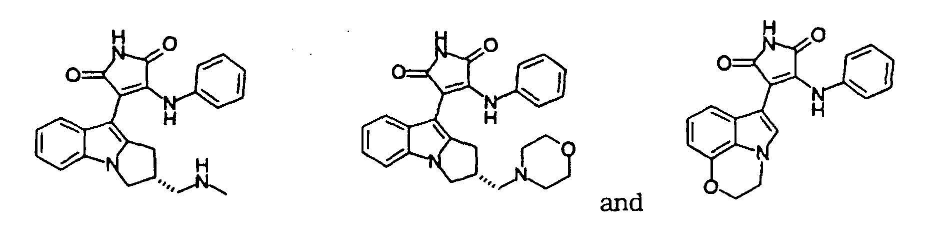
(11) A pharmaceutical composition comprising the disubstituted maleimide compound of any of (1) to (10) or a pharmaceutically acceptable salt thereof and a pharmaceutically acceptable carrier.
(12) A protein kinase C inhibitor containing the disubstituted maleimide compound of any of (1) to (10) or a pharmaceutically acceptable salt thereof as an active ingredient.
(13) A protein kinase C isozyme β selective inhibitor containing the disubstituted maleimide compound of any of (1) to (10) or a pharmaceutically acceptable salt thereof as an active ingredient.
(14) A therapeutic agent for diabetic complications, which contains the disubstituted maleimide compound of any of (1) to (10) or a pharmaceutically acceptable salt thereof as an active ingredient.

Each substituent and moiety used in the present specification are defined as follows.

The expression, C₁-C₆, means that the number of carbon atom is 1 to 6.

The protein kinase C isozyme β selective inhibitor is used for a pharmaceutical agent having a particularly high inhibitory activity against isozyme β among the PKC isozymes. This PKC inhibitor shows at least two times, preferably at least 10 times, and more preferably at least 30 times, as high an inhibitory activity against isozyme β as isozyme α.

Halogen atom is fluorine atom, chlorine atom, bromine atom or iodine atom, which is preferably fluorine atom, chlorine atom or bromine atom, particularly preferably fluorine atom.

Lower alkyl has linear or branched chain and 1 to 6 carbon atoms, and is specifically exemplified by methyl, ethyl, propyl, isopropyl, butyl, isobutyl, sec-butyl, tert-butyl, pentyl, isopentyl, tert-pentyl, hexyl and the like. It is preferably a linear or branched alkyl having 1 to 4 carbon atoms. The lower alkyl at R¹ is particularly preferably methyl, and lower alkyl at R¹⁰, R¹¹, R¹², R¹⁶, R¹⁷, R¹⁸, R¹⁹ and R²⁰ is particularly preferably methyl or ethyl.

The lower alkoxy means alkyloxy wherein the alkyl moiety is lower alkyl as defined above, which preferably has a linear or branched C₁-C₄ alkyl as the alkyl moiety. Examples thereof include methoxy, ethoxy, propoxy, isopropyloxy, butoxy, isobutyloxy, tert-butyloxy, pentyloxy, hexyloxy and the like.

Lower alkylthio is that wherein the alkyl moiety is lower alkyl as defined above, which preferably has a linear or branched C₁-C₄ alkyl as the alkyl moiety. Examples thereof include methylthio, ethylthio, propylthio, isopropylthio, butylthio, isobutylthio, tert-butylthio, pentylthio, hexylthio and the like.

Lower alkanoyl is alkylcarbonyl wherein the alkyl moiety is lower alkyl as defined above, which preferably has a linear or branched C₁-C₄ alkyl as the alkyl moiety. Examples thereof include acetyl, propionyl, butyryl, isobutyryl, pivaloyl and the like. At Z, it is particularly preferably acetyl.

Lower alkylamino is that wherein the alkyl moiety is lower alkyl as defined above, which preferably has a linear or branched C₁-C₄ alkyl as the alkyl moiety. Examples thereof include methylamino, ethylamino, propylamino, isopropylamino, butylamino, isobutylamino, tert-butylamino, pentylamino, hexylamino and the like.

Di(lower)alkylamino is dialkylamino wherein the alkyl moiety is lower alkyl as defined above, which preferably has a linear or branched C₁-C₄ alkyl as the alkyl moiety. Examples thereof include dimethylamino, diethylamino, methylethylamino, N-isopropyl-N-isobutylamino and the like.

Lower alkoxycarbonyl is alkyl-oxy-carbonyl wherein the alkoxy moiety is lower alkoxy as defined above, which preferably has a linear or branched C₁-C₄ alkyl as the alkyl moiety. Examples thereof include methoxycarbonyl, ethoxycarbonyl, propoxycarbonyl, isopropyloxycarbonyl, butoxycarbonyl, isobutyloxycarbonyl, tert-butyloxycarbonyl, pentyloxycarbonyl, hexyloxycarbonyl and the like.

Lower alkylaminocarbonyl is alkylaminocarbonyl wherein the alkylamino moiety is lower alkylamino as defined above, which preferably has a linear or branched C₁-C₄ alkyl as the alkyl moiety. Examples thereof include methylaminocarbonyl, ethylaminocarbonyl, propylaminocarbonyl, isopropylaminocarbonyl, butylaminocarbonyl, isobutylaminocarbonyl, tert-butylaminocarbonyl and the like.

Aryl is an aromatic hydrocarbon group having 6 to 18 carbon atoms, which is exemplified by phenyl, naphthyl, anthryl, indenyl, azulenyl, fluorenyl, phenanthryl, pyrenyl and the like, with preference given to phenyl.

Cycloalkyl is a saturated cycloalkyl having 3 to 8, preferably 5 to 7, carbon atoms, which is specifically cyclopropyl, cyclobutyl, cyclopentyl, cyclohexyl, cycloheptyl and cyclooctyl.

Heterocyclic ring is a saturated or unsaturated heterocyclic ring having 1 to 4 hetero atoms selected from oxygen atom, nitrogen atom and sulfur atom and the number of atoms constituting the ring is 5 to 12, which may be a monocyclic ring or a condensed ring.

The monocyclic heterocyclic ring is exemplified by 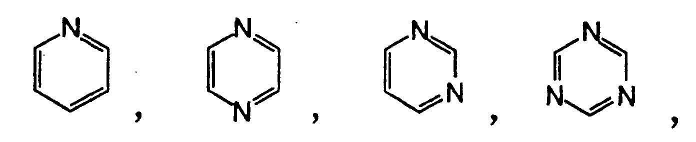 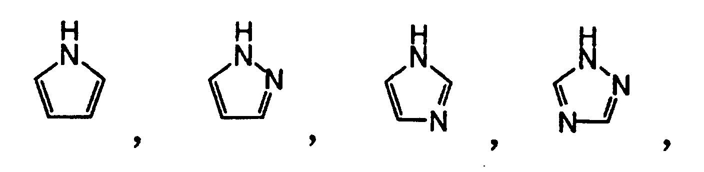 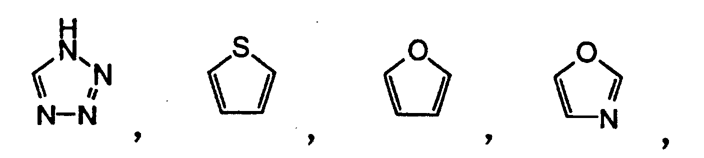 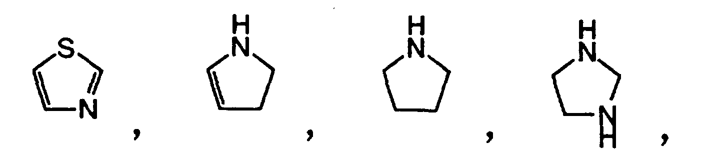 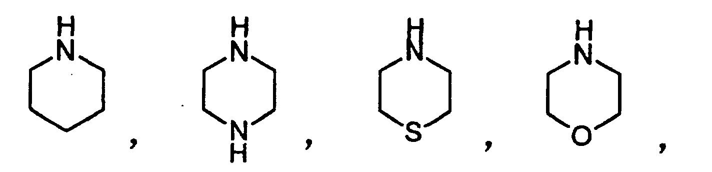 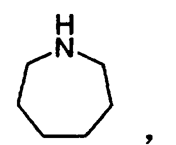 and the like, the condensed heterocyclic ring is exemplified by 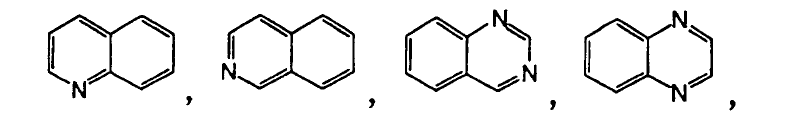 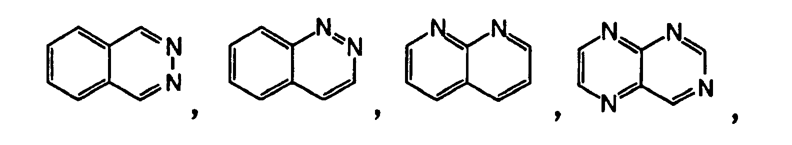 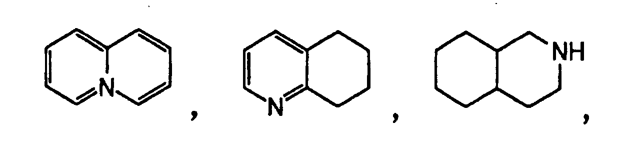 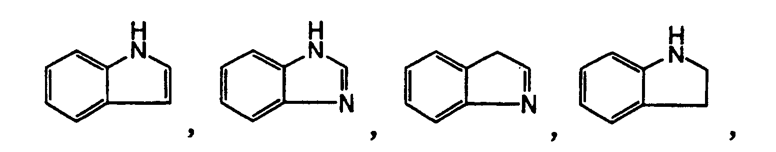 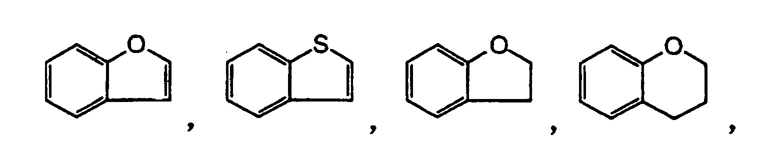 and the like.

More preferably, it is a saturated or unsaturated, 5- to 7-membered monocyclic heterocyclic ring having at least one nitrogen atom, and optionally having one oxygen atom or one sulfur atom as a second hetero atom.

Particularly preferably, it is a 5- or 6-membered monocyclic aromatic heterocyclic ring having 1 to 4 nitrogen atoms.

Optionally substituted lower alkyl is that wherein the lower alkyl as defined above, preferably a linear or branched alkyl having 1 to 4 carbon atoms, is optionally substituted by 1 to 3 substituents, and includes unsubstituted lower alkyl. Such substituent is selected from halogen atom, hydroxyl group, amino, the above-defined lower alkylamino and the above-defined di(lower)alkylamino. Optionally substituted lower alkyl is exemplified by methyl, ethyl, propyl, isopropyl, tert-butyl, hydroxymethyl, 2-hydroxyethyl, 2,3-dihydroxypropyl, trifluoromethyl, aminomethyl, 2-methylaminoethyl, 2-dimethylaminoethyl and the like, which is particularly preferably methyl at R³.

Optionally substituted lower alkoxy is that wherein the lower alkoxy as defined above, preferably a linear or branched alkoxy having 1 to 4 carbon atoms, is optionally substituted by 1 to 3 substituents, and includes unsubstituted lower alkoxy. Such substituent is selected from the above-defined halogen atom, hydroxyl group, amino, the above-defined lower alkylamino and the above-defined di(lower)alkylamino. Optionally substituted lower alkoxy is exemplified by methoxy, ethoxy, propoxy, isopropyloxy, tert-butyloxy, hydroxymethyloxy, 2-hydroxyethyloxy, 2-bromoethyloxy, 2-chloroethyloxy, aminomethyloxy, 2-methylaminoethyloxy, 2-dimethylaminoethyloxy and the like. At Z and R⁷, methoxy is particularly preferable.

Optionally substituted amidino is lower alkyl-substituted-amidino, or amidino itself, which may be substituted by the above-defined lower alkyl, preferably, a linear or branched alkyl having 1 to 4 carbon atoms on the nitrogen atom of amidino. Examples thereof include amidino, 1,2-dimethylamidino, 1,2-diethylamidino, 1-ethyl-2-methylamidino and the like. At Z, amidino is particularly preferable.

Optionally substituted guanidino is lower alkyl-substituted-guanidino, or guanidino itself, which may be substituted by the above-defined lower alkyl, preferably, a linear or branched alkyl having 1 to 4 carbon atoms on the nitrogen atom of guanidino. Examples thereof include guanidino, 2,3-dimethylguanidino, 2,3-diethylguanidino, 2-ethyl-3-methylguanidino and the like.

Optionally substituted aryl is the above-defined aryl optionally substituted by 1 to 5 substituents and includes unsubstituted one. Such substituent is selected from the above-defined halogen atom, hydroxyl group, the above-defined optionally substituted lower alkyl, the above-defined optionally substituted lower alkoxy, the above-defined lower alkylthio, the above-defined lower alkoxycarbonyl, amino, the above-defined lower alkylamino, the above-defined di(lower)alkylamino, nitro, cyano, carboxy, sulfo, carbamoyl, the above-defined lower alkylaminocarbonyl, the above-defined optionally substituted amidino, the above-defined optionally substituted guanidino, phenyl, benzyl, phenoxy, phenylsulfonyl, pyrrolidinyl, piperidyl and methylenedioxy.

Aryl of the optionally substituted aryl at R² and Z is particularly preferably phenyl.

The substituent for optionally substituted aryl at R² is more preferably the above-defined halogen atom, hydroxyl group, the above-defined optionally substituted lower alkyl, the above-defined optionally substituted lower alkoxy, the above-defined lower alkylthio, carbamoyl, the above-defined lower alkylaminocarbonyl, phenyl, benzyl, phenoxy and methylenedioxy, with particular preference given to fluorine atom, bromine atom, chlorine atom, hydroxyl group, methyl, isopropyl, trifluoromethyl, methoxy, carbamoyl, methylaminocarbonyl, phenyl, benzyl, phenoxy, methylthio and methylenedioxy (for example, 3,4-methylenedioxyphenyl and the like wherein phenyl has been substituted), which is particularly preferably fluorine atom, chlorine atom and methoxy.

Optionally substituted cycloalkyl is that wherein the cycloalkyl as defined above is optionally substituted by 1 to 3 substituents, and includes unsubstituted one. Such substituent is the same as those in the above-mentioned optionally substituted aryl, and selected from the above-defined halogen atom, hydroxyl group, the above-defined optionally substituted lower alkyl, the above-defined optionally substituted lower alkoxy, the above-defined lower alkylthio, the above-defined lower alkoxycarbonyl, amino, the above-defined lower alkylamino, the above-defined di(lower)alkylamino, nitro, cyano, carboxy, sulfo, carbamoyl, the above-defined lower alkylaminocarbonyl, the above-defined optionally substituted amidino, the above-defined optionally substituted guanidino, phenyl, benzyl, phenoxy, phenylsulfonyl, pyrrolidinyl, piperidyl and methylenedioxy.

The particularly preferable optionally substituted cycloalkyl at R² and Z is cyclopentyl, cyclohexyl or cycloheptyl.

Optionally substituted heterocyclic group is that where the above-defined heterocyclic ring is optionally substituted by 1 to 5 substituents, and includes unsubstituted one. Such substituent is the same as those in the above-mentioned optionally substituted aryl, and is selected from the above-defined halogen atom, hydroxyl group, the above-defined optionally substituted lower alkyl, the above-defined optionally substituted lower alkoxy, the above-defined lower alkylthio, the above-defined lower alkoxycarbonyl, amino, the above-defined lower alkylamino, the above-defined di(lower)alkylamino, nitro, cyano, carboxy, sulfo, carbamoyl, the above-defined lower alkylaminocarbonyl, the above-defined optionally substituted amidino, the above-defined optionally substituted guanidino, phenyl, benzyl, phenoxy, phenylsulfonyl, pyrrolidinyl, piperidyl and methylenedioxy.

The heterocyclic ring of the optionally substituted heterocyclic group at Z is preferably a saturated or unsaturated 5- to 7-membered monocyclic heterocyclic ring having at least one nitrogen atom and optionally having one oxygen atom or one sulfur atom as a second hetero atom. Examples thereof include the following.
5- or 6-membered aromatic heterocycle having 1 to 4 nitrogen atoms 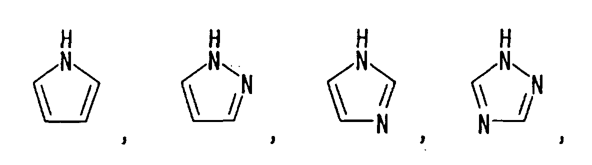 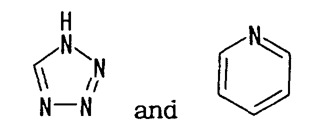 5- to 7-membered saturated monocyclic heterocyclic ring having at least one nitrogen atom and optionally having one oxygen atom or one sulfur atom as a second hetero atom

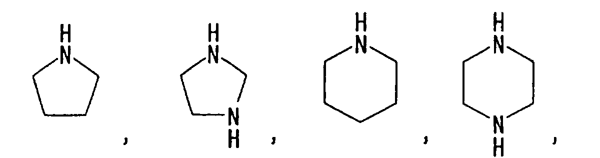

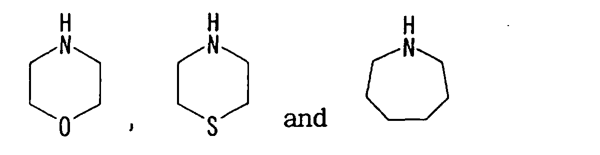

More preferably, it is pyrrolidinyl, piperidyl, piperazinyl, morpholinyl or thiomorpholinyl, particularly preferably pyrrolidinyl or piperidyl.

The substituent for the optionally substituted heterocyclic group at Z is preferably hydroxyl group, the above-defined optionally substituted lower alkyl, the above-defined lower alkoxycarbonyl, the above-defined di(lower)alkylamino, carbamoyl, the above-defined lower alkylaminocarbonyl, the above-defined optionally substituted amidino, benzyl, pyrrolidinyl and piperidyl, particularly preferably hydroxyl group, methyl, hydroxymethyl, 2-hydroxyethyl, 2,3-dihydroxypropyl, tert-butoxycarbonyl, dimethylamino, carbamoyl, tert-butylaminocarbonyl, amidino, benzyl, 1-pyrrolidinyl and piperidino.

The position of the substituent is not particularly limited as long as it allows synthesis.

In the formula [I], m at W is preferably 0. When m is 0,l+n is preferably an integer of 1 to 4. When R⁴ is independently W, it is particularly preferable that m be 0 and l+n be 3 or 4, and when R⁴ and R³ jointly form a group of the formula 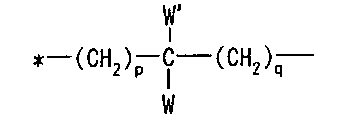 or 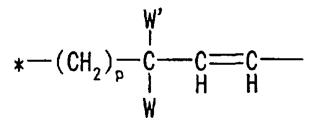 or R⁴ and R⁵ jointly form a group of the formula 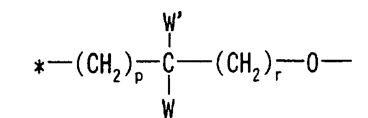 m is particularly preferably 0 and l+n is 1.

In the formula [I], p+q of the group formed by R⁴ and R³ in combination is preferably an integer of 2 to 4, particularly preferably p=1 and q=1, p=1 and q=2, or p=2 and q=1, and
p+r of the group formed by R⁴ and R⁵ in combination is preferably 0, 1 or 2.

The heterocyclic ring formed by R¹⁶ and R¹⁷ in combination together with the nitrogen atom they bind to is a heterocyclic ring having a nitrogen atom in the heterocyclic ring and capable of binding via said nitrogen atom, as defined in the above with respect to the optionally substituted heterocyclic group.

The pharmaceutically acceptable salt thereof may be any salt as long as it can form a nontoxic salt with the compound of the above-mentioned formula [I]. Examples thereof include salts with inorganic acid such as hydrochloric acid, sulfuric acid, phosphoric acid, hydrobromic acid and the like ; salts with organic acid such as oxalic acid, malonic acid, citric acid, fumaric acid, lactic acid, malic acid, succinic acid, tartaric acid, acetic acid, gluconic acid, ascorbic acid, methylsulfonic acid, benzylsulfonic acid and the like ; salts with inorganic base such as sodium hydroxide, potassium hydroxide, calcium hydroxide, magnesium hydroxide, ammonium hydroxide and the like ; salts with organic base such as methylamine, diethylamine, triethylamine, triethanolamine, ethylenediamine, tris(hydroxymethyl)methylamine, guanidine, choline, cinchonine and the like ; or salts with amino acid such as lysine, arginine, alanine and the like. The present invention further encompasses water-containing compounds and hydrates and solvates of each compound.

The compound of the formula [I] includes various isomers. For example, there exist geometric E- and Z-isomers. When asymmetrical carbon atom(s) exist(s), stereoisomers (e.g., enantiomer and diastereomer) exist. Depending on the case, tautomers may exist in the present invention. Therefore, the present invention encompasses all these isomers and mixtures thereof.

The present invention further encompasses prodrugs and metabolites of each compound. Prodrug is a derivative of the compound of the present invention, which has a chemically or metabolically decomposable group and which shows efficacy upon restoration to its original form after administration to a body, wherein included therein are a complex without a covalent bond and salts.

When the compound of the present invention is used as a pharmaceutical preparation, it is generally admixed with conventionally known pharmaceutically acceptable carrier, excipient, diluent, extender, disintegrator, stabilizer, preservative, buffer, emulsifier, aromatic, coloring agent, sweetener, tackifier, flavor, solubilizer and other additive, specifically water, vegetable oil, alcohol (e.g., ethanol, benzyl alcohol and the like), polyethylene glycol, glycerol triacetate, gelatin, carbohydrate (e.g., lactose, starch and the like), magnesium stearate, talc, lanolin, petrolatum and the like, and formulated into tablets, pills, powders, granules, suppositories, injections, eye drops, liquids, capsules, troches, aerosols, elixirs, suspensions, emulsions, syrups and the like by a conventional method, which can be administered systemically or locally by oral or parenteral administration.

While the dose varies depending on age, body weight, symptom, therapeutic effect, administration route and the like, it is generally 0.1 mg to 1 g per dose which is given once to several times a day for an adult.

One example of the production method of the compound to practice the present invention is explained in the following, to which the production method of the compound of the present invention is not limited.

In each step, the treatment of reaction may be a conventional one, such as isolation and purification, crystallization, recrystallization, silica gel column chromatography, preparative HPLC and the like, which may be appropriately selected and combined. Where necessary, a protecting group may be introduced into a functional group and deprotected for production.

In the method of the present invention, except the method for forming an optically active compound, each step using an optically active compound can be also applied to the production of a racemate, and each step using a racemate can be also applied to the production of an optically active compound.

### Production method 1-1

In this production method, acetamide derivative and oxalic acid ester are subjected to condensation cyclization to give a maleimide compound and an amine compound is substituted. 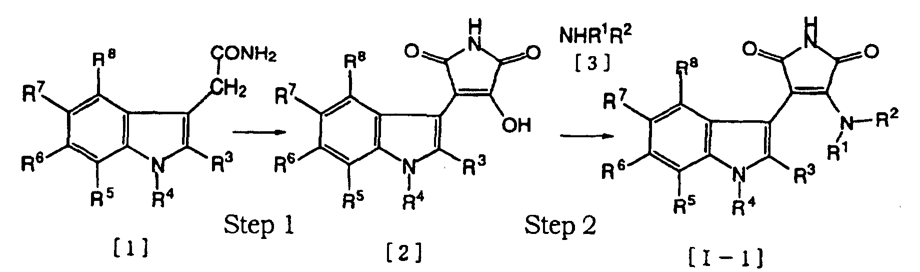 wherein each symbol is as defined above.

### Step 1

Compound [1] and dialkyl ester of oxalic acid such as dimethyl oxalate, diethyl oxalate and the like are subjected to condensation cyclization in a solvent in the presence of a base such as potassium tert-butoxide, sodium hydride, potassium hydride and the like under an argon atmosphere from under cooling to under heating to give compound [2].

As the solvent, alcohol solvent (e.g., methanol, ethanol, n-propanol, isopropanol and the like); hydrocarbon solvent (e.g., benzene, toluene, hexane, xylene and the like); halogen solvent (e.g., dichloromethane, chloroform, carbon tetrachloride, 1,2-dichloroethane and the like); ether solvent (e.g., 1,4-dioxane, diethyl ether, 1,2-dimethoxyethane, tetrahydrofuran and the like); polar solvent (e.g., dimethylformamide, dimethyl sulfoxide, acetonitrile and the like); or a mixed solvent thereof can be used.

### Step 2

Compound [2] and compound [3] are reacted in an organic solvent such as chloroform, carbon tetrachloride, methylene chloride, toluene, nitrobenzene, acetic acid and the like under heating to give compound [I-1].

### Production method 1-2

In this production method, aminoacetic acid ester and half ester of malonic acid are subjected to condensation cyclization to give a pyrrol-2-one compound, and after substitution with indole, amine compound is substituted. 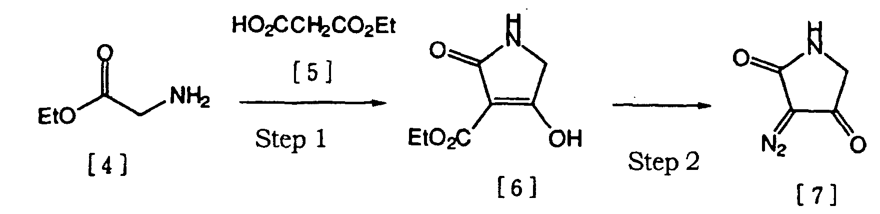 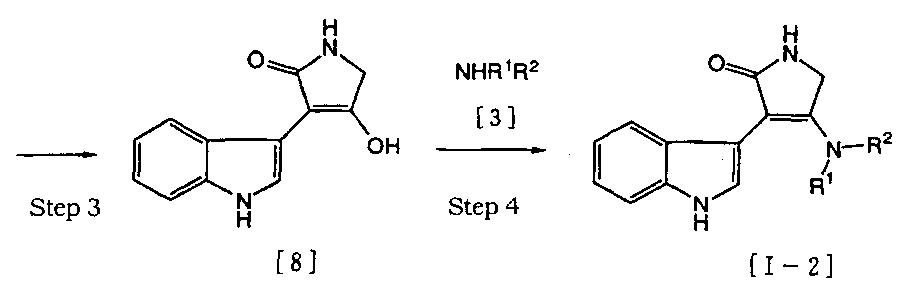 wherein each symbol is as defined above.

### Step 1 - Step 3

In the same manner as in reference publication, *J*. *American Chemical Society,* Vol. 119, No. 41, p. 9641-9651, 1997, compound [8] can be obtained from compound [4].

### Step 4

In the same manner as in Production method 1-1, Step 2, compound [I-2] can be obtained from compound [8].

### Production method 2-1

In this production method, a substituent is introduced into the nitrogen atom on the indole ring in the above-mentioned formula [I]. 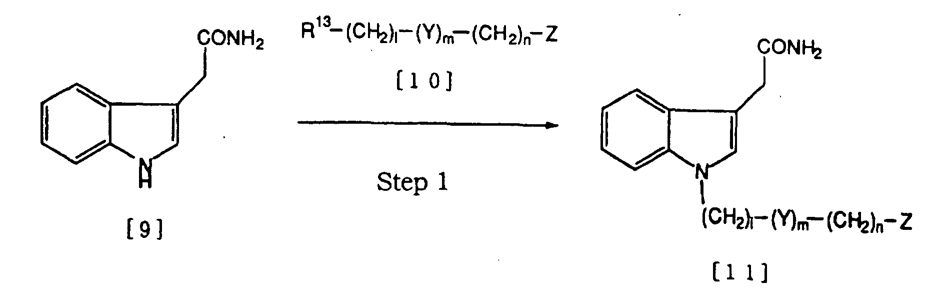 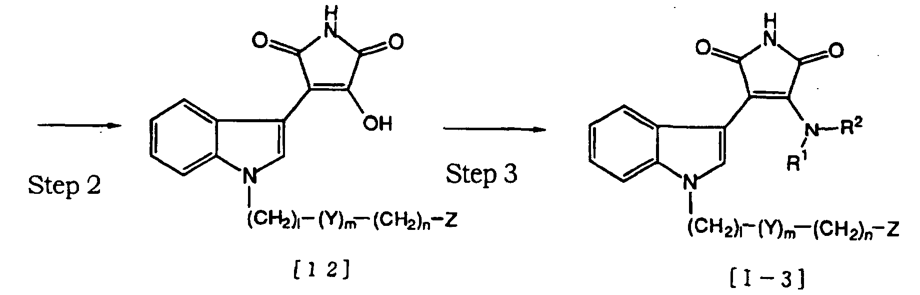 wherein Y, Z, l, m, n, R¹ and R² are as defined above, and R¹³ is halogen atom or a leaving group such as tosyloxy, mesyloxy and the like, with the proviso that when l=m=n=0, Z is not hydrogen atom.

### Step 1

Compound [9] and compound [10] are reacted in a solvent in the presence of a base such as potassium tert-butoxide, sodium hydride, potassium hydride, sodium methoxide, sodium ethoxide and the like, under an argon atmosphere preferably under cooling to give compound [11].

As the solvent, ether solvent such as 1,4-dioxane, diethyl ether, 1,2-dimethoxyethane, tetrahydrofuran and the like; polar solvent such as dimethylformamide, dimethyl sulfoxide, acetonitrile and the like; and alcohol solvent such as methanol, ethanol, n-propanol, isopropanol and the like are preferable.

### Step 2

In the same manner as in Production method 1-1, Step 1, compound [12] can be obtained from compound [11].

### Step 3

In the same manner as in Production method 1-1, Step 2, compound [1-3] can be obtained from compound [12].

When Z is a substituent having nitrogen atom, a compound wherein nitrogen atom of Z has been protected is preferably applied to this production method, and then is deprotected in the later step.

### Production method 2-2

This production method comprises substituting maleimide with an amine compound by the use of an intermediate of the above-mentioned formula [I] wherein Z at W is the protected hydroxyl group, removing the hydroxy-protecting group at Z, halogenation, and substitution with amine compound. 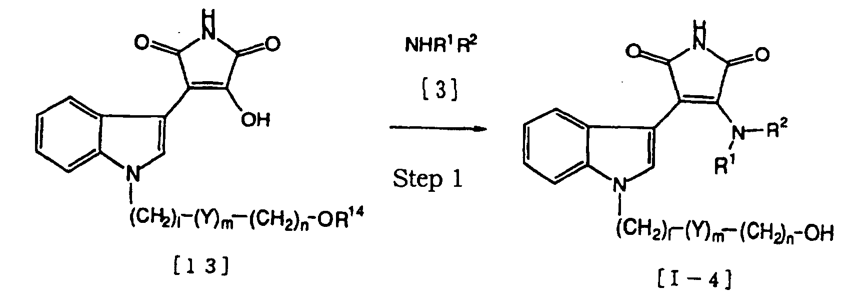 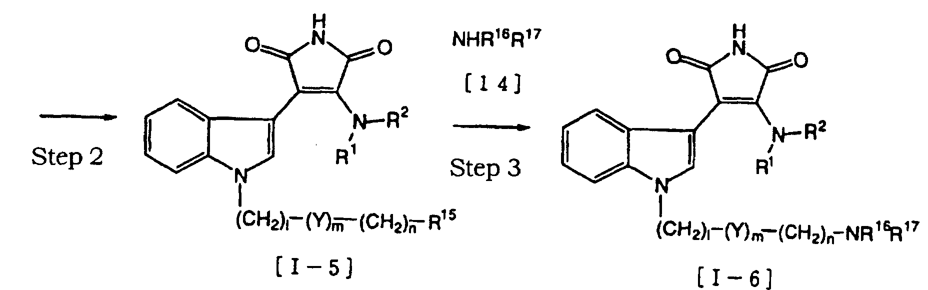 wherein Y,l, m, n, R¹ and R² are as defined above, R¹⁴ is hydroxy-protecting group, R¹⁵ is halogen atom or a leaving group such as tosyloxy, mesyloxy, trifluoromethanesulfonyloxy and the like, R¹⁶ and R¹⁷ are the same or different and each is hydrogen atom or lower alkyl, or R¹⁶ and R¹⁷ jointly form a heterocyclic ring together with the nitrogen atom they bind with.

### Step 1

Compound [13] and compound [3] are reacted in the same manner as in Production method 1-1, Step 2, and the hydroxy-protecting group is removed by a conventional method to give compound [I-4].

As the hydroxy-protecting group, tert-butyldimethylsilyl, acetyl, benzyl, methoxyethoxymethyl and the like are exemplified. For example, when R¹⁴ is tert-butyldimethylsilyl, the deprotection includes treatment with tetrabutylammonium fluoride in tetrahydrofuran at room temperature, treatment with acetic acid-water-tetrahydrofuran at room temperature to under heating, and the like.

### Step 2

Compound [I-4] is halogenated using a halogenating agent such as hypohalogenite (e.g., hypochlorite and the like), N-bromosuccinimide and the like in the presence of a reducing agent such as triphenylphosphine and the like, in a solvent under an argon atmosphere under cooling to give compound [I-5].

As the solvent, ether solvent such as 1,4-dioxane, diethyl ether, 1,2-dimethoxyethane, tetrahydrofuran and the like; and halogen solvent such as dichloromethane, chloroform, carbon tetrachloride, 1,2-dichloroethane and the like are preferable.

Alternatively, hydroxyl group may be subjected to mesylation, tosylation, trifluoromethanesulfonylation and the like to give a leaving group, instead of halogenation.

For example, compound [1-4] is reacted with sulfonic anhydride such as trifluoromethanesulfonic anhydride and the like in a solvent in the presence of a base such as 2,4,6-collidine and the like under an argon atmosphere under cooling to make hydroxyl group a leaving group.

As the solvent, an organic solvent such as chloroform, carbon tetrachloride, methylene chloride, toluene, nitrobenzene, acetic acid and the like is preferable.

### Step 3

Compound [I-5] is reacted with compound [14] in a solvent under heating to give compound [I-6].

When compound [14] is an unsaturated heterocyclic ring such as imidazole, pyrazole, 1,2,4-triazole and the like, compound [I-5] is reacted with compound [14] in a solvent in the presence of a strong base such as sodium hydride and the like under an argon atmosphere under cooling to give compound [I-6].

As the solvent, ether solvent such as 1,4-dioxane, diethyl ether, 1,2-dimethoxyethane, tetrahydrofuran and the like; and polar solvent such as dimethylformamide, dimethyl sulfoxide, acetonitrile and the like are preferable.

When compound [14] has a low boiling point, this step is preferably conducted in a sealed reactor.

### Production method 2-3

In this production method, maleimide is substituted with amine compound using an intermediate of the above-mentioned formula [I] wherein Y at W is the protected hydroxyl group, then after deprotection of hydroxy-protecting group at Y and halogenation, amine compound or thiourea compound is substituted. 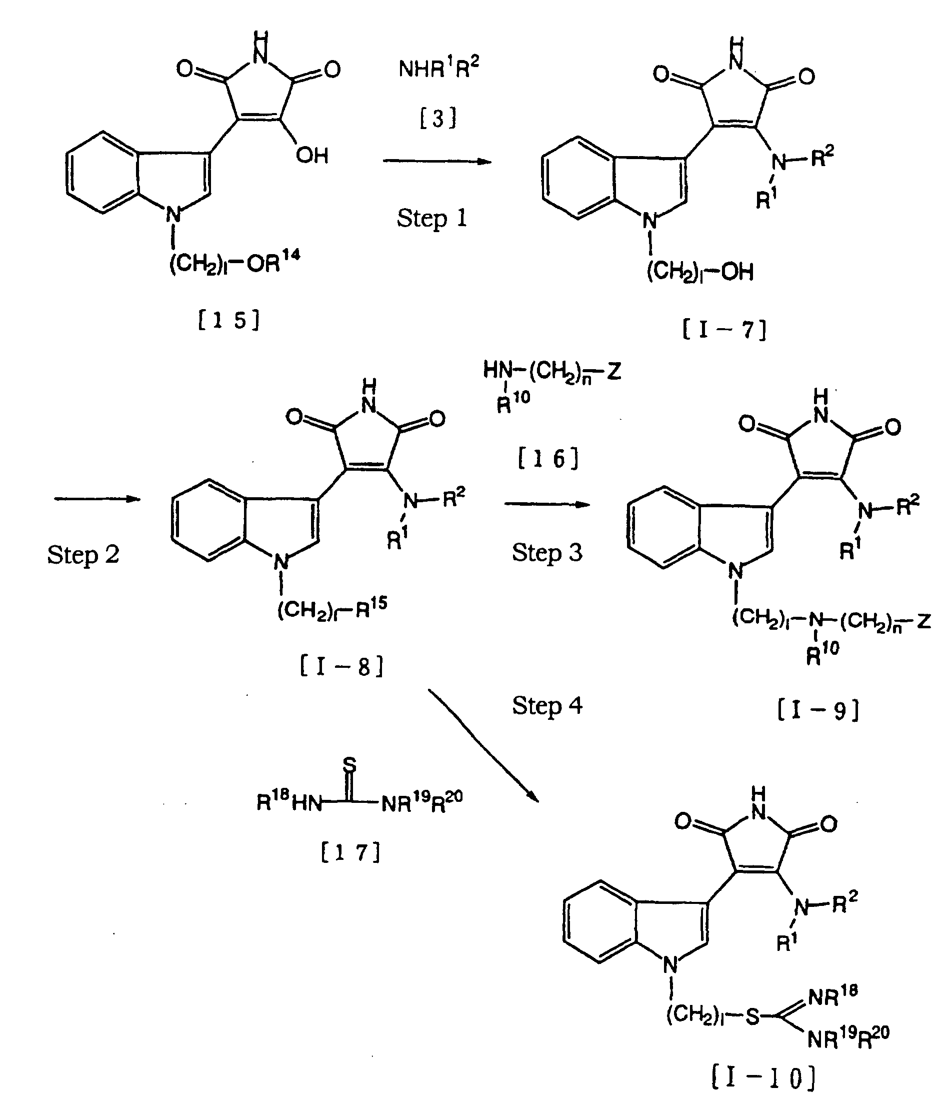 wherein Z, l, n, R¹, R², R¹⁰, R¹⁴ and R¹⁵ are as defined above, and R¹⁸, R¹⁹ and R²⁰ are the same or different and each is lower alkyl.

### Step 1

By reacting compound [15] and compound [3] in the same manner as in Production method 2-2, Step 1, compound [1-7] can be obtained.

### Step 2

By reacting compound [I-7] in the same manner as in Production method 2-2, Step 2, compound [I-8] can be obtained.

### Step 3

By reacting compound [I-8] and compound [16] in the same manner as in Production method 2-2, Step 3, compound [I-9] can be obtained.

### Step 4

By reacting compound [I-8] with compound [17] in a solvent under heating, compound [I-10] can be obtained.

As the solvent, ether solvent such as 1,4-dioxane, diethyl ether, 1,2-dimethoxyethane, tetrahydrofuran and the like; polar solvent such as dimethylformamide, dimethyl sulfoxide, acetonitrile and the like; and alcohol solvent such as methanol, ethanol, n-propanol, isopropanol and the like are preferable.

### Production method 3-1

This production method is for introducing a substituent into a saturated heterocyclic ring when Z at W in the above-mentioned formula [I] is a saturated heterocyclic ring having a protected amino or protected nitrogen atom. The following are examples wherein Z is a saturated heterocyclic ring having a protected nitrogen atom. 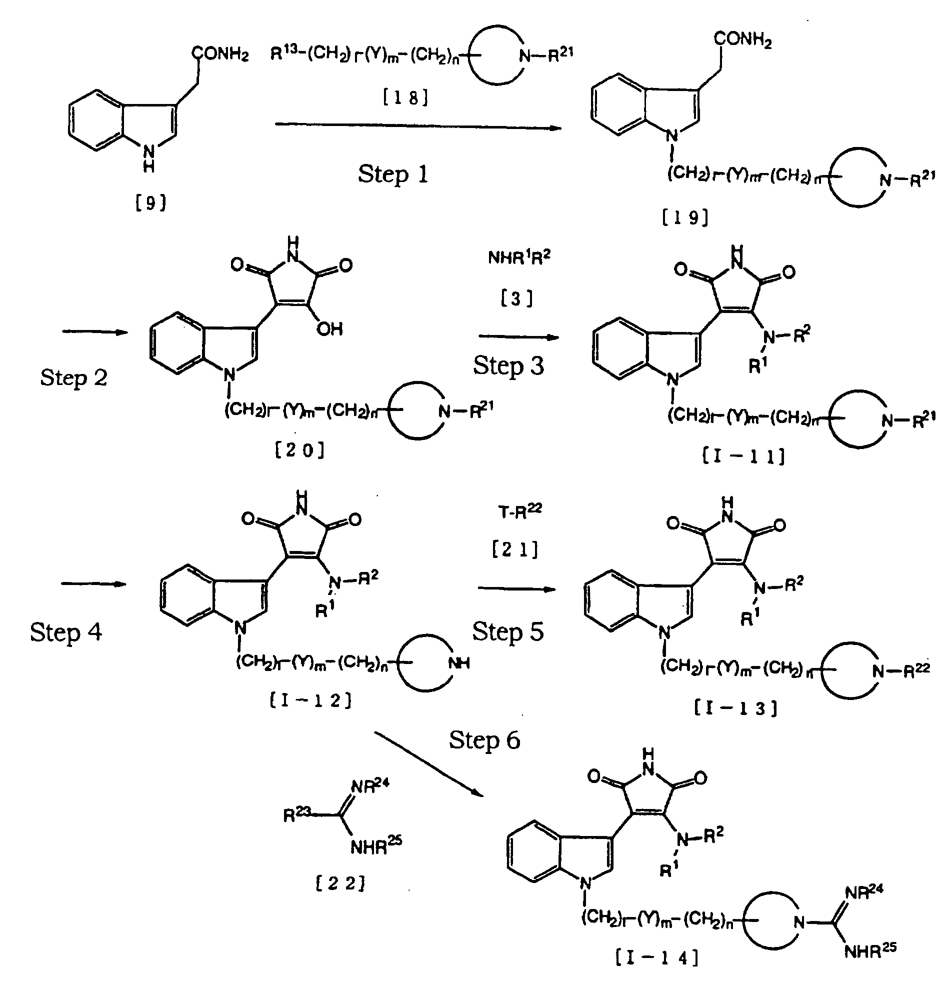 wherein Y, l, m, n, R¹, R² and R¹³ are as defined above, R²¹ is amine protecting group, R²² is lower alkyl, T-R²² is an alkylating agent, R²³ is leaving group, and R²⁴ and R²⁵ are the same or different and each is hydrogen atom or lower alkyl.

### Step 1

By reacting compound [9] and compound [18] in the same manner as in Production method 2-1, Step 1, compound [19] can be obtained.

### Step 2

By reacting compound [19] in the same manner as in Production method 1-1, Step 1, compound [20] can be obtained.

### Step 3

By reacting compound [20] and compound [3] in the same manner as in Production method 1-1, Step 2, compound [I-11] can be obtained.

### Step 4

By deprotection of the amine protecting group of compound [I-11] by a conventional method, compound [I-12] can be obtained.

As the amine-protecting group, tert-butoxycarbonyl, benzyloxycarbonyl, trifluoroacetyl and the like are exemplified. For example, when R²¹ is tert-butoxycarbonyl, deprotection includes treatment with hydrochloric acid in tetrahydrofuran at room temperature, treatment with hydrochloric acid-dioxane at room temperature, and the like.

### Step 5

Compound [I-12] is reacted with compound [21], which is an alkylating agent, and the like in a solvent in the presence of a base such as potassium carbonate, sodium carbonate, sodium hydroxide, potassium hydroxide and the like at room temperature to give compound [I-13].

As the alkylating agent, alkylsulfonic acid ester such as methyl methanesulfonate and the like, alkyl halide such as methyl iodide and the like, and the like are exemplified.

As the solvent, alcohol solvent (e.g., methanol, ethanol, n-propanol, isopropanol and the like); ether solvent (e.g., 1,4-dioxane, diethyl ether, 1,2-dimethoxyethane, tetrahydrofuran and the like); polar solvent (e.g., dimethylformamide, dimethyl sulfoxide, acetonitrile and the like) are preferable.

### Step 6

Compound [I-12] is reacted with compound [22] in a solvent in the presence of a base such as potassium carbonate, sodium carbonate, sodium hydroxide, potassium hydroxide, triethylamine and the like at room temperature to give compound [I-14].

The leaving group at R²³ is exemplified by pyrazol-1-yl, methylthio and the like.

As the solvent, ether solvent such as 1,4-dioxane, diethyl ether, 1,2-dimethoxyethane, tetrahydrofuran and the like; and polar solvent such as dimethylformamide, dimethyl sulfoxide, acetonitrile and the like are preferable.

A compound wherein R²⁴ and R²⁵ are protecting groups such as tert-butoxycarbonyl and the like in an amidino compound [22] may be used and deprotected after this step to introduce amidino onto the heterocyclic ring.

In this production method, similar reaction is carried out when Z is protected amino, i.e., Z is -N (R²¹)₂, to give a compound wherein Z is -NHR²², -N(R²²)₂ or -NHC (=NR²⁴)NHR²⁵.

### Production method 4-1

In this production method, carboxylic acid ester is reduced, hydroxyl group is protected, maleimido group is formed on the ring, amine compound is substituted and a substituent is introduced into the deprotected hydroxyl group.

### Production method 4-1-1

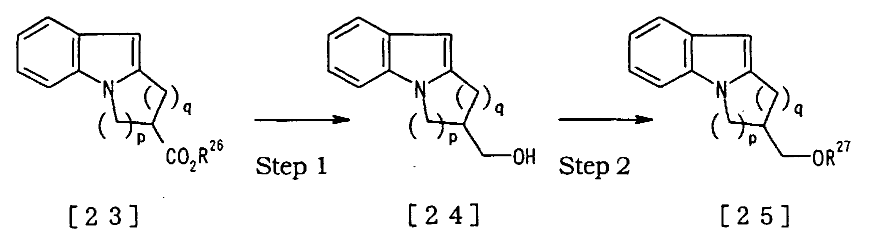

wherein R²⁶ is lower alkyl such as methyl, ethyl and the like, R²⁷ is a hydroxy-protecting group, and p and q are as defined above.

### Step 1

Compound [23] is reduced by a conventional method comprising adding a reducing agent such as lithium aluminum hydride, lithium borohydride, sodium borohydride, diborane and the like in a solvent preferably under cooling, and the like to give compound [24].

As the solvent, alcohol solvent such as methanol, ethanol, n-propanol, isopropanol and the like; hydrocarbon solvent such as benzene, toluene, hexane, xylene and the like; halogen solvent such as dichloromethane, chloroform, carbon tetrachloride, 1,2-dichloroethane and the like; ether solvent such as 1,4-dioxane, diethyl ether, 1,2-dimethoxyethane, tetrahydrofuran and the like; or a mixed solvent thereof can be exemplified.

### Step 2

The hydroxyl group of compound [24] is protected by a conventional method to give compound [25].

The hydroxy-protecting group is exemplified by tert-butyldiphenylsilyl, acetyl, benzyl, methoxyethoxymethyl and the like. For example, when R²⁷ is tert-butyldiphenylsilyl, protection comprises treatment with tert-butyldiphenylsilyl chloride and imidazole in dimethylformamide at room temperature, and the like.

### Production method 4-1-2

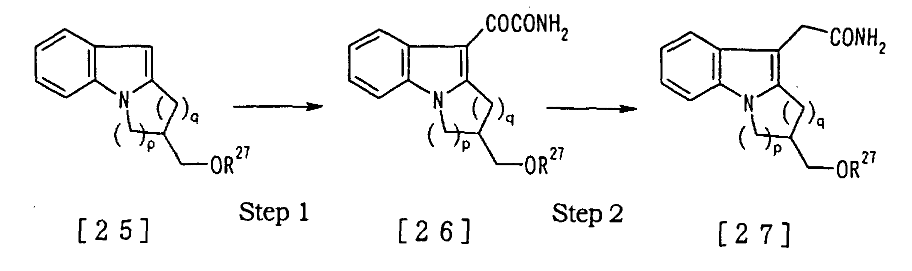

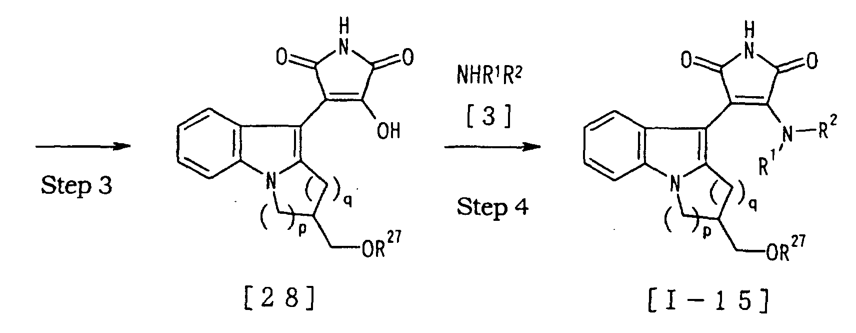

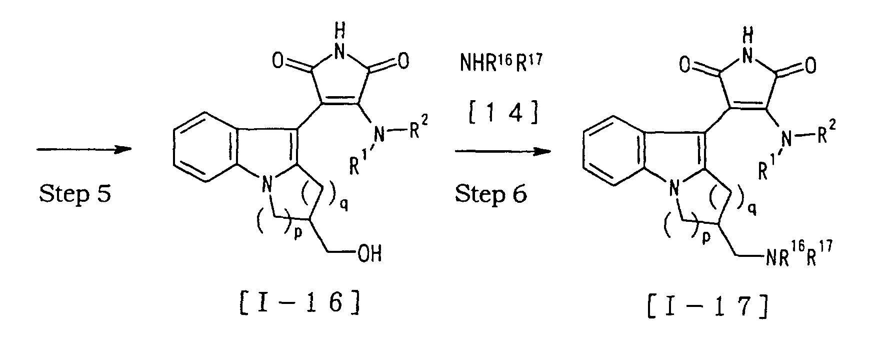

wherein each symbol is as defined above.

### Step 1

The compound [25] obtained in the same manner as in the method described in *Tetrahedron*, 47, 4645, 1991 and *J*. *Med. Chem.*, 36, 21-29, 1993 and the like is reacted with oxalyl chloride in a solvent under an argon atmosphere at room temperature and is reacted with concentrated aqueous ammonia under cooling to give compound [26]. Where necessary, the reaction proceeds in the presence of a tertiary amine such as triethylamine and the like.

As the solvent, an organic solvent such as chloroform, carbon tetrachloride, methylene chloride, toluene, nitrobenzene, tetrahydrofuran, acetic acid, ethyl acetate and the like is preferable.

### Step 2

Only the carbonyl directly bonded to the ring of compound [26] is hydrogenated to give compound [27].

In this step, a conventional reduction method such as reduction using a reducing agent (e.g., lithium borohydride and the like), catalytic reduction using hydrogen gas in the presence of a metal catalyst (e.g., palladium carbon, Raney-nickel and the like) at room temperature or refluxing temperature, and the like, with preference given to weak reduction without reduction of amide and hydrogenation. For example, compound [26] is reduced with sodium borohydride in alcohol solvent (e.g., methanol, ethanol, n-propanol, isopropanol and the like) at room temperature under an argon atmosphere, and is reacted with acid catalyst (e.g., trifluoroacetic acid and the like) and trialkylsilane (e.g., triethylsilane and the like) in an organic solvent such as chloroform, carbon tetrachloride, methylene chloride, toluene, nitrobenzene, acetic acid and the like at room temperature to hydrogenate the carbonyl directly bonded to the ring.

### Step 3

In the same manner as in Production method 1-1, Step 1, compound [28] can be obtained from compound [27].

### Step 4

In the same manner as in Production method 1-1, Step 2, compound [I-15] can be obtained from compound [28] and compound [3].

### Step 5

Removal of hydroxy-protecting group from compound [I-15] by a conventional method gives compound [I-16].

For example, when R²⁷ is tert-butyldiphenylsilyl, deprotection involves treatment with tetrabutylammonium fluoride in tetrahydrofuran at room temperature, treatment with acetic acid-water-tetrahydrofuran at room temperature, or from room temperature to under heating, and the like.

### Step 6

In the same manner as in Production method 2-2, Step 2 and Step 3, compound [I-17] can be obtained from compound [I-16] and compound [14].

### Production method 4-2

This production method relates to forming an optically active compound when obtaining an indole condensed ring as in Production method 4-1.

### Production method 4-2-1

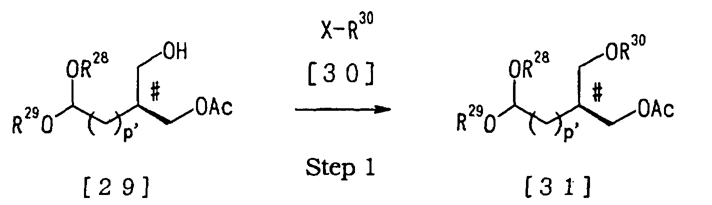

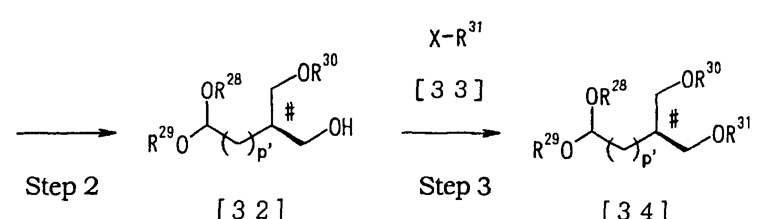

wherein R²⁸, R²⁹ and R³¹ are protecting groups relatively stable under acidic and alkaline conditions, and tolerable in multi-stage reactions, -OR³⁰ is a leaving group such as mesyloxy and the like, p' is an integer of 1 to 3, X is halogen atom, and # means that a carbon atom shown with # is an asymmetric carbon atom and that the compound is optically active.

### Step 1

The compound [30] is introduced into hydroxyl group of compound [29] by a conventional method to make hydroxyl group a leaving group, whereby compound [31] is obtained.

R²⁸ and R²⁹ are, for example, lower alkyl such as methyl and ethyl.

For example, when R³⁰ is mesyl, the compound [29] is treated with mesyl chloride in tetrahydrofuran solvent under an argon atmosphere in the presence of a base such as triethylamine, pyridine and the like, and the like.

Anhydride R³⁰-O-R³⁰ may be used instead of halide [30].

### Step 2

Acetyl of compound [31] is removed by a conventional method to give compound [32].

Deacetylation proceeds under the conditions not affecting -OR³⁰. For example, compound [31] is reacted in the presence of a base such as potassium carbonate, sodium carbonate and the like in an argon atmosphere.

The acetyl of compound [29] may be a different protecting group as long as it is a protecting group that did not react in Step 1, and removed in Step 2 without affecting -OR³⁰.

### Step 3

The compound [33] is introduced into hydroxyl group of compound [32] by a conventional method to give a protected compound [34].

For example, when R³¹ is tert-butyldiphenylsilyl, the compound [32] is treated with tert-butyldiphenylchlorosilane and imidazole in dimethylformamide, and the like.

### Production method 4-2-2

In this production method, compound [37] which is an enantiomer of compound [34], is obtained from compound [29]. 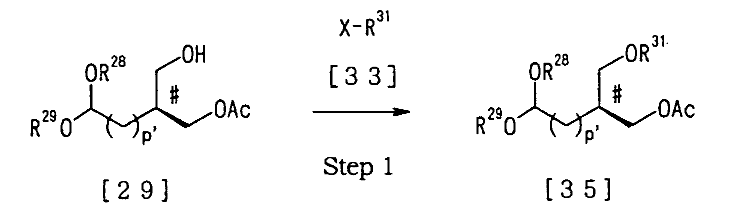 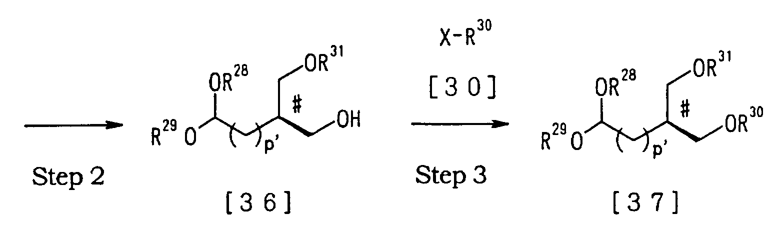 wherein each symbol is as defined above.

### Step 1

Compound [29] is reacted with compound [33] in the same manner as in Production method 4-2-1, Step 3, to give compound [35].

### Step 2

Acetyl of compound [35] is removed by a conventional method to give compound [36].

Acetyl of compound [29] may be a different protecting group as long as it can be removed without affecting R³¹.

### Step 3

Compound [36] is reacted with compound [30] in the same manner as in Production method 4-2-1, Step 1, to give compound [37].

### Production method 4-2-3

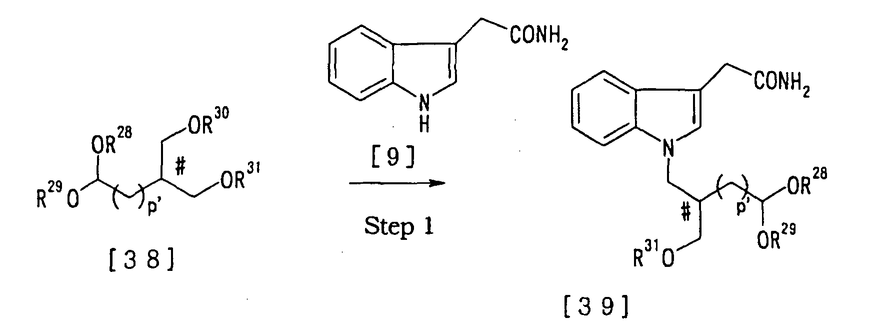

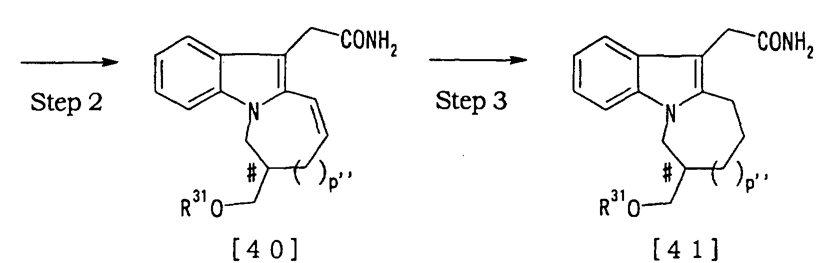

wherein p" is an integer less 1 from p', and R²⁸, R²⁹, R³⁰, R³¹ and # are as defined above.

### Step 1

Compound [38] is reacted with compound [9] in a solvent in the presence of a base such as potassium tert-butoxide, sodium hydride, potassium hydride and the like and, where necessary, sodium halide such as sodium iodide and the like under an argon atmosphere to give compound [39].

As the solvent, alcohol solvent such as methanol, ethanol, n-propanol, isopropanol and the like; hydrocarbon solvent such as benzene, toluene, hexane, xylene and the like; halogen solvent such as dichloromethane, chloroform, carbon tetrachloride, 1,2-dichloroethane and the like; ether solvent such as 1,4-dioxane, diethyl ether, 1,2-dimethoxyethane, tetrahydrofuran and the like; polar solvent such as dimethylformamide, dimethyl sulfoxide, acetonitrile and the like; or a mixed solvent thereof are exemplified.

### Step 2

Compound [39] is reacted in a solvent such as chloroform and the like and in the presence of an acid catalyst such as trifluoroacetic acid and the like to give compound [40].

### Step 3

Compound [40] is reduced by a conventional method to give compound [41].

For example, catalytic reduction under a hydrogen atmosphere in alcohol solvent such as ethanol and the like in the presence of a catalyst such as palladium carbon and the like, and the like may be applied. 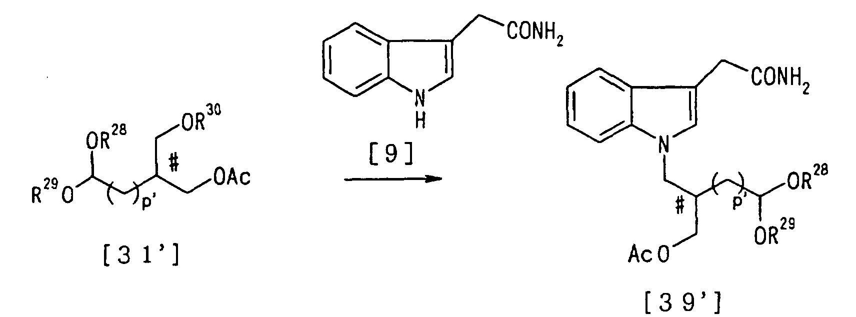 wherein each symbol is as defined above.

Compound [31' ] is reacted with compound [9] instead of compound [38] in the same manner as in Production method 4-2-3, Step 1, to give compound [39'].

### Production method 4-3

This production method is a different method to obtain indole-condensed ring having optical activity. 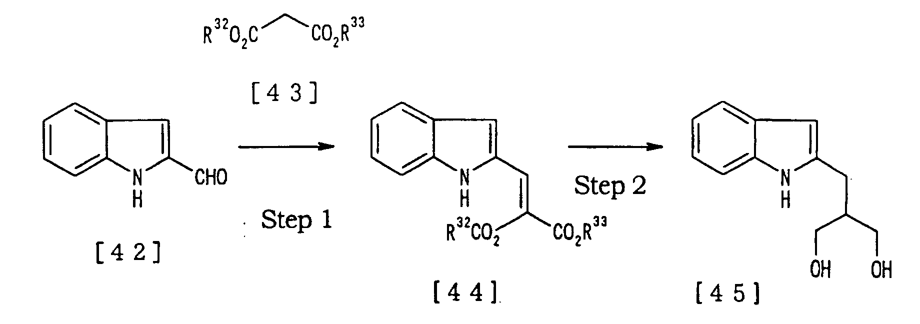 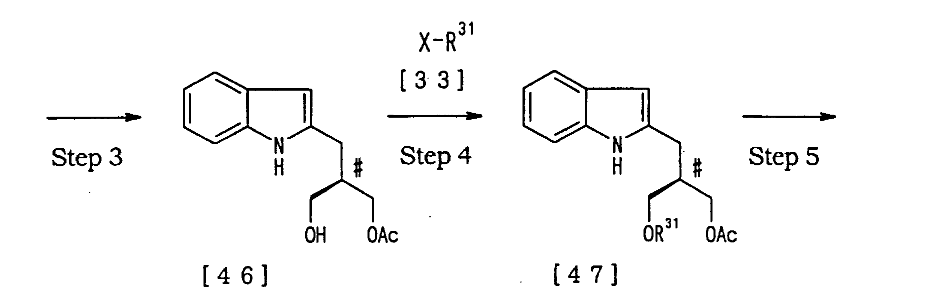 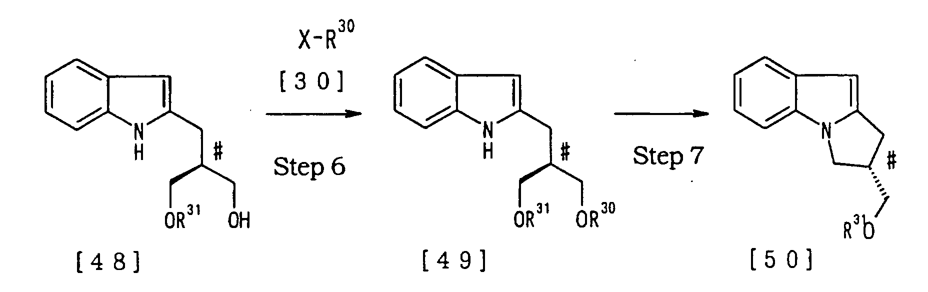 wherein R³² and R³³ are hydrogen atom, or alkyl such as methyl, ethyl and the like, and R³⁰ and R³¹ are as defined above.

### Step 1

Compound [42] is reacted with compound [43] in a solvent in the presence of a base, an acid or both of them under an argon atmosphere to give compound [44].

As the base, pyridine, piperidine, potassium carbonate, sodium carbonate, sodium hydroxide, potassium hydroxide, lithium hydroxide and the like are exemplified and as the acid, acetic acid, hydrochloric acid, nitric acid, sulfuric acid and the like are exemplified.

As the solvent, alcohol solvent such as methanol, ethanol, n-propanol, isopropanol and the like; hydrocarbon solvent such as benzene, toluene, hexane, xylene and the like; halogen solvent such as dichloromethane, chloroform, carbon tetrachloride, 1,2-dichloroethane and the like; ether solvent such as 1,4-dioxane, diethyl ether, 1,2-dimethoxyethane, tetrahydrofuran and the like; polar solvent such as dimethylformamide, dimethyl sulfoxide, acetonitrile and the like; or a mixed solvent thereof can be used.

### Step 2

Compound [44] is reduced by a conventional method to give compound [45].

For example, compound [44] is treated with a reducing agent such as lithium aluminum hydride, sodium borohydride, lithium borohydride and the like or a combination thereof under cooling in alcohol such as ethanol and the like.

### Step 3

Compound [45] is reacted with LipasePS enzyme in a solvent in the presence of vinyl acetate to give compound [46] which is an optically active compound (R compound).

As the solvent, hydrocarbon solvent such as benzene, toluene, hexane, xylene and the like; halogen solvent such as dichloromethane, chloroform, carbon tetrachloride, 1,2-dichloroethane and the like; ether solvent such as 1,4-dioxane, diethyl ether, 1,2-dimethoxyethane, tetrahydrofuran and the like; polar solvent such as dimethylformamide, dimethyl sulfoxide, acetonitrile and the like; vinyl acetate; or a mixed solvent thereof can be used.

### Step 4

Compound [46] is reacted with compound [33] in the same manner as in Production method 4-2-1, Step 3, to give compound [47].

### Step 5

Compound [47] is deacetylated by a conventional method to give compound [48].

### Step 6

Compound [48] is reacted with compound [30] in the same manner as in Production method 4-2-1, Step 1, to give compound [49].

### Step 7

Compound [49] is reacted in the same manner as in Production method 4-2-3, Step 1, to give compound [50].

As the solvent, hydrocarbon solvent such as benzene, toluene, hexane, xylene and the like; halogen solvent such as dichloromethane, chloroform, carbon tetrachloride, 1,2-dichloroethane and the like; ether solvent such as 1,4-dioxane, diethyl ether, 1,2-dimethoxyethane, tetrahydrofuran and the like; polar solvent such as dimethylformamide, dimethyl sulfoxide, acetonitrile and the like; or a mixed solvent thereof can be used.

Alternatively, compound [45] is obtained by similar reaction using malononitrile instead of compound [43] in Step 1, followed by reduction.

In this production method, an enantiomer can be produced by in a similar manner by utilizing the replacement of protecting groups conducted in Production method 4-2-2.

### Production method 4-4

This production method relates to forming a maleimido group on the indole condensed ring and introduction of a substituent. 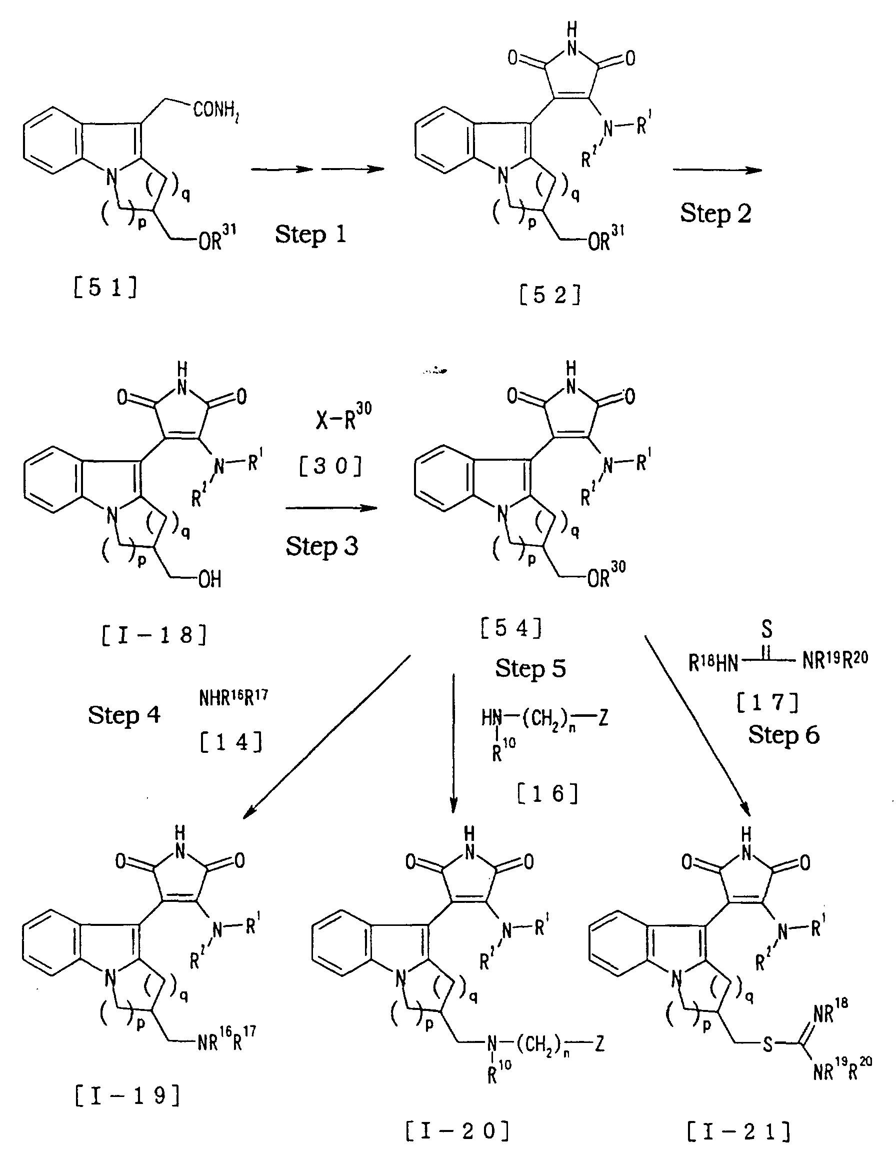 wherein each symbol is as defined above.

### Step 1

The compound [51] obtained in the same manner as in compound [27] obtained in Production method 4-1-2, compounds [40] and [41] obtained in Production method 4-2-3 or compound [50] obtained in Production method 4-3 is reacted in the same manner as in Production method 1-1 to give compound [52].

### Step 2

Compound [52] is deprotected in the same manner as in Production method 4-1-2, Step 5, to give compound [I-18].

### Step 3

Compound [I-18] is reacted with compound [30] in the same manner as in Production method 4-2-1, Step 1, to give compound [54].

### Step 4

Compound [54] is reacted with compound [14] in the same manner as in Production method 2-2, Step 3, to give compound [I-19].

### Step 5

Compound [54] is reacted with compound [16] in the same manner as in Production method 2-3, Step 3, to give compound [I-20].

### Step 6

Compound [54] is reacted with compound [17] in the same manner as in Production method 2-3, Step 4, to give compound [I-21].

### Production method 4-5

In this production method, a substituent is introduced into hydroxyl group and maleimido group is formed on the indole ring. 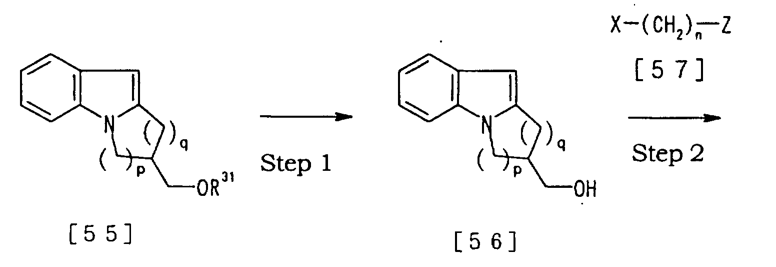 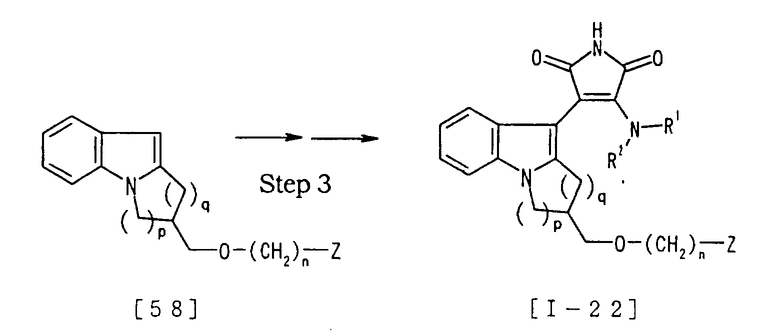 wherein each symbol is as defined above.

### Step 1

The compound [55] obtained in the same manner as in compound [25] obtained in Production method 4-1-1 or compound [50] obtained in Production method 4-3 is reacted in the same manner as in Production method 4-1-2, Step 5, to give compound [56].

### Step 2

Compound[56] is reacted with compound [57] in the presence of a base such as sodium hydride, lithium hydride and the like to give compound [58].

### Step 3

Compound [58] is treated in the same manner as in Production method 4-1-2, Step 1 to Step 2, and Production method 1-1, Step 1 to Step 2, to give compound [I-22].

### Production method 5-1

This production method relates to the synthesis of 1,7-cyclized indole compound. 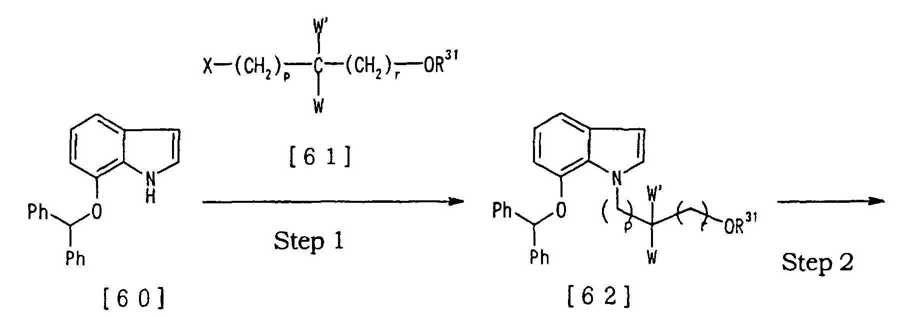 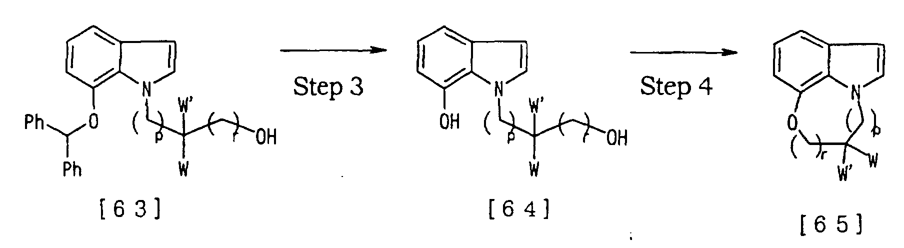 wherein each symbol is as defined above.

### Step 1

Compound [60] is reacted with compound [61] in the same manner as in Production method 2-1, Step 1 to give compound [62].

### Step 2

Compound [62] is reacted in the same manner as in Production method 4-1-2, Step 5, to give compound [63].

### Step 3

Diphenylmethyl of compound [63] is removed by a conventional method to give compound [64].

### Step 4

Compound [64] is treated with dialkyl azodicarboxylate in the presence of triphenylphosphine and the like in a solvent to give compound [65].

As the solvent, alcohol solvent such as methanol, ethanol, n-propanol isopropanol and the like; hydrocarbon solvent such as benzene, toluene, hexane, xylene and the like; halogen solvent such as dichloromethane, chloroform, carbon tetrachloride, 1,2-dichloroethane and the like; ether solvent such as 1,4-dioxane, diethyl ether, 1,2-dimethoxyethane, tetrahydrofuran and the like; polar solvent such as dimethylformamide, dimethyl sulfoxide, acetonitrile and the like; or a mixed solvent thereof can be used. 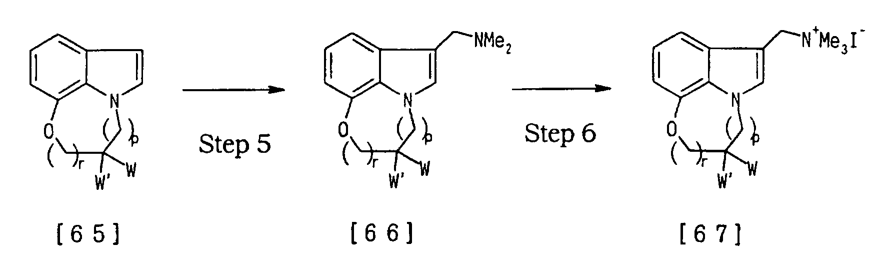 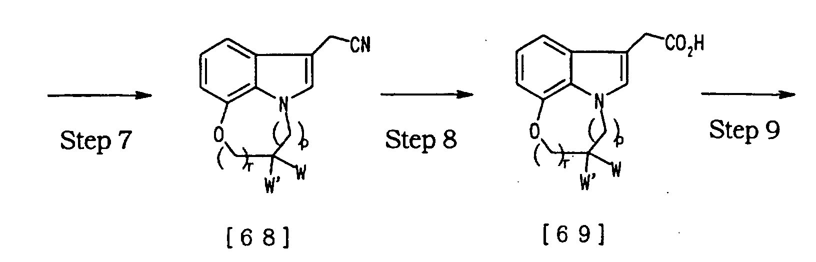 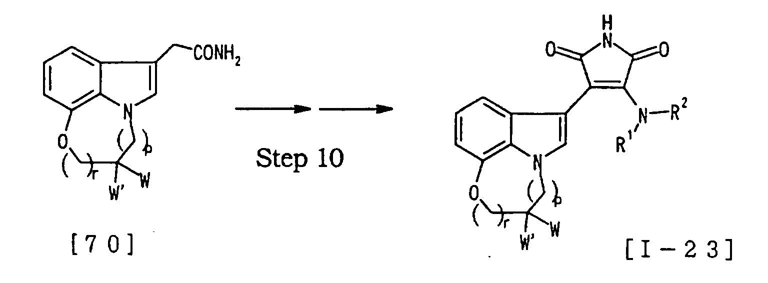 wherein each symbol is as defined above, and Me is methyl.

### Step 5

Dimethylamine and formalin were added to compound [65] in an acidic alcohol mixed solvent (e.g., a mixture of acetic acid and ethanol and the like) under cooling and reacted at room temperature to give compound [66].

### Step 6

Compound [66] in acetic acid and an alkylation agent such as methyl iodide and the like are reacted to give compound [67].

### Step 7

Compound [67] is reacted with a cyanation agent such as potassium cyanide and the like in a solvent under heating to give compound [68].

As the solvent, polar solvent such as dimethylformamide, dimethyl sulfoxide, acetonitrile and the like, and the like are exemplified.

### Step 8

Compound [68] is hydrolyzed by a conventional method in a solvent to give compound [69].

As the solvent, alcohol solvent such as methanol, ethanol, n-propanol, isopropanol and the like, and the like are exemplified.

### Step 9

Compound [69] is treated with a halogenation agent such as thionyl chloride, oxalyl chloride and the like in a solvent, and an amine source such as aqueous ammonia and the like is added to give compound [70].

### Step 10

Compound [70] is reacted in the same manner as in Production method 1-1 to give compound [I-23].

The compound of the formula [I] of the present invention and the production method thereof are specifically explained by referring to Examples, to which the present invention is not limited.

### Example 1-1

### 3-(1H-indol-3-yl)-4-[(3-methoxyphenyl)amino]-1H-pyrrole-2,5-dione

### Step 1

To a solution of indole-3-acetamide (10.0 g, 57.4 mmol) and dimethyl oxalate (7.46 g, 63.1 mmol) in dimethylformamide (DMF, 100 mL) was added potassium tert-butoxide (20 g, 178 mmol) under an argon atmosphere at 0°C and the mixture was stirred at room temperature for 1 hour. The reaction mixture was poured into a 10% aqueous citric acid solution and the mixture was extracted three times with ethyl acetate. The organic layer was washed with saturated brine and the solvent was evaporated under reduced pressure to give 3-hydroxy-4-(1H-indol-3-yl)-1H-pyrrole-2,5-dione (39.0 g, containing DMF by 55%) as a crude product.

The obtained crude product was used for the next reaction without purification.

### Step 2

3-Hydroxy-4-(1H-indol-3-yl)-1H-pyrrole-2,5-dione (350 mg, 0.69 mmol, containing DMF by 55%) obtained in the same manner as in Example 1-1, Step 1, and m-anisidine (258 mg, 2.09 mmol) were stirred under heating in acetic acid (2 mL) at 100°C. Three hours later, the reaction mixture was cooled to room temperature and the solvent was evaporated. The obtained residue was purified by silica gel column chromatography (developing solvent:hexane/ethyl acetate=4/1→3/1) to give the title compound as yellow crystals (105 mg, 46% yield). The property values are shown in Table 1.

In the same manner as in Example 1-1, the compounds of Examples 1-2 to 1-18 were obtained. The property values are shown in Tables 1 to 6.

### Example 2-1

### 3-[1-(3-hydroxypropyl)-1H-indol-3-yl]-4-(phenylamino)-1H-pyrrole-2,5-dione

### Step 1

To a solution of indole-3-acetamide (2.0 g, 11.5 mmol) in DMF (20 mL) was added sodium hydride (964 mg, 24.1 mmol) at 0°C under an argon atmosphere and the mixture was stirred. After 15 minutes, 1-bromo-3-(tert-butyldimethylsilyloxy)propane (3.05 g, 12.1 mmol) was added and the mixture was stirred at room temperature for 2 hours. The reaction mixture was poured into a 10% aqueous citric acid solution, and the mixture was extracted with ethyl acetate. The organic layer was washed with saturated brine and the solvent was evaporated under reduced pressure. The obtained residue was purified by silica gel column chromatography (developing solvent:hexane/ethyl acetate=2/1) to give 1-[3-(tert-butyldimethylsilyloxy)propyl]-1H-indol-3-ylacetamide as a colorless wax (3.39 g, 85% yield).

### Step 2

To a solution of 1-[3- (tert-butyldimethylsilyloxy)propyl]-1H-indol-3-ylacetamide (3.39 g, 9.78 mmol) obtained in Example 2-1, Step 1, and dimethyl oxalate (1.27 g, 10.8 mmol) in tetrahydrofuran (THF, 30 mL) was added potassium tert-butoxide (2.31 g, 20.5 mmol) at 0°C under an argon atmosphere. The mixture was stirred at room temperature for 1 hour. The reaction mixture was poured into a 10% aqueous citric acid solution and the mixture was extracted with ethyl acetate. The organic layer was washed with saturated brine and the solvent was evaporated under reduced pressure. The obtained residue was purified by silica gel column chromatography (developing solvent: hexane/ethyl acetate=3/1) to give 3-[1-{3-(tert-butyldimethylsilyloxy)propyl}-1H-indol-3-yl]-4-hydrooy-1H-pyrrole-2,5-dione as orange crystals (2.84 g, 72% yield).

### Step 3

A solution of 3-[1-{3-(tert-butyldimethylsilyloxy)propyl}-1H-indol-3-yl]-4-hydroxy-1H-pyrrole-2,5-dione (2.82 g, 7.04 mmol) obtained in Example 2-1, Step 2, and aniline (2.62 g, 28.2 mmol) in acetic acid (10 mL) was stirred at 100°C for 1 hour. The reaction mixture was concentrated under reduced pressure, and to the residue were successively added THF (10 mL) and 1M tetrabutylammonium fluoride/THF solution (7.74 mL, 7.74 mmol). The mixture was stirred overnight at room temperature. The reaction mixture was diluted with ethyl acetate and washed with saturated brine. The solvent was evaporated under reduced pressure, and the obtained residue was purified by silica gel column chromatography (developing solvent:hexane/ethyl acetate=1/1) to give the title compound as an orange amorphous (2.35 g, 92% yield). The property values are shown in Table 7.

In the same manner as in Example 2-1, the compounds of Examples 2-2 to 2-7 and Examples 2-44 to 2-46 were obtained. The property values are shown in Tables 7 to 9, 21 and 22.

### Example 2-8

### 3-[1-(3-dimethylaminopropyl)-1H-indol-3-yl]-4-(phenylamino)-1H-pyrrole-2,5-dione

### Step 1

To a solution of 3-[1-(3-hydroxypropyl)-1H-indol-3-yl]-4-(phenylamino)-1H-pyrrole-2,5-dione (1.84 g, 5.09 mmol) obtained in Example 2-1 in THF (30 mL) were successively added triphenylphosphine (2.67 g, 10.2 mmol) and N-bromosuccinimide (1.81 g, 10.2 mmol) under an argon atmosphere at 0°C and the mixture was stirred at 0°C for 20 minutes. To the reaction mixture was added a saturated aqueous sodium hydrogencarbonate solution and the mixture was extracted with chloroform. The organic layer was concentrated under reduced pressure. The obtained residue was purified by silica gel column chromatography (developing solvent:hexane/ethyl acetate=2/1) to give 3-[1-(3-bromopropyl)-1H-indol-3-yl]-4-(phenylamino)-1H-pyrrole-2,5-dione as an orange amorphous (1.35 g, 62% yield).

### Step 2

To a solution of 3-[1-(3-bromopropyl)-1H-indol-3-yl]-4-(phenylamino)-1H-pyrrole-2,5-dione (53 mg, 0.12 mmol) obtained in Example 2-8, Step 1, in THF (1 mL) was added a 2M dimethylamine/THF solution (1 mL, 2.0 mmol) and the mixture was stirred in a sealed tube at 60°C for 10 hours. The reaction mixture was concentrated under reduced pressure and the obtained residue was purified by silica gel thin layer chromatography (developing solvent: chloroform/methanol=4/1) to give the title compound as an orange amorphous (45mg, 93% yield). The property values are shown in Table 9.

In the same manner as in Example 2-8, the compounds of Examples 2-9 to 2-35 and Examples 2-47 to 2-57 were obtained. The property values are shown in Tables 9 to 18 and 22 to 25.

### Example 2-36

### 3-[1-(3-imidazol-1-ylpropyl)-1H-indol-3-yl]-4-(phenylamino)-1H-pyrrole-2,5-dione

To a solution of imidazole (40 mg, 0.59 mmol) in DMF (1 mL) was added 60% sodium hydride (24 mg, 0.59 mmol) under an argon atmosphere at 0°C and the mixture was stirred. After 10 minutes, 3-[1-(3-bromopropyl)-1H-indol-3-yl]-4-(phenylamino)-1H-pyrrole-2,5-dione (50 mg, 0.12 mmol) obtained in Example 2-8, Step 1, was added and the mixture was stirred at room temperature for 30 minutes. The reaction mixture was poured into a 10% aqueous citric acid solution and the mixture was extracted with ethyl acetate. The organic layer was washed with saturated brine and the solvent was evaporated under reduced pressure. The obtained residue was purified by silica gel thin layer chromatography (developing solvent:hexane/ethyl acetate=1/1) to give the title compound as an orange amorphous (37 mg, 76% yield). The property values are shown in Table 18.

In the same manner as in Example 2-36, the compounds of Examples 2-37 to 2-42 and Examples 2-58 to 2-73 were obtained. The property values are shown in Tables 19 to 20 and 26 to 31.

### Example 2-43

### 3-(1-(3-amidinothiopropyl)-1H-indol-3-yl]-4-(phenylamino)-1H-pyrrole-2,5-dione hydrobromide

3-[1-(3-Bromopropyl)-1H-indol-3-yl]-4-(phenylamino)-1H-pyrrole-2,5-dione (90 mg, 0.21 mmol) obtained in the same manner as in Example 2-8, Step 1, was dissolved in ethanol (1.0 ml), and thiourea (14 mg, 0.18 mmol) was added at room temperature, which was followed by reflux under heating for 11 hours. The reaction mixture was concentrated to dryness and the residue was purified by silica gel chromatography (developing solvent:chloroform/methanol=9/1) to give the title compound as an orange amorphous (91 mg, 85% yield). The property values are shown in Table 21.

### Example 3-1

### 3-(phenylamino)-4-[1-(3-piperidylmethyl)-1H-indol-3-yl]-1H-pyrrole-2,5-dione

### Step 1

In the same manner as in Example 2-1, Step 1, 1-[(1-tert-butoxycarbonylpiperidin-3-yl)methyl]-1H-indol-3-ylacetamide was obtained as a yellow amorphous (959 mg, 45% yield) from indole-3-acetamide (1.0 g, 5.74 mmol) and 1-tert-butoxycarbonyl-3-tosyloxymethylpiperidine (2.33 g, 6.31 mmol).

### Step 2

In the same manner as in Example 1-1, Step 1, 3-[1-{(1-tert-butoxycarbonylpiperidin-3-yl)methyl}-1H-indol-3-yl]-4-hydroxy-1H-pyrrole-2,5-dione was obtained as an orange amorphous (316 mg, 32% yield) from 1-[(1-tert-butoxycarbonylpiperidin-3-yl)methyl]-1H-indol-3-ylacetamide (860 mg, 2.32 mmol) obtained in Example 3-1, Step 1. At the same time, 1-[(1-tert-butoxycarbonylpiperidin-3-yl)methyl]-1H-indol-3-ylacetamide (545 mg, 63% yield) was recovered.

### Step 3

From 3-[1-{(1-tert-butoxycarbonylpiperidin-3-yl)methyl}-1H-indol-3-yl]-4-hydroxy-1H-pyrrole-2,5-dione (220 mg, 0.52 mmol) obtained in Example 3-1, Step 2, 3-[1-{(1-tert-butoxycarbonylpiperidin-3-yl)methyl}-1H-indol-3-yl]-4-(phenylamino)-1H-pyrrole-2,5-dione was obtained as a red amorphous (202 mg, 78% yield) in the same manner as in Example 1-1, Step 2.

### Step 4

To 3-[1-{(1-tert-butoxycarbonylpiperidin-3-yl)methyl}-1H-indol-3-yl]-4-(phenylamino)-1H-pyrrole-2,5-dione (195 mg, 0.39 mmol) obtained in Example 3-1, Step 3, was added 4N hydrochloric acid/dioxane (4.0 ml) at room temperature and the mixture was stirred for 15 minutes. To the reaction mixture was added diethyl ether, and the precipitated solid was collected by filtration. The crude product thereof was purified by thin layer chromatography (developing solvent: chloroform/methanol/aqueous ammonia=9/ 1/0.1) to give the title compound as orange crystals (54 mg, 35% yield). The property values are shown in Table 31.

In the same manner as in Example 3-1, the compound of Example 3-2 was obtained. The property values are shown in Table 31.

### Example 3-3

### 3-[1-{(1-methylpiperidin-3-yl)methyl}-1H-indol-3-yl]-4-(phenylamino)-1H-pyrrole-2,5-dione

3-(Phenylamino)-4-[1-(3-piperidylmethyl)-1H-indol-3-yl]-1H-pyrrole-2,5-dione (46 mg, 0.115 mmol) obtained in Example 3-1 was dissolved in ethanol (0.5 mL), and potassium carbonate (27 mg, 0.196 mmol) and methyl methanesulfonate (14.7 µL, 0.173 mmol) were added. The mixture was stirred at room temperature for 3 hours. To the reaction mixture was added a saturated aqueous sodium hydrogencarbonate solution and the mixture was extracted with ethyl acetate. The organic layer was washed with saturated brine and dried over sodium sulfate. Sodium sulfate was filtered off and the filtrate was concentrated to dryness. The obtained residue was purified by thin layer chromatography (developing solvent:chloroform/methanol/aqueous ammonia=9/1/0.1) to give the title compound as orange crystals (11 mg, 24% yield). The property values are shown in Table 32.

In the same manner as in Example 3-3, the compounds of Examples 3-4 to 3-6 were obtained. The property values are shown in Tables 32 and 33.

### Example 3-7

### 3-[1-{(1-amidinopiperidin-4-yl)methyl}-1H-indol-3-yl]-4-(phenylamino)-1H-pyrrole-2,5-dione hydrochloride

### Step 1

3-(Phenylamino)-4-[1-(3-piperidylmethyl)-1H-indol-3-yl]-1H-pyrrole-2,5-dione hydrochloride (100 mg, 0.23 mmol) obtained in the same manner as in Example 3-2 was dissolved in DMF (1.0 ml), and triethylamine (38 µl, 0.28 mmol) and di-tert-butoxycarbonyl-1H-pyrazole-1-carboxamidine (110 mg, 0.35 mmol) were successively added at room temperature. The mixture was stirred for 18 hours. To the reaction mixture was added saturated brine and the mixture was extracted with ethyl acetate. The organic layer was dried over sodium sulfate. Sodium sulfate was filtered off and the filtrate was concentrated to dryness, and the residue was purified by thin layer silica gel chromatography (developing solvent:chloroform/methanol=95/5) to give 3-[1-{[1-(1,2-di-tert-butoxycarbonylamidino)piperidin-4-yl]methyl}-1H-indol-3-yl]-4-(phenylamino)-1H-pyrrole-2,5-dione as a yellow oil (150 mg, 100% yield).

### Step 2

3-[1-{[1-(1,2-Di-tert-butoxycarbonylamidino)piperidin-4-yl]methyl}-1H-indol-3-yl]-4-(phenylamino)-1H-pyrrole-2,5-dione (145 mg, 0.22 mmol) obtained in Example 3-7, Step 1, was dissolved in methanol (1.0 ml). 4N Hydrochloric acid/dioxane (1.0 ml) was added at room temperature and the mixture was stirred for 24 hours. The reaction mixture was concentrated to dryness and the residue was dissolved in methanol (0.5 ml). This solution was gradually added to diethyl ether (50 ml) at room temperature, and the mixture was stirred at room temperature for 3 hours. The obtained crystals were collected by filtration and washed with diethyl ether to give the title compound as orange crystals (70 mg, 65% yield). The property values are shown in Table 33.

### Example 4-1

### 3-[8-Hydroxymethyl-6,7,8,9-tetrahydropyrido[ [1,2-a]indol-10-yl]-4-(phenylamino)-1H-pyrrole-2,5-dione

### Step 1

Ethyl 6,7,8,9-tetrahydropyrido[1,2-a]indol-8-ylcarboxylate (8.1 g, 33.3 mmol) synthesized by the method described in *Tetrahedron*, 47, 4645, 1991 was dissolved in THF (100 mL). This solution was added to a suspension of lithium aluminum hydride (1.0 g, 26.6 mmol) in THF (300 mL) at 0°C, and the mixture was stirred for 1 hour. To the reaction mixture were successively added ethyl acetate, water and 1N hydrochloric acid, and the mixture was extracted with diethyl ether. The organic layer was washed with saturated brine and the solvent was evaporated under reduced pressure. The obtained residue was concentrated to dryness to give a crude product.

The crude product was subsequently dissolved in DMF (80 mL), and imidazole (5.44 g, 79.9 mmol) and tert-butyldiphenylsilyl chloride (10.98 g, 40.0 mmol) were successively added at room temperature, and the mixture was stirred for 4 hours. To the reaction mixture was added a 0.5N aqueous potassium hydrogensulfate solution and the mixture was extracted with ethyl acetate. The organic layer was washed with water and dried over sodium sulfate. Sodium sulfate was filtered off, and the filtrate was concentrated to dryness. The residue was purified by silica gel column chromatography (developing solvent:hexane/ethyl acetate=20/1) to give 8-tert-butyldiphenylsilyloxymethyl-6,7,8,9-tetrahydropyrido[1,2-a]indole as an oil (10.5 g, 72% yield).

### Step 2

8-tert-Butyldiphenylsilyloxymethyl-6,7,8,9-tetrahydropyrido[1,2-a]indole (10.0 g, 22.7 mmol) obtained in Example 4-1, Step 1 was dissolved in methylene chloride (60 mL), and triethylamine (3.79 mL, 27.2 mmol) was added. Thereto was further added oxalyl chloride (2.18 mL, 25.0 mmol) under an argon atmosphere at 0°C and the mixture was stirred. After 30 minutes, the reaction mixture was added to 28% aqueous ammonia at 0°C and the mixture was stirred for 20 minutes. To the reaction mixture was added water and the mixture was extracted with ethyl acetate. The organic layer was washed with saturated brine and dried over sodium sulfate. Sodium sulfate was filtered off and the filtrate was concentrated to dryness. The obtained residue was purified by silica gel column chromatography (developing solvent:hexane/ethyl acetate=10/1→6/1→2/1) to give 8-tert-butyldiphenylsilyloxymethyl-10-oxamoyl-6,7,8,9-tetrahydropyrido[1,2-a)indole as a white solid (10.15 g, 88% yield).

### Step 3

8-tert-Butyldiphenylsilyloxymethyl-10-oxamoyl-6,7,8,9-tetrahydropyrido[1,2-a]indole (3.10 g, 6.07 mmol) obtained in Example 4-1, Step 2, was dissolved in ethanol (80 mL). Sodium borohydride (1.15 g, 30.4 mmol) was added under an argon atmosphere at room temperature and the mixture was stirred for 1 hour. To the reaction mixture was added a 2N aqueous solution of potassium hydrogensulfate at 0°C and the mixture was extracted with ethyl acetate. The organic layer was washed with saturated brine and dried over sodium sulfate. Sodium sulfate was filtered off and the filtrate was concentrated to dryness to give a crude product.

Subsequently, the crude product was dissolved in methylene chloride (40 mL) and triethylsilane (1.94 mL, 12.1 mmol) and trifluoroacetic acid (4 mL) were added at room temperature. The mixture was stirred for 3.5 hours. To the reaction mixture was added a saturated aqueous sodium hydrogencarbonate solution at 0°C and the mixture was extracted with chloroform. The organic layer was washed successively with a saturated aqueous sodium hydrogencarbonate solution and saturated brine, and dried over sodium sulfate. Sodium sulfate was filtered off and the filtrate was concentrated to dryness. The obtained residue was purified by silica gel column chromatography (developing solvent:hexane/ethyl acetate=5/1→2/1) to give [8-tert-butyldiphenylsilyloxymethyl-6,7,8,9-tetrahydropyrido[1,2-a]indol-10-yl]acetamide as a brown white oil (2.15 g, 71% yield).

### Step 4

[8-tert-Butyldiphenylsilyloxymethyl-6,7,8,9-tetrahydropyrido[1,2-a]indol-10-yl]acetamide (1.63 g, 3.28 mmol) obtained in Example 4-1, Step 3, and dimethyl oxalate (426 mg, 3.61 mmol) were dissolved in DMF (20 mL), and potassium tert-butoxide (405 mg, 3.61 mmol) was added under an argon atmosphere at 0°C. The mixture was stirred for 30 minutes and potassium tert-butoxide (405 mg, 3.61 mmol) was added. After 30 minutes, an aqueous solution of 2N potassium hydrogensulfate was added to the reaction mixture at 0°C, and the mixture was extracted with ethyl acetate. The organic layer was washed with saturated brine and dried over sodium sulfate. Sodium sulfate was filtered off and the filtrate was concentrated to dryness. The residue was purified by silica gel column chromatography (developing solvent:hexane/ethyl acetate=5/1→2/1) to give 3-[8-(tert-butyldiphenylsilyloxymethyl)-6,7,8,9-tetrahydropyrido[1,2-a]indol-10-yl]-4-hydroxy-1H-pyrrole-2,5-dione as an oil (180 mg, 10% yield).

### Step 5

In the same manner as in Example 2-1, Step 3, the title compound (70 mg, 55% yield) was obtained from 3-[8-(tert-butyldiphenylsilyloxymethyl)-6,7,8,9-tetrahydropyrido[1,2-a]indol-10-yl]-4-hydroxy-1H-pyrrole-2,5-dione (180 mg, 0.327 mmol) obtained in Example 4-1, Step 4. The property values are shown in Table 33.

In the same manner as in Example 4-1, the compounds of Examples 4-4 to 4-10 were obtained. The property values are shown in Tables 34 to 36.

### Example 4-2

### 3-[8-(dimethylaminomethyl)-6,7,8,9-tetrahydropyrido[1,2-a]indol-10-yl]-4-(phenylamino)-1H-pyrrole-2,5-dione

To a solution of trifluoromethanesulfonic anhydride (39 µL, 232 µmol) in methylene chloride (4 mL) was dropwise added at 0°C under an argon atmosphere a mixture of 3-[8-hydroxymethyl-6,7,8,9-tetrahydropyrido[1,2-a]indol-10-yl]-4-(phenylamino)-1H-pyrrole-2,5-dione (30 mg, 77.4 mmol) obtained in Example 4-1 and 2,4,6-collidine (31 µL, 232 µmol) dissolved in methylene chloride (4 mL), and the mixture was stirred for 40 minutes. To the reaction mixture was added a solution of 2M dimethylamine (1.55 mL, 3.10 mmol) in THF at 0°C, and the mixture was stirred for 2.5 hours. To the reaction mixture was added an aqueous solution of sodium hydrogencarbonate and the mixture was extracted with ethyl acetate. The organic layer was washed with saturated brine and dried over sodium sulfate. Sodium sulfate was filtered off and the filtrate was concentrated to dryness. The residue was purified by thin layer chromatography (developing solvent:chloroform/methanol=9/1) to give the title compound as an orange amorphous (16 mg, 50% yield). The property values are shown in Table 34.

In the same manner as in Example 4-2, the compounds of Examples 4-11 to 4-35 were obtained. The property values are shown in Tables 37 to 45.

### Example 4-3

### 3-[8-(1-imidazolylmethyl)-6,7,8,9-tetrahydropyrido[1,2-a]indol-10-yl]-4-(phenylamino)-1H-pyrrole-2,5-dione

To a solution of trifluoromethanesulfonic anhydride (51 µL, 303 µmol) in methylene chloride (4 mL) was dropwise added at 0°C under an argon atmosphere a mixture of 3-[8-hydroxymethyl-6,7,8,9-tetrahydropyrido[1,2-a]indol-10-yl]-4-(phenylamino)-1H-pyrrole-2,5-dione (39 mg, 101 µmol) obtained in Example 4-1 and 2,4,6-collidine (40 µL, 303 µmol) dissolved in methylene chloride (4 mL) and the mixture was stirred for 40 minutes. To the reaction mixture was added a mixture of imidazole (69 mg, 1.01 mmol) and 60% sodium hydride (40 mg, 1.01 mmol) stirred in DMF (1 mL) for 1.5 hours, using methylene chloride (4 mL) at 0°C. After 30 minutes, an aqueous solution of sodium hydrogencarbonate was added to the reaction mixture, and the mixture was extracted with ethyl acetate. The organic layer was washed with saturated brine and dried over sodium sulfate. Sodium sulfate was filtered off and the filtrate was concentrated to dryness. The residue was purified by thin layer chromatography (developing solvent:chloroform/methanol=9/1) to give the title compound as an orange amorphous (5 mg, 11% yield). The property values are shown in Table 34.

In the same manner as in Example 4-3, the compounds of Examples 4-36 to 4-39 were obtained. The property values are shown in Tables 45 and 46.

### Example 4-40

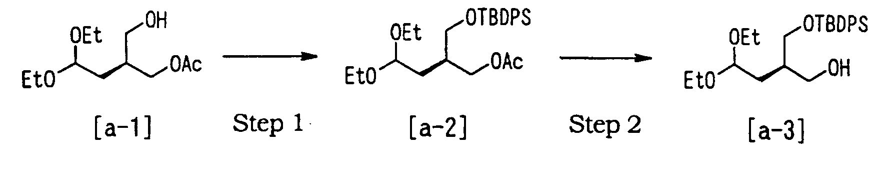

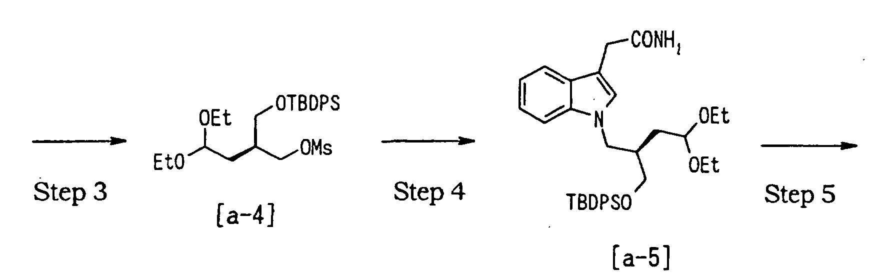

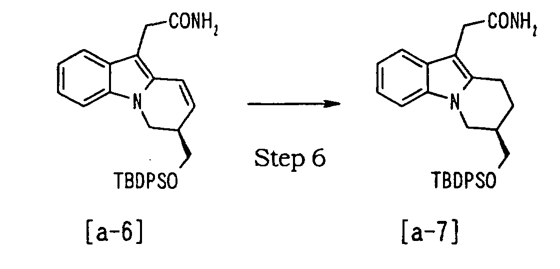

wherein Et means ethyl, Ac means acetyl, TBDPS means tert-butyldiphenylsilyl and Ms means mesyl, hereinafter the same)

### Step 1

To a solution of the compound [a-1] (1.00 g, 4.27 mmol) obtained according to the method described in *Chem*. *Pharm. Bull.,* 39(3), 823-825, 1991 and imidazole (581 mg, 8.54 mmol) in DMF (5 mL) was added tert-butyldiphenylchlorosilane (1.22 mL,4.70 mmol) at 0°C and the mixture was stirred overnight at room temperature. The reaction mixture was poured into a saturated aqueous sodium hydrogencarbonate solution and the mixture was extracted with ethyl acetate. The organic layer was washed with saturated brine and concentrated. The obtained residue was purified by silica gel column chromatography (developing solvent;hexane/ethyl acetate=9/1) to give compound [a-2] (2.00 g, 99% yield) as a colorless oil.

### Step 2

To a solution of compound [a-2] (2.00 g, 4.23 mmol) obtained in Example 4-40, Step 1, in ethanol (20 mL) was added potassium carbonate (643 mg, 4.65 mmol) and the mixture was stirred overnight at room temperature. The reaction mixture was poured into saturated brine and the mixture was extracted with ethyl acetate. The organic layer was washed with saturated brine and concentrated. The obtained residue was purified by silica gel column chromatography (developing solvent;hexane/ethyl acetate=4/1) to give compound[a-3] (1.79 g, 98% yield) as a colorless oil.

### Step 3

To a solution of compound[a-3] (1.78 g, 4.13 mmol) obtained in Example 4-40, Step 2, in anhydrous THF (20 mL) were successively added triethylamine (1.15 mL, 8.26 mmol) and mesyl chloride (352 µl, 4.54 mmol) under an argon atmosphere at 0°C, and the mixture was stirred at 0°C for 2 hours. To the reaction mixture was added a saturated aqueous sodium hydrogencarbonate solution, and the mixture was extracted with ethyl acetate. The organic layer was washed with saturated brine, dried over anhydrous magnesium sulfate and concentrated. The obtained crude product [a-4] (crude 2.14 g) was used in the next step without purification.

### Step 4

To a solution of indole-3-acetamide (1.44 g, 8.26 mmol) in anhydrous DMF (10 mL) was added 60% sodium hydride (330 mg, 8.26 mmol) under an argon atmosphere at 0°C and the mixture was stirred at 0°C for 15 minutes. Then, a solution of compound [a-4] (2.14 g, corresponding to 4.13 mmol) obtained in Example 4-40, Step 3, in anhydrous THF (5 mL) and sodium iodide (62 mg, 0.41 mmol) were successively added and the mixture was stirred at 50°C for 4 hours. The reaction mixture was cooled to room temperature and poured into saturated aqueous ammonium chloride solution. The mixture was extracted with ethyl acetate. The organic layer was washed with saturated brine and concentrated. The obtained residue was purified by silica gel column chromatography (developing solvent;hexane/ethyl acetate=1/2) to give compound [a-5] (1.48 g, 60% yield) as a colorless oil.

### Step 5

To a solution of compound [a-5] (1.44 g, 2.40 mmol) obtained in Example 4-40, Step 4, in chloroform (100 mL) was added an aqueous solution of 50% trifluoroacetic acid (7.5 mL), and the mixture was stirred at room temperature for 30 minutes. The reaction mixture was poured into a saturated aqueous sodium hydrogencarbonate solution, and after partitioning, the organic layer was dried over anhydrous magnesium sulfate and concentrated. The obtained residue [a-6] (crude 1.20 g) was used in the next step without purification.

### Step 6

To a solution of compound [a-6] (1.20 g, corresponding to 2.40 mmol) obtained in Example 4-40, Step 5, in ethanol (50 mL) was added 5% palladium carbon (145 mg) and mixture was stirred under hydrogen atmosphere (3 atm) at room temperature for 2 hours. The catalyst was filtered off and the filtrate was concentrated. The obtained residue was purified by silica gel column chromatography (developing solvent;hexane/ethyl acetate=1/2) to give compound [a-7] (1.13 g, 93% yield) as a colorless oil.

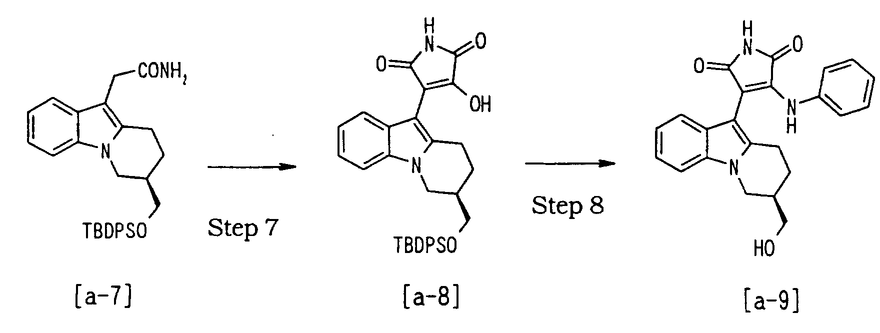

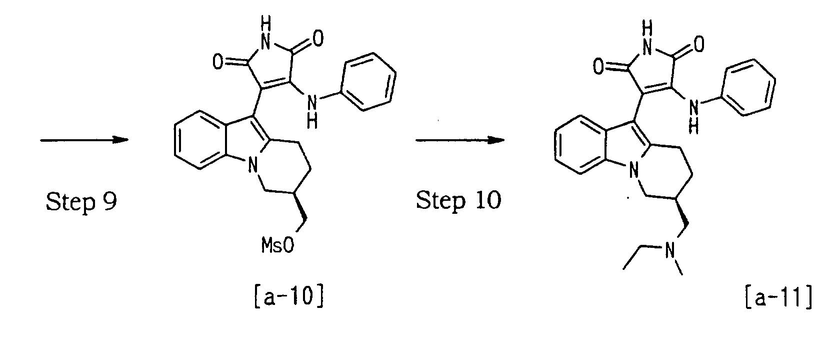

### Step 7

To a solution of compound [a-7] (1.13 g, 2.28 mmol) obtained in Example 4-40, Step 6, and dimethyl oxalate (296 mg, 2.51 mmol) in anhydrous THF (12 mL) was added potassium tert-butoxide (537 mg, 4.79 mmol) under an argon atmosphere at 0°C in two aliquots and the mixture was stirred at room temperature for 2 hours. The reaction mixture was poured into an aqueous solution of 5% potassium hydrogensulfate, and the mixture was extracted with ethyl acetate. The organic layer was washed with saturated brine and dried over anhydrous magnesium sulfate, followed by concentration. The obtained residue was purified by silica gel column chromatography (developing solvent;hexane/ethyl acetate=1/3) to give compound [a-8] (1.05 g, 84% yield) as a red amorphous.

### Step 8

To a solution of compound [a-8] (600 mg, 1.09 mmol) obtained in Example 4-40, Step 7, in acetic acid (3 mL) was added aniline (496 µl, 5.45 mmol) and the mixture was stirred at 100°C for 2 hours. The mixture was cooled to room temperature and concentrated under reduced pressure. The obtained residue was diluted with THF (10 mL) and a solution (2.2 mL, 2.2 mmol) of 1M tetrabutylammonium fluoride in THF was added. The mixture was stirred overnight at room temperature and the reaction mixture was diluted with ethyl acetate. The organic layer was washed successively with a saturated aqueous sodium hydrogencarbonate solution and saturated brine, followed by concentration. The obtained residue was purified by silica gel column chromatography (developing solvent;hexane/ethyl acetate=1/3) to give compound [a-9] (364 mg, 86% yield) as a red amorphous.

### Step 9

To a solution of compound [a-9] (300 mg, 0.77 mmol) obtained in Example 4-40, Step 8, in anhydrous THF (5 mL) were successively added pyridine (188 µl, 2.31 mmol) and methanesulfonic anhydride (270 mg, 1.54 mmol), under an argon atmosphere at 0°C and the mixture was stirred at room temperature for 2 hours. The reaction mixture was poured into an aqueous solution of 5% potassium hydrogensulfate and the mixture was extracted with ethyl acetate. The organic layer was washed with saturated brine, dried over anhydrous magnesium sulfate and concentrated. The obtained crude product [a-10] (crude 374 mg) was used in the next step without purification.

### Step 10

To a solution of compound [a-10] (50 mg, 0.11 mmol) obtained in Example 4-40, Step 9, in THF (1 mL) was added ethylmethylamine (185 µl, 2.14 mmol) and the mixture was stirred overnight in a sealed tube at 85°C. The reaction mixture was cooled to room temperature and poured into a saturated aqueous sodium hydrogencarbonate solution. The mixture was extracted with chloroform. The organic layer was concentrated and the obtained residue was purified by thin layer chromatography (developing solvent;chloroform/methanol=9/1) to give compound [a-11] (36 mg, 81% yield) as a red amorphous. The property values are shown in Table 46.

In the same manner as in Example 4-40, compounds of Examples 4-41 to 4-50 were obtained. The property values are shown in Tables 47 to 50.

### Example 4-51

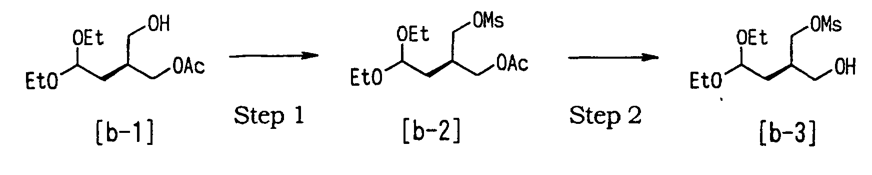

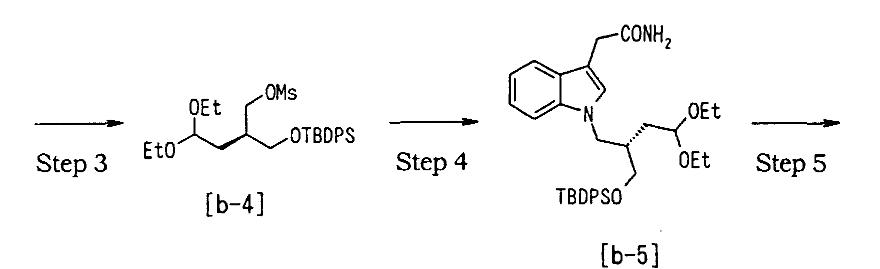

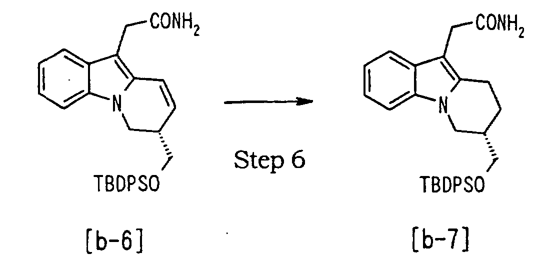

### Step 1

To a solution of a known compound [b-1] (1.09 g, 4.67 mmol) in anhydrous THF (20 mL) were successively added triethylamine (1.30 mL, 9.34 mmol) and mesyl chloride (398 µl, 5.14 mmol), under an argon atmosphere at 0°C, and the mixture was stirred at 0°C for 25 minutes. To the reaction mixture was added a saturated aqueous sodium hydrogencarbonate solution, and the mixture was extracted with ethyl acetate. The organic layer was washed with saturated brine, dried over anhydrous magnesium sulfate and concentrated. The obtained crude product [b-2] (crude 1.53 g) was used in the next step without purification.

### Step 2

To a solution of crude product [b-2] (1.53 g, corresponding to 4.67 mmol) obtained in Example 4-51, Step 1, in dioxane (20 mL) were successively added at 0°C water (10 mL) and an aqueous solution of 4N sodium hydroxide (1.40 mL, 5.60 mmol) and the mixture was stirred at 0°C for 1 hour. To the reaction mixture was added saturated brine and the mixture was extracted with ethyl acetate. The organic layer was washed successively with a saturated aqueous sodium hydrogencarbonate solution and saturated brine, dried over anhydrous magnesium sulfate and concentrated. The obtained crude product [b-3] (crude 1.31 g) was used in the next step without purification.

### Step 3

To a solution of crude product [b-3] (1.31 g, corresponding to 4.67 mmol) obtained in Example 4-51, Step 2, and imidazole (636 mg, 9.34 mmol) in DMF (5 mL) was added tert-butyldiphenylchlorosilane (1.34 mL, 5.14 mmol) at 0°C and the mixture was stirred overnight at room temperature. The reaction mixture was poured into a saturated aqueous sodium hydrogencarbonate solution and the mixture was extracted with ethyl acetate. The organic layer was washed with saturated brine and concentrated. The obtained residue was purified by silica gel column chromatography (developing solvent;hexane/ethyl acetate=4/1) to give compound [b-4] (2.11 g, 89% yield) as a colorless oil.

### Step 4

In the same manner as in Example 4-40, Step 4, using indole-3-acetamide (1.08 g, 6.23 mmol), anhydrous DMF (10 mL), 60% sodium hydride (249 mg, 6.23 mmol), compound [b-4] (2.11 g, 4.15 mmol) obtained in Example 4-51, Step 3, anhydrous THF (5 mL) and sodium iodide (62 mg, 0.42 mmol), compound [b-5) (1.42 g, 57% yield) was obtained as a colorless oil.

### Step 5

In the same manner as in Example 4-40, Step 5, using compound [b-5] (1.42 g, 2.37 mmol) obtained in Example 4-51, Step 4, chloroform (100 mL) and an aqueous solution of 50% trifluoroacetic acid (7.1 mL), a crude product [b-6] (crude 1.12 g) was obtained, which was used in the next step without purification.

### Step 6

In the same manner as in Example 4-40, Step 6, using crude product [b-6] (1.12 g, corresponding to 2.37 mmol) obtained in Example 4-51, Step 5, ethanol (50 mL) and 5% palladium carbon (100 mg), compound [b-7] (1.12 g, 96% yield) was obtained as a colorless oil.

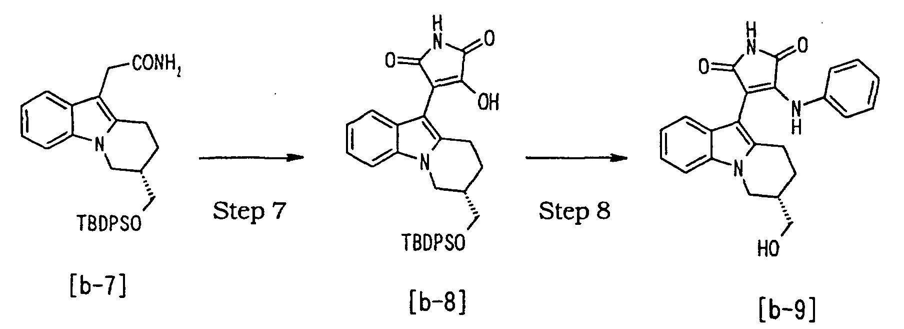

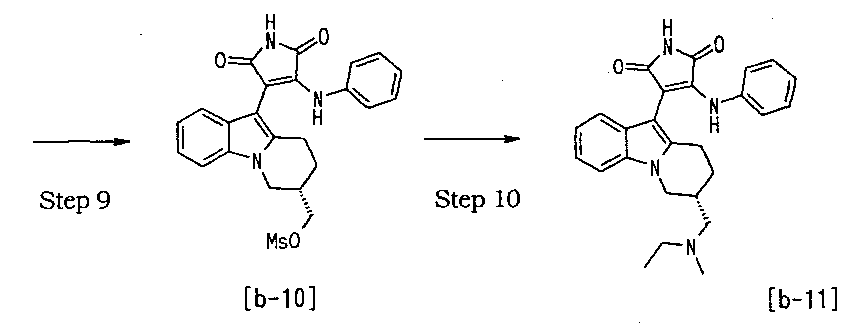

### Step 7

In the same manner as in Example 4-40, Step 7, using compound [b-7] (1.12 g, 2.26 mmol) obtained in Example 4-51, Step 6, dimethyl oxalate (294 mg, 2.51 mmol), anhydrous THF (11 mL) and potassium tert-butoxide (533 mg, 4.75 mmol), compound [b-8] (960 mg, 74% yield) was obtained as a red amorphous.

### Step 8

In the same manner as in Example 4-40, Step 8, using compound [b-8] (600 mg, 1.09 mmol) obtained in Example 4-51, Step 7, acetic acid (3 mL), aniline (496 µl, 5.45 mmol), THF (10 mL) and a solution of 1M tetrabutylammonium fluoride in THF (2.2 mL, 2.2 mmol), compound [b-9] (398 mg, 94% yield) was obtained as a red amorphous.

### Step 9

In the same manner as in Example 4-40, Step 9, using compound[b-9] (300 mg, 0.77 mmol) obtained in Example 4-51, Step 8, anhydrous THF (5 mL), pyridine (188 µl, 2.31 mmol) and methanesulfonic anhydride (270 mg, 1.54 mmol), crude product [b-10] (360 mg) was obtained, which was used in the next step without purification.

### Step 10

In the same manner as in Example 4-40, Step 10, using compound [b-10] (50 mg, 0.11 mmol) obtained in Example 4-51, Step 9, THF (1 mL) and ethylmethylamine (185 µl, 2.14 mmol), compound [b-11] (38 mg, 83% yield) was obtained as a red amorphous. The property values are shown in Table 50.

In the same manner as in Example 4-51, compounds of Examples 4-52 to 4-55 were obtained. The property values are shown in Tables 50 and 51. **Example 4-56**

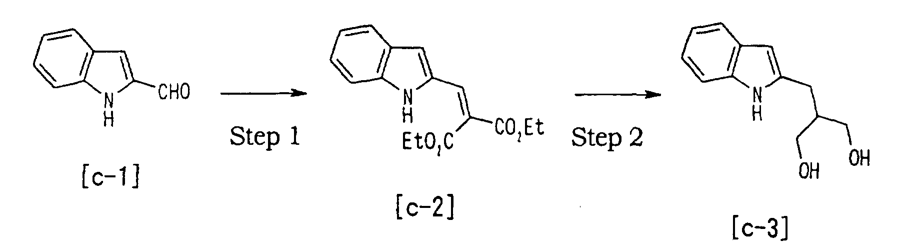

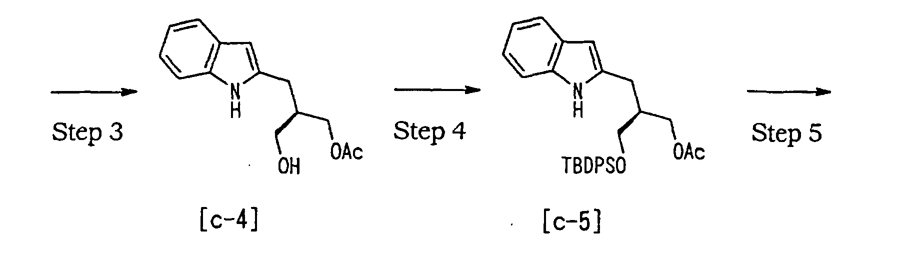

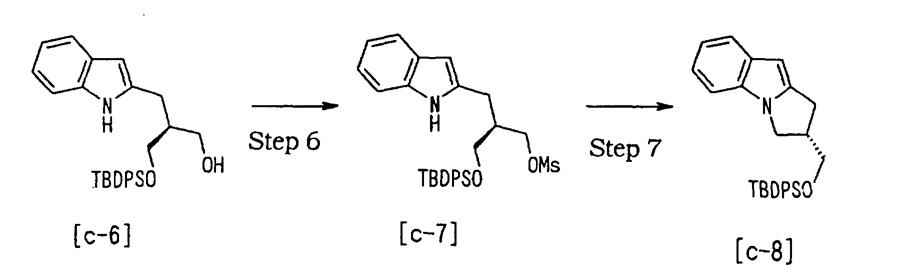

### Step 1

The compound [c-1] (10.0 g, 68.9 mmol) synthesized according to the method described *in Tetrahedron*, 50, 6299, 1994 or *Synthetic Communication*, 17, 647, 1987 was suspended in toluene (150 mL) and diethyl malonate (12.5 mL, 82.7 mmol), piperidine (0.68 mL, 6.89 mmol) and molecular sieves 4A (10 g) were added at room temperature. The mixture was stirred under heating under an argon stream at 100-105°C. After 3 hours, the reaction mixture was filtered and the solvent was evaporated under reduced pressure. The obtained residue was purified by silica gel pad (developing solvent:hexane/ethyl acetate) to give compound [c-2] (10.0 g, 50% yield), as yellow crystals.

### Step 2

The compound [c-2] (0.2 g, 0.7 mmol) obtained in Example 4-56, Step 1, was dissolved in ethanol (2.5 mL) and THF (2.5 mL) and this solution was dropwise added to a mixture of lithium chloride (150 mg, 3.5 mmol) and sodium borohydride (130 mg, 3.5 mmol) in ethanol (2.5 mL) and THF (2.5 mL) at 0°C and the mixture was stirred for 30 minutes. Then, the reaction mixture was refluxed under heating for 30 minutes. To the reaction mixture was added a 10% aqueous citric acid solution and the mixture was extracted with ethyl acetate. The organic layer was washed with saturated brine and dried over sodium sulfate. Sodium sulfate was filtered off and the filtrate was concentrated to dryness to give a crude product [c-3].

### Step 3

The crude product [c-3] (7.2 g) obtained in Example 4-56, Step 2, was dissolved in vinyl acetate (100 mL) and Lipase PS (348 mg) was added. The mixture was stirred at room temperature for 13 hours. The reaction mixture was filtered through Celite and the filtrate was concentrated to dryness to give a crude product [c-4] (9.72 g).

### Step 4

The crude product [c-4] obtained in Example 4-56, Step 3, was dissolved in DMF (35 mL), and tert-butyldiphenylchlorosilane (8.98 mL, 38.3 mmol) and imidazole (2.61 g, 38.3 mmol) were added. The mixture was stirred under an argon stream at room temperature for 1.5 hours. To the reaction mixture was added saturated brine and the mixture was extracted with ethyl acetate. The organic layer was washed with water and saturated brine and concentrated to dryness to give a crude product [c-5] (19.1 g).

### Step 5

The crude product [c-5] obtained in Example 4-56, Step 4, was dissolved in methanol (70 mL), and potassium carbonate (4.81 g, 34.8 mmol) was added. The mixture was stirred under an argon stream at room temperature for 40 minutes. To the reaction mixture were added saturated brine and an aqueous solution of 1M potassium hydrogensulfate and the mixture was extracted with ethyl acetate. The obtained organic layer was washed with saturated brine and concentrated to dryness. The obtained residue was purified by silica gel column chromatography (developing solvent:hexane/ethyl acetate=4/1) to give compound [c-6] (11.6 g).

### Step 6

The compound [c-6] (11.6 g, 26.1 mmol) obtained in Example 4-56, Step 5, was dissolved in THF (200 mL), and pyridine (6.3 mL, 78.3 mmol) and methanesulfonic anhydride (9.09 g, 52.2 mmol) were added at 0°C. The mixture was stirred under an argon stream at room temperature for 2 hours. To the reaction mixture was added saturated brine and the mixture was extracted with ethyl acetate. The obtained organic layer was washed with an aqueous solution of 1M potassium hydrogensulfate and saturated brine and concentrated to dryness to give a crude product [c-7] (14.1 g) as an yellow-orange amorphous.

### Step 7

The crude product [c-7] obtained in Example 4-56, Step 6, was dissolved in DMF (200 mL), and sodium hydride (1.15 g, 28.7 mmol) and sodium iodide (391 mg, 2.61 mmol) were added at 0°C. The mixture was stirred under an argon stream at 0°C for 40 minutes and at room temperature for 12 hours. To the reaction mixture was added an aqueous solution of 1M potassium hydrogensulfate and the mixture was extracted with ethyl acetate. The obtained organic layer was washed with water and saturated brine, and concentrated to dryness. The obtained residue was purified by silica gel column chromatography (developing solvent:hexane/ethyl acetate=95/5) to give compound [c-8] (8.94 g, 80% yield from compound [c-6]).

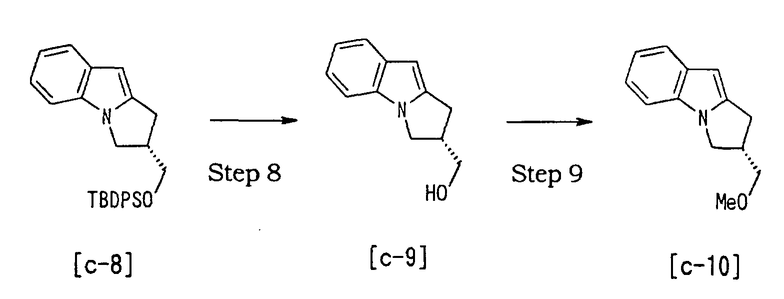

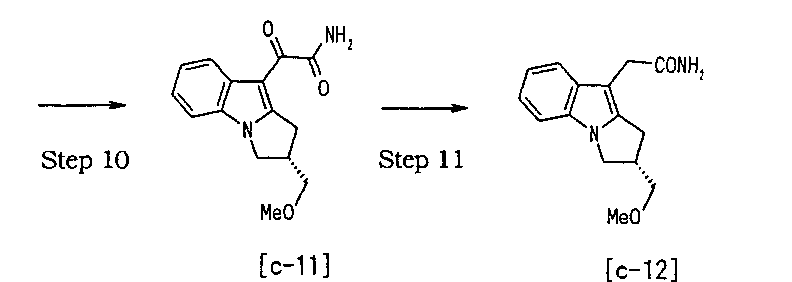

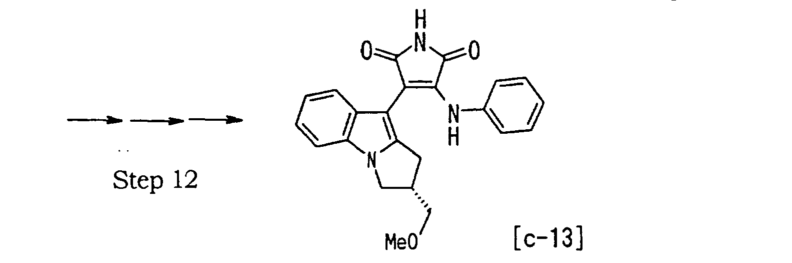

### Step 8

The compound [c-8] (4.6 g, 10.8 mmol) obtained in Example 4-56, Step 7, was dissolved in THF (30 mL) and a solution (22 mL) of 1M tetrabutylammonium fluoride in THF was added at room temperature. The mixture was stirred for 12 hours. To the reaction mixture was added a 10% aqueous citric acid solution and the mixture was extracted with ethyl acetate. The organic layer was washed with saturated brine and dried over sodium sulfate. The desiccant was filtered off and the filtrate was concentrated to dryness. The residue was purified by silica gel column chromatography (developing solvent;hexane/ethyl acetate=1/1) to give compound [c-9] (1.36 g, 67% yield) as a yellow oil.

### Step 9

The compound [c-9] (500 mg, 2.7 mmol) obtained in Example 4-56, Step 8, was dissolved in THF (5 mL) and sodium hydride (130 mg, 3.2 mmol) was added under ice-cooling. The reaction mixture was stirred at room temperature for 15 minutes and methyl iodide (0.2 mL, 3.2 mmol) was added. The mixture was stirred at room temperature for 1 hour. To the reaction mixture was added a 10% aqueous citric acid solution and the mixture was extracted with ethyl acetate. The organic layer was washed with saturated brine and dried over sodium sulfate. The desiccant was filtered off and the filtrate was concentrated to dryness to give compound [c-10] (620 mg) as a yellow oil, which was used in the next step without purification.

### Step 10

The compound [c-10] (620 mg) obtained in Example 4-56, Step 9, was dissolved in methylene chloride (10 mL) and triethylamine (0.28 mL, 3.2 mmol) was added. Oxalyl chloride (0.45 mL, 3.2 mmol) was added and the mixture was stirred under ice-cooling for 30 minutes. To the reaction mixture was added aqueous ammonia (20 mL) under ice-cooling and the mixture was stirred for 30 minutes. To the reaction mixture was added a 10% aqueous citric acid solution. After partitioning, the aqueous layer was extracted with chloroform. The organic layers were combined and dried over sodium sulfate. The desiccant was filtered off and the filtrate was concentrated to dryness to give compound [c-11] (755 mg) as a pale-brown amorphous, which was used in the next step without purification.

### Step 11

The compound [c-11] (755 mg) obtained in Example 4-56, Step 10, was dissolved in THF (15 mL) and ethanol (7 mL), and sodium borohydride (505 mg, 13.5 mmol) was added under ice-cooling. The mixture was stirred under ice-cooling for 1 hour. To the reaction mixture was added a 10% aqueous citric acid solution and the mixture was extracted with ethyl acetate. The organic layer was washed with saturated brine and dried over sodium sulfate. The desiccant was filtered off and the filtrate was concentrated to dryness. To the residue was added methylene chloride (10 mL) and then triethylsilane (0.85 mL, 5.4 mmol) and trifluoroacetic acid (3 mL), which was followed by stirring at room temperature for 12 hours. To the reaction mixture was added a saturated aqueous sodium hydrogencarbonate solution. After partitioning, the aqueous layer was extracted with chloroform. The organic layers were combined and dried over sodium sulfate. The desiccant was filtered off and the filtrate was concentrated to dryness. The residue was purified by silica gel column chromatography (developing solvent:hexane/ethyl acetate 1/2) to give compound [c-12] (590 mg, 86% yield) as a pale-brown amorphous.

### Step 12

The compound [c-12] obtained in Example 4-56, Step 11, was treated in the same manner as in Example 4-1, Step 4, and Example 1-1, Step 2, to give compound [c-13]. The property values are shown in Table 52.

In the same manner as in Example 4-56, the compound of Example 4-57 was obtained. The property values are shown in Table 52.

In the same manner as in Example 4-1, Step 2 to Step 5, Example 4-2 or Example 4-40, Step 9 to Step 10, the compounds of Examples 4-58 to 4-71 were obtained from compound [c-8] obtained in Example 4-56, Step 1 to Step 7. The property values are shown in Tables 52 to 57.

### Example 5-1

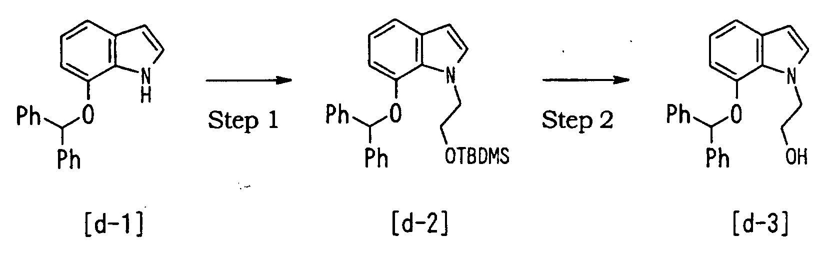

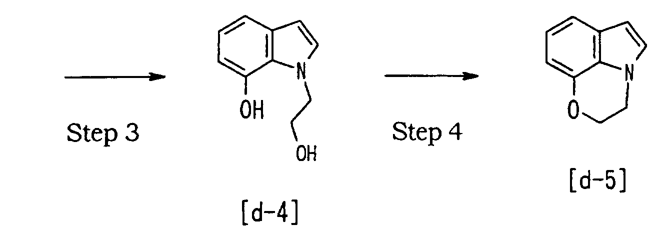

wherein Ph is phenyl and TBDMS is tert-butyldimethylsilyl.

### Step 1

The compound [d-1] (20 g) obtained in the same manner as in the method described in *Synthetic Communication*, 21 (5), 611-617, 1991, was dissolved in DMF (50 mL) and added dropwise to a suspension of sodium hydride (3.2 g, 80.2 mmol) in DMF (150 mL) under ice-cooling over 40 minutes. After the completion of the dropwise addition, the reaction mixture was stirred at room temperature for 30 minutes and 1-bromo-2-(tert-butyldimethylsilyloxy)ethane (19.2 g, 80.2 mmol) was added, which was followed by stirring at room temperature for 1 more hour. After the completion of the reaction, a 10% aqueous citric acid solution was added and the mixture was extracted with ethyl acetate. The mixture was washed with saturated brine and dried over sodium sulfate. The desiccant was filtered off and the filtrate was concentrated to dryness to give the crude product [d-2] (38.7 g) as a pale-brown oil.

### Step 2

The crude product [d-2] obtained in Example 5-1, Step 1, was dissolved in THF (100 mL) and a solution (150 mL) of 1M tetrabutylammonium fluoride in THF was added at room temperature. The mixture was stirred for 1 hour. To the reaction mixture was added a 10% aqueous citric acid solution and the mixture was extracted with ethyl acetate. The organic layer was washed with saturated brine and dried over sodium sulfate. The desiccant was filtered off and the filtrate was concentrated to dryness to give a crude product [d-3] (21.7 g) as a pale-brown oil.

### Step 3

The crude product [d-3] obtained in Example 5-1, Step 2, was dissolved in methanol (200 mL) and palladium hydroxide (13.0 g) was added. The mixture was stirred under a hydrogen atmosphere (3 atm) at room temperature for 1 hour. The catalyst was filtered off and the filtrate was concentrated to dryness. The residue was purified by silica gel column chromatography (developing solvent:hexane/ethyl acetate=3/2) to give compound [d-4] (3.2 g, 27% yield from compound [d-1]) as pale-yellow crystals.

### Step 4

The compound [d-4] (3.2 g) obtained in Example 5-1, Step 3, was dissolved in THF (90 mL) and triphenylphosphine (5.7 g, 21.7 mmol) was added. The mixture was stirred at room temperature for 30 minutes. A solution of 40% diethyl azodicarboxylate in toluene was added dropwise over 20 minutes under ice-cooling. After the dropwise addition, the mixture was stirred at room temperature for 2 hours, and the reaction mixture was concentrated under reduced pressure. The residue was purified by silica gel column chromatography (developing solvent:hexane/ethyl acetate=9/1) to give compound [d-5] (2.34 g, 82% yield) as a yellow oil.

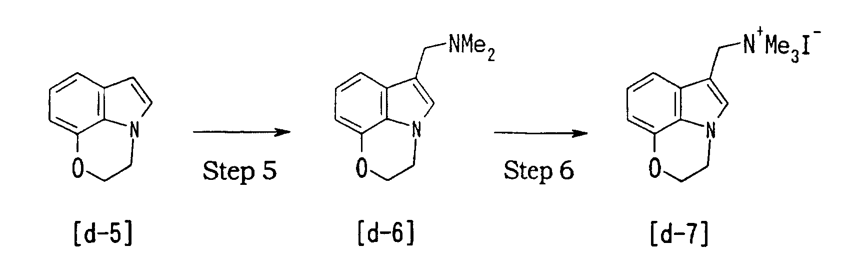

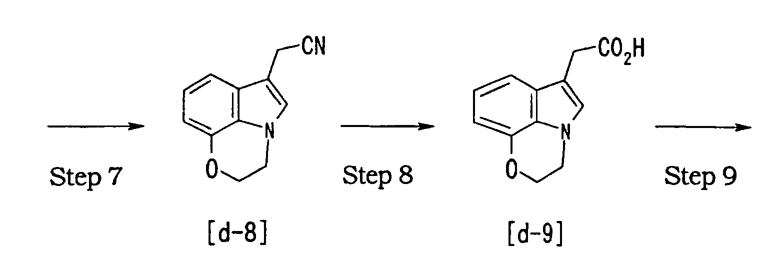

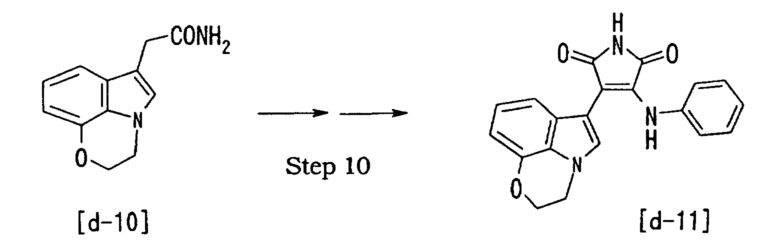

### Step 5

An aqueous 40% dimethylamine solution (4.1 g, 36.0 mmol) and an aqueous 37% formalin solution (2.6 mL, 36.0 mmol) were added to a mixed solvent of acetic acid (40 mL) and ethanol (40 mL) under ice-cooling. Then, a solution of compound [d-5] (2.3 g) obtained in Example 5-1, Step 4, in a mixture of acetic acid (15 mL) and ethanol (15 mL) was added under ice-cooling, and the mixture was stirred at room temperature for 12 hours. To the reaction mixture was added an aqueous 40% sodium hydroxide solution, and the mixture was extracted with chloroform. The organic layer was washed with saturated brine and dried over sodium sulfate. The desiccant was filtered off and the filtrate was concentrated to dryness to give a crude product [d-6] (3.4 g) as a yellow oil, which was used in the next reaction without purification.

### Step 6

Methyl iodide (95 mL) was added to ethanol (150 mL) and a solution of the crude product [d-6] (3.4 g) obtained in Example 5-1, Step 5, in acetic acid (150 mL) was added under ice-cooling, and the mixture was stirred at room temperature for 5 hours. The reaction mixture was concentrated to dryness to give a crude product [d-7] (6.1 g) as a yellow amorphous.

### Step 7

The crude product [d-7] (6.1g) obtained in Example 5-1, Step 6, was dissolved in DMF (75 mL), and a solution of potassium cyanide (9.4 g, 144 mol) in water (37.5 mL) was added, which was followed by reflux under heating for 1 hour. To the reaction mixture was added a saturated aqueous sodium hydrogencarbonate solution, and the mixture was extracted with ethyl acetate. The organic layer was washed with saturated brine and dried over sodium sulfate. The desiccant was filtered off and the filtrate was concentrated to dryness. The residue was purified by silica gel column chromatography (developing solvent:hexane/ethyl acetate=7/3) to give compound [d-8] (1.33 g, 47% yield, from compound [d-5]) as a yellow oil.

### Step 8

The compound [d-8] (1.0 g) obtained in Example 5-1, Step 7, was dissolved in methanol (10 mL), and an aqueous 40% sodium hydroxide solution (6.0 mL) was added, which was followed by reflux under heating for 2 hours. To the reaction mixture was added conc. hydrochloric acid, and the mixture was extracted with chloroform. The organic layer was washed with saturated brine and dried over sodium sulfate. The desiccant was filtered off and the filtrate was concentrated to dryness to give a crude product [d-9] (1.1 g) as a brown oil, which was used in the next reaction without purification.

### Step 9

The crude product [d-9] (1.1 g) obtained in Example 5-1, Step 8, was dissolved in methylene chloride (20 mL), and oxalyl chloride (0.8 mL, 9.2 mmol) was added under ice-cooling. The mixture was stirred under ice-cooling for 1 hour. Then, aqueous ammonia (30 mL) was added under ice-cooling and the mixture was stirred for 30 minutes. To the reaction mixture was added saturated brine, and the mixture was extracted with chloroform. The organic layer was washed with saturated brine and dried over sodium sulfate. The desiccant was filtered off and the filtrate was concentrated to dryness to give compound [d-10] (850 mg, 85% yield) as a brown amorphous.

### Step 10

The compound [d-10] obtained in Example 5-1, Step 9, was treated in the same manner as in Example 4-1, Step 4, and Example 1-1, Step 2, to give compound [d-11]. The property values are shown in Table 57.

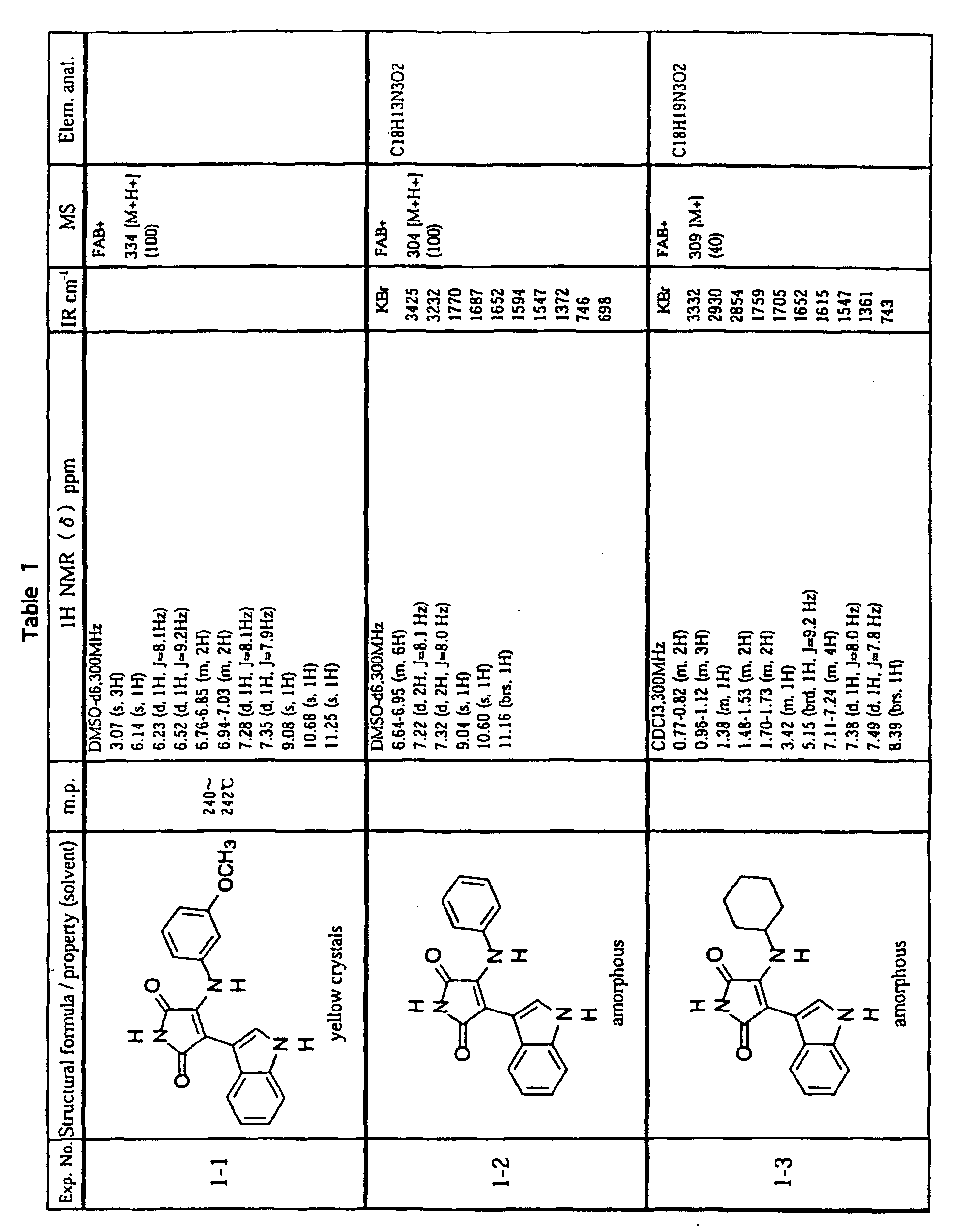

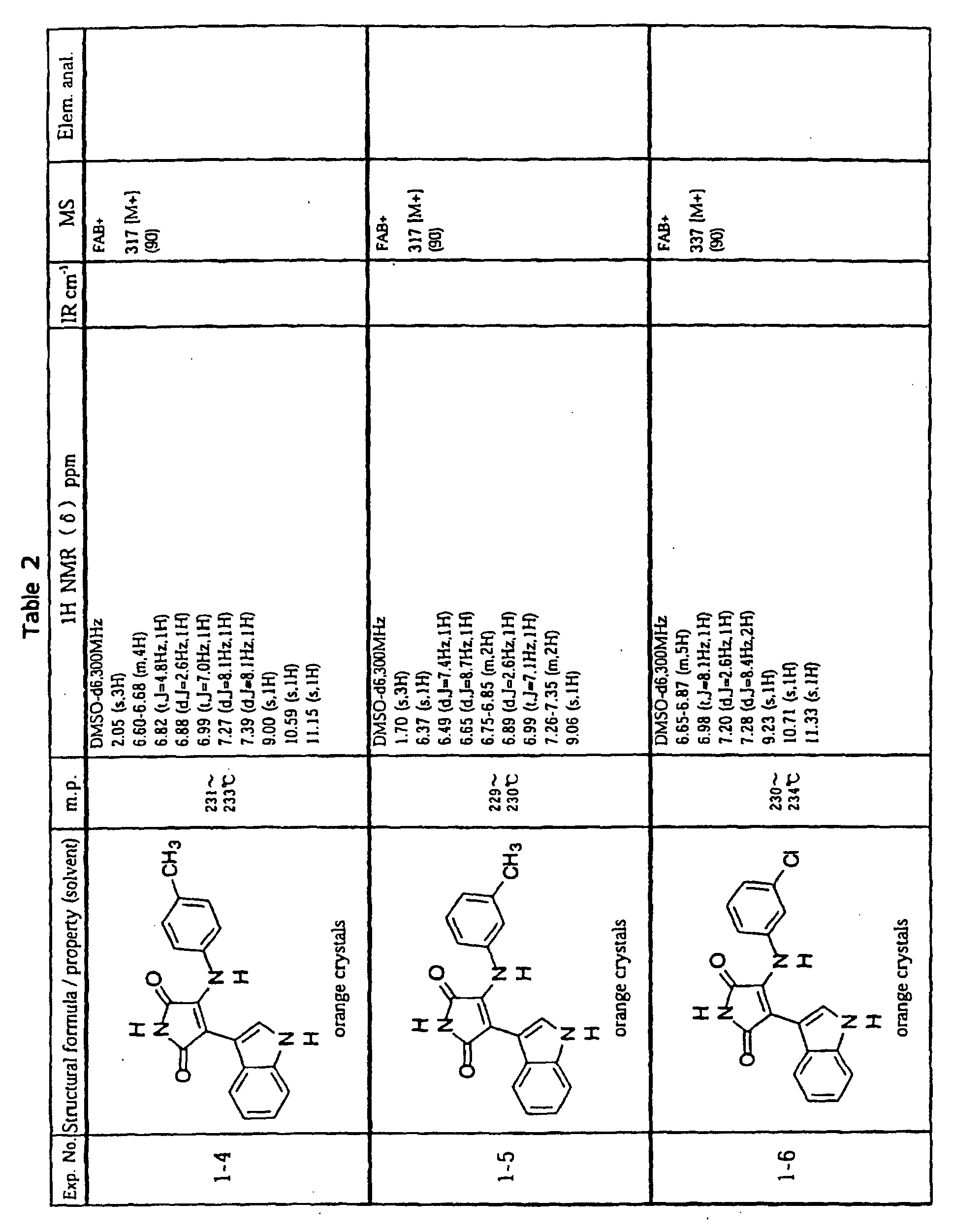

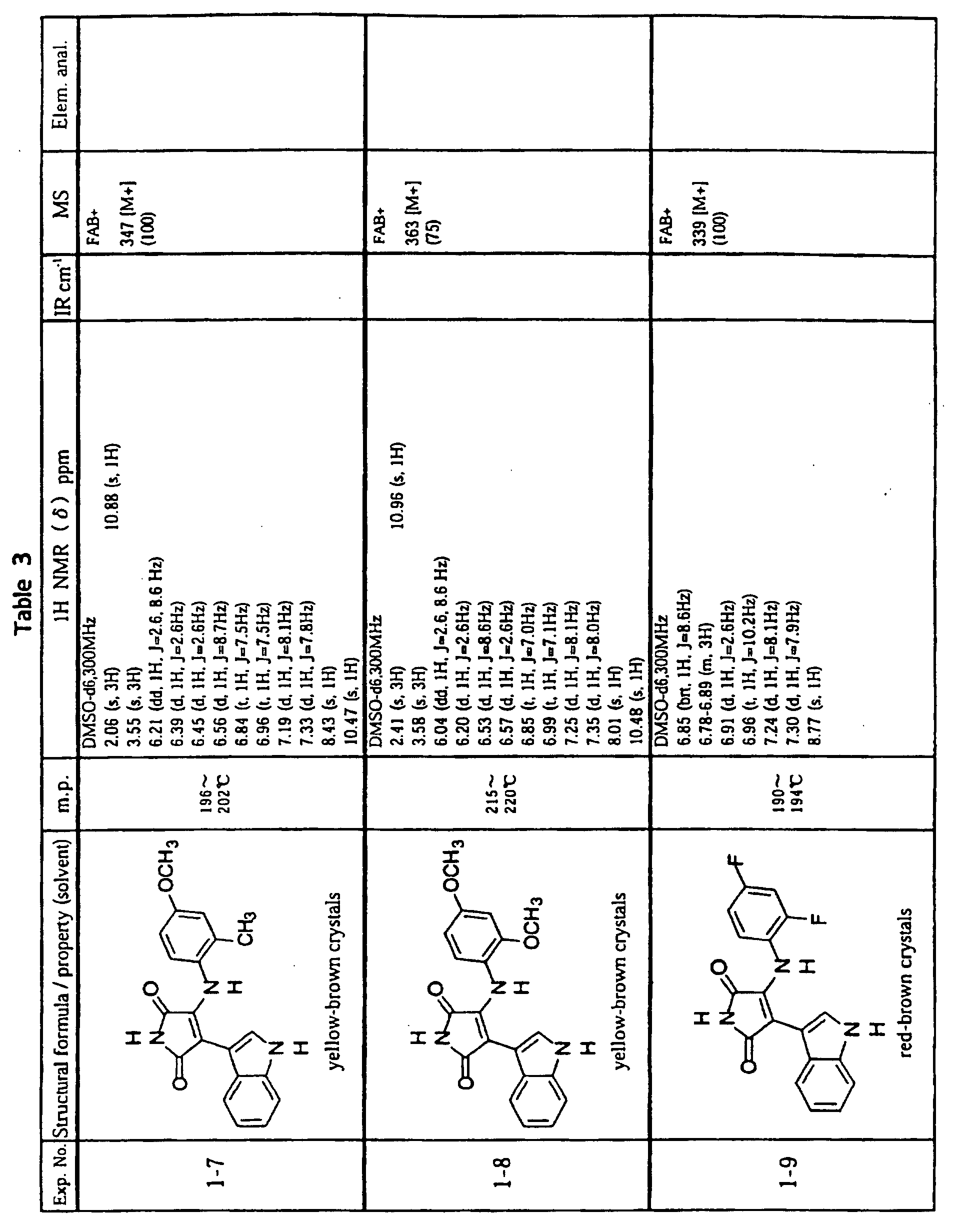

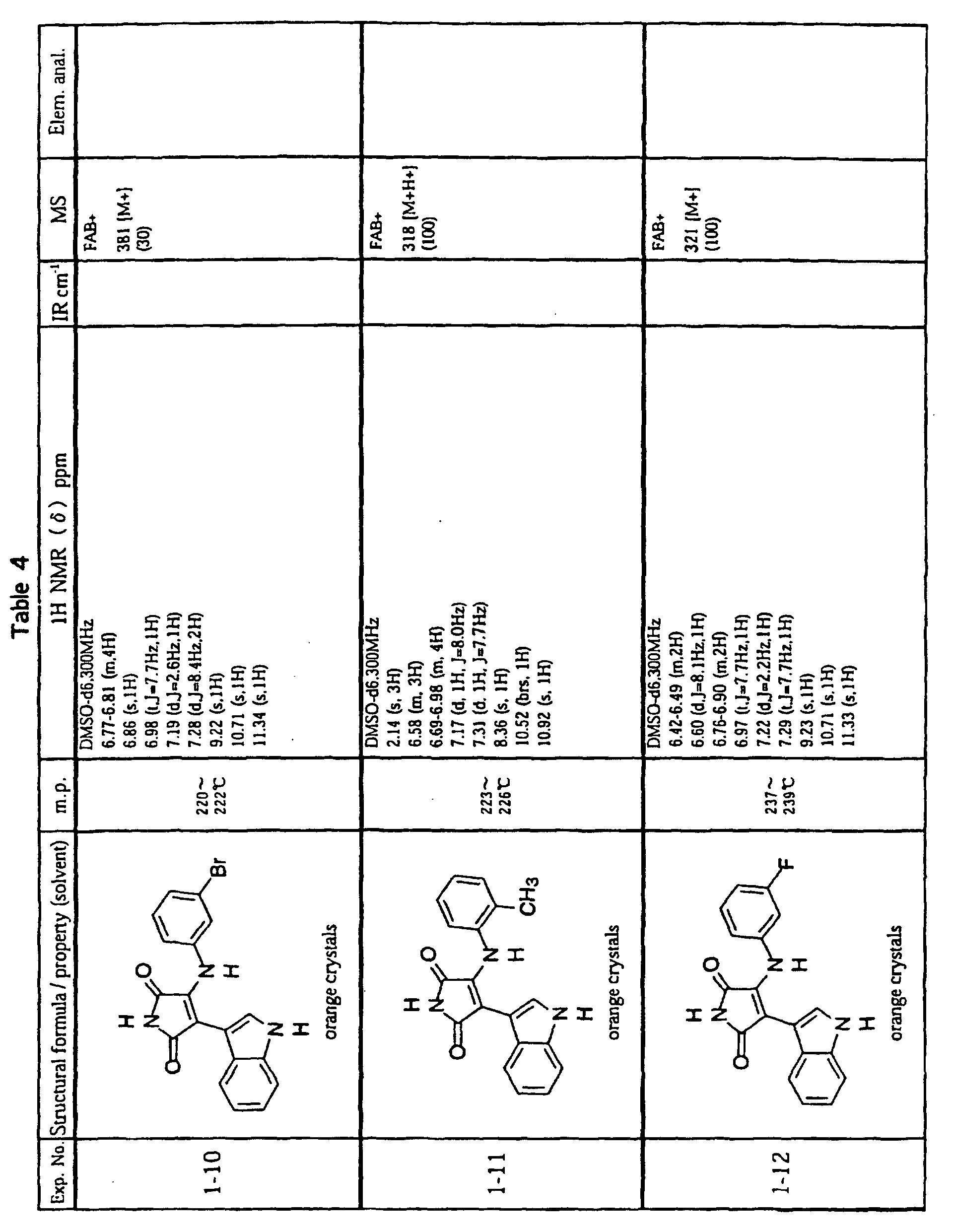

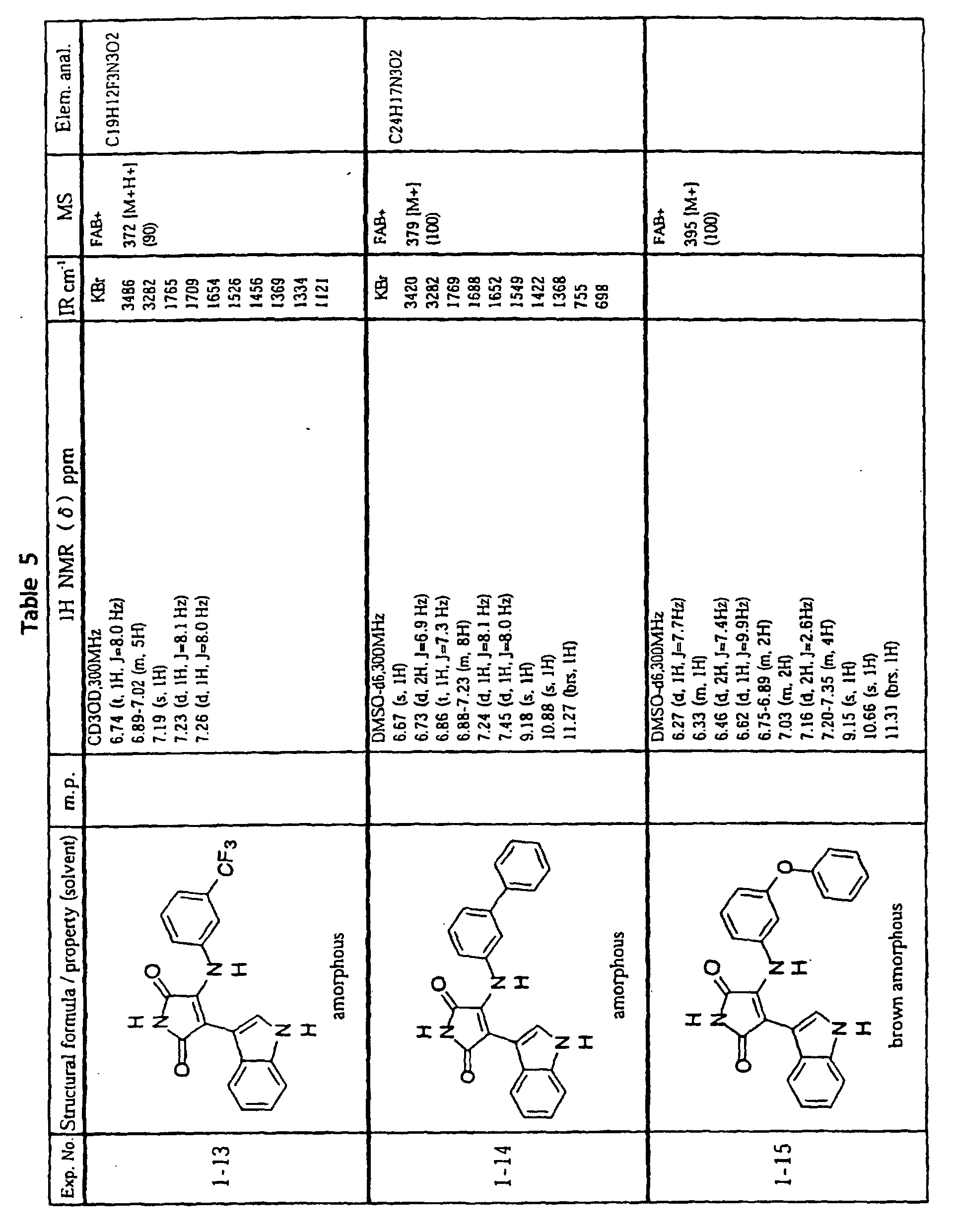

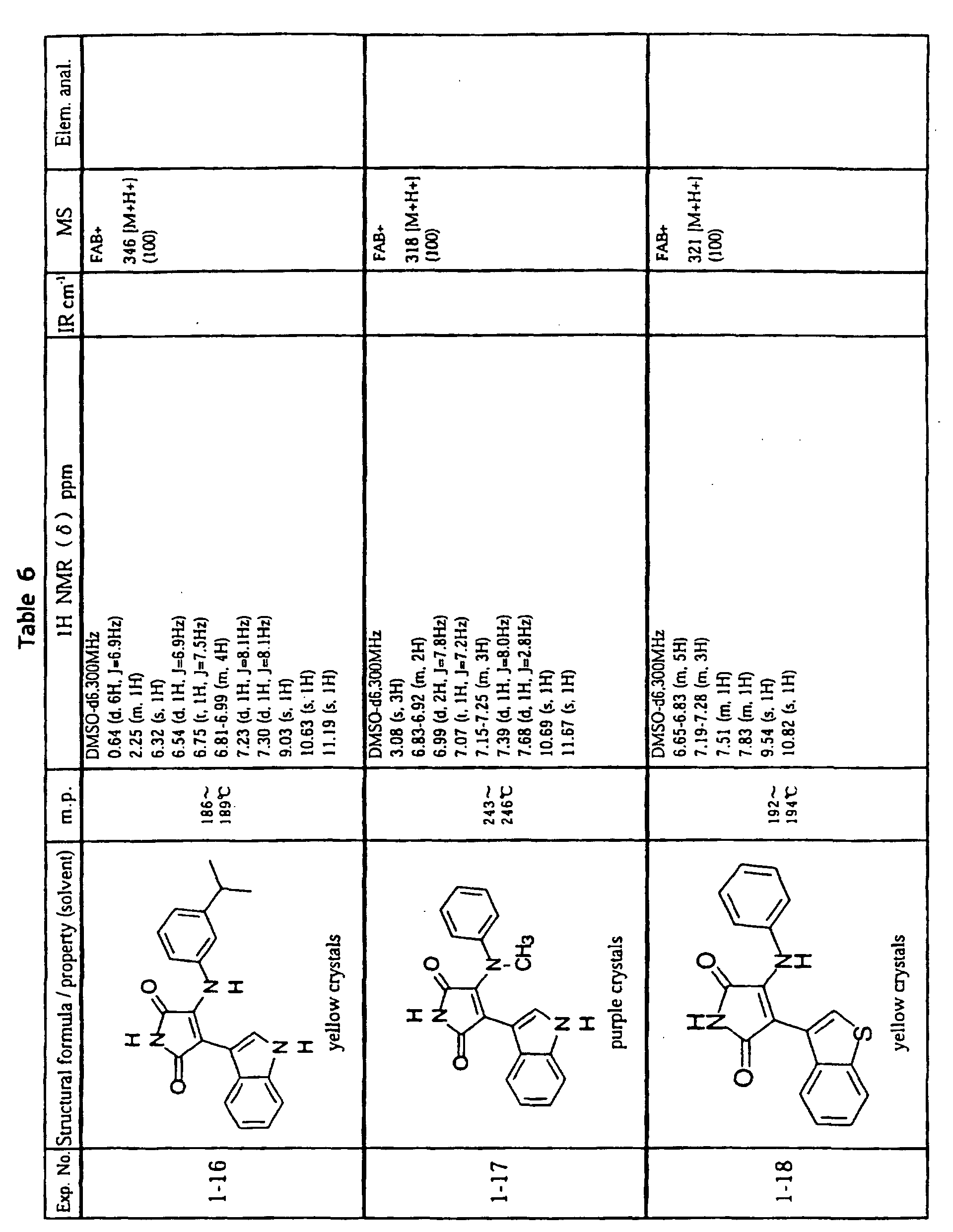

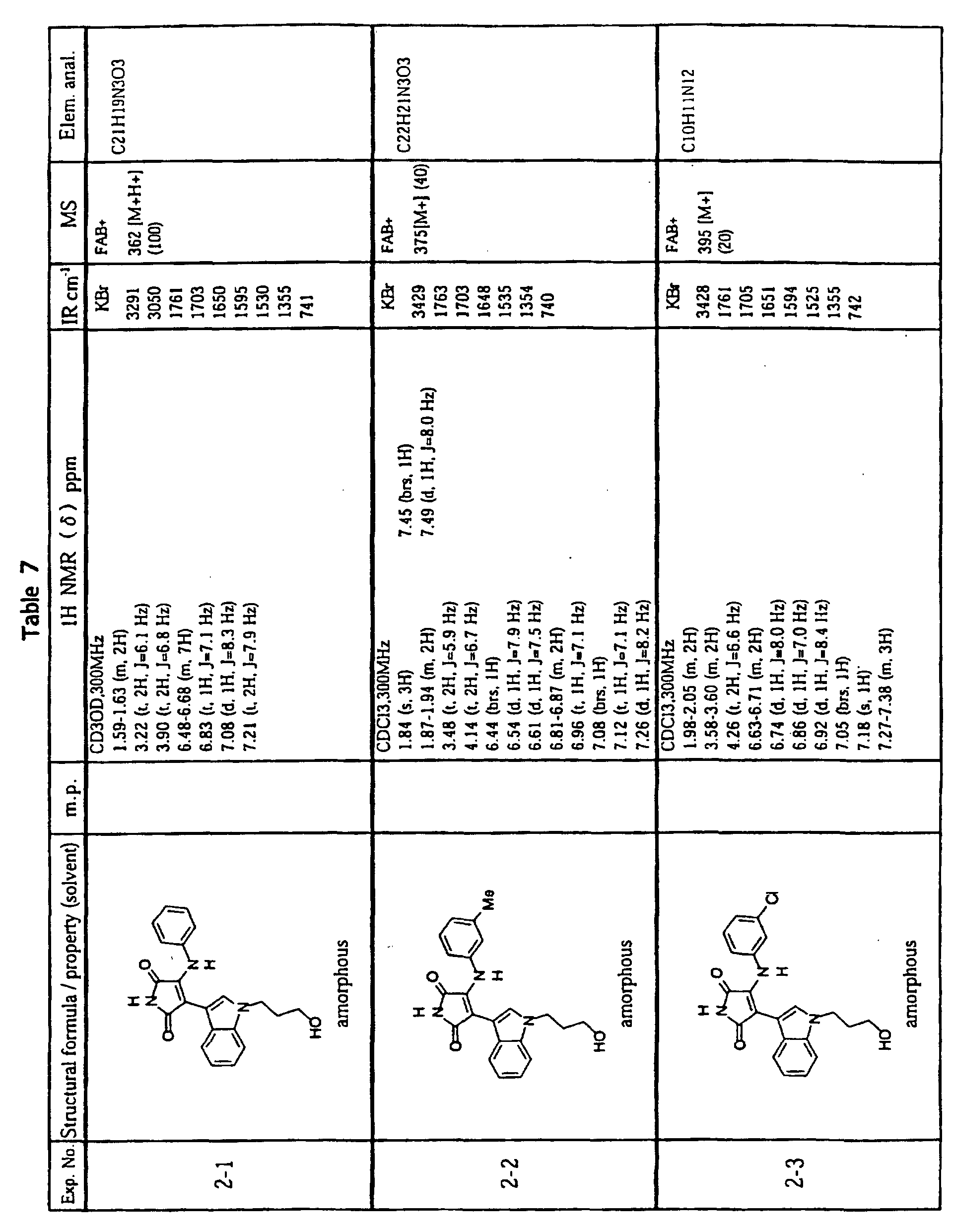

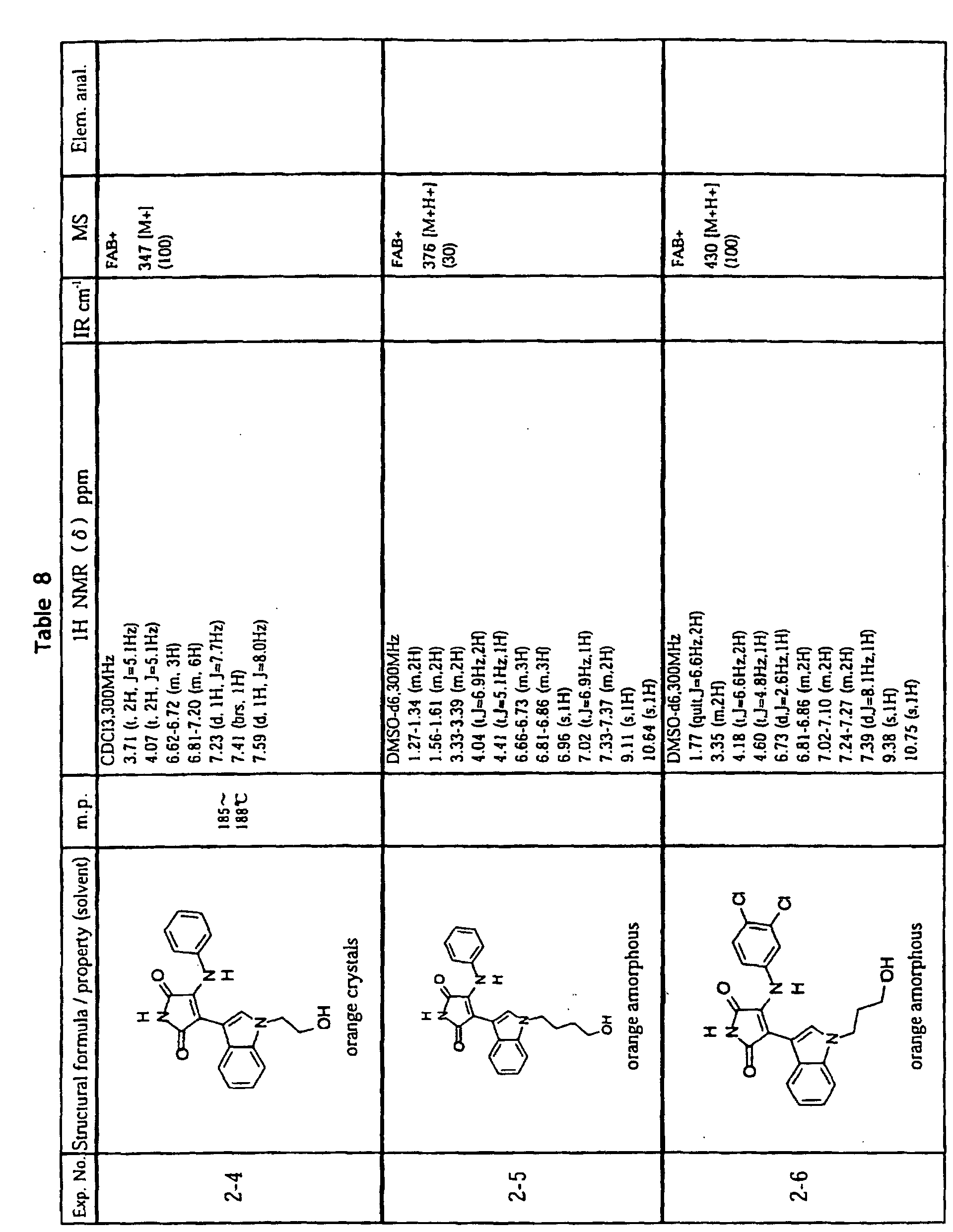

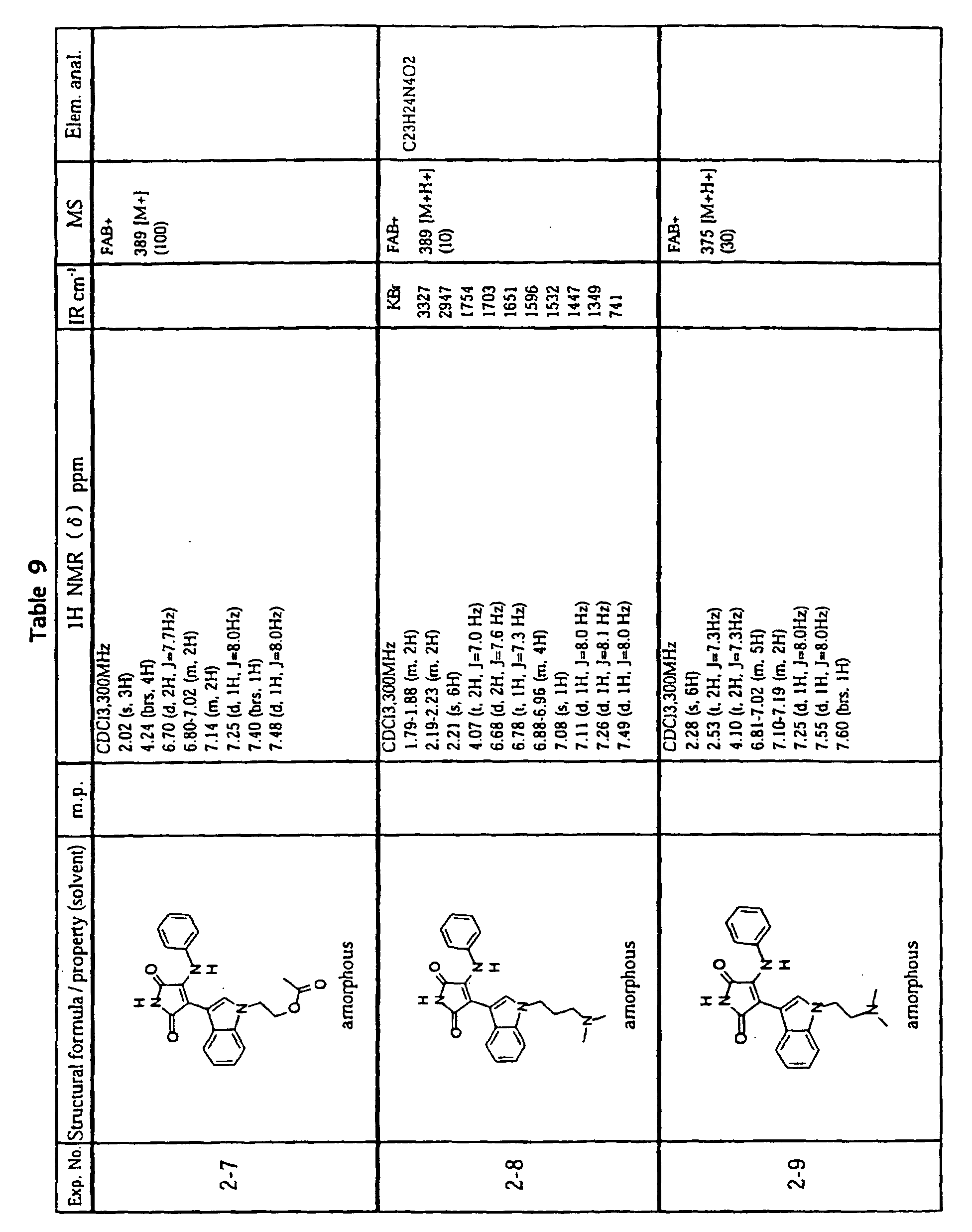

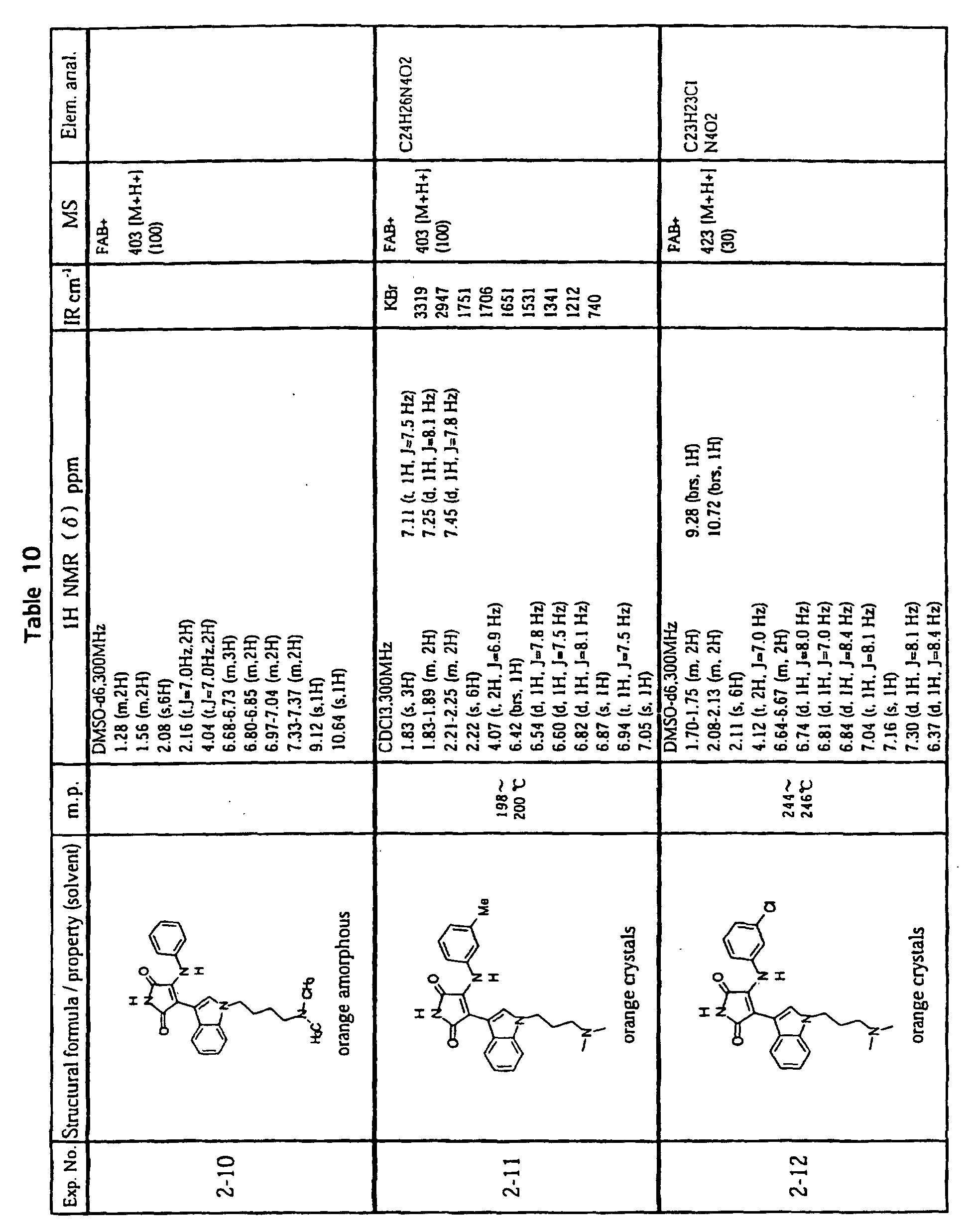

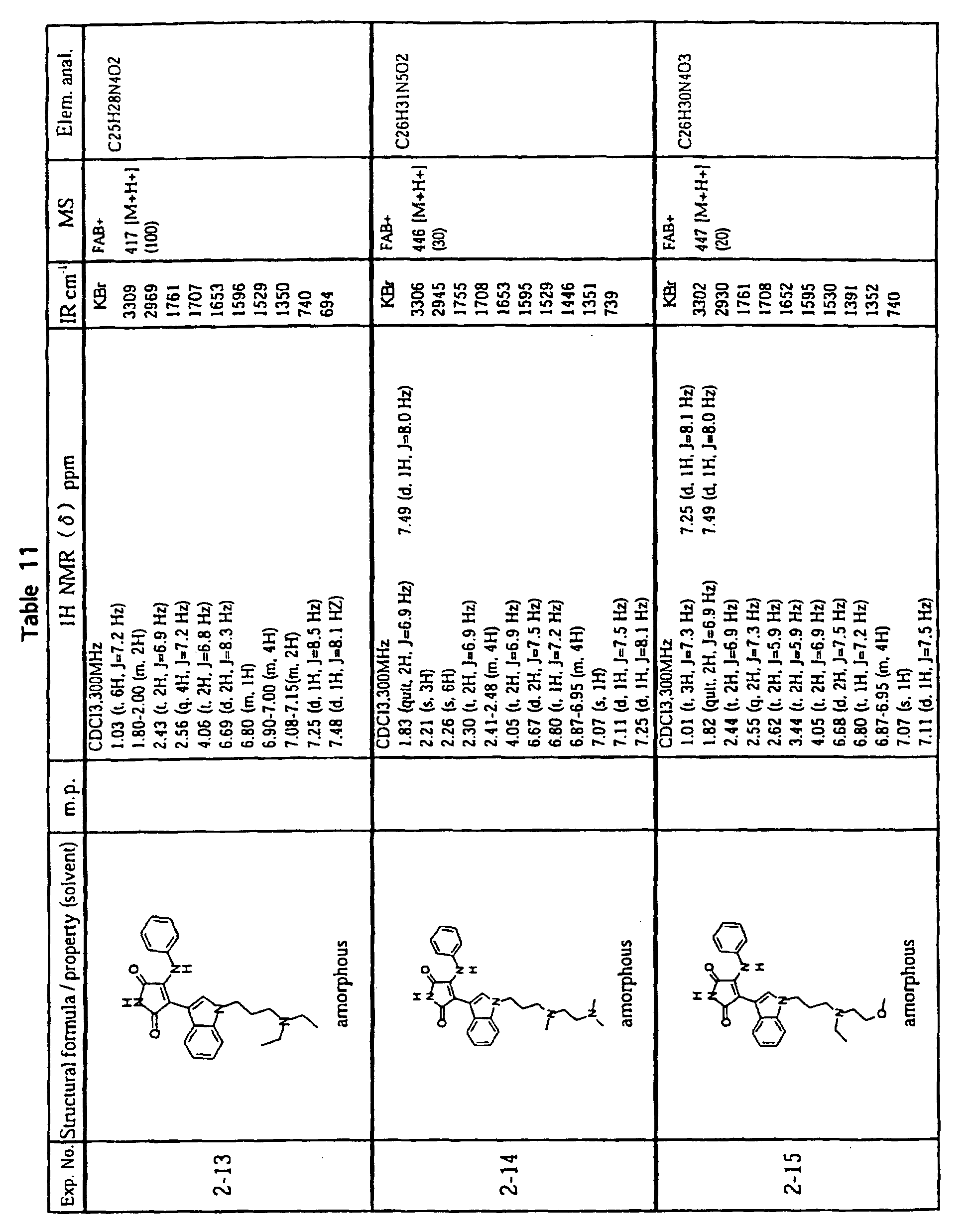

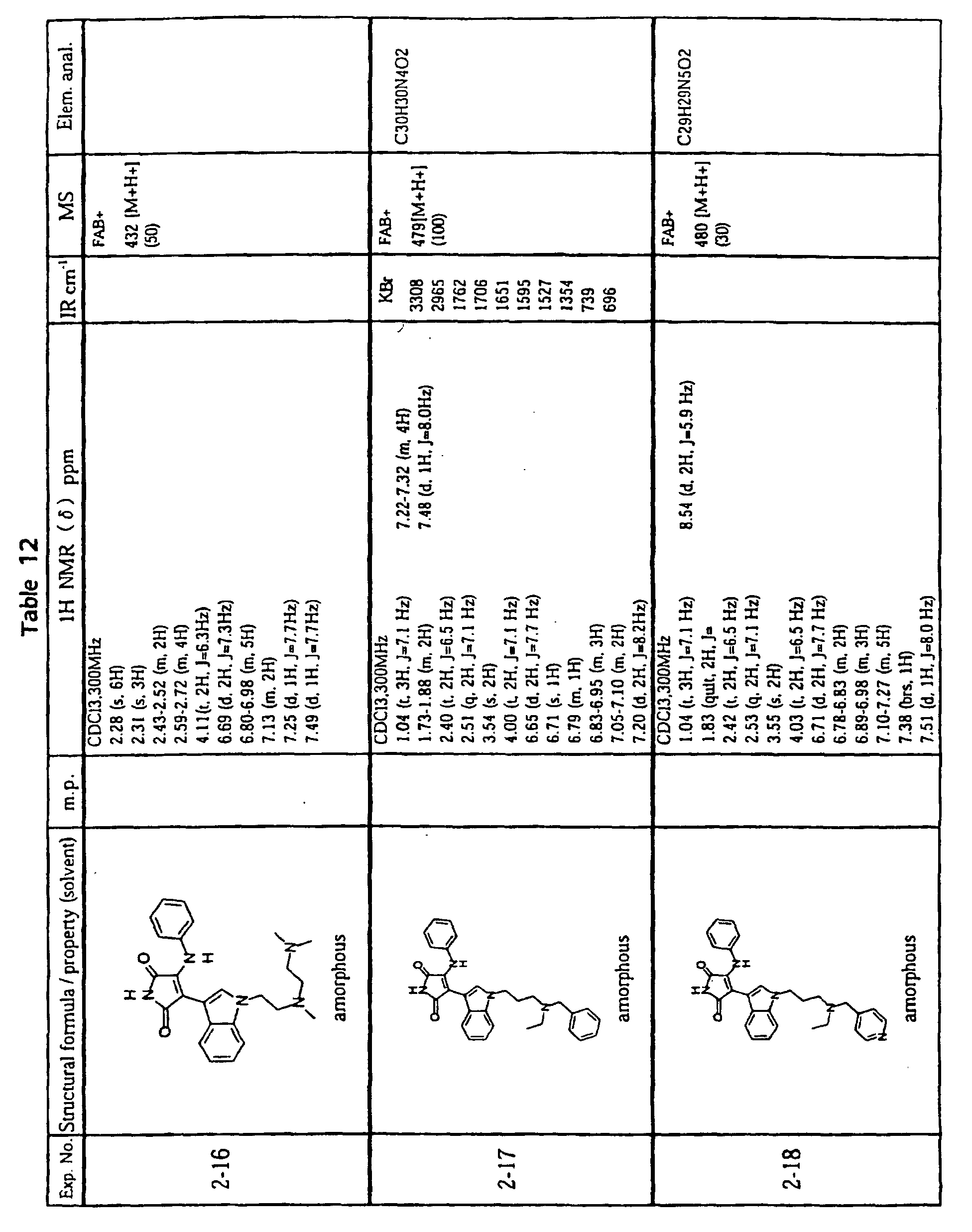

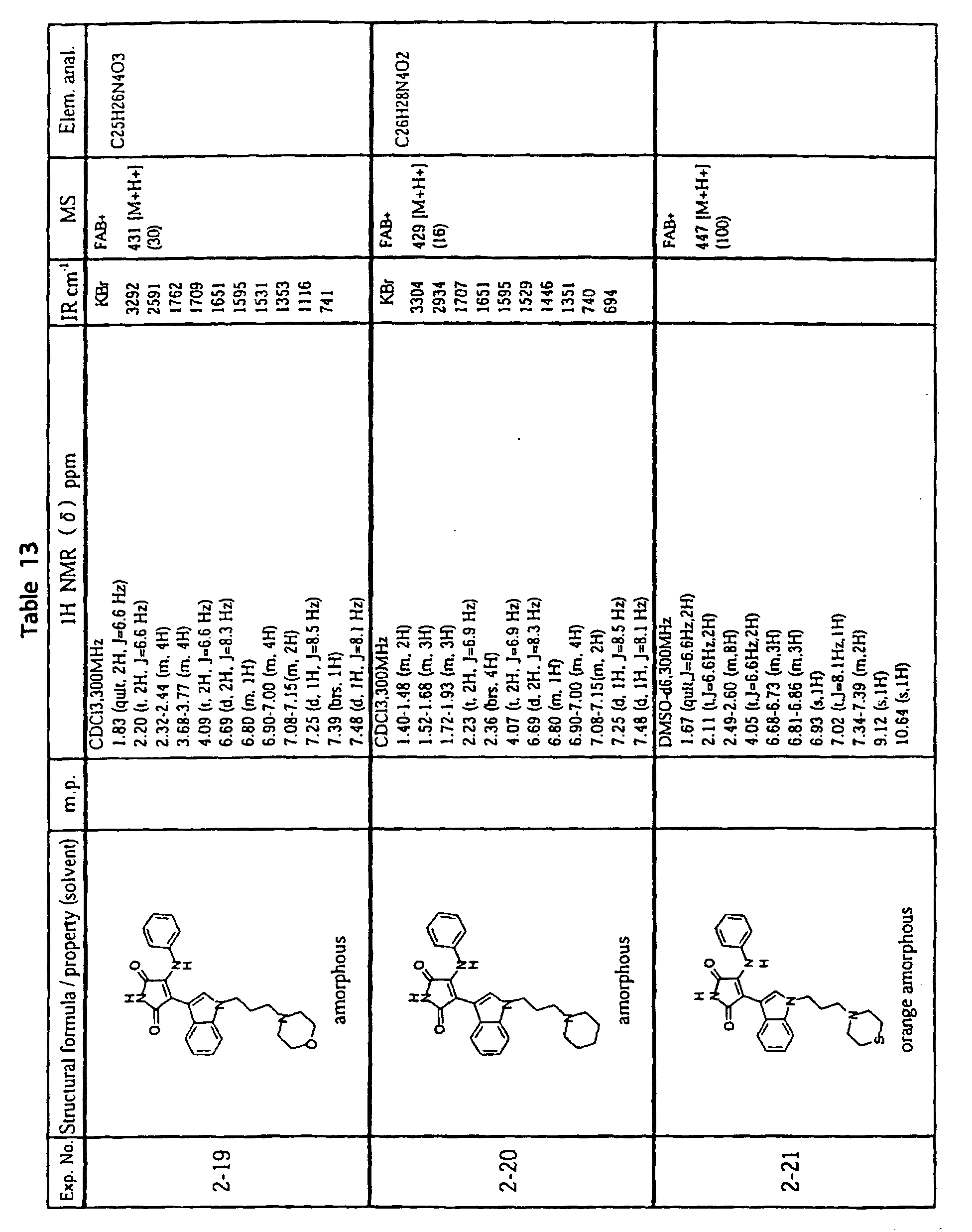

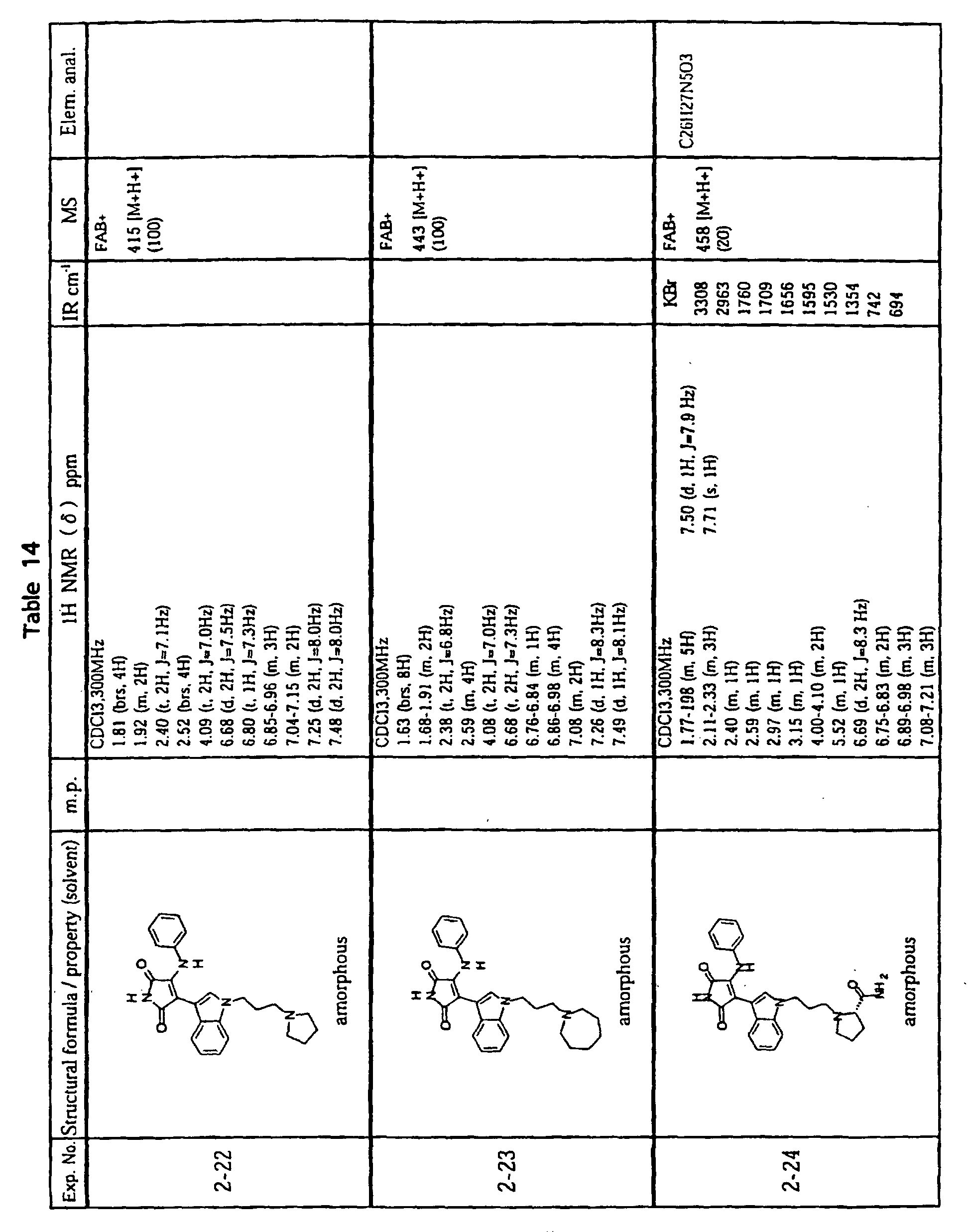

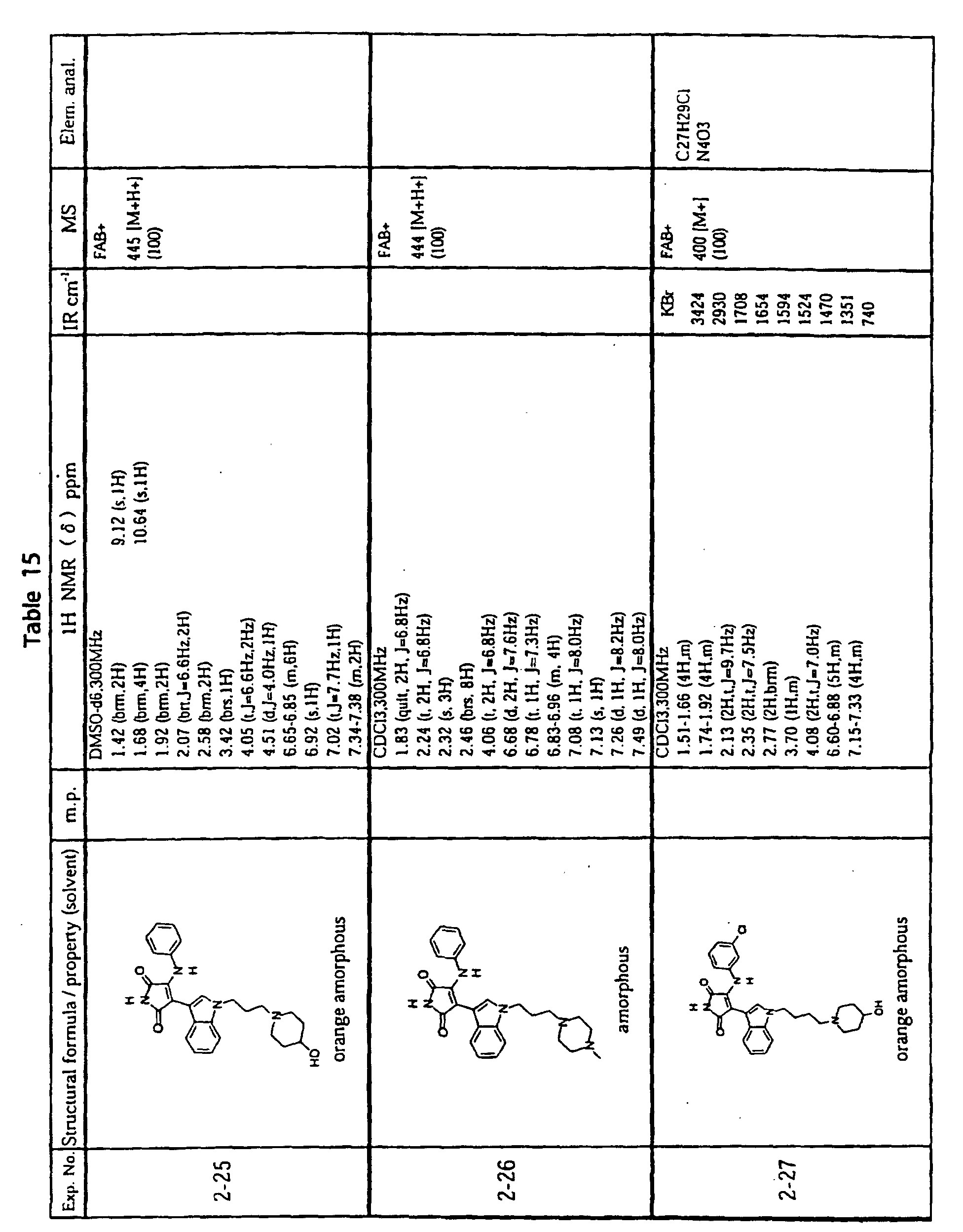

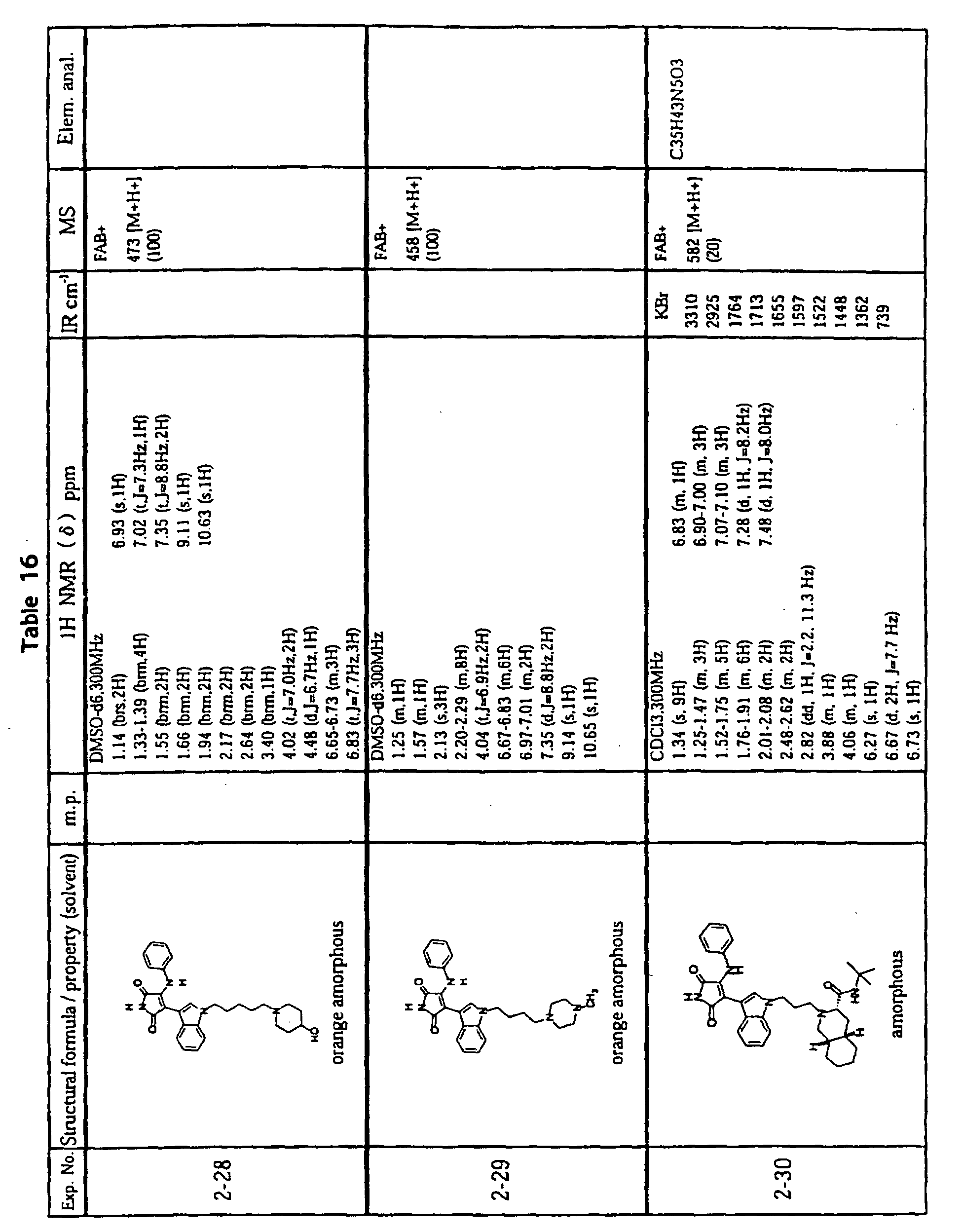

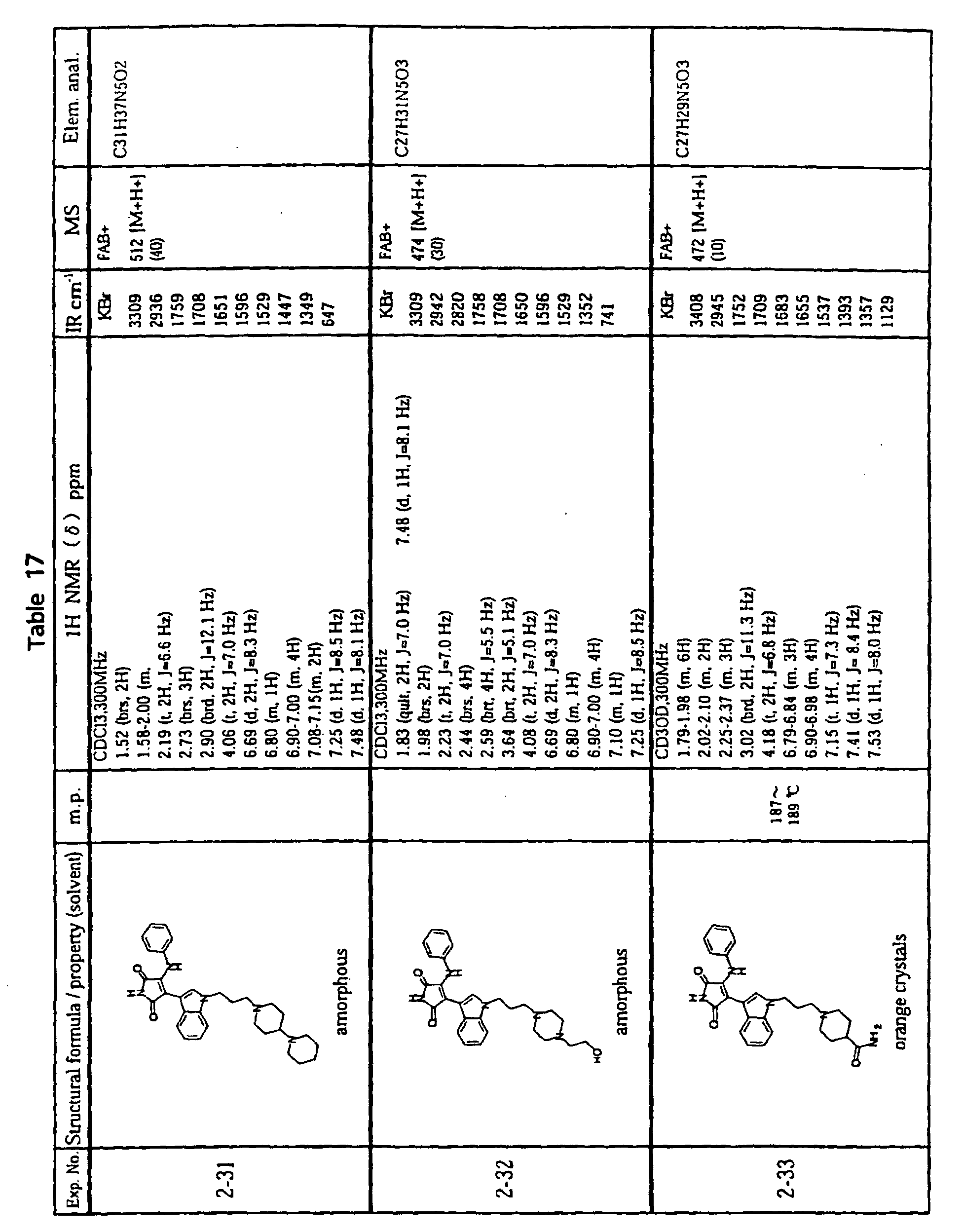

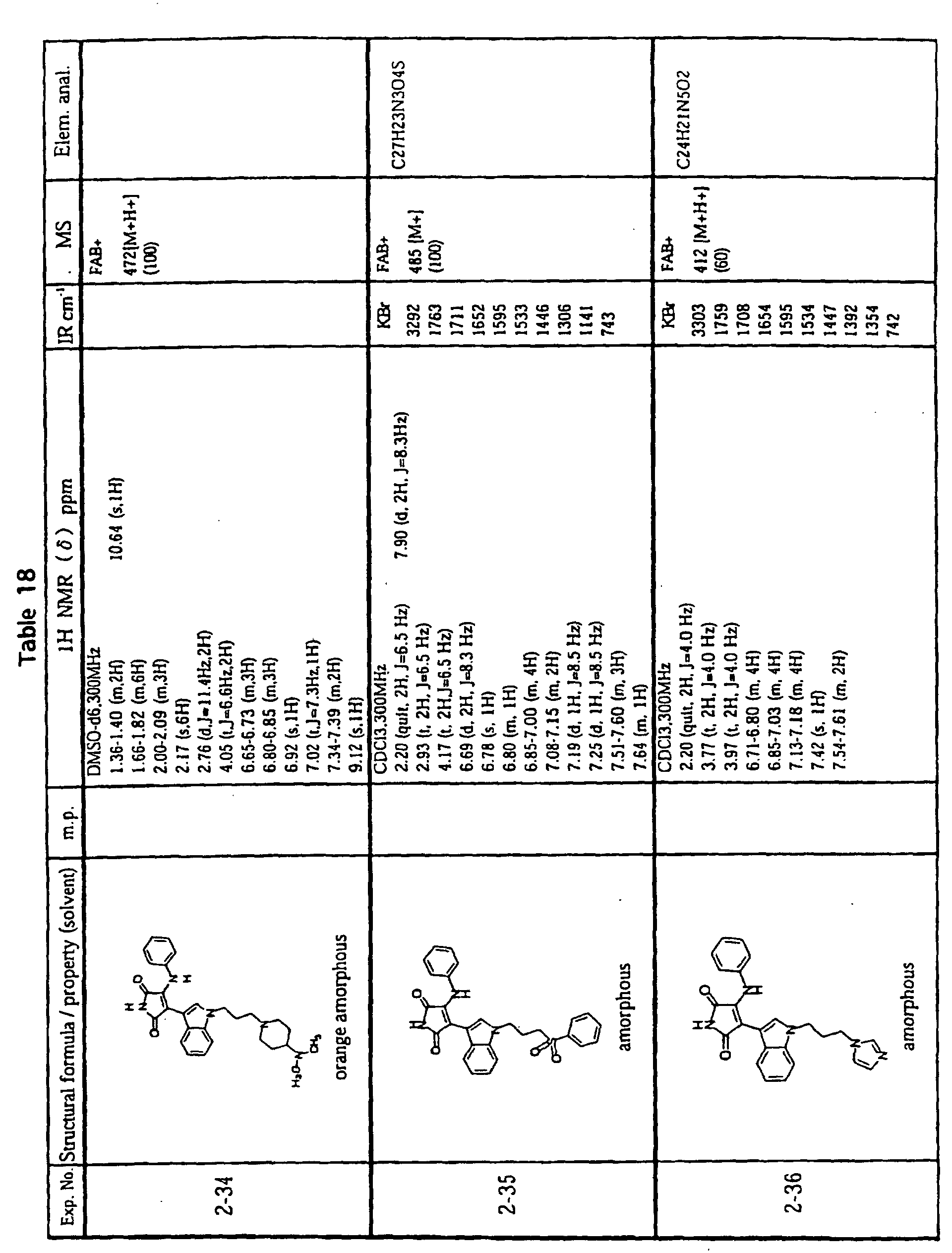

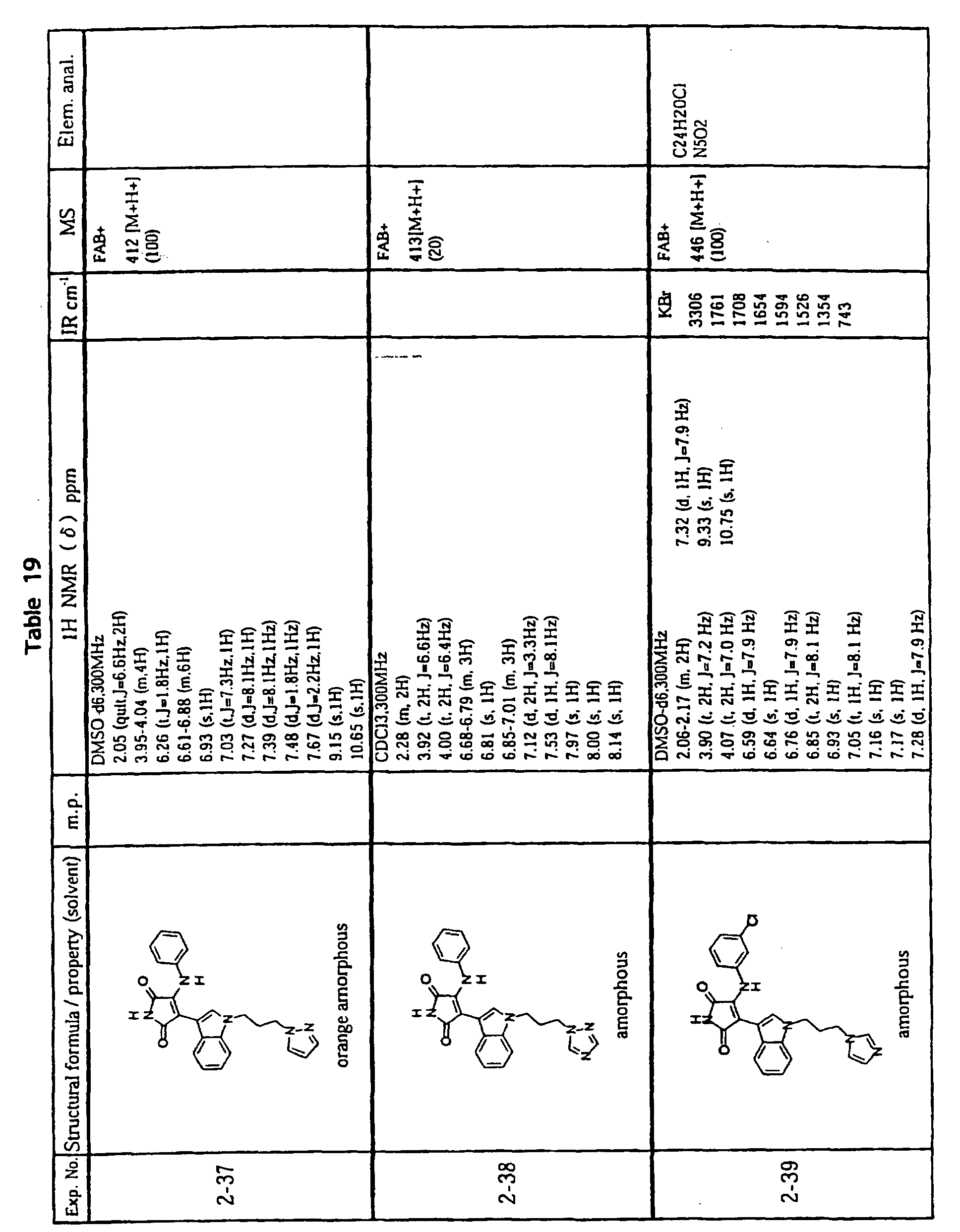

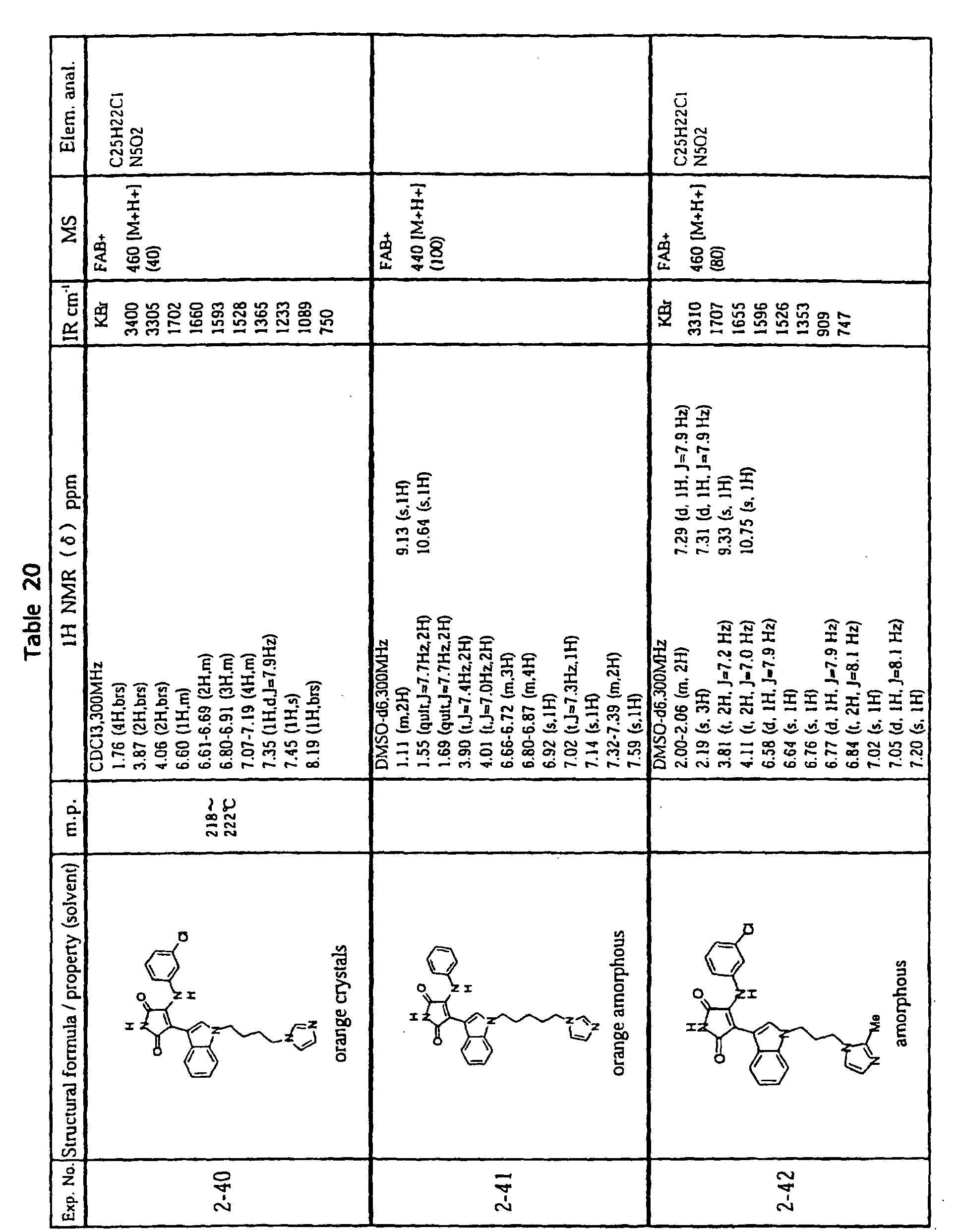

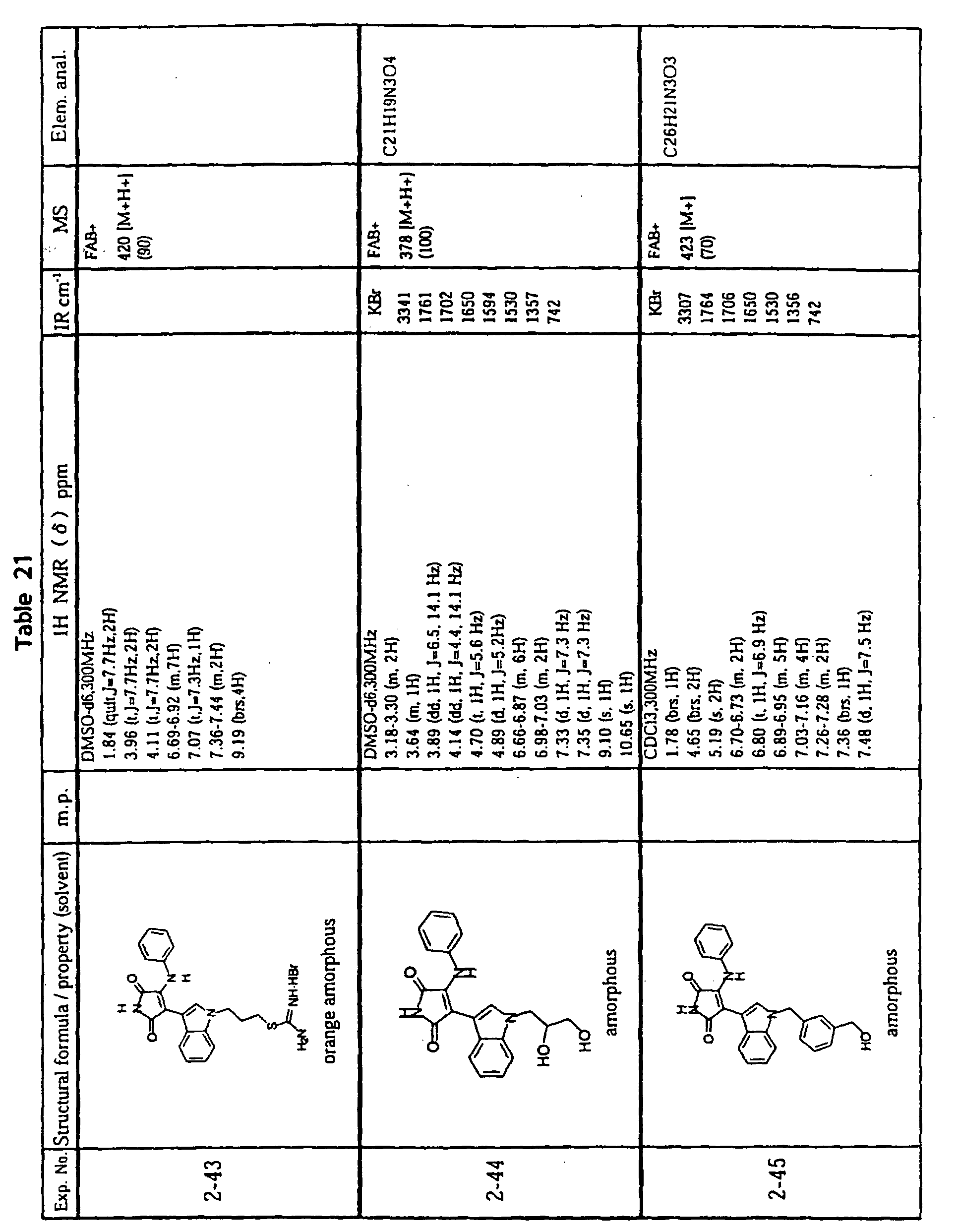

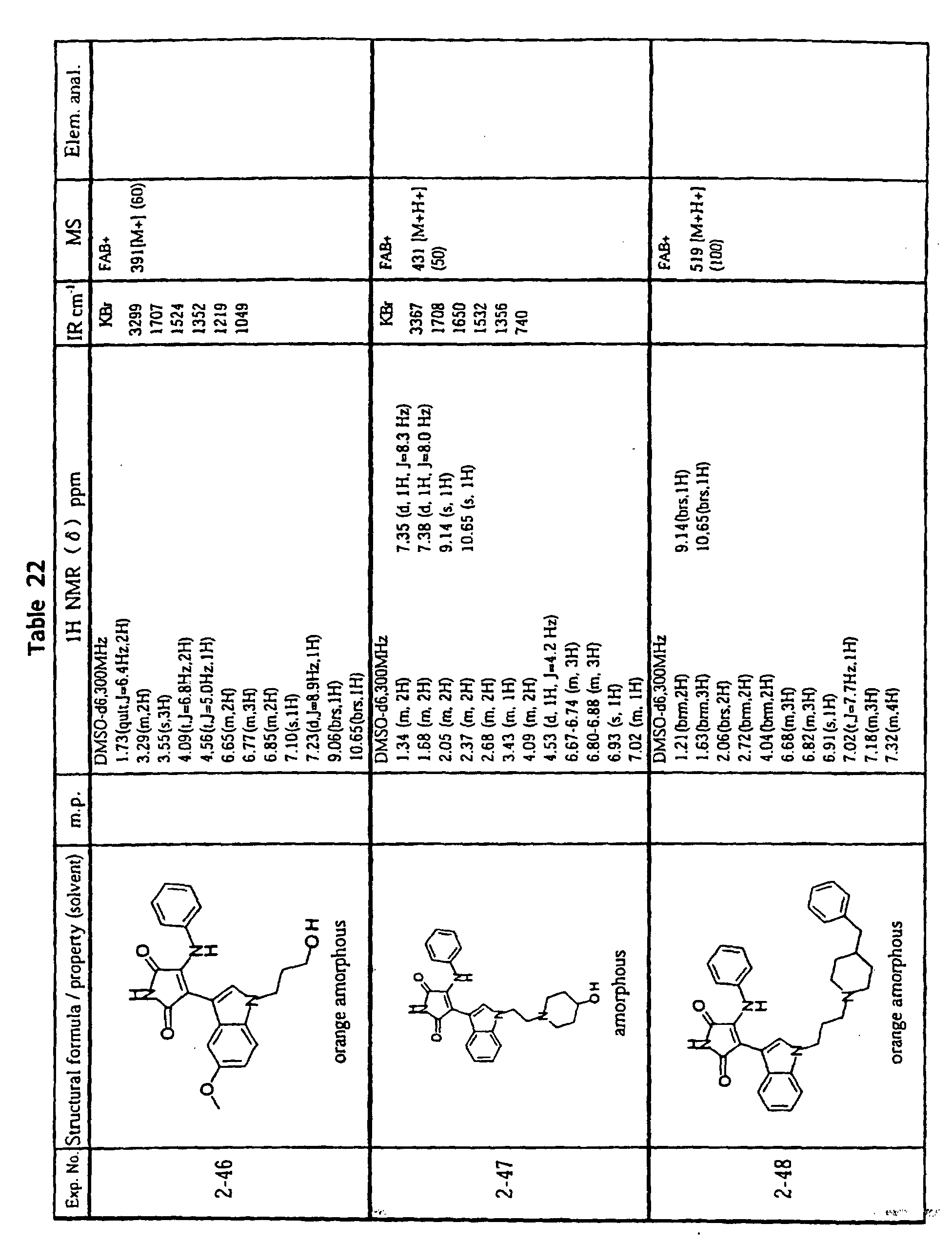

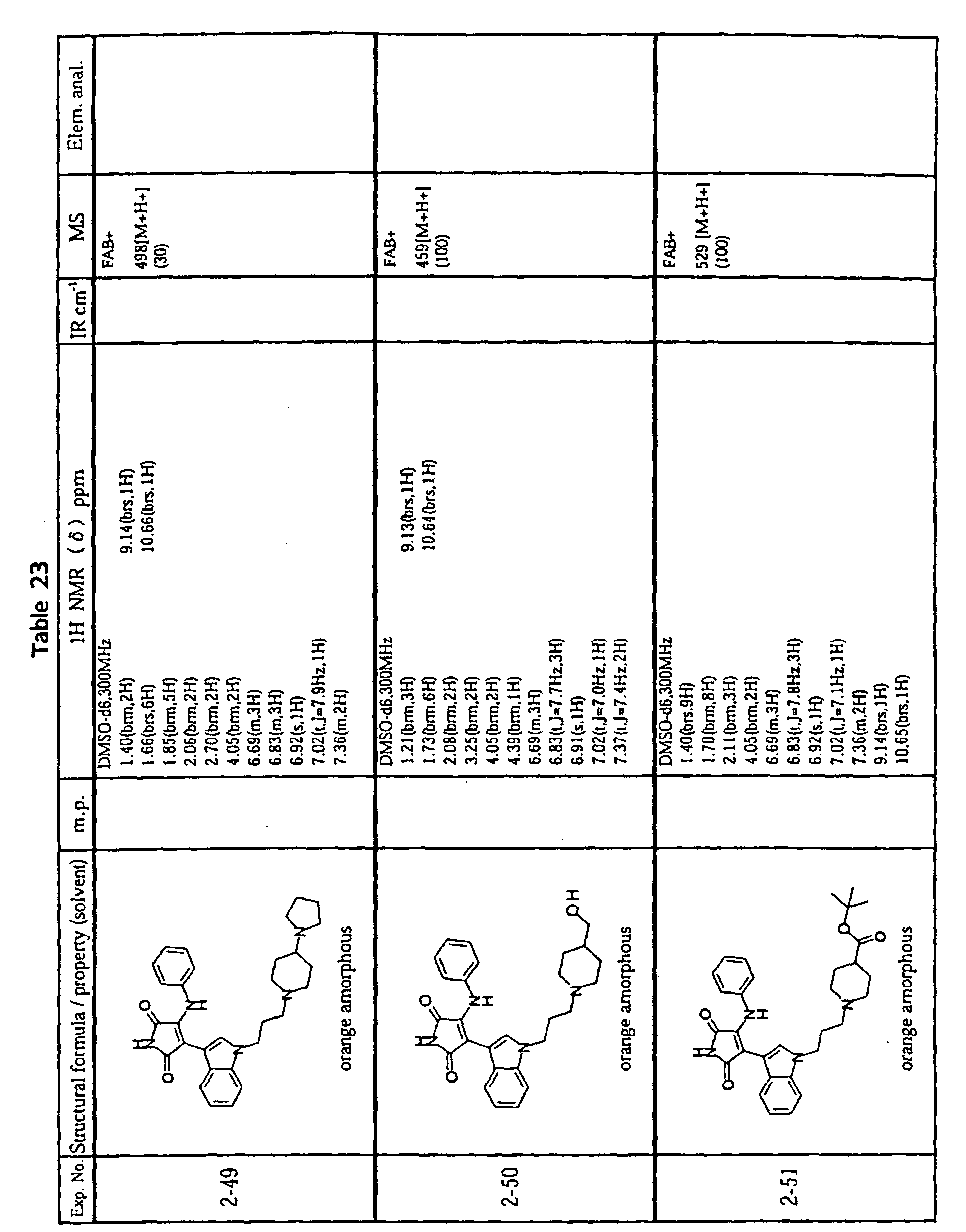

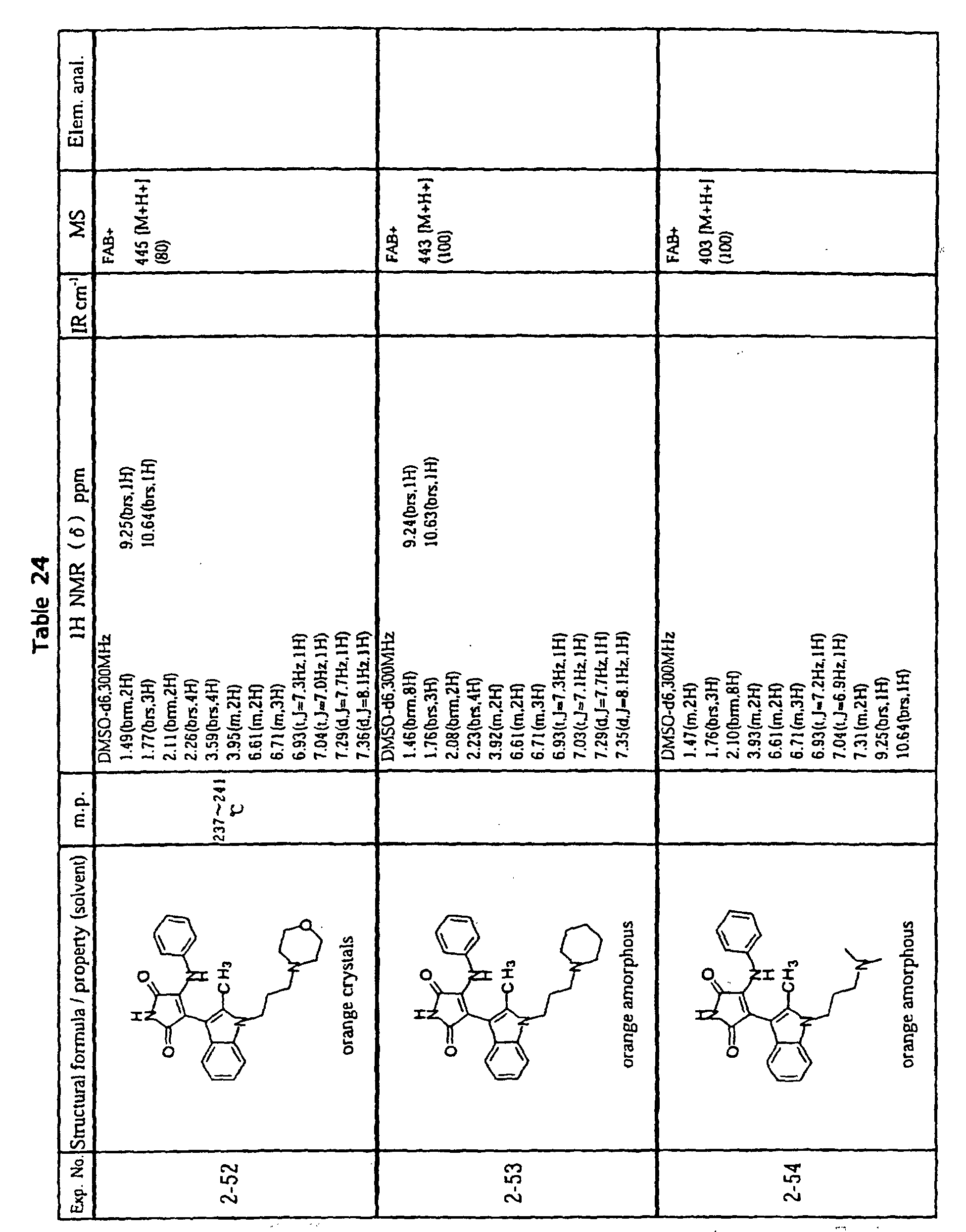

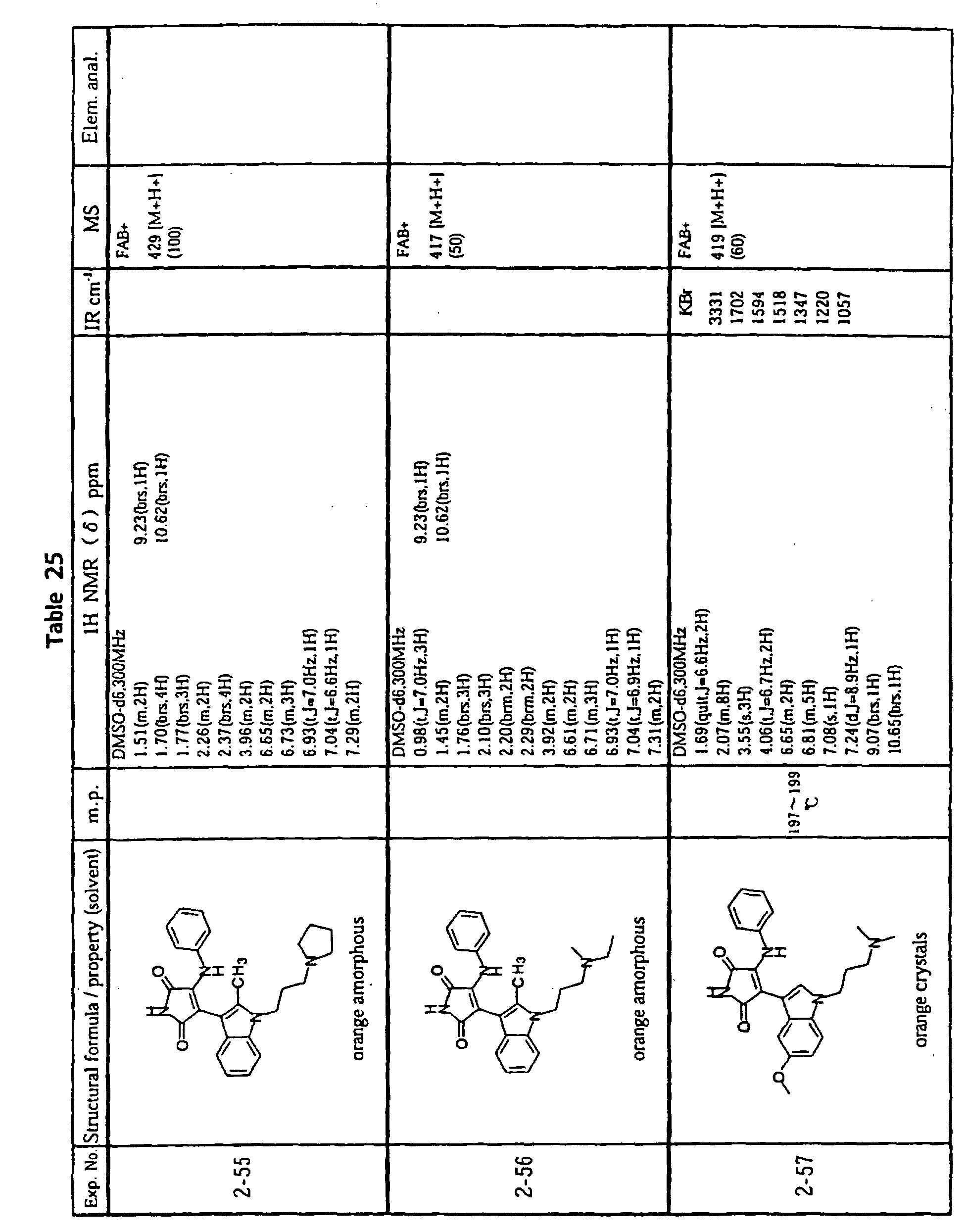

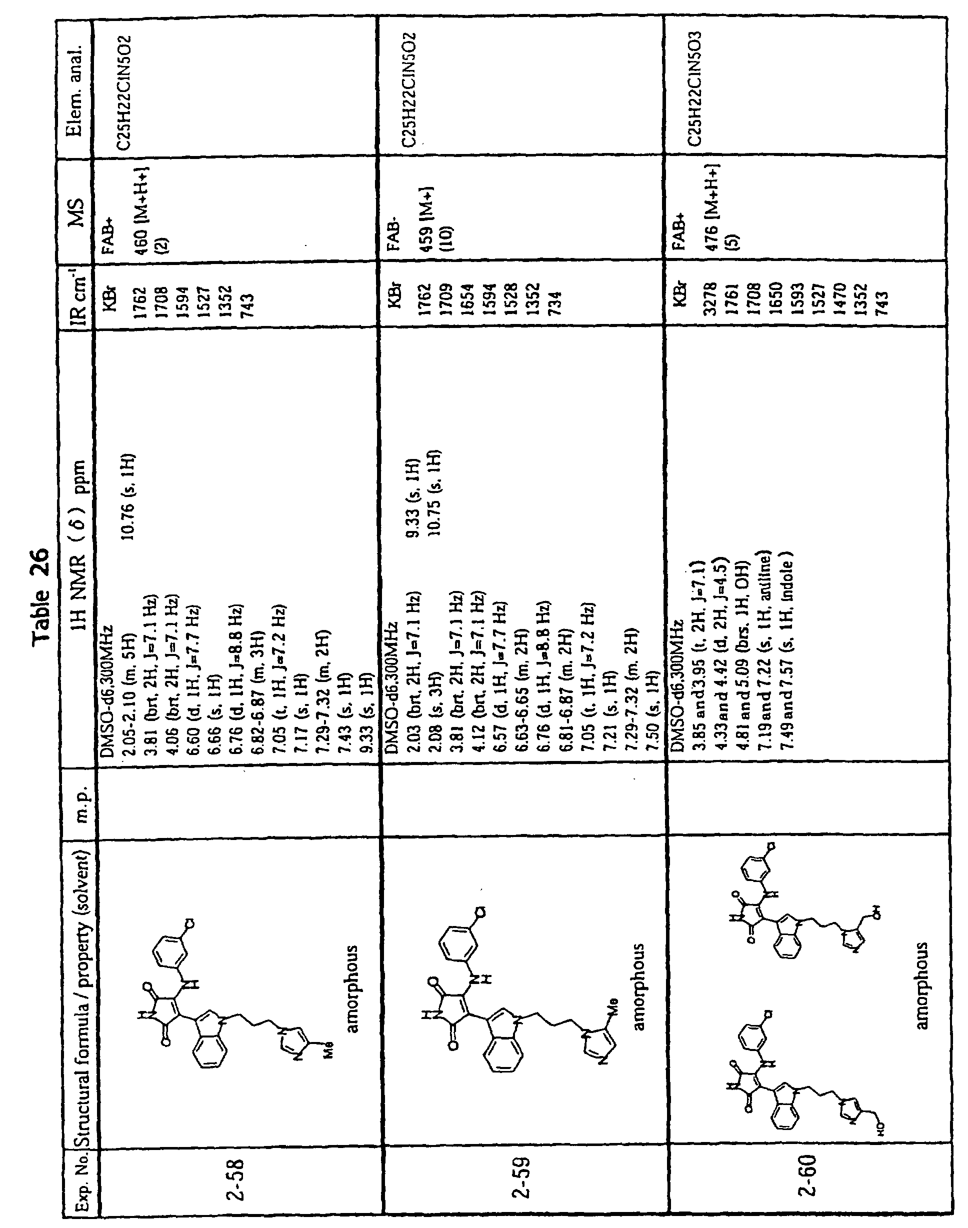

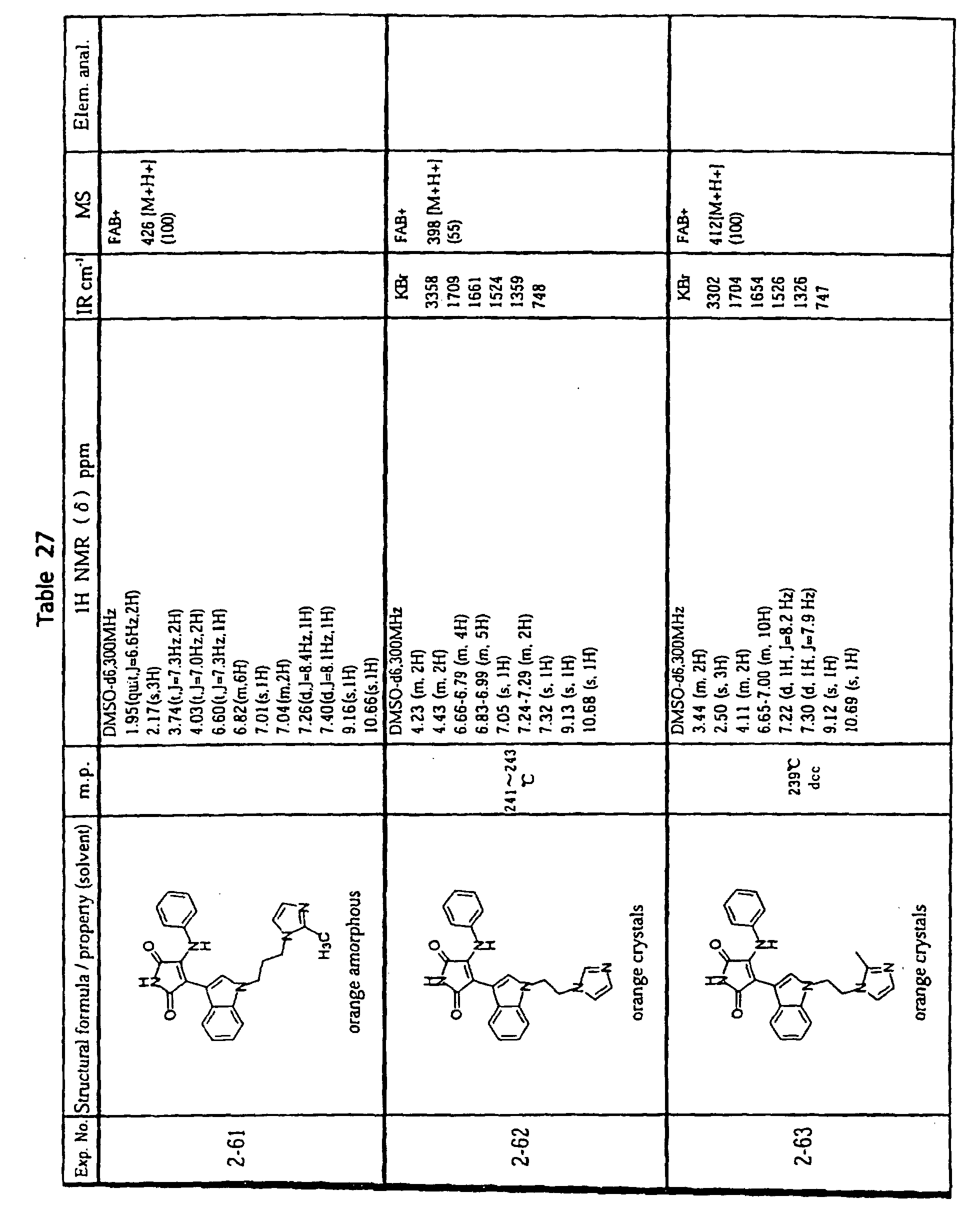

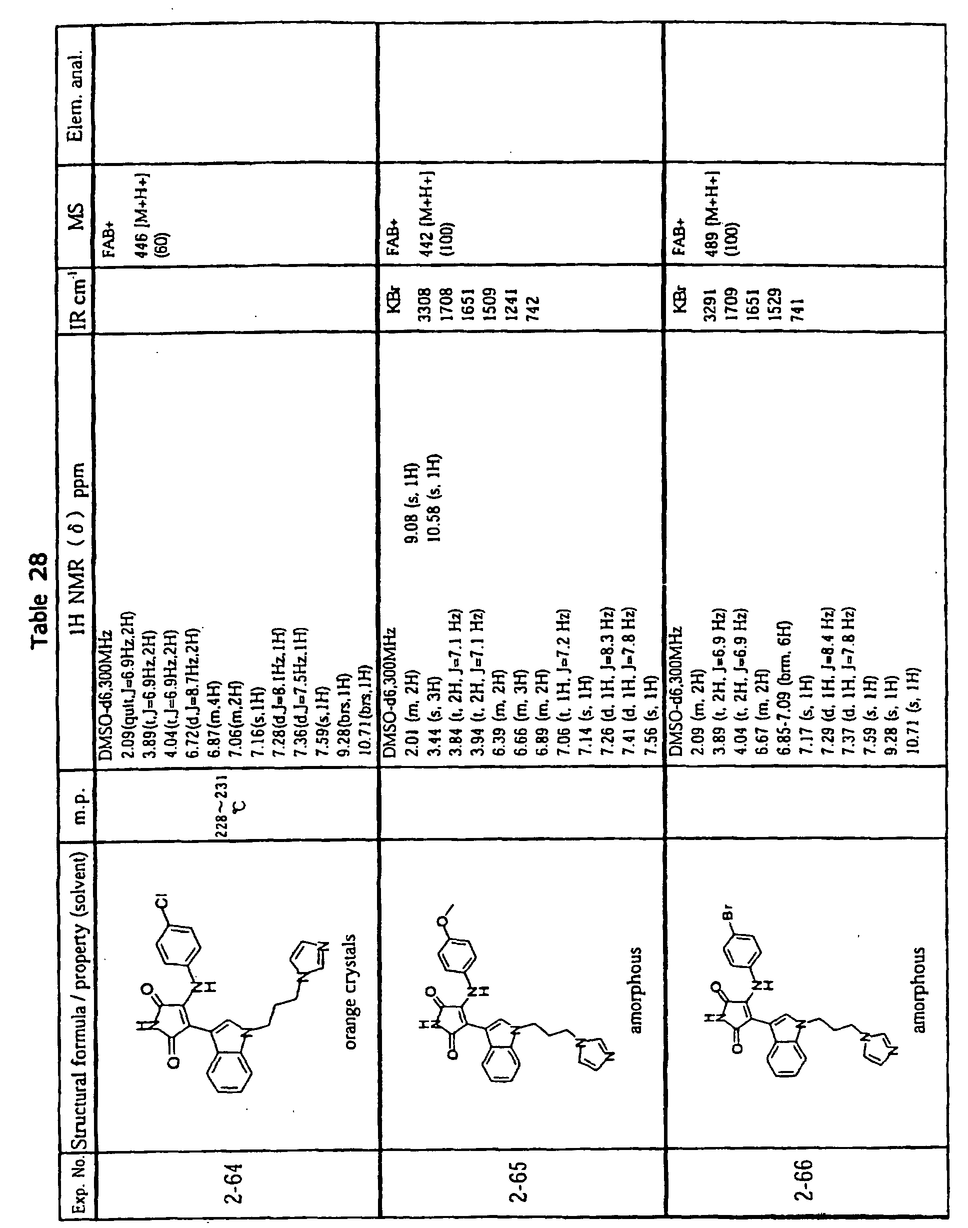

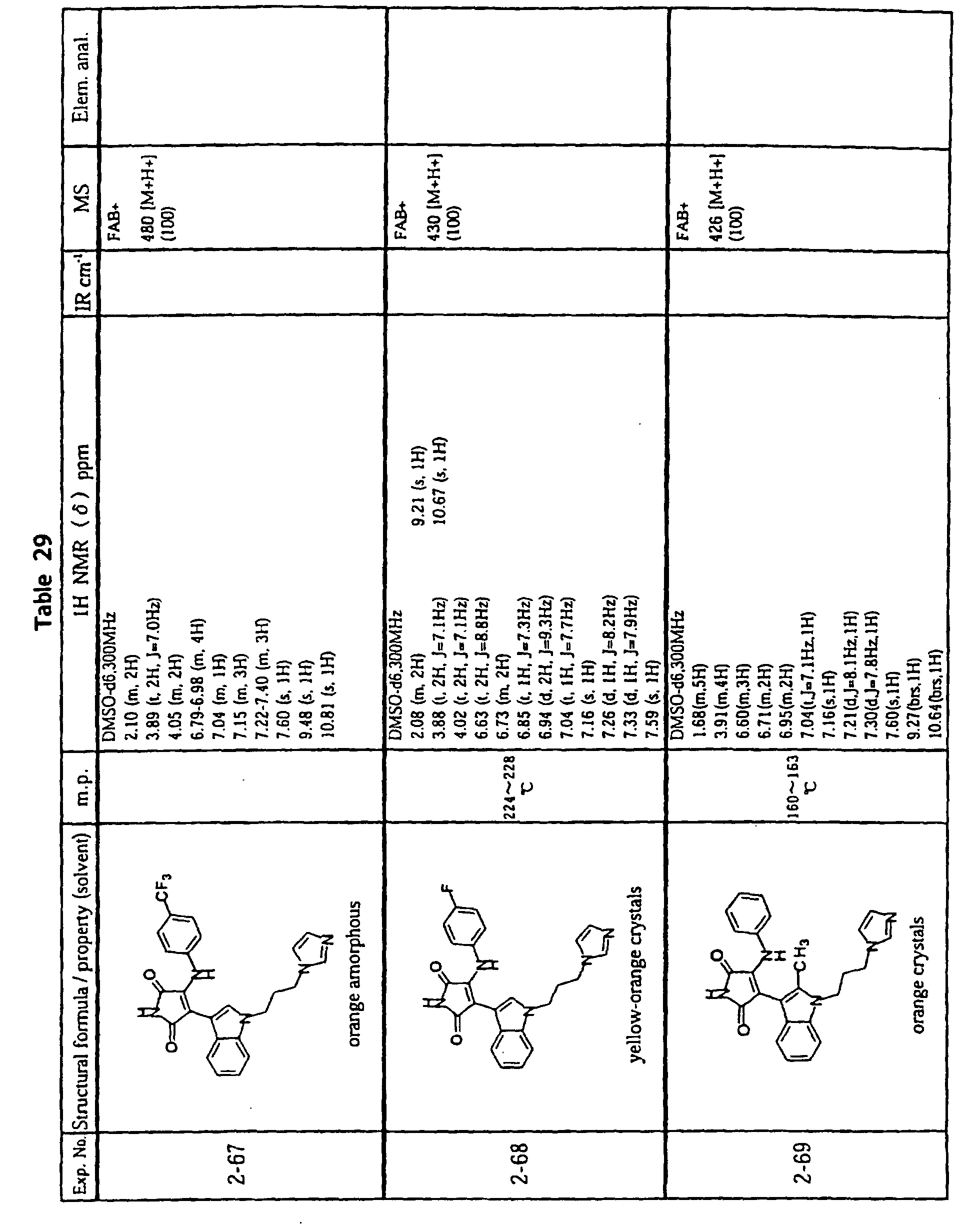

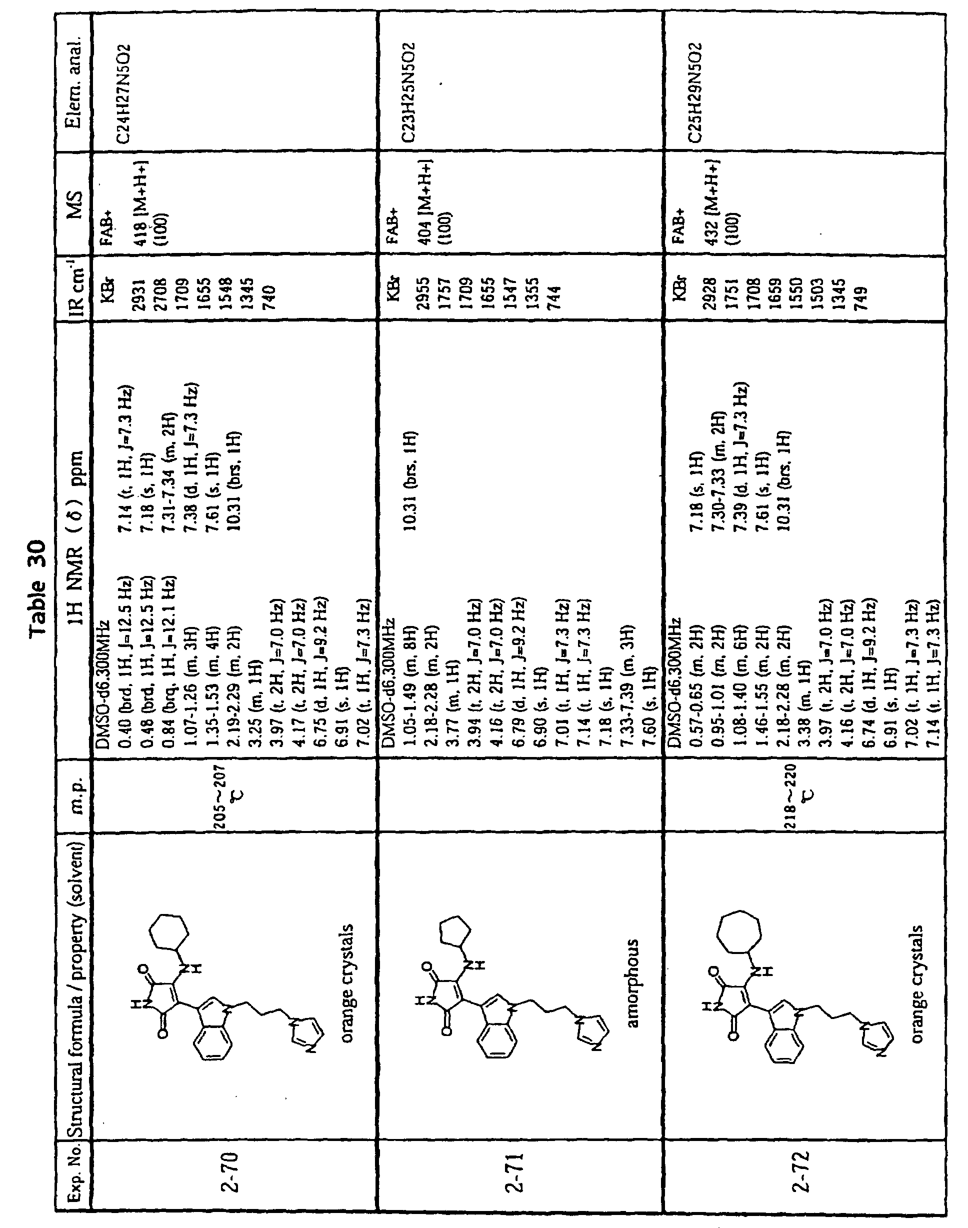

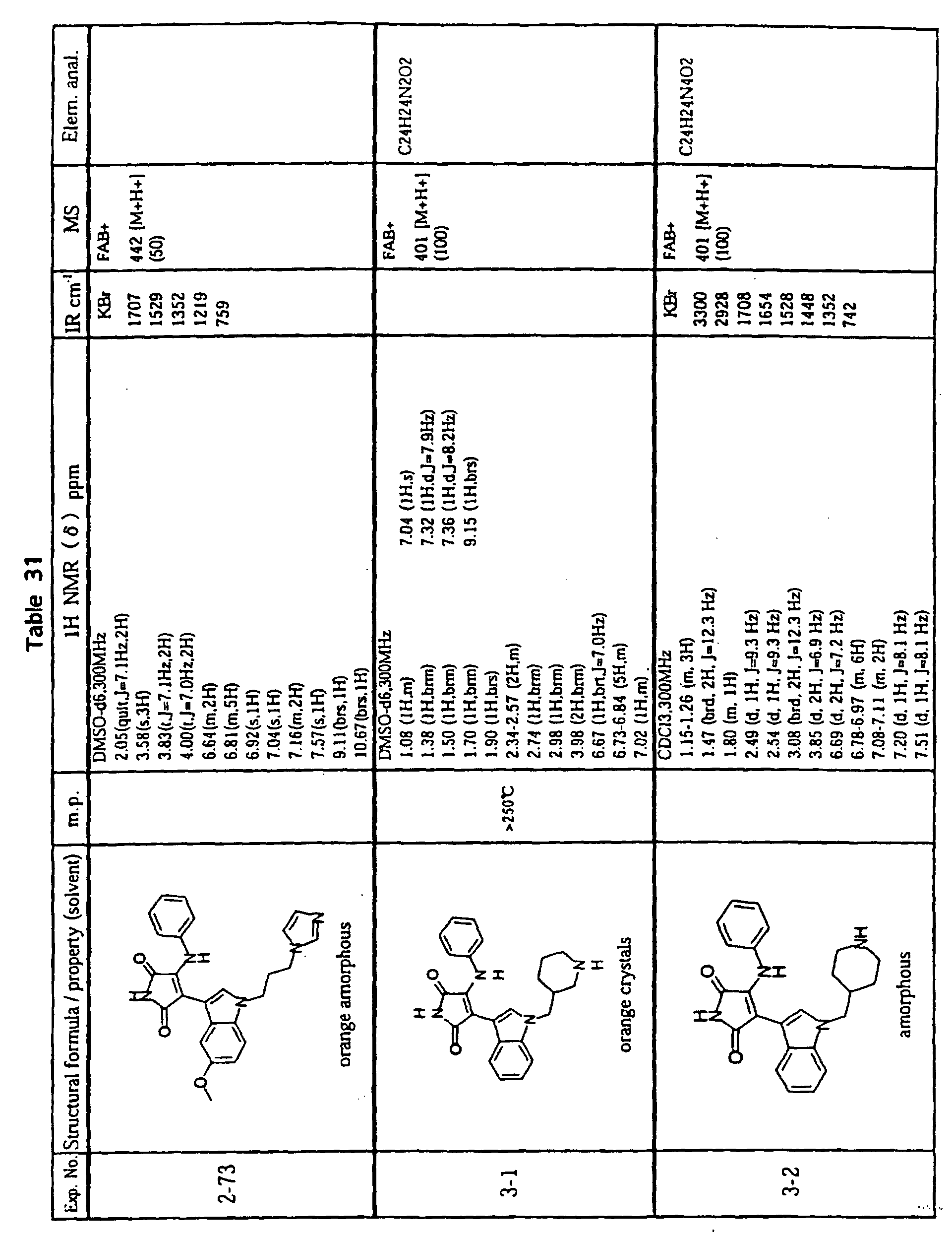

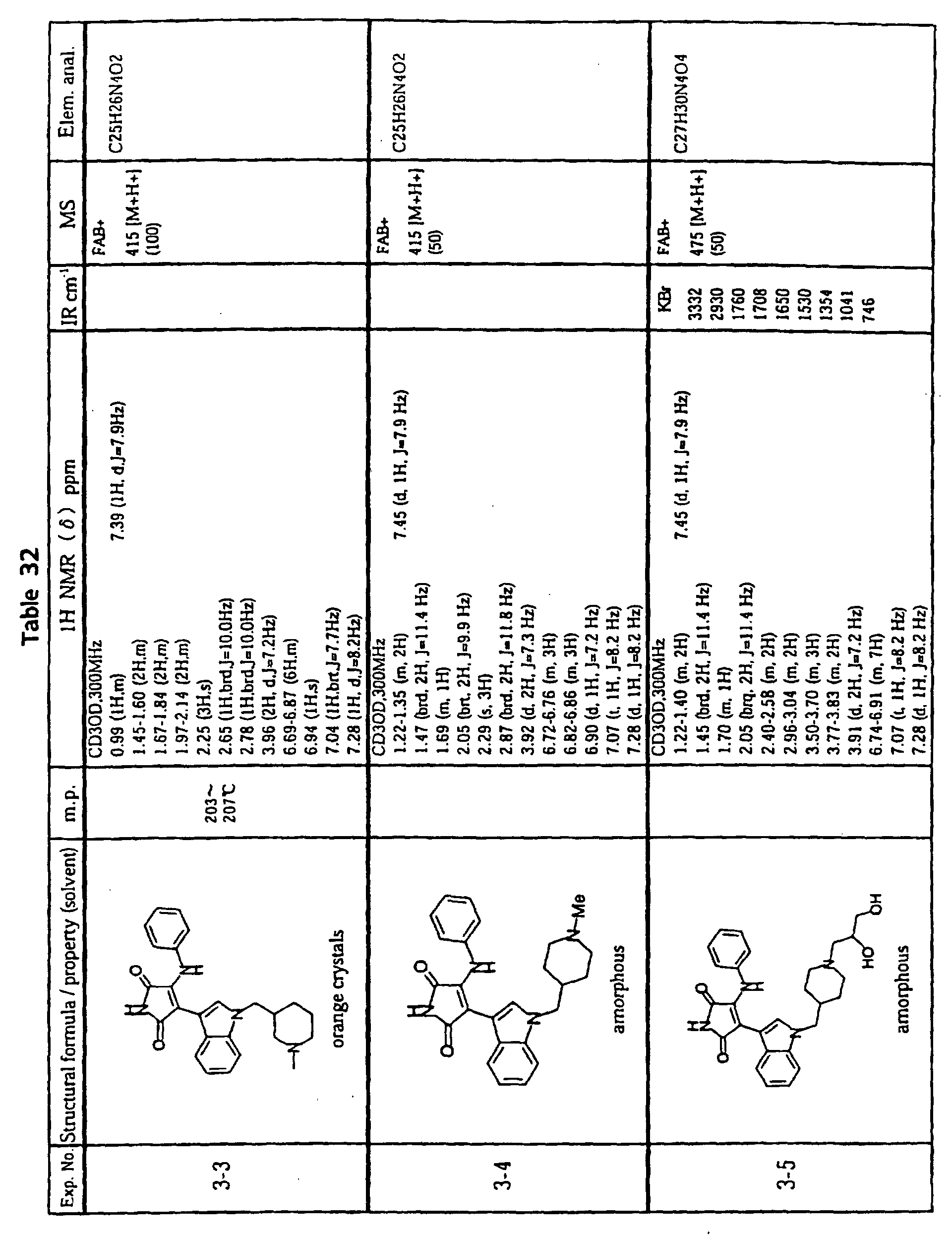

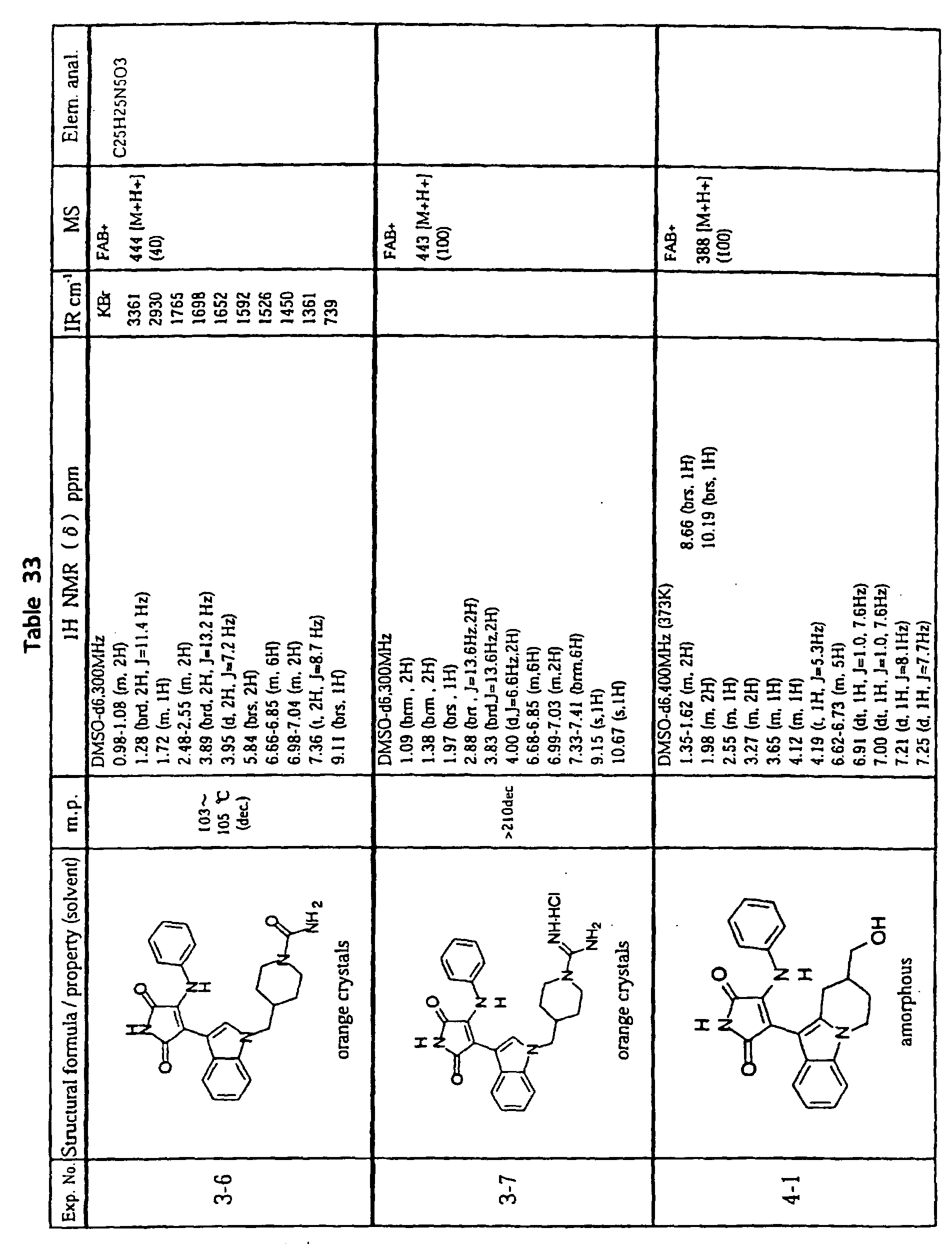

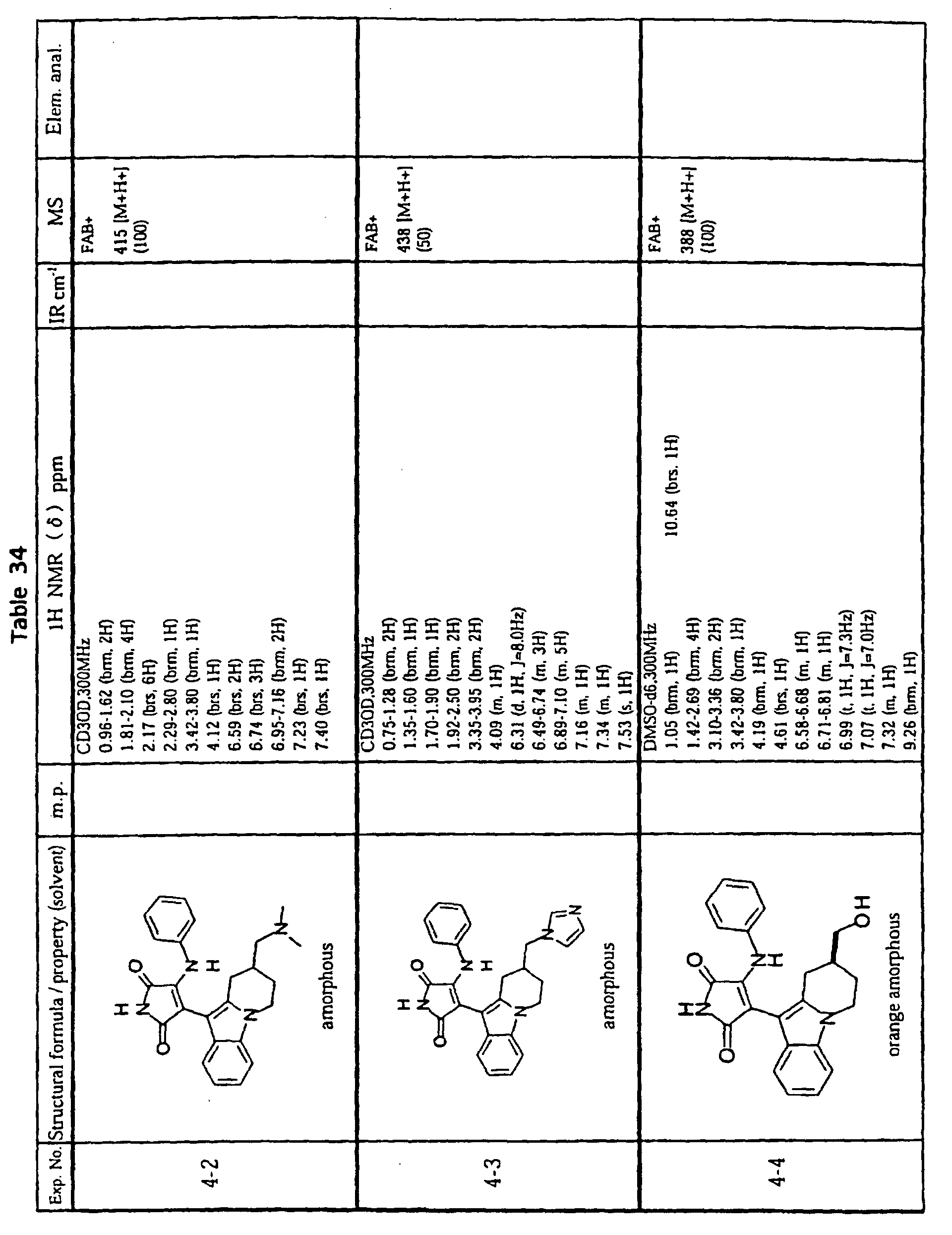

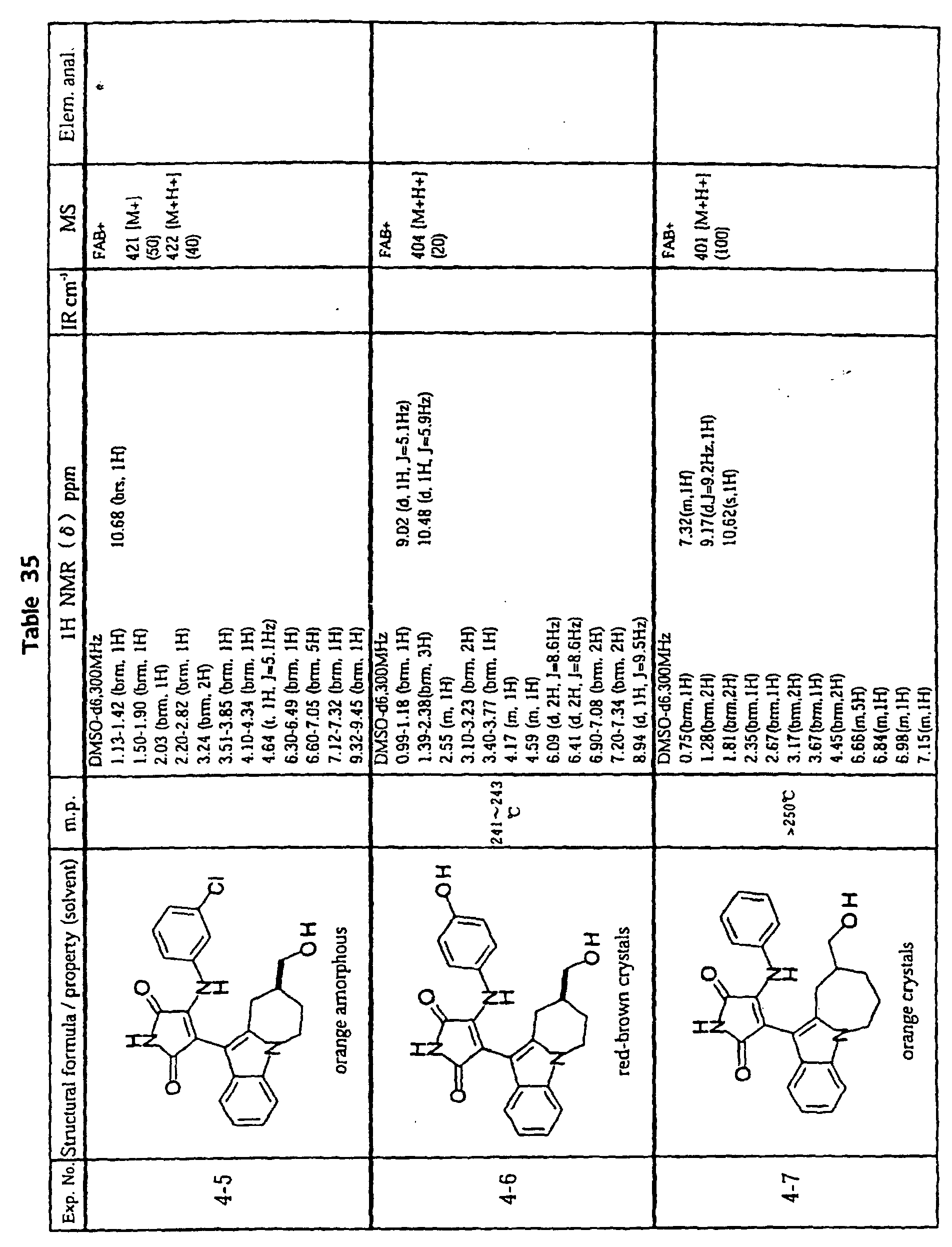

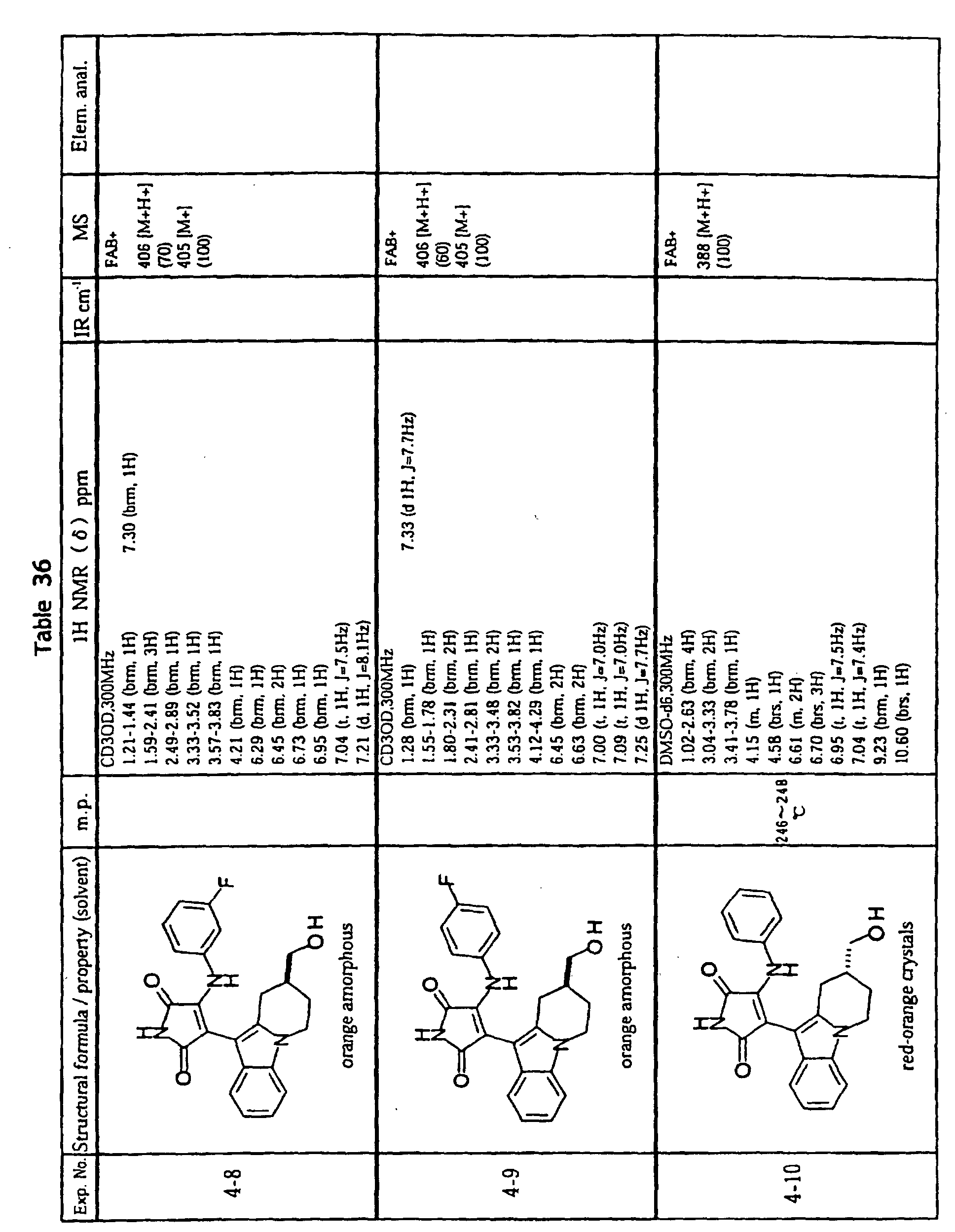

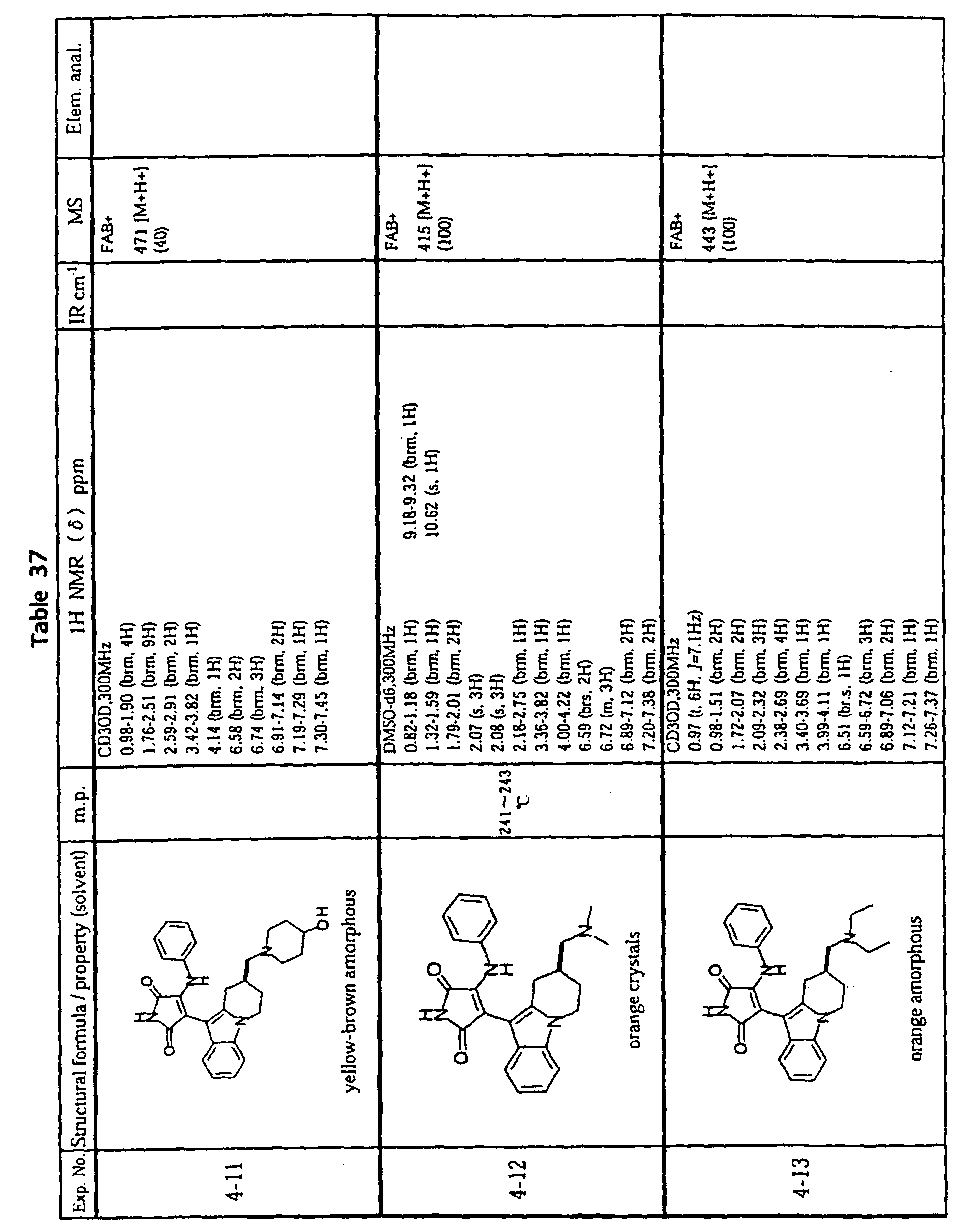

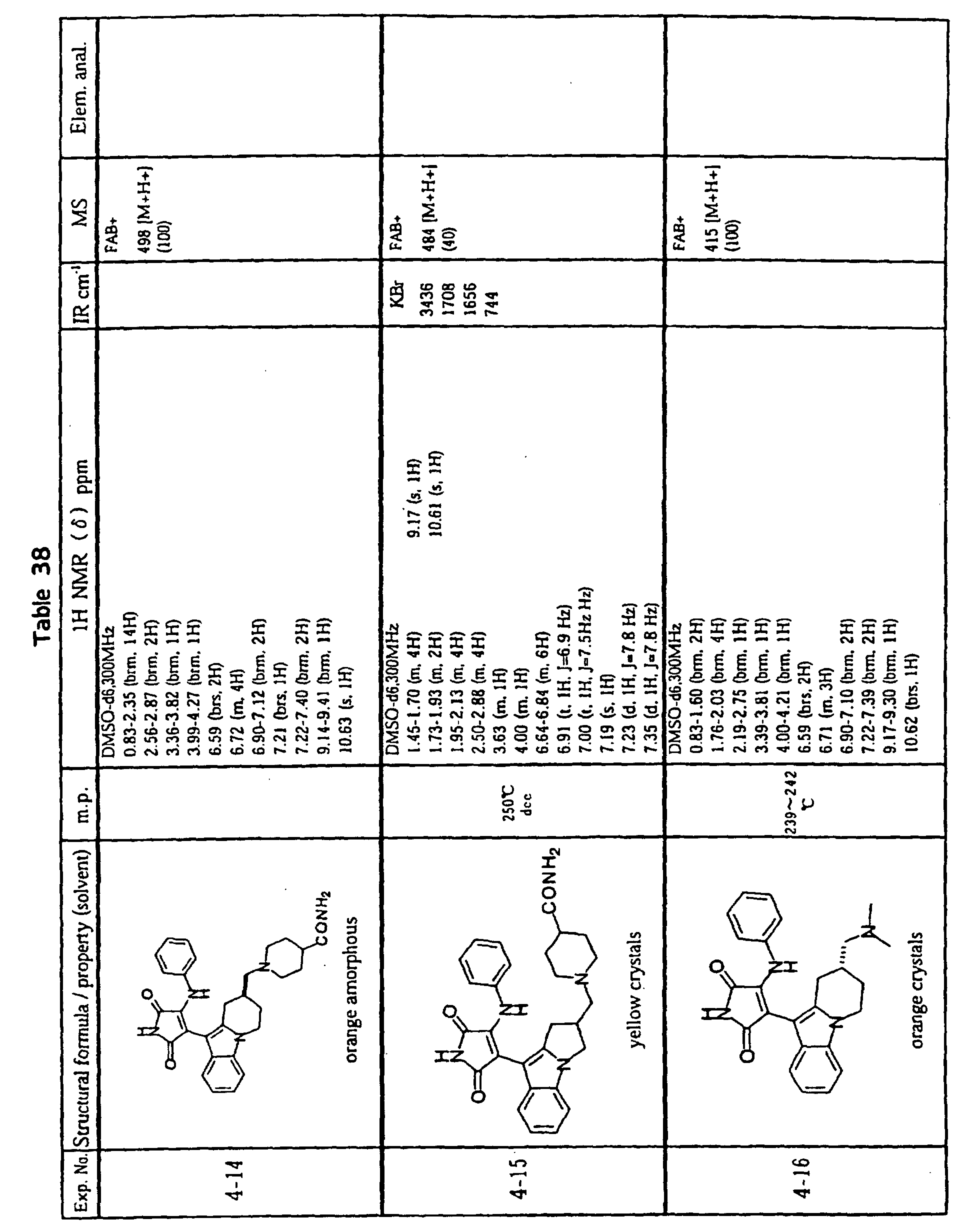

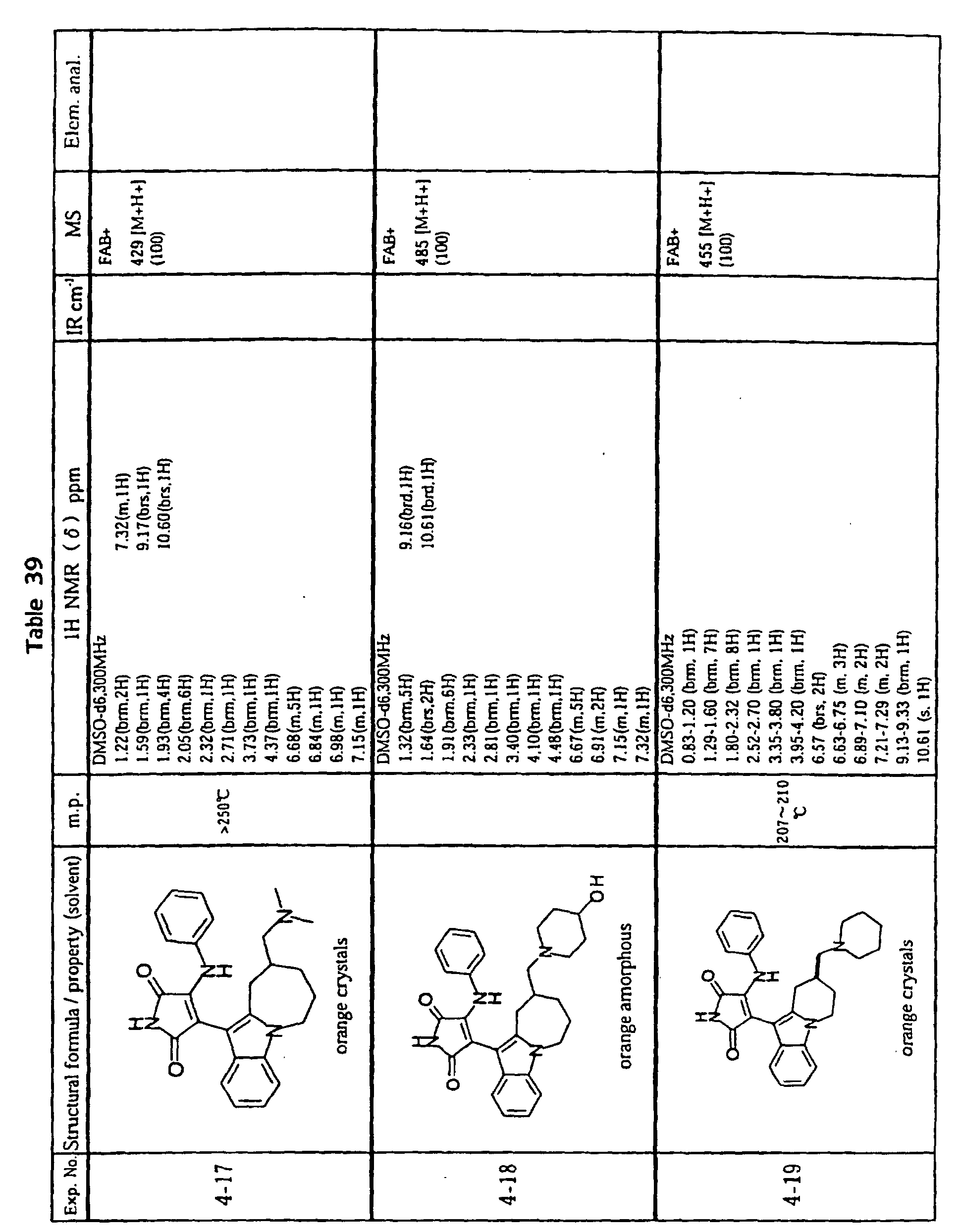

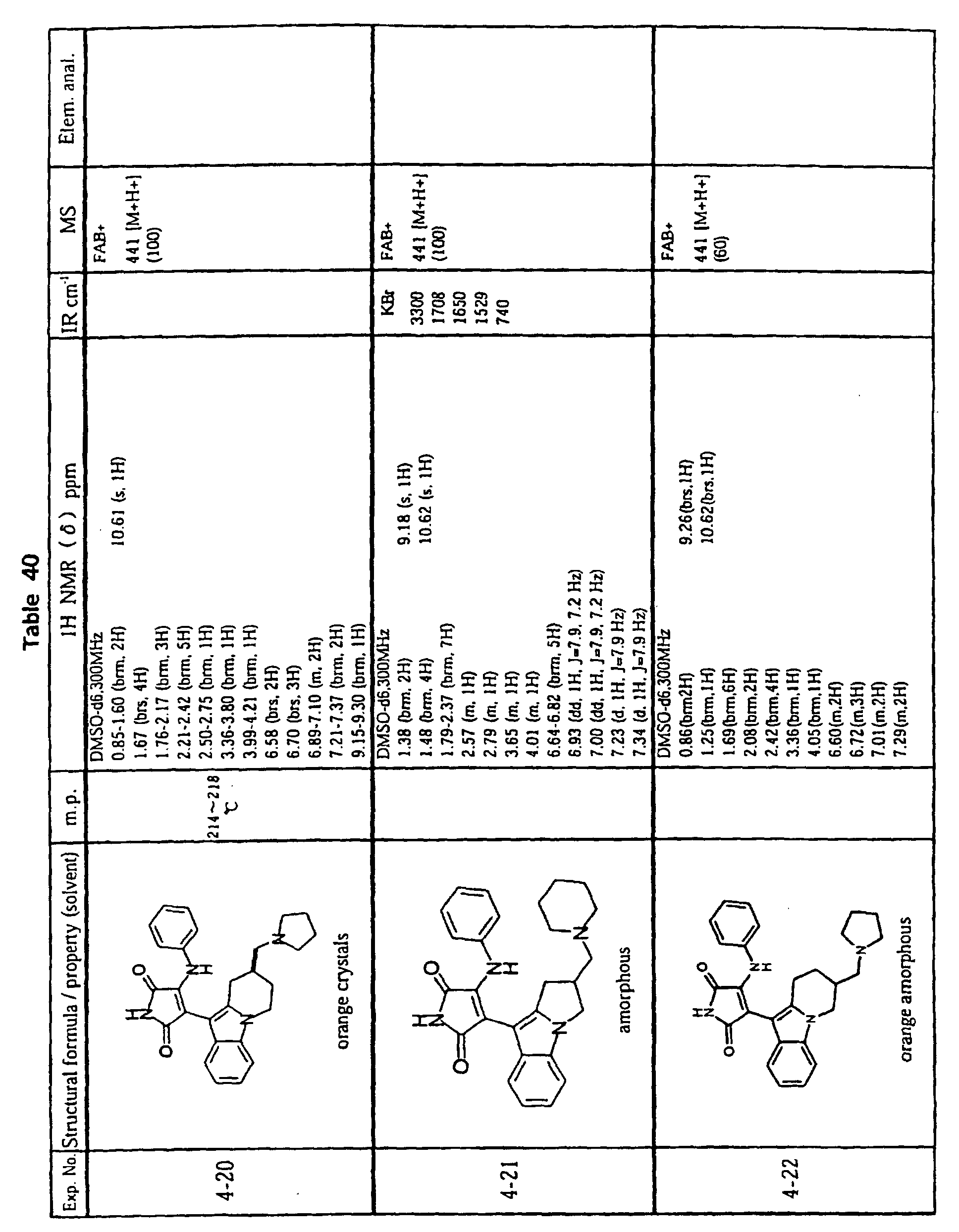

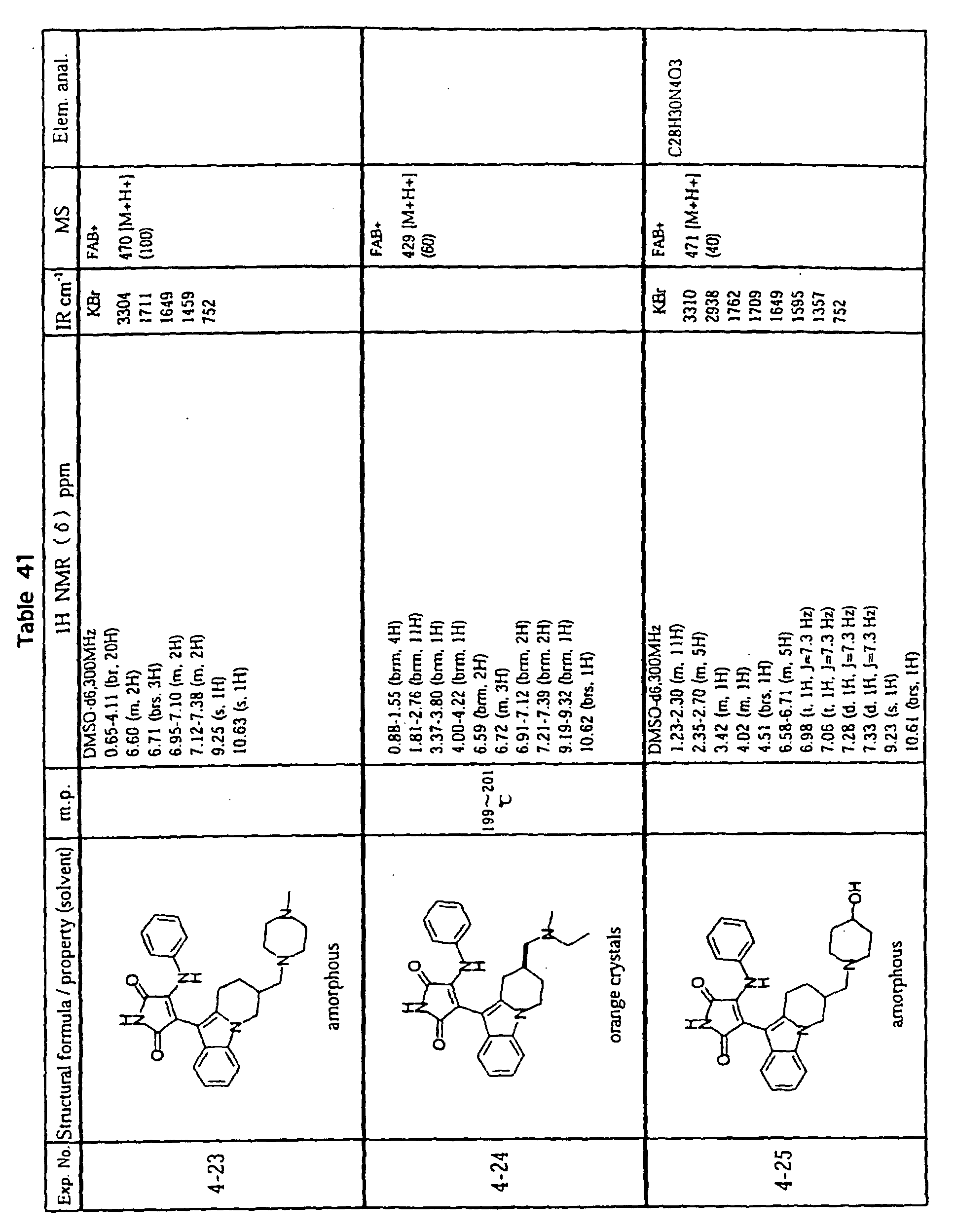

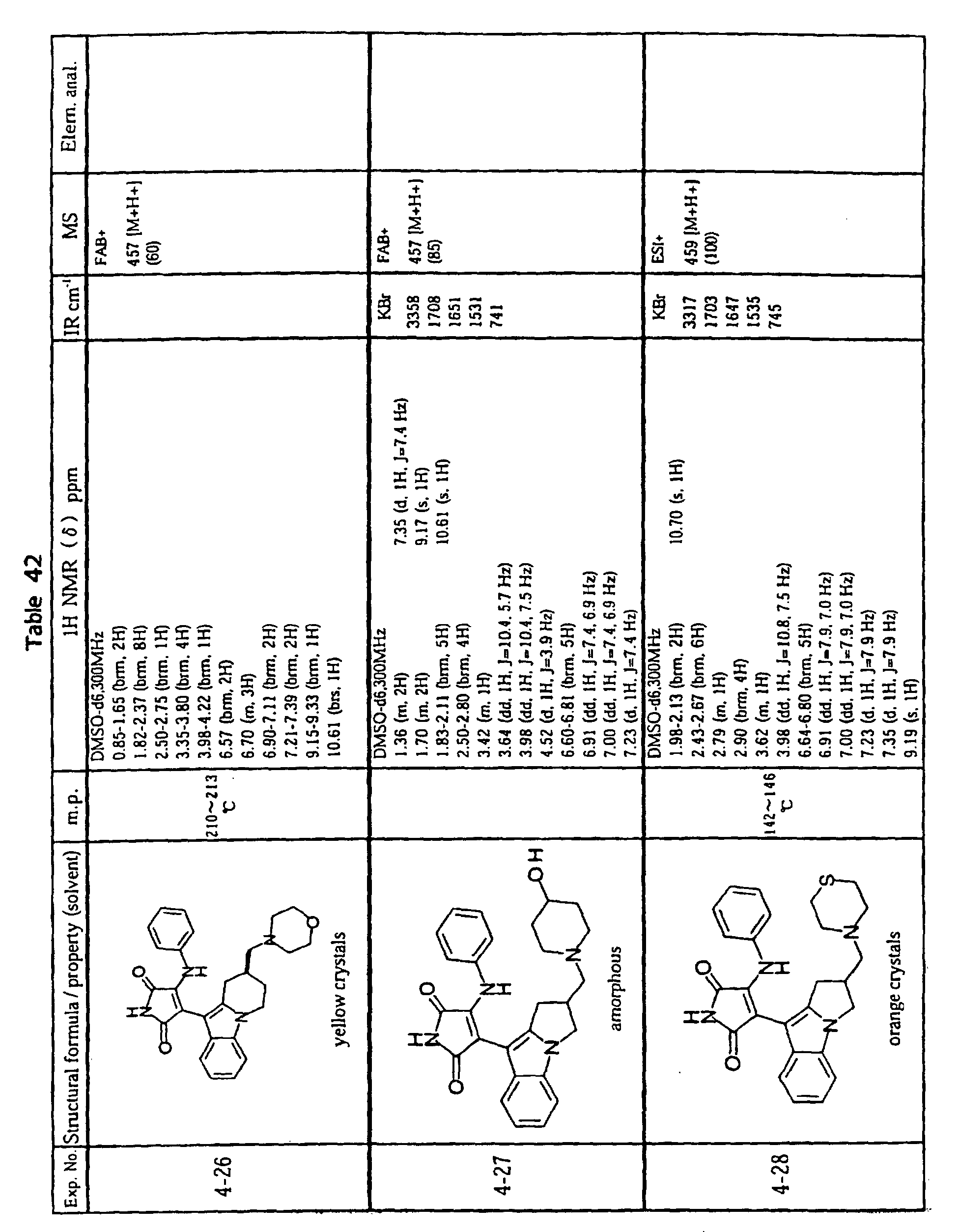

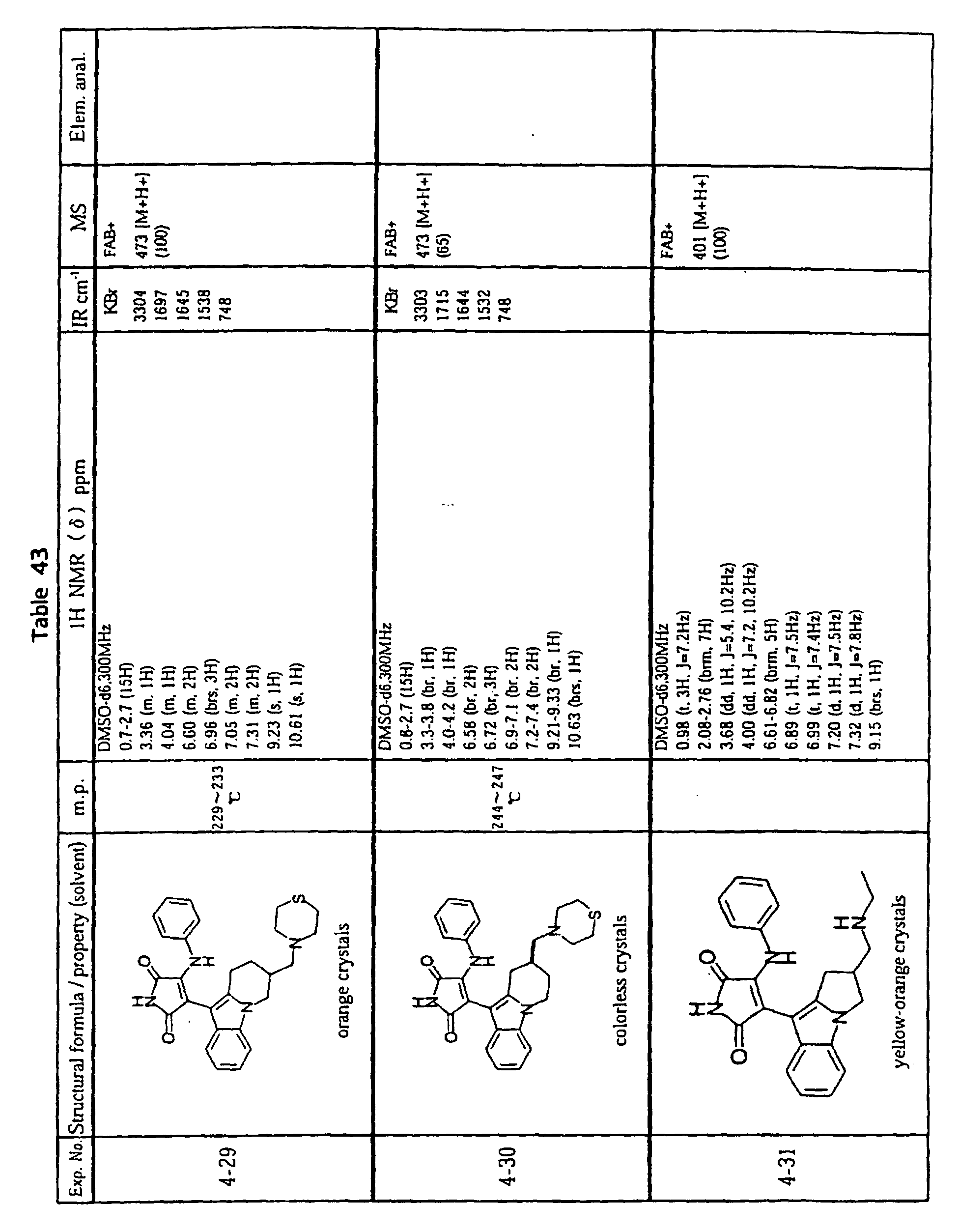

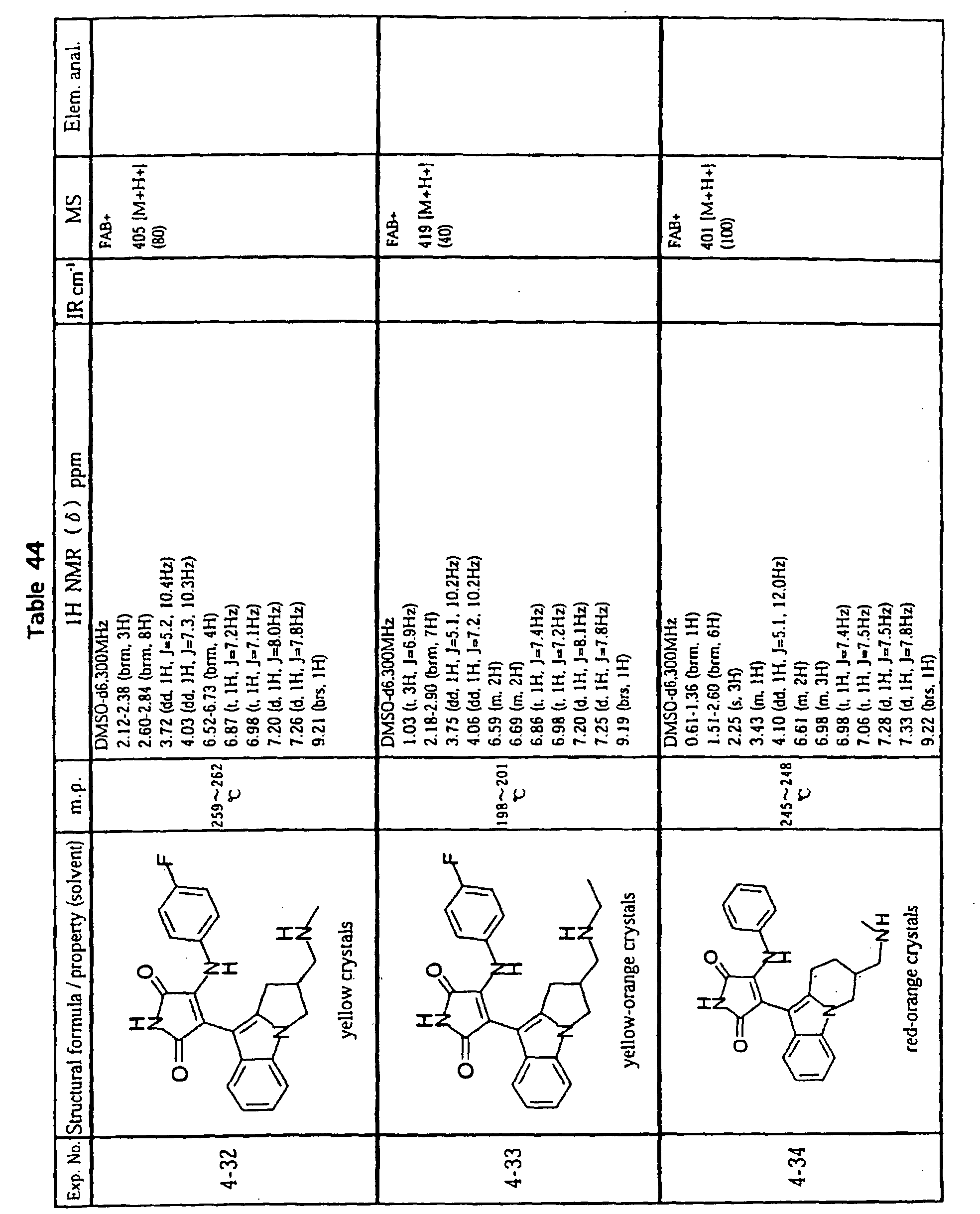

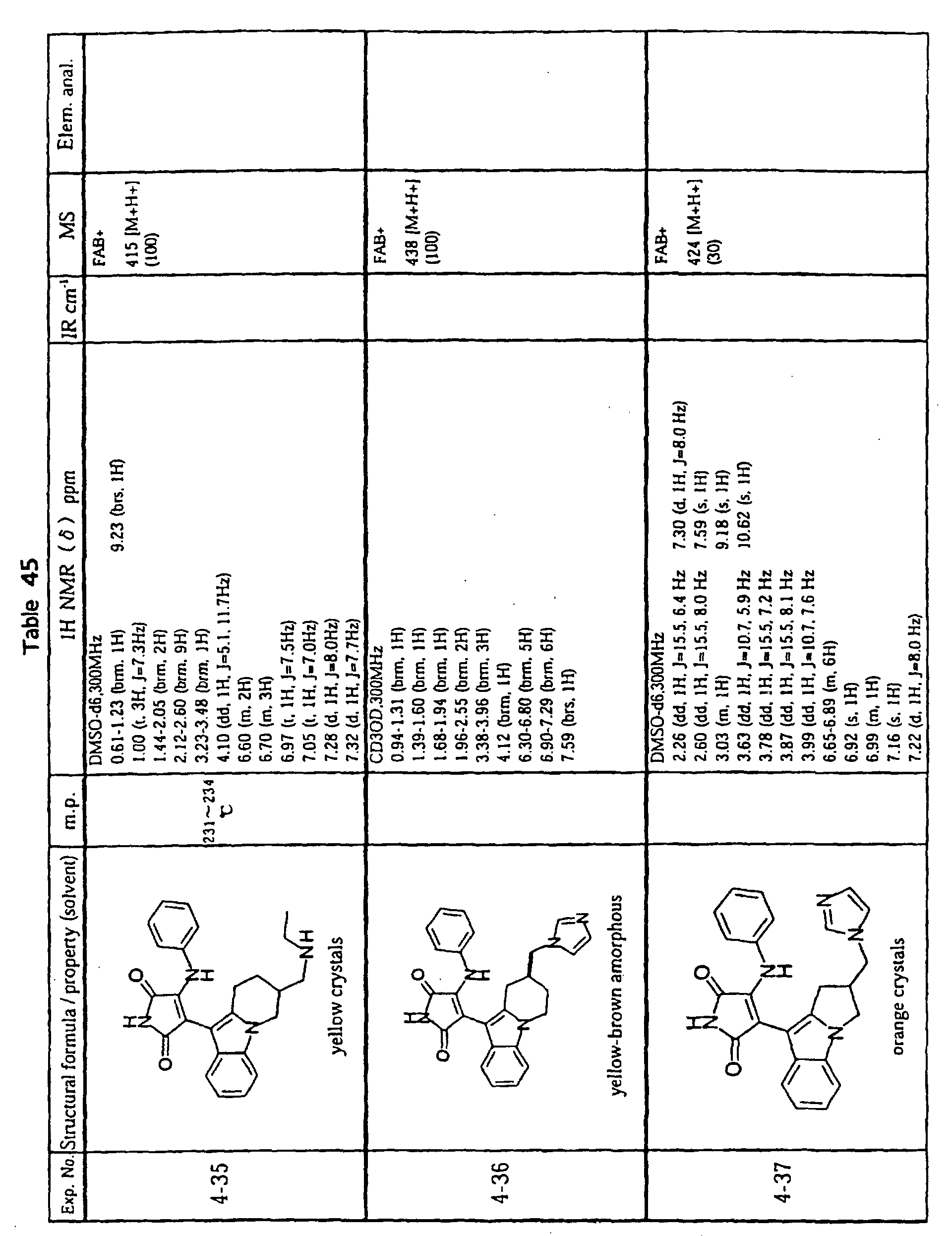

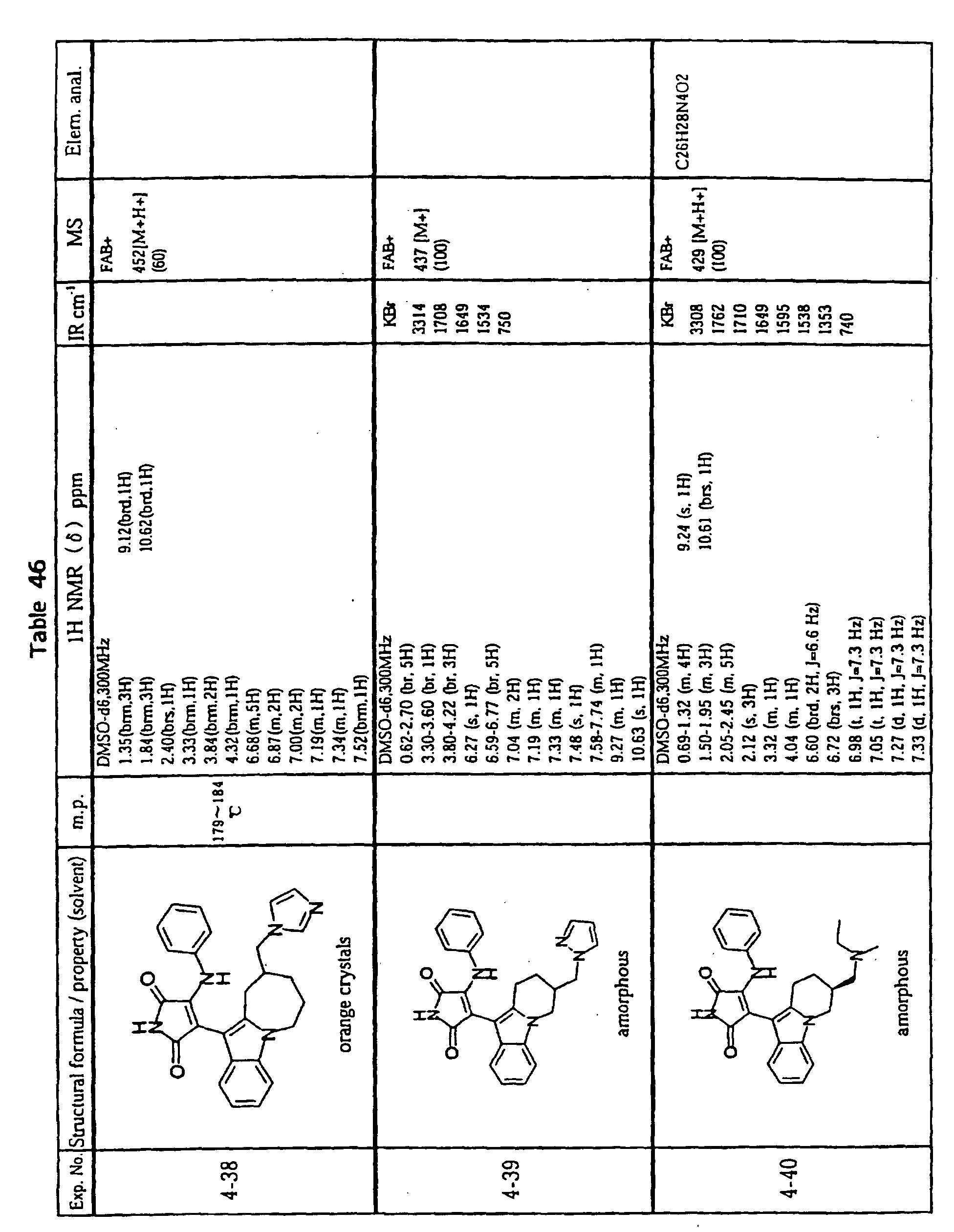

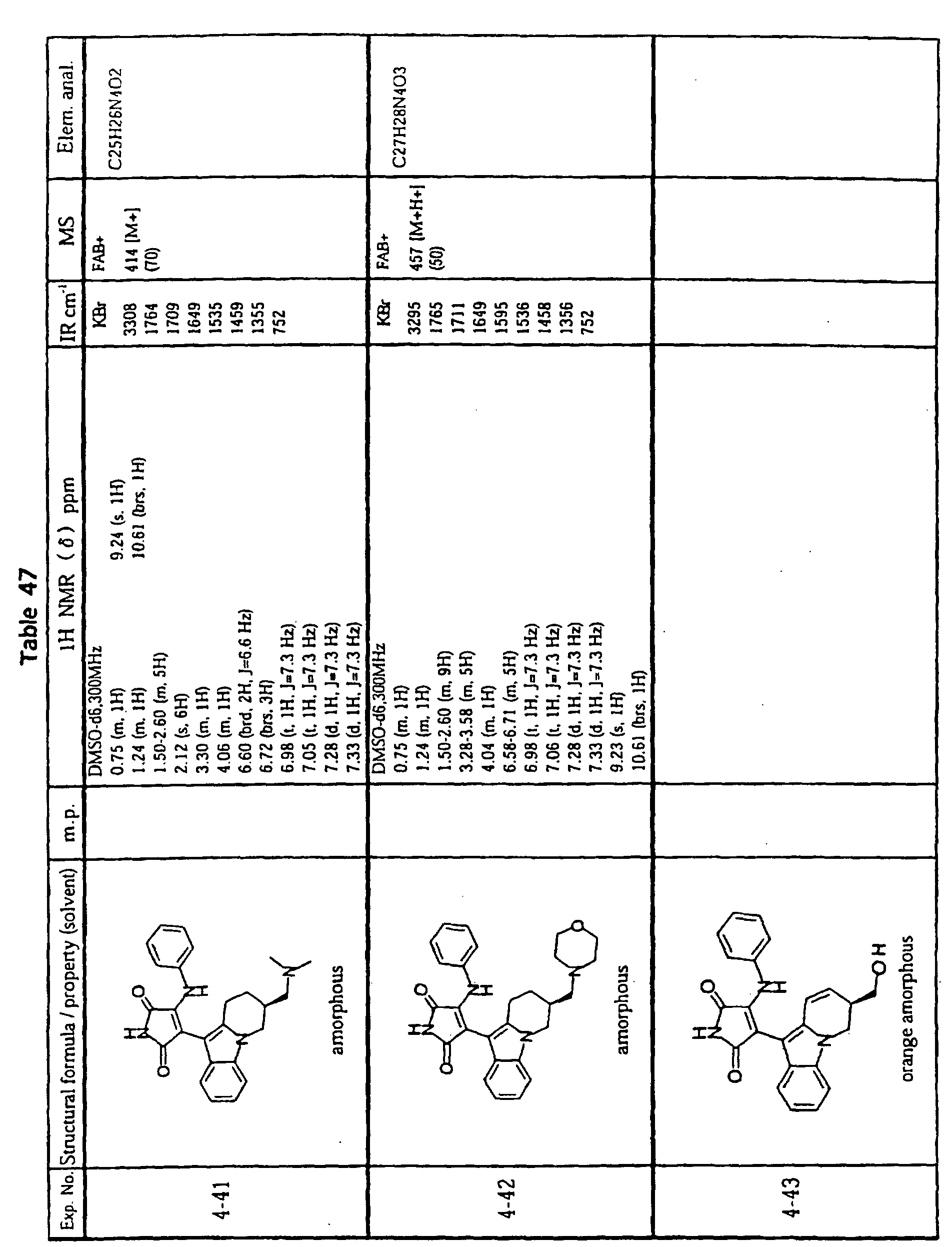

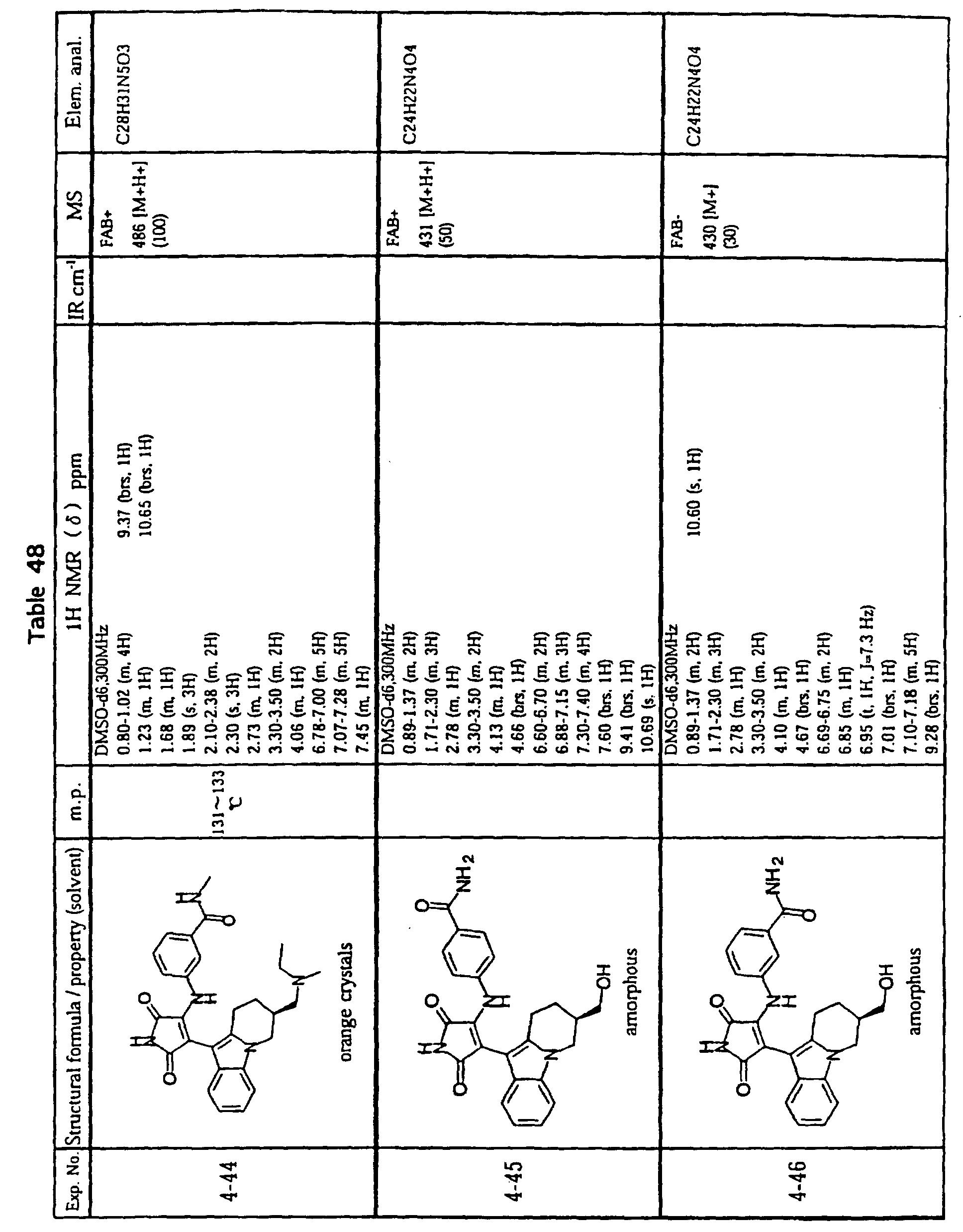

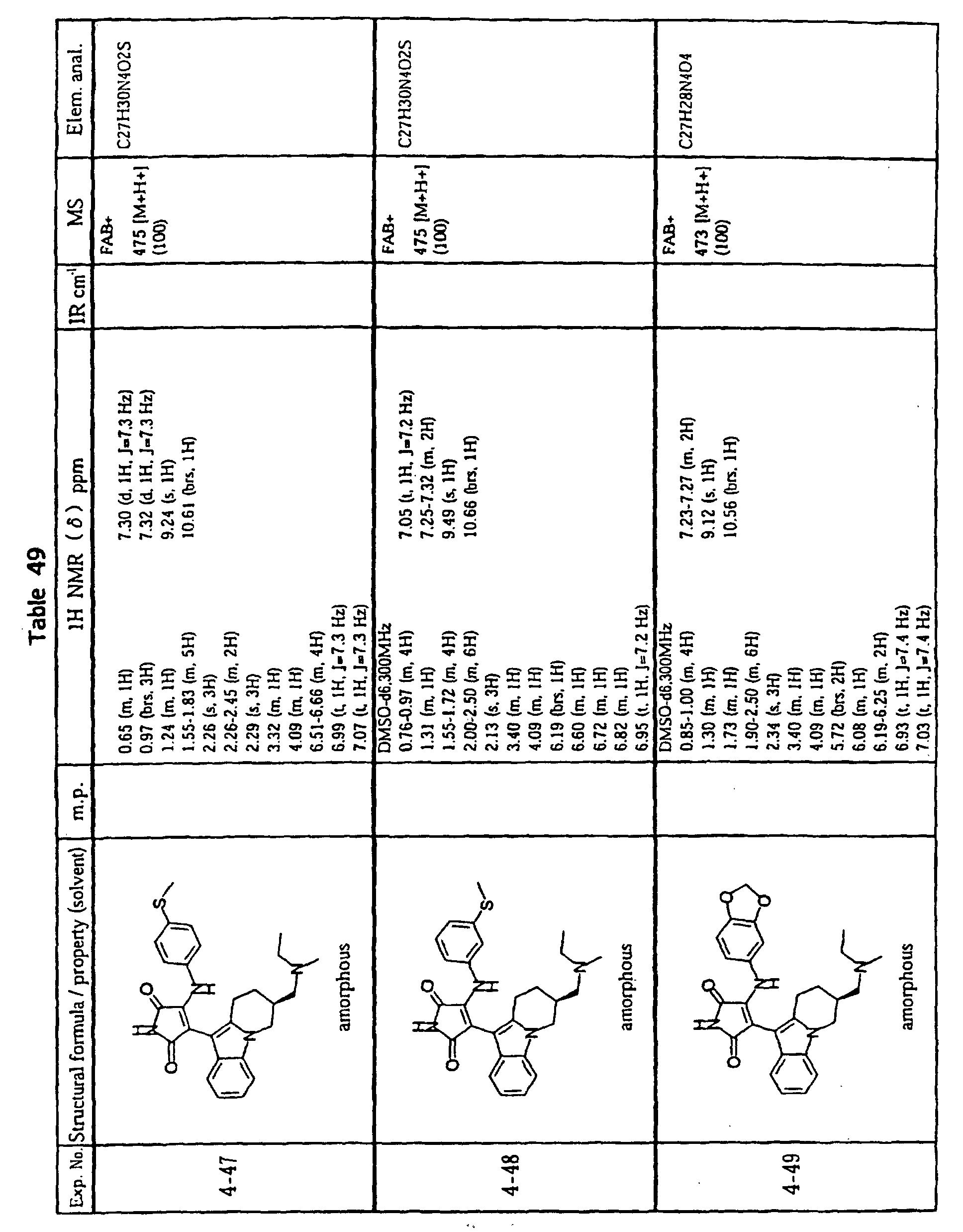

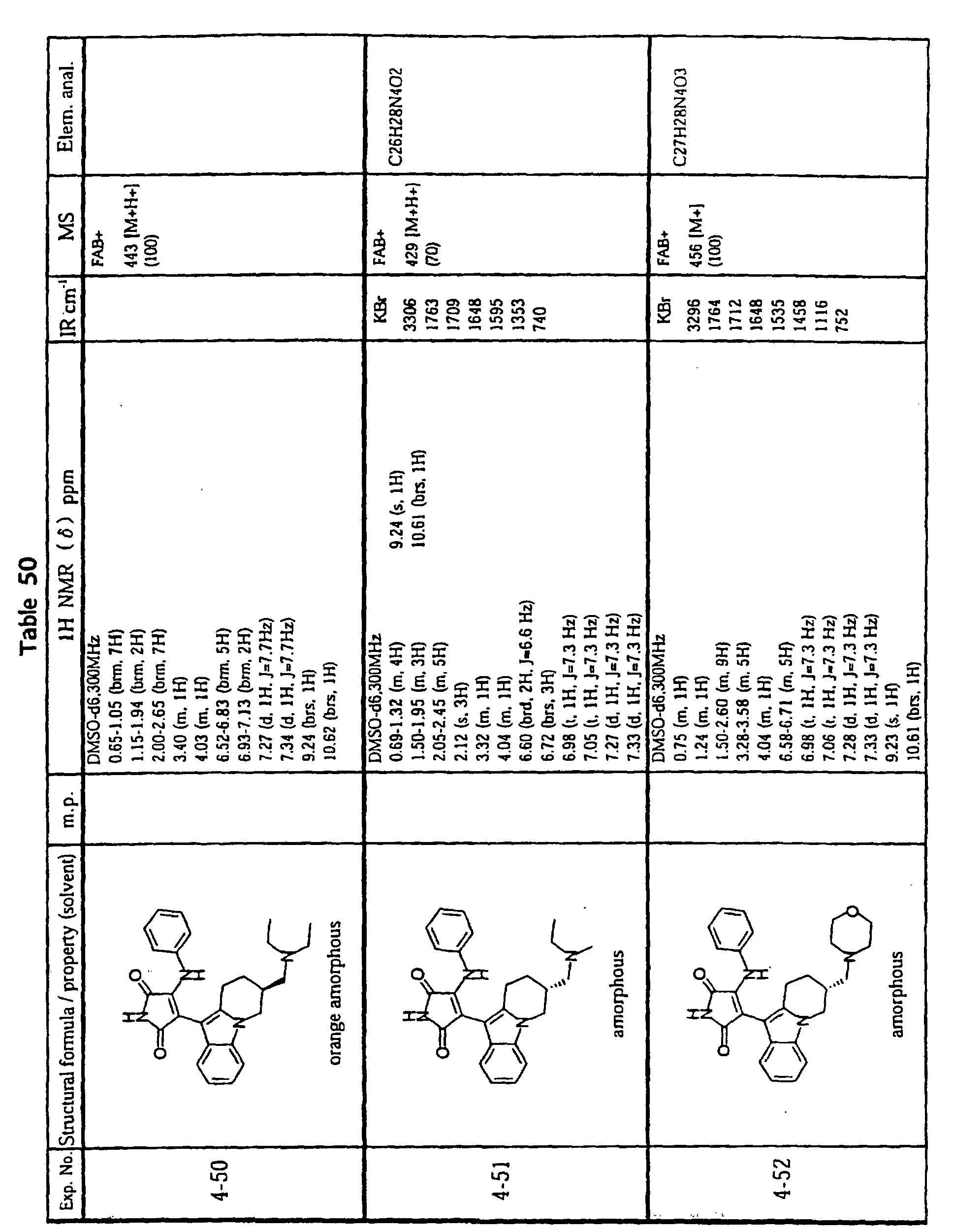

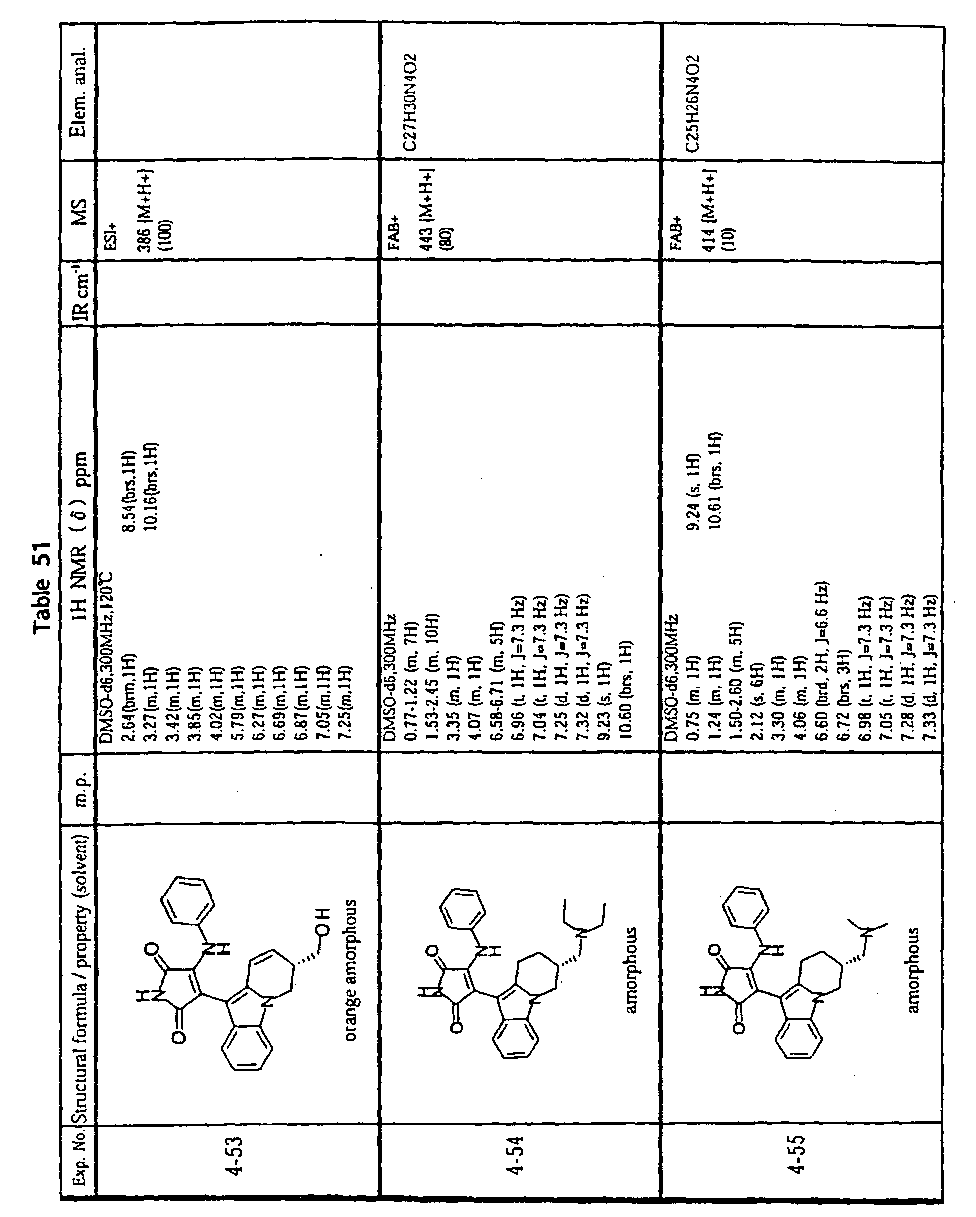

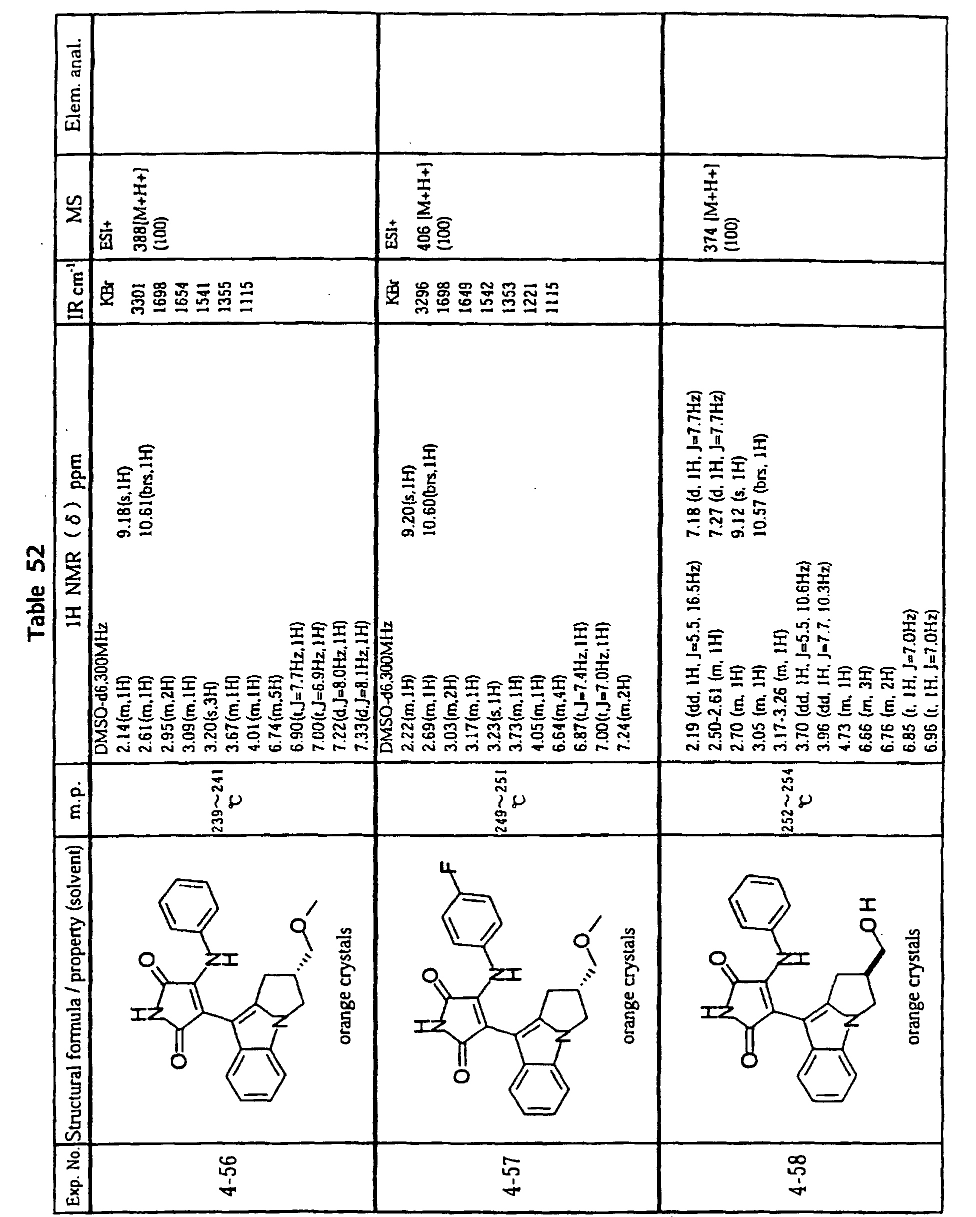

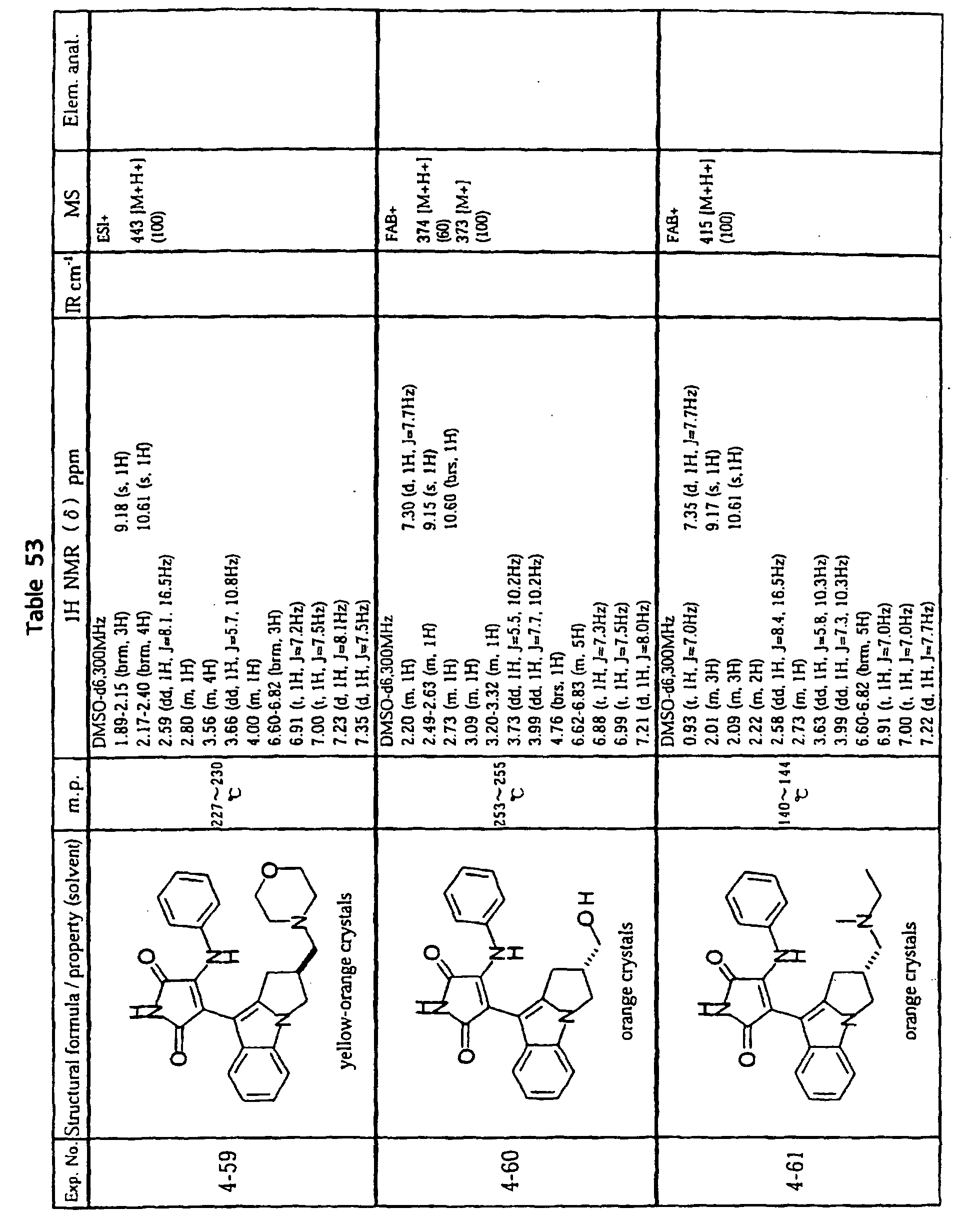

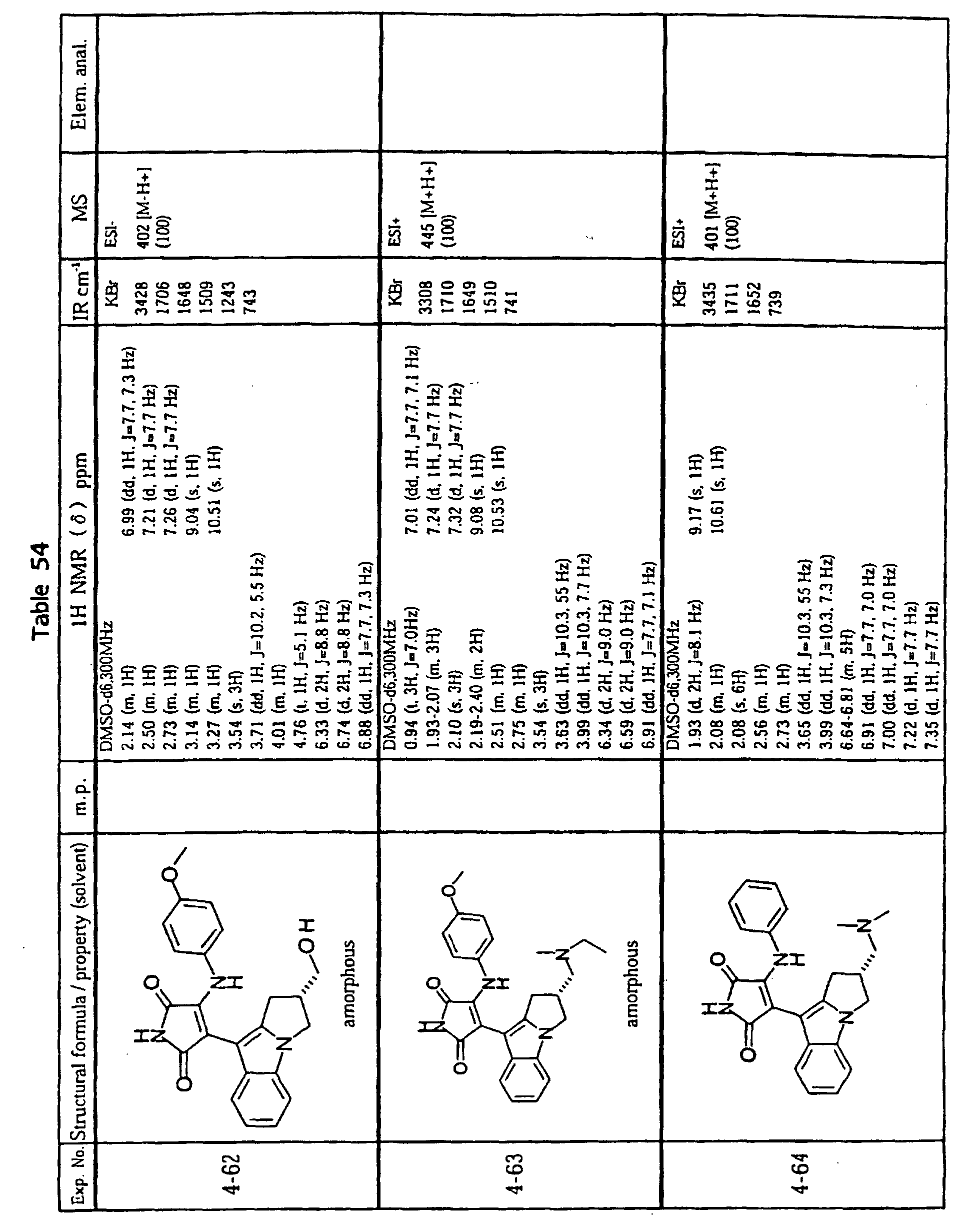

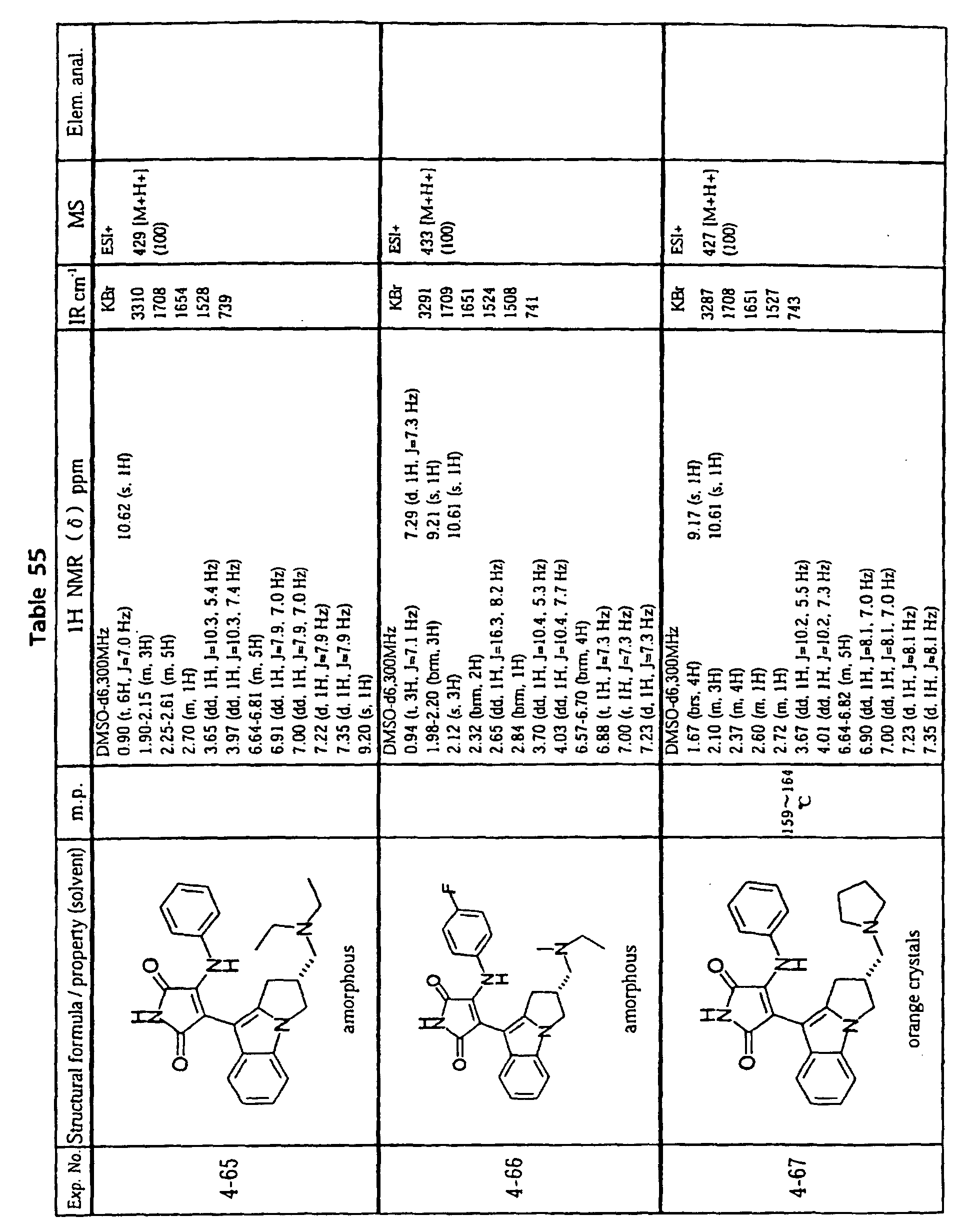

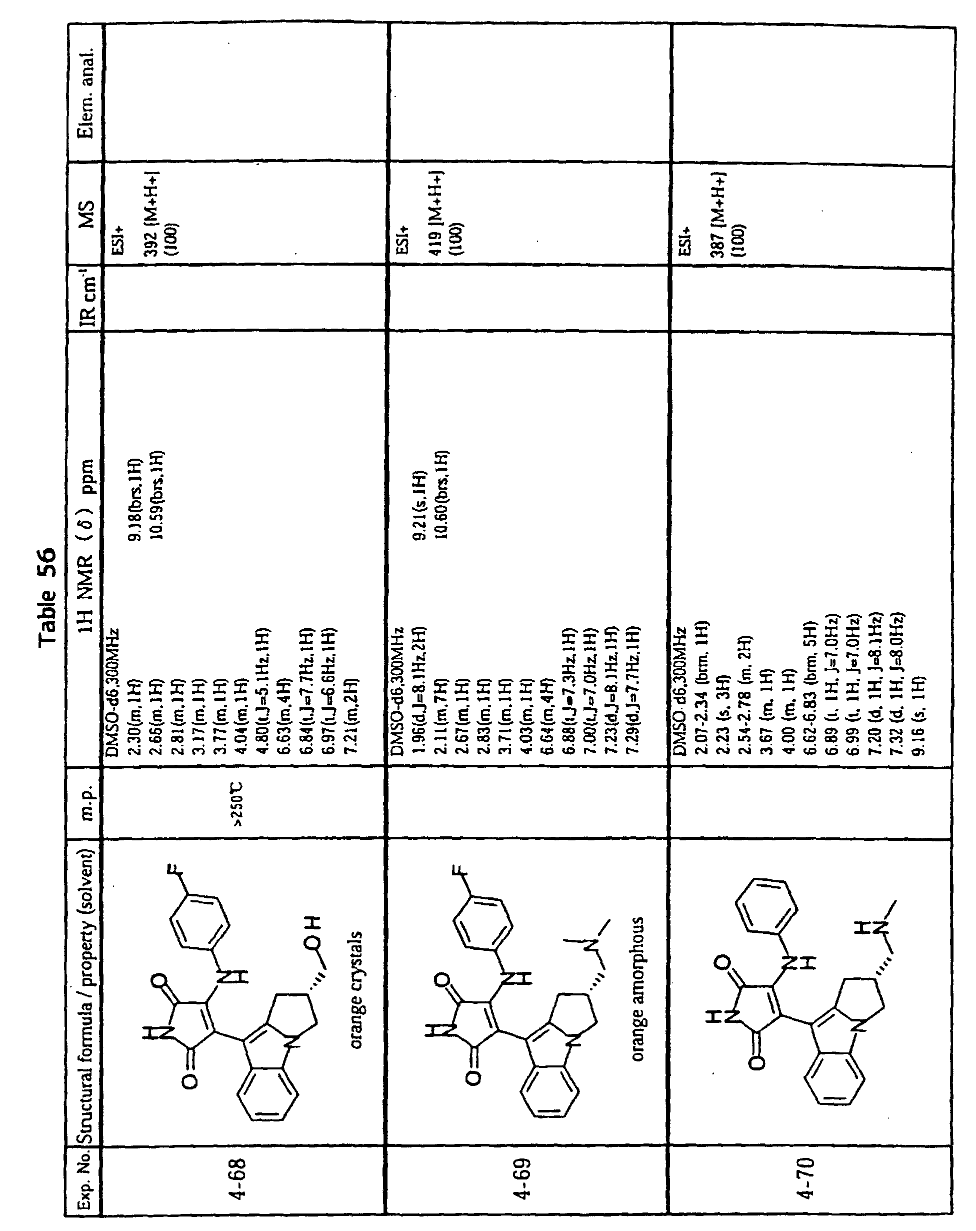

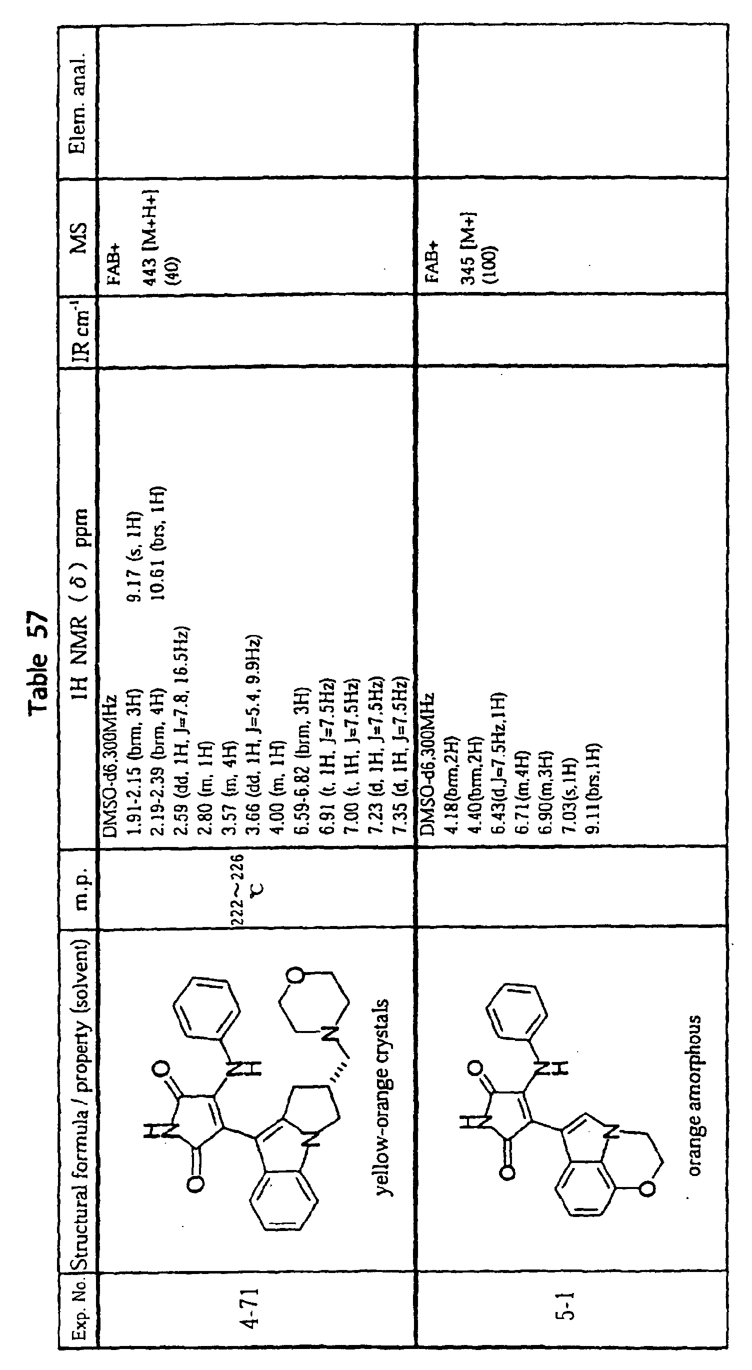

A formulation example is given in the following, to which the invention is not limited.

### Formulation example

| | |
|---|---|
| (a) Compound of Example 1-1 | 10 g |
| (b) Lactose | 50 g |
| (c) Corn starch | 15 g |
| (d) Sodium carboxymethylcellulose | 44 g |
| (e) Magnesium stearate | 1 g |

The entire amounts of (a), (b) and (c) and (d) (30 g) were kneaded with water and dried in vacuo, followed by granulation. To the granules are added 14 g of (d) and 1 g of (e) and the admixture was tableted by a tableting machine to give 1000 tablets, each containing 10 mg of (a).

The method for determining the PKC inhibitory activity of the compound of the present invention is explained in the following. Inasmuch as PKCβII is present in greater number in intravascular cells than PKCβI, PKCβII was mainly used here to test the enzyme activity.

### Experimental Example [I] PKC enzyme assay

As the reagent for enzyme assay, BIOTRAK Protein kinase C enzyme assay system (hereinafter the system, Amersham), and as the PKC enzyme standard product, Protein Kinase C, Human Recombinant (CALBIOCHEM) were purchased and used in the assay. A substrate mixture was prepared by mixing calcium buffer (12 mM calcium acetate, 50 mM Tris/HCl pH 7.5, 0.05% (w/v) sodium azide), Lipid (0.3 mg/ml La-phosphatidyl-L-serine, 24 mg/ml phorbol 12-myristate 13-acetate, 50 mM Tris/HCl pH 7.5, 0.05% (w/v) sodium azide), peptide buffer (900 µM peptide RKRTLRRL), 50 mM Tris/HCl pH 7.5, 0.05% (w/v) sodium azide), DTT buffer (30 mM dithiothreitol, 50 mM Tris/HCl pH 7.5, 0.05% (w/v) sodium azide), and magnesium ATP buffer (1.2 mM ATP, 30 m Hepes pH 7.4, 72 mM magnesium chloride) all attached to the system in a ratio of 1:1:1:1:0.8 and adding [ y -32P]ATP (Amersham, cat. No. PB168) to a concentration of 6.7 µM. The PKC enzyme standard product was diluted with an assay buffer (10 mM Hepes pH 7.4, 0.01% Triton X-100) to a concentration of 400 ng/ml and used as the enzyme solution. The substrate mixture and a test substance (diluted to the final concentration of 1 nM-10 µM with dimethyl sulfoxide (DMSO)) were mixed at a ratio of 20:1. The enzyme solution was added in the amount equivalent to the substrate mixture and mixed, which was followed by incubation at 37°C for 15 minutes. The reaction terminator (300 mM orthophosphoric acid containing carmosine red) attached to the system was added in the amount equivalent to the substrate mixture to end the reaction, and the reaction mixture was spotted on a phosphocellulose paper (Whatman, P-81), washed twice with 75 mM orthophosphoric acid and assayed for radioactivity by BAS2000 (Fuji film).

The ratio of the radioactivity of the addition of the test substance to the addition of DMSO was determined, and IC₅₀ was calculated from inhibition at each concentration. The results are shown in Tables 58 to 65.

**Table 58**

| Example number | Inhibition of PKC activity IC₅₀ (µM) | | Ratio |
|---|---|---|---|
| | PKC β II | PKC α | α/β |
| 1-2 | 0.223 | 7.233 | 32 |
| 1-5 | 0.151 | 4.918 | 33 |
| 1-6 | 0.198 | 6.909 | 35 |
| 1-10 | 0.226 | 8.343 | 37 |
| 1-12 | 0.249 | 7.163 | 29 |
| 2-1 | 0.030 | 1.619 | 54 |
| 2-2 | 0.041 | 2.857 | 70 |
| 2-3 | 0.046 | 3.897 | 85 |
| 2-4 | 0.073 | 5.799 | 79 |
| 2-5 | 0.030 | 1.826 | 61 |
| 2-6 | 0.060 | 3.308 | 55 |
| 2-7 | 0.056 | 3.169 | 57 |
| 2-8 | 0.012 | 0.460 | 38 |
| 2-9 | 0.028 | 2.157 | 77 |
| 2-10 | 0.015 | 0.730 | 49 |
| 2-11 | 0.015 | 0.727 | 49 |
| 2-12 | 0.010 | 0.558 | 56 |
| 2-13 | 0.021 | 1.262 | 60 |

**Table 59**

| Example number | Inhibition of PKC activity IC₅₀(µM) | | Ratio |
|---|---|---|---|
| | PKC β II | PKC α | α/β |
| 2-14 | 0.040 | 2.795 | 70 |
| 2-15 | 0.035 | 2.680 | 77 |
| 2-16 | 0.080 | 6.287 | 79 |
| 2-17 | 0.103 | 4.633 | 45 |
| 2-18 | 0.183 | 7.526 | 41 |
| 2-19 | 0.017 | 1.228 | 72 |
| 2-20 | 0.017 | 0.726 | 43 |
| 2-21 | 0.017 | 0.773 | 46 |
| 2-22 | 0.018 | 0.662 | 37 |
| 2-23 | 0.010 | 0.399 | 40 |
| 2-24 | 0.066 | 2.750 | 42 |
| 2-25 | 0.009 | 0.413 | 46 |
| 2-26 | 0.023 | 0.968 | 42 |
| 2-27 | 0.026 | 0.973 | 37 |
| 2-28 | 0.025 | 1.078 | 43 |
| 2-29 | 0.025 | 1.874 | 75 |
| 2-31 | 0.012 | 0.703 | 59 |
| 2-32 | 0.019 | 1.075 | 57 |

**Table 60**

| Example number | Inhibition of PKC activity IC₅₀ (*µ*M) | | Ratio |
|---|---|---|---|
| | PKC β II | PKC α | α/β |
| 2-33 | 0.010 | 0.676 | 68 |
| 2-34 | 0.015 | 0.851 | 57 |
| 2-35 | 0.095 | 3.891 | 41 |
| 2-36 | 0.005 | 0.331 | 66 |
| 2-37 | 0.060 | >1 | 17 |
| 2-38 | 0.040 | >1 | 25 |
| 2-39 | 0.021 | 0.354 | 17 |
| 2-40 | 0.019 | 0.770 | 41 |
| 2-41 | 0.062 | 2.741 | 44 |
| 2-42 | 0.010 | 0.452 | 45 |
| 2-43 | 0.002 | 0.027 | 14 |
| 2-44 | 0.261 | 8.159 | 31 |
| 2-47 | 0.066 | 2.342 | 36 |
| 2-48 | 0.018 | 1.389 | 77 |
| 2-49 | 0.008 | 0.723 | 90 |
| 2-50 | 0.019 | 0.886 | 47 |
| 2-51 | 0.027 | 1.020 | 38 |
| 2-53 | 0.098 | >10 | 102 |

**Table 61**

| Example number | Inhibition of PKC activity IC₅₀ (*µ*M) | | Ratio |
|---|---|---|---|
| | PKC β II | PKCα | α/β |
| 2-54 | 0.061 | 6.672 | 109 |
| 2-55 | 0.096 | >10 | 104 |
| 2-56 | 0.084 | 5.490 | 65 |
| 2-58 | 0.018 | 0.842 | 47 |
| 2-59 | 0.008 | 0.356 | 45 |
| 2-60 | 0.016 | 0.762 | 48 |
| 2-61 | 0.009 | 0.401 | 45 |
| 2-62 | 0.081 | 4.434 | 55 |
| 2-63 | 0.247 | 7.115 | 29 |
| 2-64 | 0.020 | 0.739 | 37 |
| 2-65 | 0.010 | 0.473 | 47 |
| 2-66 | 0.027 | 0.908 | 34 |
| 2-68 | 0.006 | 0.315 | 53 |
| 2-69 | 0.020 | 2.345 | 117 |
| 3-1 | 0.018 | 0.484 | 27 |
| 3-2 | 0.016 | 0.298 | 19 |
| 3-3 | 0.057 | 2.651 | 47 |
| 3-4 | 0.018 | 0.518 | 29 |

**Table 62**

| Example number | Inhibition of PKC activity IC₅₀ (*µ* M) | | Ratio |
|---|---|---|---|
| | PKCβII | PKC *α* | α/β |
| 3-5 | 0.029 | 1.628 | 56 |
| 3-6 | 0.108 | 3.663 | 34 |
| 3-7 | 0.056 | 2.560 | 46 |
| 4-1 | 0.014 | 0.852 | 61 |
| 4-2 | 0.005 | 0.257 | 51 |
| 4-3 | 0.016 | 1.633 | 102 |
| 4-4 | 0.006 | 0.394 | 66 |
| 4-5 | 0.023 | 1.908 | 83 |
| 4-6 | 0.025 | 2.098 | 84 |
| 4-7 | 0.023 | 3.999 | 174 |
| 4-8 | 0.014 | 0.680 | 49 |
| 4-9 | 0.015 | 1.451 | 97 |
| 4-10 | 0.017 | 2.131 | 125 |
| 4-11 | 0.005 | 0.265 | 53 |
| 4-12 | 0.002 | 0.096 | 48 |
| 4-13 | 0.008 | 0.910 | 114 |
| 4-14 | 0.004 | 0.247 | 62 |
| 4-15 | 0.016 | 1.224 | 77 |

**Table 63**

| Example number | Inhibition of PKC activity IC₅₀ (*µ*M) | | Ratio |
|---|---|---|---|
| | PKC β II | PKCα | α/β |
| 4-16 | 0.025 | 3.057 | 122 |
| 4-17 | 0.068 | >10 | 147 |
| 4-19 | 0.004 | 0.275 | 69 |
| 4-20 | 0.003 | 0.170 | 57 |
| 4-21 | 0.011 | 0.795 | 72 |
| 4-22 | 0.003 | 0.355 | 118 |
| 4-23 | 0.023 | 2.605 | 113 |
| 4-24 | 0.003 | 0.204 | 68 |
| 4-25 | 0.006 | 0.497 | 83 |
| 4-26 | 0.009 | 0.768 | 85 |
| 4-27 | 0.013 | 0.831 | 64 |
| 4-28 | 0.025 | 3.356 | 134 |
| 4-29 | 0.014 | 1.930 | 138 |
| 4-30 | 0.005 | 0.654 | 131 |
| 4-31 | 0.003 | 0.186 | 64 |
| 4-32 | 0.004 | 0.228 | 60 |
| 4-33 | 0.004 | 0.321 | 75 |
| 4-34 | 0.002 | 0.091 | 43 |

**Table 64**

| Example number | Inhibition of PKC activity IC₅₀ (*µ*M) | | Ratio |
|---|---|---|---|
| | PKC β II | PKC α | α/β |
| 4-35 | 0.003 | 0.113 | 33 |
| 4-36 | 0.009 | 0.725 | 81 |
| 4-37 | 0.007 | 0.592 | 85 |
| 4-38 | 0.041 | >10 | 244 |
| 4-39 | 0.056 | 5.739 | 103 |
| 4-40 | 0.006 | 0.557 | 93 |
| 4-41 | 0.006 | 0.445 | 74 |
| 4-42 | 0.022 | 2.659 | 121 |
| 4-43 | 0.007 | 0.749 | 107 |
| 4-46 | 0.089 | >10 | 112 |
| 4-48 | 0.034 | 2.526 | 74 |
| 4-49 | 0.033 | 2.711 | 82 |
| 4-50 | 0.006 | 0.494 | 82 |
| 4-51 | 0.007 | 0.708 | 101 |
| 4-52 | 0.057 | 3.876 | 68 |
| 4-53 | 0.014 | 1.479 | 106 |
| 4-54 | 0.010 | 0.569 | 57 |
| 4-55 | 0.004 | 0.252 | 63 |

**Table 65**

| Example number | Inhibition of PKC activity IC₅₀ (*µ*M) | | Ratio |
|---|---|---|---|
| | PKC β II | PKCα | α/β |
| 4-56 | 0.040 | 5.218 | 131 |
| 4-57 | 0.084 | 6.46 | 77 |
| 4-58 | 0.014 | 1.021 | 73 |
| 4-59 | 0.046 | 4.992 | 109 |
| 4-60 | 0.004 | 0.458 | 115 |
| 4-61 | 0.005 | 0.481 | 96 |
| 4-62 | 0.016 | 0.794 | 50 |
| 4-63 | 0.016 | 1.103 | 69 |
| 4-64 | 0.004 | 0.148 | 37 |
| 4-65 | 0.032 | 1.955 | 61 |
| 4-66 | 0.012 | 0.632 | 53 |
| 4-67 | 0.008 | 0.542 | 68 |
| 4-68 | 0.004 | 0.341 | 85 |
| 4-69 | 0.006 | 0.455 | 76 |
| 4-70 | 0.001 | 0.159 | 159 |
| 4-71 | 0.041 | 4.446 | 108 |

### EFFECT OF THE INVENTION

As is evident from the above-mentioned results, the compounds of the present invention have high inhibitory activity against PKCβ, with part thereof showing selective inhibition of PKCβ as compared to PKCα and PKA.

Thus, these compounds can be pharmaceutical agents effective against diseases caused by PKC, inclusive of diabetic complications such as diabetic retinopathy, diabetic nephropathy, diabetic cardiomyopathy, diabetic neuropathy and the like. The selective action on PKCβ indicates realization of a safe pharmaceutical agent free of noticeable side effect.

This application is based on a patent application No. 215070/1998 filed in Japan, the content of which is hereby incorporated by reference.

## Claims

1. A disubstituted maleimide compound of the formula [I] 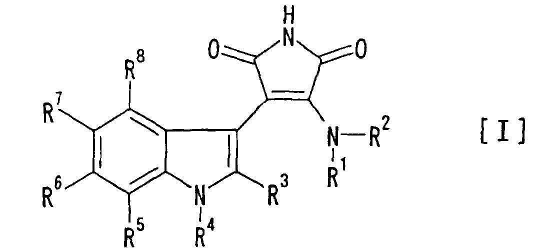 wherein
R¹ is hydrogen atom or lower alkyl;
R² is optionally substituted aryl, optionally substituted cycloalkyl or optionally substituted heterocyclic group;
R³, R⁵, R⁶, R⁷ and R⁸ are the same or different and each is hydrogen atom, halogen atom, hydroxyl group, amino, optionally substituted lower alkyl or optionally substituted lower alkoxy;
R⁴ is independently W, or R⁴ and R³ jointly form a group of the formula 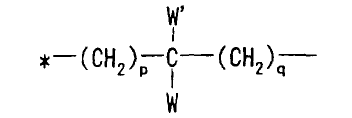 or 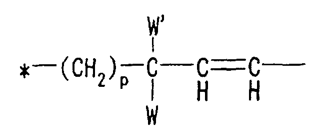 or R⁴ and R⁵ jointly form a group of the formula 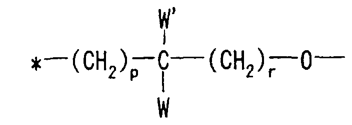
W is - (CH₂)ₗ- (Y)ₘ- (CH₂)ₙ-Z
wherein Y is -CR⁹R⁹' - (wherein R⁹ and R⁹' are the same or different and each is hydrogen atom, hydroxyl group, lower alkyl, lower alkoxy, lower alkylthio, lower alkylamino, di(lower)alkylamino, or heterocyclic group), -NR¹⁰- (wherein R¹⁰ is hydrogen atom or lower alkyl), -O-, -S-, -SO₂-, -CONH-, -NHCO-, -SONH-, -NHSO-, -SO₂NH-, -NHSO₂- or -SO₃-,
Z is hydrogen atom, halogen atom, hydroxyl group, optionally substituted lower alkoxy, lower alkanoyl, lower alkoxycarbonyl, -NR¹¹R¹² (wherein R¹¹ and R¹² are the same or different and each is hydrogen atom or lower alkyl), optionally substituted amidino, optionally substituted guanidino, carbamoyl, lower alkylaminocarbonyl, optionally substituted aryl, optionally substituted cycloalkyl or optionally substituted heterocyclic group,
1 is 0 or an integer of 1 to 4, m is 0 or 1, and n is 0 or an integer of 1 to 4},
W' is hydrogen atom or the same as or different from W and is - (CH₂)ₗ-(Y)ₘ- (CH₂)ₙ-Z (wherein each symbol is as defined above); and
p, q and r are the same or different and each is 0 or an integer of 1 to 4,
the above-mentioned symbol * means that the side marked with a * binds to the nitrogen atom of the indole ring,
or a pharmaceutically acceptable salt thereof.

2. The disubstituted maleimide compound of the formula [I] of claim 1 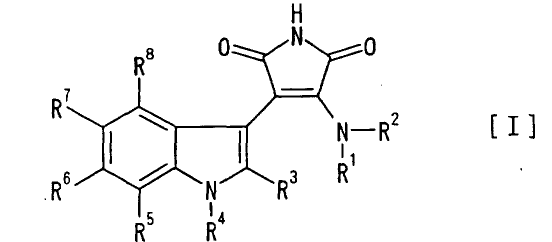 wherein R¹ is hydrogen atom or C₁-C₆ lower alkyl (wherein C₁-C₆ means having 1 to 6 carbon atoms, hereinafter the same);
R² is optionally substituted C₆-C₁₈ aryl, optionally substituted C₃-C₈ cycloalkyl or optionally substituted heterocyclic group (wherein said heterocyclic group has 1 to 4 hetero atoms selected from oxygen atom, nitrogen atom and sulfur atom, wherein the number of atoms constituting the ring is 5 to 12);
R³, R⁵, R⁶, R⁷ and R⁸ are the same or different and each is hydrogen atom, halogen atom, hydroxyl group, amino, optionally substituted C₁-C₆ lower alkyl or optionally substituted C₁-C₆ lower alkoxy;
R⁴ is independently W, or R⁴ and R³ jointly form a group of the formula 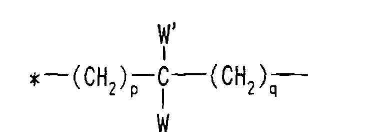 or 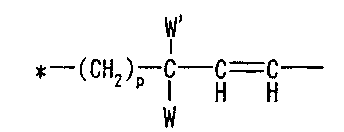 or R⁴ and R⁵ jointly form a group of the formula 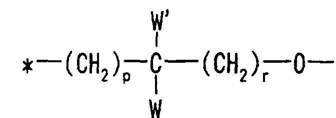
W is - (CH₂)ₗ- (Y)ₘ- (CH₂)ₙ-Z
wherein Y is -CR⁹R^{9'}- [wherein R⁹ and R^{9'} are the same or different and each is hydrogen atom, hydroxyl group, C₁-C₆ lower alkyl, C₁-C₆ lower alkoxy, C₁-C₆ lower alkylthio, C₁-C₆ lower alkylamino, di(C₁-C₆ lower)alkylamino or heterocyclic group (wherein said heterocyclic group has 1 to 4 hetero atoms selected from oxygen atom, nitrogen atom and sulfur atom, wherein the number of atoms constituting the ring is 5 to 12)], -NP¹⁰- (wherein R¹⁰ is hydrogen atom or C₁ -C₆ lower alkyl), -O-, -S-, -SO₂-, -CONH-, -NHCO-, -SONH-, -NHSO-, -SO₂NH-, -NHSO₂- or -SO₃-,
Z is hydrogen atom, halogen atom, hydroxyl group, optionally substituted C₁-C₆ lower alkoxy, C₁-C₆ lower alkanoyl, C₁-C₆ lower alkoxycarbonyl, -NR¹¹R¹² (wherein R¹¹ and R¹² are the same or different and each is hydrogen atom or C₁-C₆ lower alkyl), optionally substituted amidino, optionally substituted guanidino, carbamoyl, C₁-C₆ lower alkylaminocarbonyl, optionally substituted C₆-C₁₈ aryl, optionally substituted C₃-C₈ cycloalkyl or optionally substituted heterocyclic group (said heterocyclic group is as defined above),
l is 0 or an integer of 1 to 4, m is 0 or 1, and n is 0 or an integer of 1 to 4;
W' is hydrogen atom or the same as or different from W and is - (CH₂)ₗ- (Y)ₘ- (CH₂)ₙ-Z (wherein each symbol is as defined above); and
p, q and r are the same or different and each is 0 or an integer of 1 to 4,
the above-mentioned symbol * means that the side marked with a * binds to the nitrogen atom of the indole ring, or a pharmaceutically acceptable salt thereof.

3. The disubstituted maleimide compound of claim 2, wherein R² is optionally substituted C₆-C₁₈ aryl or optionally substituted C₃-C₈ cycloalkyl;
R³, R⁵, R⁶, R⁷ and R⁸ are the same or different and each is hydrogen atom, optionally substituted C₁-C₆ lower alkyl or optionally substituted C₁-C₆ lower alkoxy;
Y at W is -CR⁹R⁹' -, -NR¹⁰- (wherein R⁹, R⁹' and R¹⁰ are as defined in claim 2), -O-, -S- or -SO₂-;
Z at W is hydrogen atom, hydroxyl group, optionally substituted C₁-C₆ lower alkoxy, C₁-C₆ lower alkanoyl, -NR¹¹R¹² (wherein R¹¹ and R¹² are as defined in claim 2), optionally substituted amidino or optionally substituted heterocyclic group; and
W' is hydrogen atom,
or a pharmaceutically acceptable salt thereof.

4. The disubstituted maleimide compound of claim 2, wherein R¹ is hydrogen atom, and R² is optionally substituted C₆-C₁₈ aryl, or a pharmaceutically acceptable salt thereof.

5. The disubstituted maleimide compound of claim 4, wherein R⁴ is independently W or R⁴ and R³ jointly form a group of the formula 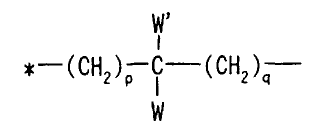 wherein W, p and q are as defined in claim 2, and W' is hydrogen atom, or a pharmaceutically acceptable salt thereof.

6. The disubstituted maleimide compound of claim 5, wherein R⁴ and R³ jointly form a group of the formula 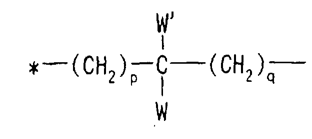 wherein W, p and q are as defined in claim 2, and W' is hydrogen atom, or a pharmaceutically acceptable salt thereof.

7. The disubstituted maleimide compound of claim 6, wherein R⁵, R⁶, R⁷ and R⁸ are each hydrogen atom, and R² is phenyl, or a pharmaceutically acceptable salt thereof.

8. The disubstituted maleimide compound of claim 7, wherein Z at W is hydroxyl group, -NR¹¹R¹² (wherein R¹¹ and R¹² are as defined in claim 2) or optionally substituted heterocyclic group, or a pharmaceutically acceptable salt thereof.

9. The disubstituted maleimide compound of claim 1 or a pharmaceutically acceptable salt thereof, which is selected from the group consisting of
3-(1H-indol-3-yl)-4-[(3-methoxyphenyl)amino]-1H-pyrrole-2,5-dione,
3-(1H-indol-3-yl)-4-(phenylamino)-1H-pyrrole-2,5-dione,
3-(cyclohexylamino)-4-(1H-indol-3-yl)- 1H-pyrrole-2,5-dione,
3-(1H-indol-3-yl)-4-[(4-methylphenyl)amino]-1H-pyrrole-2,5-dione,
3-(1H-indol-3-yl)-4-[(3-methylphenyl)amino]-1H-pyrrole-2,5-dione,
3-[(3-chlorophenyl)amino]-4-(1H-indol-3-yl)-1H-pyrrole-2,5-dione,
3-(1H-indol-3-yl)-4-[(4-methoxy-2-methylphenyl)amino]-1H-pyrrole-2,5-dione,
3-[(2,4-dimethoxyphenyl)amino]-4-(1H-indol-3-yl)-1H-pyrrole-2,5-dione,
3-[(2,4-difluorophenyl)amino]-4-(1H-indol-3-yl)-1H-pyrrole-2,5-dione,
3-[(3-bromophenyl)amino]-4-(1H-indol-3-yl)-1H-pyrrole-2,5-dione,
3-(1H-indol-3-yl)-4-[(2-methylphenyl)amino]-1H-pyrrole-2,5-dione,
3-[(3-fluorophenyl)amino]-4-(1H-indol-3-yl)-1H-pyrrole-2,5-dione,
3-(1H-indol-3-yl)-4-[(3-trifluoromethylphenyl)amino]-1H-pyrrole-2,5-dione,
3-(1H-indol-3-yl)-4-(biphenyl-3-ylamino)-1H-pyrrole-2,5-dione,
3-(1H-indol-3-yl)-4-[(3-phenoxyphenyl)amino)-1H-pyrrole-2,5-dione,
3-(1H-indol-3-yl)-4-[(3-isopropylphenyl)amino]-1H-pyrrole-2,5-dione,
3-(1H-indol-3-yl)-4-(N-methyl-N-phenylamino)-1H-pyrrole-2,5-dione,
3-[1-(3-hydroxypropyl)-1H-indol-3-yl]-4-(phenylamino)-1H-pyrrole-2,5-dione,
3-[1-(3-hydroxypropyl)-1H-indol-3-yl]-4-[(3-methylphenyl)amino]-1H-pyrrole-2,5-dione,
3-[(3-chlorophenyl)amino]-4-[1-(3-hydroxypropyl)-1H-indol-3-yl]-1H-pyrrole-2,5-dione,
3-[1-(2-hydroxyethyl)-1H-indol-3-yl]-4-(phenylamino)-1H-pyrrole-2,5-dione,
3-[1-(4-hydroxybutyl)-1H-indol-3-yl]-4-(phenylamino)-1H-pyrrole-2,5-dione,
3-[(3,4-dichlorophenyl)amino]-4-[1-(3-hydroxypropyl)-1H-indol-3-yl]-1H-pyrrole-2,5-dione,
3-[1-(2-acetoxyethyl)-1H-indol-3-yl]-4-(phenylamino)-1H-pyrrole-2,5-dione,
3-[1-(3-dimethylaminopropyl)-1H-indol-3-yl]-4-(phenylamino)-1H-pyrrole-2,5-dione,
3-[1-(2-dimethylaminoethyl)-1H-indol-3-yl]-4-(phenylamino)-1H-pyrrole-2,5-dione,
3-[1-(4-dimethylaminobutyl)-1H-indol-3-yl]-4-(phenylamino)-1H-pyrrole-2,5-dione,
3-[1-(3-dimethylaminopropyl)-1H-indol-3-yl]-4-[(3-methylphenyl)amino]-1H-pyrrole-2,5-dione,
3-[1-(3-dimethylaminopropyl)-1H-indol-3-yl]-4-[(3-chlorophenyl)amino]-1H-pyrrole-2,5-dione,
3-[1-(3-diethylaminopropyl)-1H-indol-3-yl]-4-(phenylamino)-1H-pyrrole-2,5-dione,
3-[1-{3-[N-(2-dimethylaminoethyl)-N-methylamino]propyl}-1H-indol-3-yl]-4-(phenylamino)-1H-pyrrole-2,5-dione,
3-[1-{3-[N-ethyl-N-(2-methoxyethyl)amino]propyl}-1H-indol-3-yl]-4-(phenylamino)-1H-pyrrole-2,5-dione,
3-[1-{2-[N-(2-dimethylaminoethyl)-N-methylamino)ethyl}-1H-indol-3-yl]-4-(phenylamino)-1H-pyrrole-2,5-dione,
3-[1-{3-(N-benzyl-N-ethylamino)propyl}-1H-indol-3-yl]-4-(phenylamino)-1H-pyrrole-2,5-dione,
3-[1-{3-[N-ethyl-N-(4-pyridylmethyl)amino]propyl}-1H-indol-3-yl]-4-(phenylamino)-1H-pyrrole-2,5-dione,
3-[1-(3-morpholinopropyl)-1H-indol-3-yl]-4-(phenylamino)-1H-pyrrole-2,5-dione,
3-[1-(3-piperidinopropyl)-1H-indol-3-yl]-4-(phenylamino)-1H-pyrrole-2,5-dione,
3-(phenylamino)-4-[1-(3-thiomorpholinopropyl)-1H-indol-3-yl]-1H-pyrrole-2,5-dione,
3-(phenylamino)-4-[1-(3-pyrrolidin-1-ylpropyl)-1H-indol-3-yl]-1H-pyrrole-2,5-dione,
3-[1-(3-azacycloheptan-1-ylpropyl)-1H-indol-3-yl]-4-(phenylamino)-1H-pyrrole-2,5-dione,
3-[1-{3-(2-carbamoylpyrrolidin-1-yl)propyl}-1H-indol-3-yl]-4-(phenylamino)-1H-pyrrole-2,5-dione,
3-[1-{3-(4-hydroxypiperidino)propyl}-1H-indol-3-yl]-4-(phenylamino)-1H-pyrrole-2,5-dione,
3-[1-{3-(4-methylpiperazin-1-yl)propyl}-1H-indol-3-yl]-4-(phenylamino)-1H-pyrrole-2,5-dione,
3-[(3-chlorophenyl)amino]-4-[1-(4-(4-hydroxypiperidino)butyl}-1H-indol-3-yl]-1H-pyrrole-2,5-dione,
3-[1-{5-(4-hydroxypiperidino)pentyl}-1H-indol-3-yl]-4-(phenylamino)-1H-pyrrole-2,5-dione,
3-[1-{4-(4-methylpiperazin-1-yl)butyl}-1H-indol-3-yl]-4-(phenylamino)-1H-pyrrole-2,5-dione,
3-[1-{3-[3-(tert-butylaminocarbonyl)-decahydro-(4aS,8aS)-isoquinolin-2-yl]propyl}-1H-indol-3-yl]-4-(phenylamino)-1H-pyrrole-2,5-dione,
3-(phenylamino)-4-[1-{3-(4-piperidinopiperidino)propyl}-1H-indol-3-yl]-1H-pyrrole-2,5-dione,
3-[1-{3-[4-(2-hydroxyethyl)piperazin-1-yl]propyl}-1H-indol-3-yl]-4-(phenylamino)-1H-pyrrole-2,5-dione,
3-[1-{3-(4-carbamoylpiperidino)propyl)-1H-indol-3-yl]-4-(phenylamino)-1H-pyrrole-2,5-dione,
3-[1-{3-(4-dimethylaminopiperidino)propyl}-1H-indol-3-yl]-4-(phenylamino)-1H-pyrrole-2,5-dione,
3-[1-{3-(phenylsulfonyl)propyl}-1H-indol-3-yl]-4-(phenylamino)-1H-pyrrole-2,5-dione,
3-[1-(3-imidazol-1-ylpropyl)-1H-indol-3-yl]-4-(phenylamino)-1H-pyrrole-2,5-dione,
3-(phenylamino)-4-[1-(3-pyrazol-1-ylpropyl)-1H-indol-3-yl]-1H-pyrrole-2,5-dione,
3-(phenylamino)-4-[1-{3-(1,2,4-triazol-1-yl)propyl}-1H-indol-3-yl]-1H-pyrrole-2,5-dione,
3-[(3-chlorophenyl)amino]-4-[1-(3-imidazol-1-ylpropyl)-1H-indol-3-yl]-1H-pyrrole-2,5-dione,
3-[(3-chlorophenyl)amino]-4-[1-(4-imidazol-1-ylbutyl)-1H-indol-3-yl]-1H-pyrrole-2,5-dione,
3-[1-(5-imidazol-1-ylpentyl)-1H-indol-3-yl]-4-(phenylamino)-1H-pyrrole-2,5-dione,
3-[(3-chlorophenyl)amino]-4-[1-{3-(2-methylimidazol-1-yl)propyl}-1H-indol-3-yl]-1H-pyrrole-2,5-dione,
3-[1-(3-amidinothiopropyl)-1H-indol-3-yl]-4-(phenylamino)-1H-pyrrole-2,5-dione hydrobromide,
3-[1-(2,3-dihydroxypropyl)-1H-indol-3-yl]-4-(phenylamino)-1H-pyrrole-2,5-dione,
3-[1-{3-(hydroxymethyl)benzyl}-1H-indol-3-yl]-4-(phenylamino)-1H-pyrrole-2,5-dione,
3-[1-(3-hydroxypropyl)-5-methoxy-1H-indol-3-yl]-4-(phenylamino)-1H-pyrrole-2,5-dione,
3-[1-{2-(4-hydroxypiperidino)ethyl}-1H-indol-3-yl]-4-(phenylamino)-1H-pyrrole-2,5-dione,
3-[1-{3-(4-benzylpiperidino)propyl}-1H-indol-3-yl]-4-(phenylamino)-1H-pyrrole-2,5-dione,
3-[1-{3-(4-pyrrolidinylpiperidino)propyl}-1H-indol-3-yl]-4-(phenylamino)-1H-pyrrole-2,5-dione,
3-[1-{3-[4-(hydroxymethyl)piperidino]propyl}-1H-indol-3-yl]-4-(phenylamino)-1H-pyrrole-2,5-dione,
3-[1-{3-(4-(tert-butoxycarbonyl)piperidino]propyl}-1H-indol-3-yl]-4-(phenylamino)-1H-pyrrole-2,5-dione,
3-[2-methyl-1-(3-morpholinopropyl)-1H-indol-3-yl]-4-(phenylamino)-1H-pyrrole-2,5-dione,
3-(2-methyl-1-(3-piperidinopropyl)-1H-indol-3-yl]-4-(phenylamino)-1H-pyrrole-2,5-dione,
3-[1-(3-dimethylaminopropyl)-2-methyl-1H-indol-3-yl]-4-(phenylamino]-1H-pyrrole-2,5-dione,
3-[2-methyl-1-(3-pyrrolidin-1-ylpropyl)-1H-indol-3-yl]-4-(phenylamino)-1H-pyrrole-2,5-dione,
3-[1-{3-(ethylmethylamino)propyl}-2-methyl-1H-indol-3-yl]-4-(phenylamino)-1H-pyrrole-2,5-dione,
3-[1-(3-dimethylaminopropyl)-5-methoxy-1H-indol-3-yl]-4-(phenylamino)-1H-pyrrole-2,5-dione,
3-[(3-chlorophenyl)amino]-4-[1-{3-(4-methyl-imidazol-1-yl)propyl}-1H-indol-3-yl]-1H-pyrrole-2,5-dione,
3-[(3-chlorophenyl)amino]-4-[1-{3-(5-methyl-imidazol-1-yl)propyl}-1H-indol-3-yl]-1H-pyrrole-2,5-dione,
3-[(3-chlorophenyl)amino]-4-[1-{3-(4-hydroxymethyl-imidazol-1-yl)propyl}-1H-indol-3-yl]-1H-pyrrole-2,5-dione,
3-[(3-chlorophenyl)amino]-4-[1-{3-(5-hydroxymethyl-imidazol-1-yl)propyl}-1H-indol-3-yl]-1H-pyrrole-2,5-dione,
3-[1-{3-(2-methylimidazol-1-yl)propyl}-1H-indol-3-yl]-4-(phenylamino)-1H-pyrrole-2,5-dione,
3-[1-(2-imidazol-1-ylethyl)-1H-indol-3-yl]-4-(phenylamino)-1H-pyrrole-2,5-dione,
3-[1-{2-(2-methyl-imidazol-1-yl)ethyl}-1H-indol-3-yl]-4-(phenylamino)-1H-pyrrole-2,5-dione,
3-[(4-chlorophenyl)amino]-4-[1-(3-imidazol-1-ylpropyl)-1H-indol-3-yl]-1H-pyrrole-2,5-dione,
3-[1-(3-imidazol-1-ylpropyl)-1H-indol-3-yl]-4-[(4-methoxyphenyl)amino]-1H-pyrrole-2,5-dione,
3-[(4-bromophenyl)amino]-4-[1-(3-imidazol-1-ylpropyl)-1H-indol-3-yl]-1H-pyrrole-2,5-dione,
3-[1-(3-imidazol-1-ylpropyl)-1H-indol-3-yl]-4-[(4-trifluoromethylphenyl)amino]-1H-pyrrole-2,5-dione,
3-[(4-fluorophenyl)amino]-4-[1-(3-imidazol-1-ylpropyl)-1H-indol-3-yl]-1H-pyrrole-2,5-dione,
3-[1-(3-imidazol-1-ylpropyl)-2-methyl-1H-indol-3-yl]-4-(phenylamino)-1H-pyrrole-2,5-dione,
3-(cyclohexylamino)-4-[1-(3-imidazol-1-ylpropyl)-1H-indol-3-yl]-1H-pyrrole-2,5-dione,
3-(cyclopentylamino)-4-[1-(3-imidazol-1-ylpropyl)-1H-indol-3-yl]-1H-pyrrole-2,5-dione,
3-(cycloheptylamino)-4-[1-(3-imidazol-1-ylpropyl)-1H-indol-3-yl]-1H-pyrrole-2,5-dione,
3-[1-(3-imidazol-1-ylpropyl)-5-methoxy-1H-indol-3-yl]-4-(phenylamino)-1H-pyrrole-2,5-dione,
3-(phenylamino)-4-[1-(3-piperidylmethyl)-1H-indol-3-yl]-1H-pyrrole-2,5-dione,
3-(phenylamino)-4-[1-(4-piperidylmethyl)-1H-indol-3-yl]-1H-pyrrole-2,5-dione,
3-[1-{(1-methylpiperidin-3-yl)methyl}-1H-indol-3-yl]-4-(phenylamino)-1H-pyrrole-2,5-dione,
3-[1-{(1-methylpiperidin-4-yl)methyl)-1H-indol-3-yl]-4-(phenylamino)-1H-pyrrole-2,5-dione,
3-[1-{[1-(2,3-dihydroxypropyl)piperidin-4-yl]methyl}-1H-indol-3-yl]-4-(phenylamino)-1H-pyrrole-2,5-dione,
3-[1-{(1-carbamoylpiperidin-4-yl)methyl}-1H-indol-3-yl]-4-(phenylamino)-1H-pyrrole-2,5-dione, and
3-[1-{(1-amidinopiperidin-4-yl)methyl}-1H-indol-3-yl]-4-(phenylamino)-1H-pyrrole-2,5-dione hydrochloride.

10. The disubstituted maleimide compound of claim 1 or a pharmaceutically acceptable salt thereof, which is selected from the group consisting of

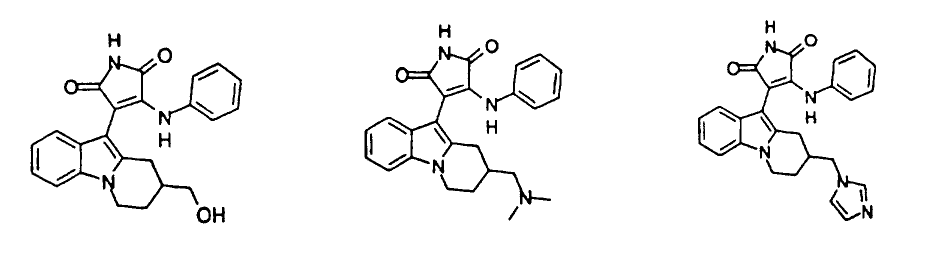

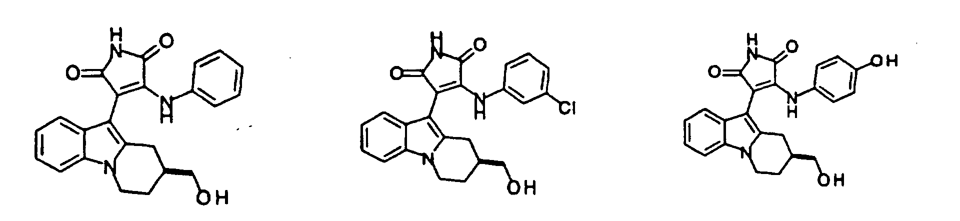

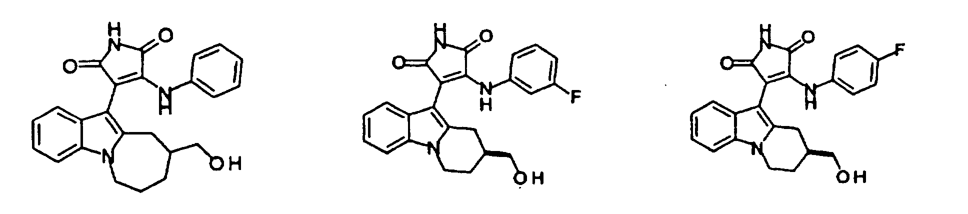

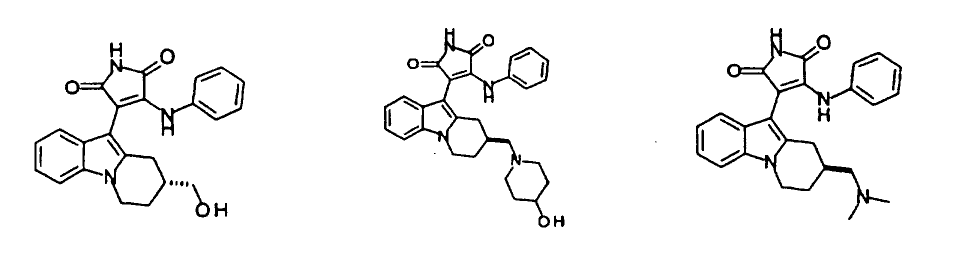

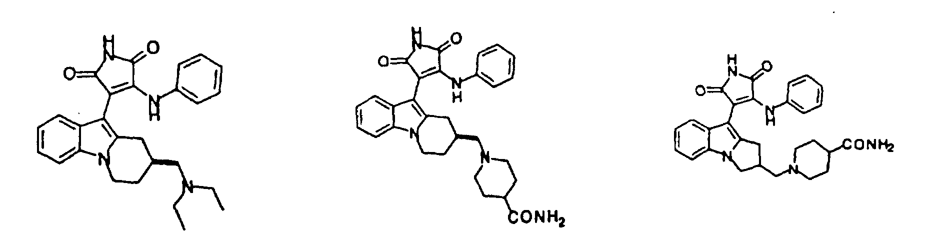

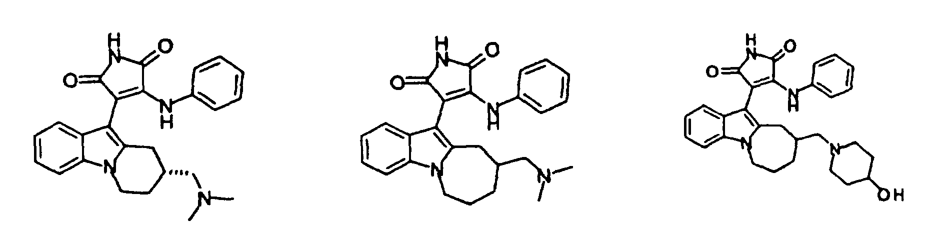

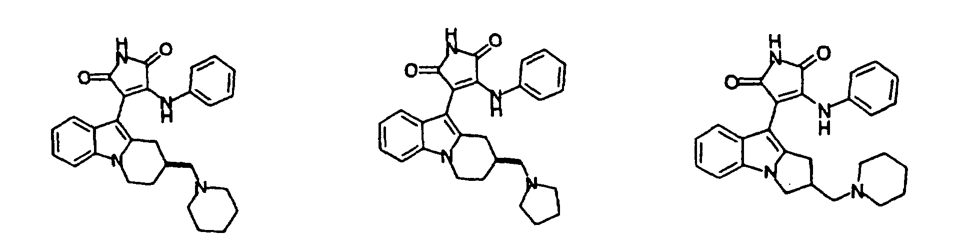

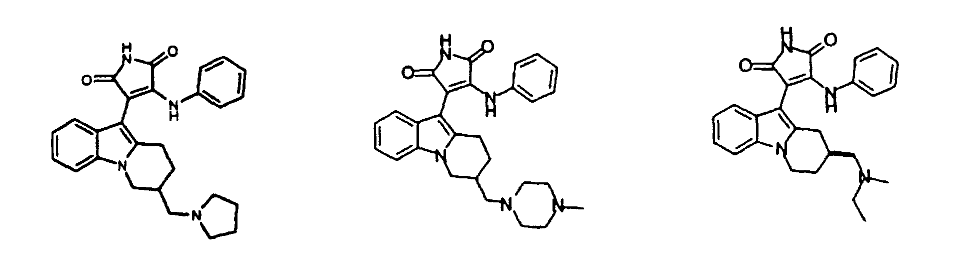

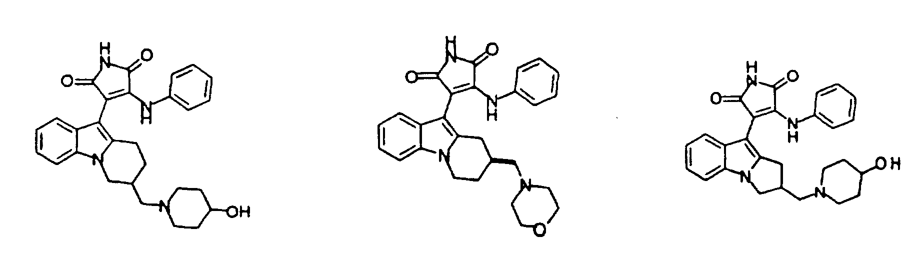

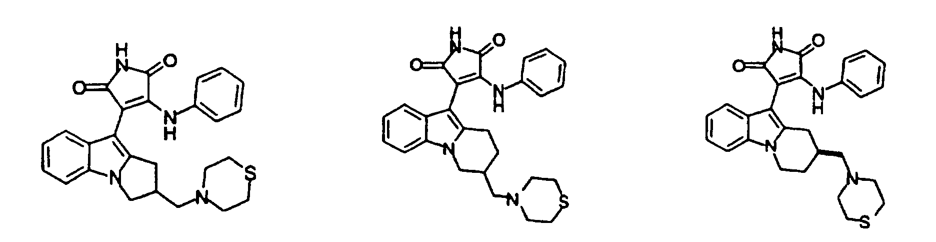

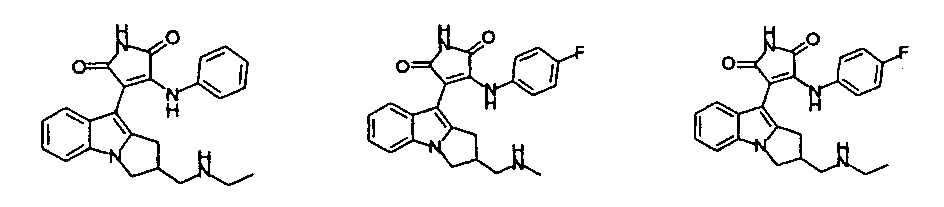

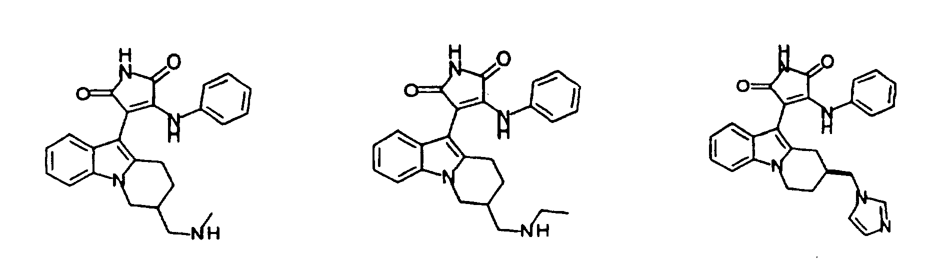

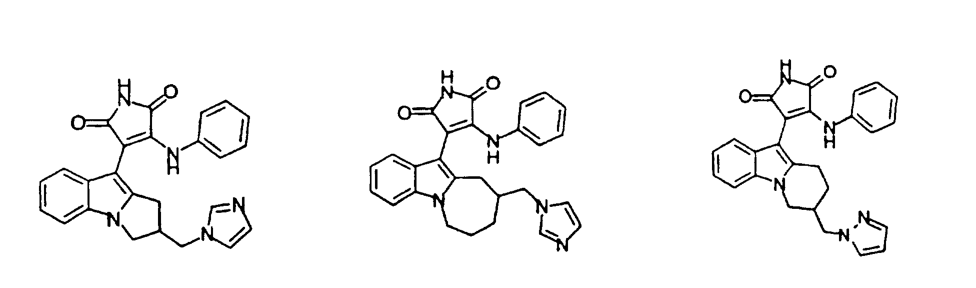

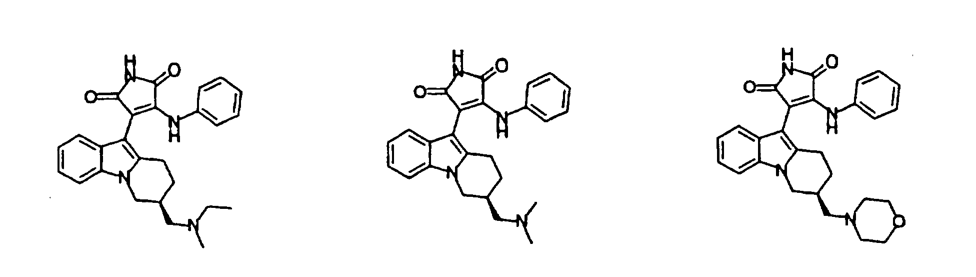

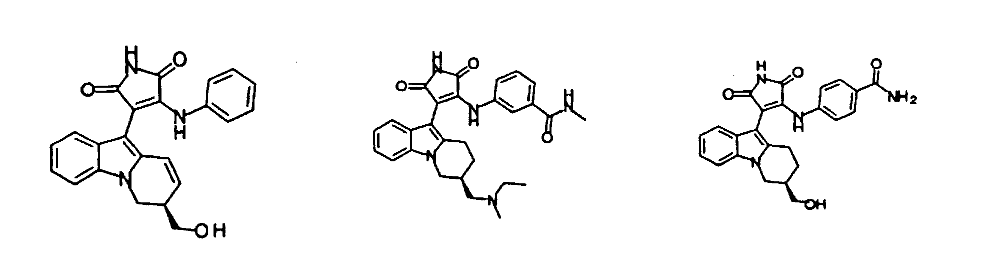

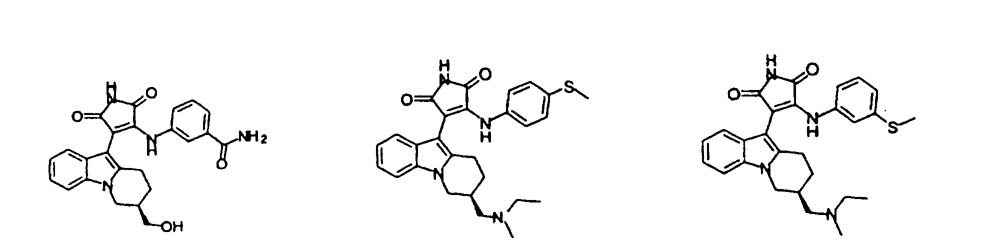

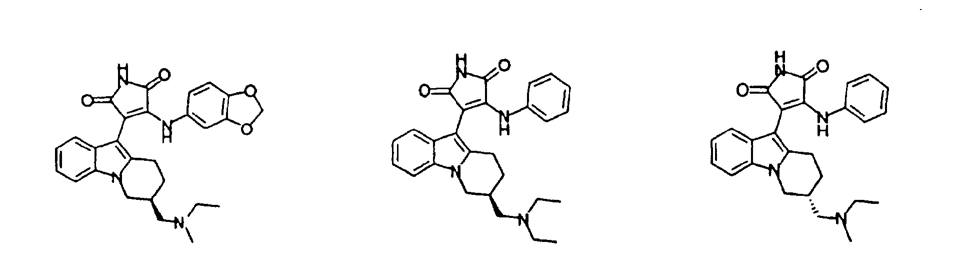

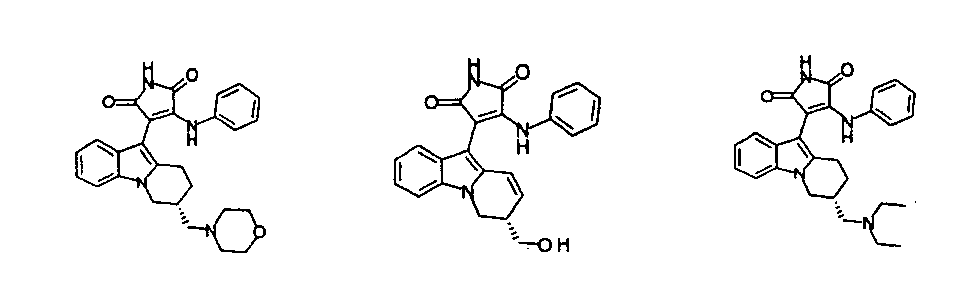

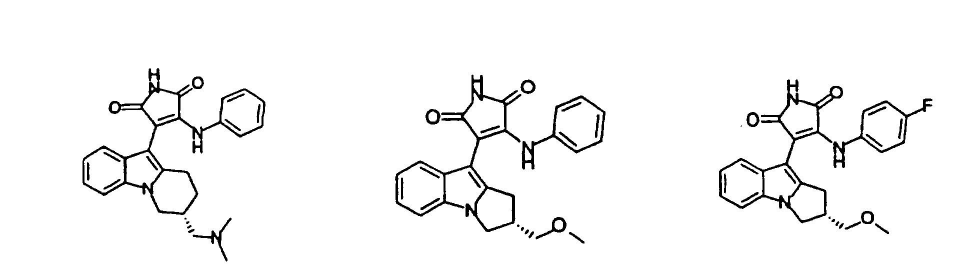

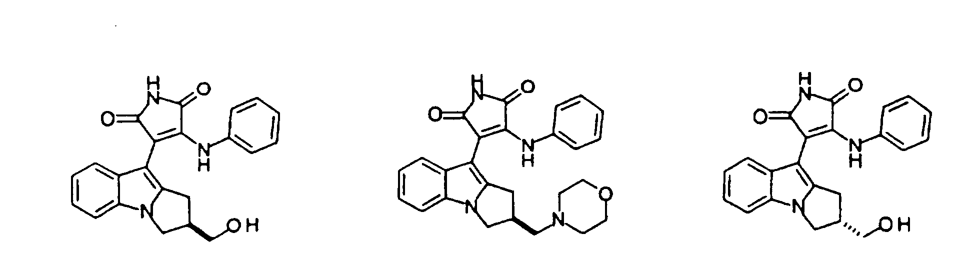

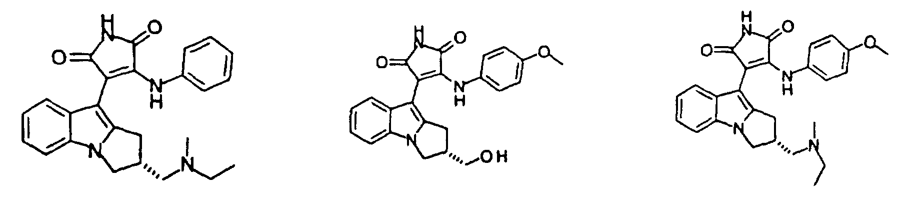

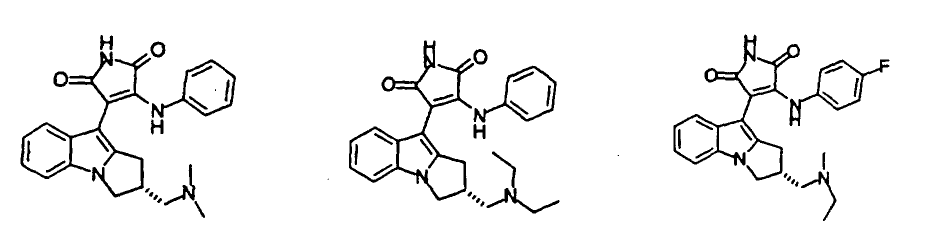

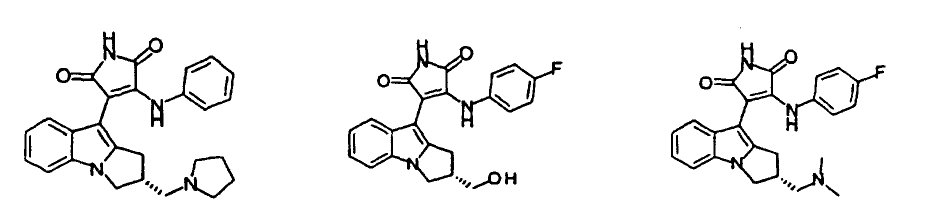

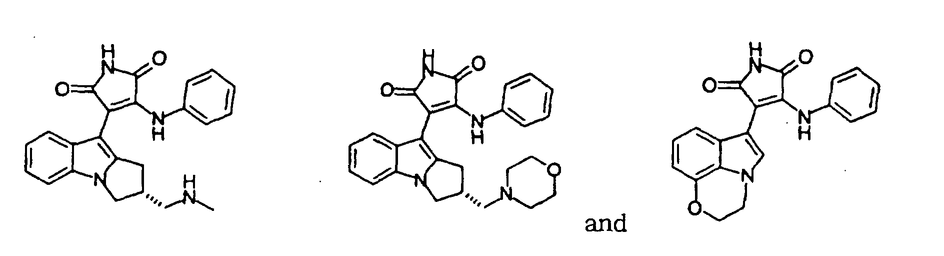

11. A pharmaceutical composition comprising the disubstituted maleimide compound of any of claim 1 to claim 10 or a pharmaceutically acceptable salt thereof and a pharmaceutically acceptable carrier.

12. A protein kinase C inhibitor containing the disubstituted maleimide compound of any of claim 1 to claim 10 or a pharmaceutically acceptable salt thereof as an active ingredient.

13. A protein kinase C isozyme β selective inhibitor containing the disubstituted maleimide compound of any of claim 1 to claim 10 or a pharmaceutically acceptable salt thereof as an active ingredient.

14. A therapeutic agent for diabetic complications, which contains the disubstituted maleimide compound of any of claim 1 to claim 10 or a pharmaceutically acceptable salt thereof as an active ingredient.
